# EUROPEAN PATENT SPECIFICATION

(11) **EP 3 056 576 B1**
(45) Date of publication and mention of the grant of the patent: **13.02.2019**
(21) Application number: 16160209.9
(22) Date of filing: 23.10.2008
(51) Int. Cl.: C12Q 1/68

(54) **A METHOD OF DIAGNOSING NEOPLASMS**
VERFAHREN ZUR DIAGNOSE VON NEOPLASMEN
PROCÉDÉ PERMETTANT DE DIAGNOSTIQUER DES NÉOPLASMES

(30) Priority: 23.10.2007 US 982114 P
(43) Date of publication of application: 17.08.2016
(62) Divisional of application: 08842750.5
(73) Proprietor: Clinical Genomics Pty Ltd, North Ryde, New South Wales 2113 (AU); Commonwealth Scientific and Industrial Research Organisation, Campbell, ACT 2612 (AU)
(72) Inventor: LAPOINTE, Lawrence C, West Pennant Hills, New South Wales 2125 (AU); DUNNE, Robert, Darlington, New South Wales 2008 (AU); YOUNG, Graeme P, Malvern, South Australia 5061 (AU); LOCKETT, Trevor John, Denistone, New South Wales 2114 (AU); WILSON, William J, Hornsby Heights, New South Wales 2077 (AU)
(74) Representative: Maschio, Antonio

(56) References cited:
- WO-A1-2013/052480
- KR-A- 20110 036 559
- US-A1- 2005 276 812
- US-A1- 2006 188 889
- WOLF D A ET AL: "Expression of a highly conserved oncofetal gene, TA1/E16, in human colon carcinoma and other primary cancers: homology to Schistosoma mansoni amino acid permease and Caenorhabditis elegans gene products.", CANCER RESEARCH 1 NOV 1996, vol. 56, no. 21, 1 November 1996 (1996-11-01), pages 5012-5022, XP002757266, ISSN: 0008-5472

## Description

### FIELD OF THE INVENTION

The present invention relates generally to nucleic acid molecules, the RNA and protein expression profiles of which are indicative of the onset, predisposition to the onset and/or progression of a neoplasm. More particularly, the present invention is directed to nucleic acid molecules, the expression profiles of which are indicative of the onset and/or progression of a large intestine neoplasm, such as an adenocarcinoma. The expression profiles of the present invention are useful in a range of applications including, but not limited to, those relating to the diagnosis and/or monitoring of colorectal neoplasms, such as colorectal adenocarcinomas. Accordingly, in a related aspect the present invention is directed to a method of screening a subject for the onset, predisposition to the onset and/or progression of a neoplasm by screening for modulation in the expression profile of one or more nucleic acid molecule markers.

### BACKGROUND OF THE INVENTION

Bibliographic details of the publications referred to by author in this specification are collected alphabetically at the end of the description.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Adenomas are benign tumours, or neoplasms, of epithelial origin which are derived from glandular tissue or exhibit clearly defined glandular structures. Some adenomas show recognisable tissue elements, such as fibrous tissue (fibroadenomas) and epithelial structure, while others, such as bronchial adenomas, produce active compounds that might give rise to clinical syndromes.

Adenomas may progress to become an invasive neoplasm and are then termed adenocarcinomas. Accordingly, adenocarcinomas are defined as malignant epithelial tumours arising from glandular structures, which are constituent parts of many organs of the body. The term adenocarcinoma is also applied to tumours showing a glandular growth pattern. These tumours may be sub-classified according to the substances that they produce, for example mucus secreting and serous adenocarcinomas, or to the microscopic arrangement of their cells into patterns, for example papillary and follicular adenocarcinomas. These carcinomas may be solid or cystic (cystadenocarcinomas). Each organ may produce tumours showing a variety of histological types, for example the ovary may produce both mucinous and cystadenocarcinoma.

Adenomas in different organs behave differently. In general, the overall chance of carcinoma being present within an adenoma (i.e. a focus of cancer having developed within a benign lesion) is approximately 5%. However, this is related to size of an adenoma. For instance, in the large bowel (colon and rectum specifically) occurrence of a cancer within an adenoma is rare in adenomas of less than 1 centimetre. Such a development is estimated at 40 to 50% in adenomas which are greater than 4 centimetres and show certain histopathological change such as villous change, or high grade dysplasia. Adenomas with higher degrees of dysplasia have a higher incidence of carcinoma. In any given colorectal adenoma, the predictors of the presence of cancer now or the future occurrence of cancer in the organ include size (especially greater than 9mm) degree of change from tubular to villous morphology, presence of high grade dysplasia and the morphological change described as "serrated adenoma". In any given individual, the additional features of increasing age, familial occurrence of colorectal adenoma or cancer, male gender or multiplicity of adenomas, predict a future increased risk for cancer in the organ - so-called risk factors for cancer. Except for the presence of adenomas and its size, none of these is objectively defined and all those other than number and size are subject to observer error and to confusion as to precise definition of the feature in question. Because such factors can be difficult to assess and define, their value as predictors of current or future risk for cancer is imprecise.

Once a sporadic adenoma has developed, the chance of a new adenoma occurring is approximately 30% within 26 months.

Colorectal adenomas represent a class of adenomas which are exhibiting an increasing incidence, particularly in more affluent countries. The causes of adenoma, and of progression to adenocarcinoma, are still the subject of intensive research. To date it has been speculated that in addition to genetic predisposition, environmental factors (such as diet) play a role in the development of this condition. Most studies indicate that the relevant environmental factors relate to high dietary fat, low fibre, low vegetable intake, smoking, obesity, physical inactivity and high refined carbohydrates.

Colonic adenomas are localised areas of dysplastic epithelium which initially involve just one or several crypts and may not protrude from the surface, but with increased growth in size, usually resulting from an imbalance in proliferation and/or apoptosis, they may protrude. Adenomas can be classified in several ways. One is by their gross appearance and the major descriptors include degrees of protrusion: flat sessile (i.e. protruding but without a distinct stalk) or pedunculated (i.e. having a stalk). Other gross descriptors include actual size in the largest dimension and actual number in the colon/rectum. While small adenomas (less than say 5 or 10 millimetres) exhibit a smooth tan surface, pedunculated and especially larger adenomas tend to have a cobblestone or lobulated red-brown surface. Larger sessile adenomas may exhibit a more delicate villous surface. Another set of descriptors include the histopathological classification; the prime descriptors of clinical value include degree of dysplasia (low or high), whether or not a focus of invasive cancer is present, degree of change from tubular gland formation to villous gland formation (hence classification is tubular, villous or tubulovillous), presence of admixed hyperplastic change and of so-called "serrated" adenomas and its subgroups. Adenomas can be situated at any site in the colon and/or rectum although they tend to be more common in the rectum and distal colon. All of these descriptors, with the exception of number and size, are relatively subjective and subject to interobserver disagreement.

The various descriptive features of adenomas are of value not just to ascertain the neoplastic status of any given adenomas when detected, but also to predict a person's future risk of developing colorectal adenomas or cancer, Those features of an adenoma or number of adenomas in an individual that point to an increased future risk for cancer or recurrence of new adenomas include: size of the largest adenoma (especially 10mm or larger), degree of villous change (especially at least 25% such change and particularly 100% such change), high grade dysplasia, number (3 or more of any size or histological status) or presence of serrated adenoma features. None except size or number is objective and all are relatively subjective and subject to interobserver disagreement. These predictors of risk for future neoplasia (hence "risk") are vital in practice because they are used to determine the rate and need for and frequency of future colonoscopic surveillance. More accurate risk classification might thus reduce workload of colonoscopy, make it more cost-effective and reduce the risk of complications from unnecessary procedures.

Adenomas are generally asymptomatic, therefore rendering difficult their diagnosis and treatment at a stage prior to when they might develop invasive characteristics and so became cancer. It is technically impossible to predict the presence or absence of carcinoma based on the gross appearance of adenomas, although larger adenomas are more likely to show a region of malignant change than are smaller adenomas, Sessile adenomas exhibit a higher incidence of malignancy than pedunculated adenomas of the same size. Some adenomas result in blood loss which might be observed or detectable in the stools; while sometimes visible by eye, it is often, when it occurs, microscopic or "occult". Larger adenomas tend to bleed more than smaller adenomas. However, since blood in the stool, whether overt or occult, can also be indicative of non-adenomatous conditions, the accurate diagnosis of adenoma is rendered difficult without the application of highly invasive procedures such as colonoscopy combined with tissue acquisition by either removal (i.e. polypectomy) or biopsy and subsequent histopathological analysis.

Accordingly, there is an on-going need to elucidate the causes of adenoma and to develop more informative diagnostic protocols or aids to diagnosis that enable one to direct colonoscopy at people more likely to have adenomas. These adenomas may be high risk, advanced or neither of these. Furthermore, it can be difficult after colonoscopy to be certain that all adenomas have been removed, especially in a person who has had multiple adenomas. An accurate screening test may minimise the need to undertake an early second colonoscopy to ensure that the colon has been cleared of neoplasms. Accordingly, the identification of molecular markers for adenomas would provide means for understanding the cause of adenomas and cancer, improving diagnosis of adenomas including development of useful screening tests, elucidating the histological stage of an adenoma, characterising a patient's future risk for colorectal neoplasia on the basis of the molecular state of an adenoma and facilitating treatment of adenomas.

To date, research has focused on the identification of gene mutations which lead to the development of colorectal neoplasms. In work leading up to the present invention, however, it has been determined that changes in expression profiles of genes which are also expressed in healthy individuals are indicative of the development of neoplasms of the large intestine, such as adenomas and adenocarcinomas. It has been further determined that in relation to neoplasms of the large intestine, diagnosis can be made based on screening for one or more of a panel of these differentially expressed genes. In a related aspect, it has still further been determined that to the extent that neoplastic tissue has been identified either by the method of the invention or by some other method, the present invention provides still further means of characterising that tissue as a cancer. In yet another aspect, it has been determined that a proportion of these genes are characterised by gene expression which occurs in the context of a neoplastic state but not in the context of a non-neoplastic state, thereby facilitating the development of qualitative analyses which do not require a relative analysis to be performed against a non-neoplastic or normal control reference level. Accordingly, the inventors have identified a panel of genes which facilitate the diagnosis of adenocarcinoma and adenoma development and/or the monitoring of conditions characterised by the development of these types of neoplasms.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.

The subject specification contains amino acid and nucleotide sequence information prepared using the programme PatentIn Version 3.4, presented herein after the bibliography. Each amino acid and nucleotide sequence is identified in the sequence listing by the numeric indicator <210> followed by the sequence identifier (eg. <210>1, <210>2, etc). The length, type of sequence (amino acid, DNA, etc.) and source organism for each sequence is indicated by information provided in the numeric indicator fields <211>m <212> and <213>, respectively. Amino acid and nucleotide sequences referred to in the specification are identified by the indicator SEQ ID NO: followed by the sequence identifier (eg. SEQ ID NO:1, SEQ ID NO: 2, etc). The sequence identifier referred to in the specification correlates to the information provided in numeric indicator field <400> in the sequence listing, which is followed by the sequence identifier (eg. <400>1, <400>2, etc). That is SEQ ID NO: 1 as detailed in the specification correlates to the sequence indicated as <400>1 in the sequence listing.

One aspect of the present disclosure is directed to a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 201328_at | 221577_x_at | 205828_at |
| 201341_at | 221922_at | 205886_at |
| 201416_at | 60474_at | 205890_s_at |
| 201417_at | 222696_at | 205910_s_at |
| 201468_s_at | 223447_at | 205941_s_at |
| 201506_at | 223970_at | 206224_at |
| 201563_at | 225541_at | 206976_s_at |
| 201656_at | 225835_at | 207173_x_at |
| 201925_s_at | 226360_at | 207457_s_at |
| 201926_s_at | 227174_at | 208079_s_at |
| 202286_s_at | 227475_at | 208712_at |
| 202718_at | 228303_at | 209218_at |
| 202831_at | 228653_at | 209309_at |
| 202833_s_at | 228754_at | 209752_at |
| 202935_s_at | 228915_at | 209773_s_at |
| 202936_s_at | 229215_at | 209774_x_at |
| 203124_s_at | 231832_at | 209792_s_at |
| 203256_at | 231941_s_at | 209875_s_at |
| 203313_s_at | 232176_at | 209955_s_at |
| 203510_at | 232252_at | 210052_s_at |
| 203860_at | 232481_s_at | 210511_s_at |
| 203895_at | 234331_s_at | 210559_s_at |
| 203896_s_at | 235210_s_at | 210766_s_at |
| 203961_at | 235976_at | 211506_s_at |
| 203962_s_at | 236894_at | 212281_s_at |
| 204259 at | 238017_at | 212344_at |
| 204351_at | 238021_s_at | 212353_at |
| 204401_at | 238984_at | 212354 at |
| 204404_at | 241031_at | 213905_x_at |
| 204855_at | 200660_at | 214022_s_at |
| 204885_s_at | 200832 s_at | 214974_x_at |
| 205174_s_at | 200903_s_at | 215091_s_at |
| 205366_s_at | 201014_s_at | 217430_x_at |
| 205470_s_at | 201112_s_at | 217996_at |
| 205513_at | 201195_s_at | 218507_at |
| 205765_at | 201261_x_at | 218963_s_at |
| 205825_at | 201292_at | 218984_at |
| 205927_s_at | 201338_x_at | 219787_s_at |
| 205983_at | 201479_at | 219911_s_at |
| 206239_s_at | 201577_at | 221729_at |
| 206286_s_at | 201601_x_at | 221730_at |
| 207158_at | 201666_at | 221731_x_at |
| 207850_at | 202310_s_at | 221923_s_at |
| 209369_at | 202311_s_at | 37892_at |
| 210445_at | 202404_s_at | 222449_at |
| 210519_s_at | 202431_s_at | 222450_at |
| 211429_s_at | 202504_at | 222549_at |
| 212063_at | 202779_s_at | 222608_s_at |
| 212070_at | 202859_x_at | 223062_s_at |
| 212190_at | 202954_at | 224428_s_at |
| 212531_at | 202998_s_at | 224646_x_at |
| 212942_s_at | 203083_at | 224915_x_at |
| 213880_at | 203213_at | 225295_at |
| 213975 s_at | 203878_s_at | 225520_at |
| 214235_at | 204051_s_at | 225664_at |
| 214651_s_at | 204127_at | 225681_at |
| 217523_at | 204170_s_at | 225767_at |
| 217867_x_at | 204320_at | 225799_at |
| 218086_at | 204470_at | 225806_at |
| 218211_s_at | 204475_at | 226227_x_at |
| 218704_at | 204580_at | 226237_at |
| 218796_at | 204620_s_at | 226311_at |
| 218872_at | 204702_s_at | 226777_at |
| 219630 at | 205361_ s _ at | 226835_s_at |
| 219682_s_at | 205476_at | 227140_at |
| 219727_at | 205479_s_at | 229802_at |
| 219955_at | 205713_s_at | 231766_s_at |
| 219956_at | 205815_at | 232151_at; and/or |
| 200665_s_at | | |

**(ii)**

| | | | |
|---|---|---|---|
| FOXQ1 | RNF43 | CDCA7 | LOXL2 |
| MMP1 | CCL20 | TDGF1 | AZGP1 |
| TCN1 | CTSE | MTHFD1L | MYC |
| MMP7 | MSLN | ANLN | COMP |
| WDR72 | TIMP1 | H19 | AURKA |
| INHBA | PCSK1 | FAP | PAICS |
| COL11A1 | CST1 | DACH1 | PUS7 |
| GDF15 | BGN | VCAN | ZNRF3 |
| CTHRC1 | AXIN2 | SQLE | CCND1 |
| COL1A1 | MET | REG3A | CSE1L |
| LGR5 | SOX9 | TESC | PFDN4 |
| DUSP27 | TMEPAI | UBE2C | C20orf42 |
| SERPINB5 | CDH11 | TMEM97 | SLC11A2 |
| ASCL2 | MMP11 | TRIM29 | NFE2L3 |
| SULF1 | CXCL1 | KLK11 | CEL |
| SLC6A6 | QPCT | LY6G6D | NLF1 |
| TACSTD2 | PDZK1IP1 | SLC7A5 | NPDC1 |
| NEBL | CD55 | FABP6 | ENC1 |
| PCCA | ECT2 | SLITRK6 | SERPINE2 |
| FLJ37644 | COL12A1 | MLPH | UBE2S |
| KRT23 | L1TD1 | HOXA9 | TOP2A |
| CXCL3 | WDR51B | TBX3 | CDC2 |
| MMP3 | HOXB6 | BACE2 | RFC3 |
| SFRP4 | FAM84A | GPX2 | LILRB1 |
| UBD | COL5A2 | TPX2 | NPM1 |
| SCD | MMP12 | KCNN4 | RDHE2 |
| DPEP1 | SORD | LOC541471 | PLAU |
| LCN2 | PSAT1 | CYP3A5 | HSPH1 |
| PLCB4 | CXCL5 | ANXA3 | GTF3A |
| SPINK1 | IGFBP2 | CYP3A5P2 | KLK10 |
| DUOX2 | TGIF1 | C14orf94 | GPSM2 |
| SOX4 | CXCL2 | RP5-875H10.1 | NME1 |
| THBS2 | SLCO4A1 | RPL22L1 | MUC20 |
| CLDN1 | CKS2 | APOBEC1 | RPESP |
| REG1B | PHLDA1 | HIG2 | RRM2 |
| CDH3 | SERPINA1 | COL10A1 | DKC1 |
| SPARC | COL1A2 | AHCY | CD44 |
| | | IL8 | |

in a biological sample from said individual wherein a higher level of expression of the genes or transcripts of group (i) and/or group (ii) relative to control levels is indicative of a neoplastic large intestine cell or a cell predisposed to the onset of a neoplastic state.

In another aspect there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs: 225681_at; 227140_at; and/or
**(ii)** CTHRC1
in a biological sample from said individual wherein a higher level of expression of the genes or transcripts of group (i) and/or group (ii) relative to control levels is indicative of a neoplastic large intestine cell or a cell predisposed to the onset of a neoplastic state.

In yet another aspect there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:
   227475_at
   204475_at
   202859_x_at
   202404_s_at; and/or
**(ii)** FOXQ1, MMP1, IL8, COL1A2
in a biological sample from said individual wherein a higher level of expression of the genes or transcripts of group (i) and/or group (ii) relative to control levels is indicative of a neoplastic large intestine cell or a cell predisposed to the onset of a neoplastic state.

In still another aspect there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs: 205513_at, 204259_at, 227174_at, 210511_s_at, 37892_at; and/or
**(ii)** TCN1, MMP7, WDR72, INHBA, COL11A1
in a biological sample from said individual wherein a higher level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In still yet another aspect there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 221577_x_at | 212942_s_at | 202310_s_at |
| 213880_at | 232252_at | 204855_at |
| 229215_at | 212354_at | 228754_at |
| 202286_s_at | 203962_s_at | 203860_at |
| 202935_s_at | 218963_s_at | 207850_at |
| 205828_at | 204051_s_at | 205890_s_at |
| 200832_s_at | 205983_at | 212531_at; and/or |

**(ii)**

| | | |
|---|---|---|
| LGR5 | GDF15 | COL1A1 |
| ASCL2 | DUSP27 | SERPINB5 |
| TACSTD2 | SULF1 | SLC6A6 |
| FLJ37644 | NEBL | PCCA |
| MMP3 | KRT23 | CXCL3 |
| SCD | SFRP4 | UBD |
| | DPEP1 | LCN2 |

in a biological sample from said individual wherein a higher level of expression of the genes or transcripts of group (i) and/or group (ii) relative to control levels is indicative of a neoplastic large intestine cell or a cell predisposed to the onset of a neoplastic state.

In a further aspect the present disclosure is directed to a method of screening for the onset or predisposition to the onset of a large intestine neoplasm in an individual, said method comprising measuring the level of expression of one or more genes or transcripts selected from:
(i) the gene, genes or transcripts detected by Affymetrix probeset IDs:

| | | |
|---|---|---|
| 201328_at | 205825_at | 219956_at |
| 201468_s_at | 205927_s_at | 221922_at |
| 201656_at | 206239_s_at | 223447_at |
| 201925_s_at | 207158_at | 223970_at |
| 201926_s_at | 210445_at | 225835_at |
| 202718_at | 210519_s_at | 226360_at |
| 202831_at | 211429_s_at | 228303_at |
| 202833_s_at | 212063_at | 228653_at |
| 203124_s_at | 213975_s_at | 228915_at |
| 203313_s_at | 214235_at | 231832_at |
| 203860_at | 214651_s_at | 231941_s_at |
| 203895_at | 217523_at | 232176_at |
| 203896_s_at | 217867_x_at | 232481_s_at |
| 204401_at | 218086_at | 234331_s_at |
| 204885_s_at | 218211_s_at | 235210_s_at |
| 205174_s_at | 218796_at | 235976_at |
| 205366_s_at | 219630_at | 236894_at |
| 205470_s_at | 219682_s_at | 238017_at |
| 205513_at | 219727_at | 238984_at |
| 205765_at | 219955_at; | 241031_at; and/or |

**(ii)**

| | | |
|---|---|---|
| APOBEC1 | HOXB6 | QPCT |
| BACE2 | IGFBP2 | RDHE2 |
| C20orf42 | ITGA6 | REG4 |
| CD44 | KCNN4 | RETNLB |
| CD55 | KLK11 | RP5-875H10.1 |
| CTSE | L1TD1 | RPESP |
| CYP3A5 | LILRB 1 | SERPINA1 |
| CYP3A5P2 | MLPH | SLC11A2 |
| DACH1 | MSLN | SLC12A2 |
| DUOX2 | MUC20 | SLITRK6 |
| ETS2 | NLF1 | SPINK1 |
| FABP6 | NPDC1 | TBX3 |
| FAM84A | NQO1 | TCN1 |
| GALNT6 | PCCA | TGIF1 |
| GPSM2 | PCSK1 | WDR51B |
| GPX2 | PDZK1IP1 | ZNRF3 |
| HOXA9 | PLCB4 | |

in a biological sample from said individual wherein a higher level of expression of the genes or transcripts of group (i) and/or group (ii) relative to control levels is indicative of an adenoma cell or a cell predisposed to the onset of an adenoma state.

In another further aspect of the present disclosure there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 200660_at | 205476_at | 217430_x_at |
| 200665_s_at | 205479_s_at | 217996_at |
| 200832_s_at | 205713_s_at | 218507_at |
| 200903_s_at | 205815_at | 218963_s_at |
| 201014_s_at | 205828_at | 218984_at |
| 201112_s_at | 205886_at | 219787_s_at |
| 201195_s_at | 205890_s_at | 219911_s_at |
| 201261_x_at | 205910_s_at | 221729_at |
| 201292_at | 205941_s_at | 221730_at |
| 201338_x_at | 206224_at | 221731_x_at |
| 201479_at | 206976_s_at | 221923_s_at |
| 201577_at | 207173_x_at | 37892_at |
| 201601_x_at | 207457_s_at | 222449_at |
| 201666_at | 208079_s_at | 222450_at |
| 202310_s_at | 208712_at | 222549_at |
| 202311_s_at | 209218_at | 222608_s_at |
| 202403_s_at | 209309_at | 223062_s_at |
| 202404_s_at | 209752_at | 224428_s_at |
| 202431_s_at | 209773_s_at | 224646_x_at |
| 202504_at | 209774_x_at | 224915_x_at |
| 202779_s_at | 209792_s_at | 225295_at |
| 202859_x_at | 209875_s_at | 225520_at |
| 202954_at | 209955_s_at | 225664_at |
| 202998_s_at | 210052_s_at | 225681_at |
| 203083_at | 210511_s_at | 225767_at |
| 203213_at | 210559_s_at | 225799_at |
| 203878_s_at | 210766_s_at | 225806_at |
| 204051_s_at | 211506_s_at | 226227_x_at |
| 204127_at | 212281_s_at | 226237_at |
| 204170_s_at | 212344_at | 226311_at |
| 204320_at | 212353_at | 226777_at |
| 204470_at | 212354_at | 226835_s_at |
| 204475_at | 213905_x_at | 227140_at |
| 204580_at | 214022_s_at | 229802_at |
| 204620_s_at | 214974_x_at | 231766_s_at |
| 204702_s_at 205361_s_at | 215091_s_at; | 232151_at; and/or |

**(ii)**

| | | |
|---|---|---|
| AHCY | DKC1 | PSAT1 |
| ANLN | ECT2 | PUS7 |
| AURKA | FAP | REGIA |
| AZGP1 | GTF3A | REG1B |
| BGN | H19 | REG3A |
| C14orf94 | HIG2 | RFC3 |
| C20orf199 | HSPH1 | RRM2 |
| CCL20 | IFITM1 | S100A11 |
| CCND1 | IL8 | SCD |
| CDC2 | INHBA | SFRP4 |
| CDCA7 | KLK10 | SLC39A10 |
| CDH11 | KRT23 | SLC7A5 |
| CEL | LOC541471 | SLCO4A1 |
| CKS2 | LOXL2 | SPARC |
| CLDN1 | LY6G6D | SPP1 |
| COL10A1 | MMP1 | SQLE |
| COL11A1 | MMP11 | SULF1 |
| COL12A1 | MMP12 | THBS2 |
| COL1A1 | MMP3 | TIMP1 |
| COL1A2 | MTHFD1L | TMEM97 |
| COL5A2 | MYC | TMEPAI |
| COL8A1 | NFE2L3 | TOP2A |
| COMP | NME 1 | TPX2 |
| CSE1L | NPM1 | TRIM29 |
| CST1 | PAICS | UBD |
| CTHRC1 | PFDN4 | UBE2C |
| CXCL1 | PHLDA1 | UBE2S |
| CXCL2 | PLAU | VCAN |
| CXCL5 | | |

in a biological sample from said individual wherein a higher level of expression of the genes or transcripts of group (i) and/or group (ii) relative to control levels is indicative of a cancer cell or a cell predisposed to the onset of a cancerous state.

In yet another further aspect there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 202286 s at | 235976 at | 209309 at |
| 204259_at | 236894_at | 211506_s_at |
| 204885_s_at | | 214974_x_at |
| 205174_s_at | 238984_at | 219787_s_at |
| 205825_at | 241031_at | 37892_at |
| 207850_at | 202311_ s_at | 222608_s_at |
| 213880_at | 204320_at | 223062_s_at |
| 217523_at | 204475_at | 225806_at |
| 227174_at | 204702_s_at | 226237_at |
| 228915_at | 205910_s_at | 227140_at |
| 232252_at | 206224_at | 229802_at; and/or |

**(ii)**

| | | |
|---|---|---|
| MMP1 | PCSK1 | ANLN |
| MMP7 | CST1 | DACH1 |
| LGR5 | QPCT | COL11A1 |
| WDR72 | ECT2 | C14orf94 |
| COL11A1 | SLITRK6 | AZGP1 |
| COL1A1 | L1TD1 | REG4 |
| DUSP27 | KIAA1199 | NFE2L3 |
| NLF1 | PSAT1 | CEL |
| IL8 | CXCL5 | CD44 |
| TACSTD2 | CXCL3 | COL8A1 |
| MSLN | | |

in a biological sample from said individual wherein a higher level of expression of the genes or transcripts of group (i) and/or group (ii) relative to background levels is indicative of a neoplastic cell or a cell predisposed to the onset of a neoplastic state.

Yet another aspect of the present disclosure provides a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 204885_s_at | 217523_at | 236894_at |
| 205174_s_at | 228915_at | 238984_at |
| 205825_at | 235976_at | 241031_at; and/or |

**(ii)**

| | | |
|---|---|---|
| CD44 | MSLN | QPCT |
| DACH1 | NLF1 | REG4 |
| L1TD1 | PCSK1 | SLITRK6 |

in a biological sample from said individual wherein a higher level of expression of the genes or transcripts of group (i) and/or group (ii) relative to background levels is indicative of an adenoma cell or a cell predisposed to the onset of an adenoma state.

In yet still another aspect there is provided a method of screening for the onset or predisposition to the onset of a large intestine neoplasm in an individual, said method comprising measuring the level of expression of one or more genes or transcripts selected from:
**(i)** the gene or genes detected by Affymetrix probeset IDs:

| | | |
|---|---|---|
| 202311 s at | 209309 at | 223062 s at |
| 204320_at | 211506_s_at | 225806_at |
| 204475_at | 214974_x_at | 226237_at |
| 204702_s_at | 219787_s_at | 227140_at |
| 205910_s_at | 37892_at | 229802_at; and/or |
| 206224 at | 222608 s at | |

**(ii)**

| | | |
|---|---|---|
| ANLN | COL1A1 | IL8 |
| AZGP1 | COL8A1 | MMP1 |
| C14orf94 | CST1 | NFE2L3 |
| CEL | CXCL5 | PSAT1 |
| COL11A1 | ECT2 | |

in a biological sample from said individual wherein a higher level of expression of the genes or transcripts of group (i) and/or group (ii) relative to background levels is indicative of a cancer cell or a cell predisposed to the onset of a cancerous state.

In still yet another aspect of the present disclosure, there is provided a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 200884_at | 214234_s_at | 226248_s_at |
| 203240_at | 214235_at | 226302_at |
| 203963_at | 214433_s_at | 227676_at |
| 204508_s_at | 215125_s_at | 227719_at |
| 204607_at | 215867_x_at | 227725_at |
| 204811_s_at | 217109_at | 228232_s_at |
| 204895_x_at | 217110_s_at | 229070_at |
| 204897_at | 218211_s_at | 231832_at |
| 205259_at | 219543_at | 232176_at |
| 205765_at | 219955_at | 232481_s_at |
| 205927_s_at | 221841_s_at | 235976_at |
| 208063_s_at | 221874_at | 236894_at |
| 208937_s_at | 223969_s_at | 237521_x_at |
| 210107_at | 223970_at | 242601_at |
| 213106_at; and/or | | |

**(ii)**

| | | |
|---|---|---|
| CLCA1 | CTSE | ATP8B1 |
| FCGBP | C6orf105 | CACNA2D2 |
| HMGCS2 | CKB | KLF4 |
| RETNLB | ATP8A1 | CYP3A5P2 |
| L1TD1 | MUC4 | CAPN9 |
| SLITRK6 | UGT1A1 | NR3C2 |
| VSIG2 | SELENBP1 | PBLD |
| LOC253012 | PTGER4 | CA12 |
| ST6GALNAC1 | MLPH | WDR51B |
| ID1 | KIAA1324 | FAM3D |
| CYP3A5 | | |

in said cell or cellular population wherein a higher level of expression of the genes or transcripts of group (i) and/or group (ii) relative to a gastrointestinal cancer cell level is indicative of an adenoma cell or a cell predisposed to the onset of an adenoma state.

In another aspect there is provided a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 200600_at | 204006_s_at | 213428_s_at |
| 200665_s_at | 204051_s_at | 213524_s_at |
| 200832_s_at | 204122_at | 213869_x_at |
| 200974_at | 204320_at | 213905_x_at |
| 200986_at | 204475_at | 214247_s_at |
| 201058_s_at | 204620_s_at | 215049_x_at |
| 201069_at | 205479_s_at | 215076_s_at |
| 201105_at | 205547_s_at | 21S646_s_at |
| 201141_at | 205828_at | 216442_x_at |
| 201147_s_at | 207173_x_at | 217430_x_at |
| 201150_s_at | 207191_s_at | 217762_s_at |
| 201162_at | 208747_s_at | 217763_s_at |
| 201163_s_at | 208782_at | 217764_s_at |
| 201I85_at | 208788_at | 218468_s_at |
| 201261_x_at | 208850_s_at | 218469_at |
| 201289_at | 208851_s_at | 218559_s_at |
| 201426_s_at | 209101_at | 218638_s_at |
| 201438_at | 209156_s_at | 219087_at |
| 201616_s_at | 209218_at | 221011_s_at |
| 201645_at | 209395_at | 221729_at |
| 201667_at | 209396_s_at | 221730_at |
| 201744_s_at | 209596_at | 221731_x_at |
| 201792_at | 209875_s_at | 37892_at |
| 201842_s_at | 209955_s_at | 223122_s_at |
| 201852_x_at | 210095_s_at | 223235_s_at |
| 201859_at | 210495_x_at | 224560_at |
| 201893_x_at | 210511_s_at | 224694_at |
| 202237_at | 210764_s_at | 224724_at |
| 202238_s_at | 210809_s_at | 225664_at |
| 202283_at | 211161_s_at | 225681_at |
| 202291_s_at | 211571_s_at | 225710_at |
| 202310_s_at | 211719_x_at | 225799_at |
| 202311_s_at | 211813_x_at | 226237_at |
| 202403_s_at | 211896_s_at | 226311_at |
| 202404_s_at | 211959_at | 226694_at |
| 202450_s_at | 211964_at | 226777_at |
| 202620_s_at | 211966_at | 226930_at |
| 202766_s_at | 211980_at | 227099_s_at |
| 202859_x_at | 211981_at | 227140_at |
| 202878_s_at | 212077_at | 227566_at |
| 202917_s_at | 212344_at | 229218_at |
| 202998_s_at | 212353_at | 229802_at |
| 203083_at | 212354_at | 231579_s_at |
| 203325_s_at | 212464_s_at | 231766_s_at |
| 203382_s_at | 212488_at | 231879_at |
| 203477_at | 212489_at | 232458_at |
| 203570_at | 212667_at | 233555_s_at |
| 203645_s_at | 213125_at | 234994_at |
| 203878_s_at; and/or | | |

**(ii)**

| | | |
|---|---|---|
| COL1A2 | LGALS1 | SRGN |
| CTHRC1 | ELOVL5 | LBH |
| FN1 | MGP | CTGF |
| POSTN | MMP2 | TNC |
| SPP1 | LOXL2 | G0S2 |
| MMP1 | MYL9 | SQLE |
| SPARC | DCN | EFEMP1 |
| LUM | CALD1 | APOE |
| GREM1 | FBN1 | MSN |
| IL8 | MMP3 | IGFBP3 |
| IGFBP5 | IGFBP7 | SERPINF1 |
| SFRP2 | FSTL1 | ISLR |
| SULF1 | COL4A2 | HNT |
| ASPN | VCAN | COL5A1 |
| COL6A3 | SMOC2 | OLFML2B |
| COL8A1 | HTRA1 | KIAA1913 |
| COL12A1 | CYR61 | PALM2-AKAP2 |
| COL5A2 | FAP | SERPING1 |
| CDH11 | VIM | TYROBP |
| THBS2 | TIMP2 | ACTA2 |
| COL15A1 | SCD | COL3A1 |
| COL11A1 | TIMP3 | PLOD2 |
| S100A8 | AEBP1 | MMP11 |
| FNDC1 | GJA1 | CD163 |
| SFRP4 | NNMT | FCGR3B |
| INHBA | COL1A1 | PLAU |
| COL6A2 | SULF2 | MAFB |
| ANTXR1 | COL6A1 | LOC541471 |
| GPNMB | SPON2 | LOC387763 |
| BGN | CTSK | CHI3L1 |
| TAGLN | MXRA5 | THY1 |
| COL4A1 | C1S | LOXL1 |
| RAB31 | DKK3 | CD93 |

in a biological sample from said individual wherein a higher level of expression of the genes or transcripts of group (i) and/or group (ii) relative to a gastrointestinal adenoma cell level is indicative of a cancer or a cell predisposed to the onset of a cancerous state.

In a further aspect there is provided a method of characterising a neoplastic cell or cellular population, which cell or cellular population is derived from the large intestine of an individual, said method comprising measuring the level of expression of one or more genes or transcripts selected from:
**(i)** the gene or genes detected by Affymetrix probeset IDs: 210107_at; and/or
**(ii)** CLCA1
in a biological sample from said individual wherein a higher level of expression of the genes or transcripts of group (i) and/or group (ii) relative to a gastrointestinal cancer control level is indicative of an adenoma cell or a cell predisposed to the onset of an adenoma state.

In another aspect there is provided a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 203240_at | 219955_at | 242601_at |
| 204607_at | 232481_s_at | 227725_at |
| 223969_s_at | 228232_s_at; and/or | |

**(ii)**

| | | |
|---|---|---|
| FCGBP | L1TD1 | LOC253012 |
| HMGCS2 | SLITRK6 | ST6GALNAC1 |
| RETNLB | VSIG2 | |

in a biological sample from said individual wherein a higher level of expression of the genes or transcripts of group (i) and/or group (ii) relative to a gastrointestinal cancer control level is indicative of an adenoma cell or a cell predisposed to the onset of an adenoma state.

A further aspect of the present disclosure is directed to a method of characterising a cell or cellular population, which cell or cellular population is derived from the large intestine of an individual, said method comprising measuring the level of expression of one or more genes or transcripts selected from:
(i) the gene, genes or transcripts detected by Affymetrix probeset IDs: 202404_s_at, 210809_s_at, 227140_at, 22568 1_at, 209875_s_at, 204475_at, 212464_s_at; and/or,
(ii) COL1A2, FN1, SPP1, CTHRC1, POSTN, MMP1
in a biological sample from said individual wherein a higher level of expression of the genes or transcripts of group (i) and/or (ii) relative to a gastrointestinal adenoma control level is indicative of a cancer or a cell predisposed to the onset of a cancerous state.

In yet another further aspect the present disclosure is directed to a method of characterising a cell or cellular population, which cell or cellular population is derived from the large intestine of an individual, said method comprising measuring the level of expression of one or more genes or transcripts selected from:
(i) the gene, genes or transcripts detected by Affymetrix probeset IDs: 200665_s_at, 218468_s_at, 211959_at, 201744_s_at, 202859_x_at; and/or,
(ii) SPARC, GREM1, IGFBP5, LUM, IL8,
in a biological sample from said individual wherein a higher level of expression of the genes or transcripts of group (i) and/or (ii) relative to a gastrointestinal adenoma control level is indicative of a cancer or a cell predisposed to the onset of a cancerous state.

In still yet another further aspect the present invention is directed to a method of characterising a cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 223122_s_at | 207173_x_at | 210511_s_at |
| 212353_at | 203083_at | 209156_s_at |
| 219087_at | 203477_at | 224694_at |
| 201438_at | 37892_at | 201141_at |
| 226237_at | 202917_s_at | 213905_x_at |
| 225664_at | 226930_at | 205547_s_at |
| 221730_at | 204051_s_at; | and/or |

**(ii)**

| | | |
|---|---|---|
| SFRP2 | CDH11 | INHBA |
| SULF1 | THBS2 | COL6A2 |
| ASPN | COL15A1 | ANTXR1 |
| COL6A3 | COL11A1 | GPNMB |
| COL8A1 | S100A8 | BGN |
| COL12A1 | FNDC1 | TAGLN |
| COL5A2 | SFRP4 | |

in a biological sample from said individual wherein a higher level of expression of the genes or transcripts of group (i) and/or (ii) relative to a gastrointestinal adenoma control level is indicative of a cancer or a cell predisposed to the onset of a cancerous state.

Yet another aspect of the present disclosure provides a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:
   235976_at 236894_at; and/or
**(ii)** SLITRK6 L1TD1
in a biological sample from said individual wherein a higher level of expression of the genes or transcripts of group (i) and/or (ii) relative to neoplastic tissue background levels is indicative of an adenoma cell or a cell predisposed to the onset of an adenoma state.

In still another aspect the present disclosure provides a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs: 202311_s_t, 209396_s_at, 226237_at, 204320_at, 215646_s_at, 227140_at, 204475_at, 37892_at, 229802_at, 209395_at; and/or
**(ii)** COL1A1, VCAN, CHI3L1, MMP1, COL8A1, COL11A1
in a biological sample from said individual wherein a higher level of expression of the genes or transcripts of group (i) and/or (ii) relative to neoplastic tissue background neoplastic cell levels is indicative of a cancer or a cell predisposed to the onset of a cancerous state.

An aspect related to the present invention provides a molecular array, which array comprises a plurality of:
(i) nucleic acid molecules comprising a nucleotide sequence corresponding to any one or more of the neoplastic marker genes hereinbefore described or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (iv) is indicative of the neoplastic state of a cell or cellular subpopulation derived from the large intestine.

### BRIEF DESCRIPTION OF THE DRAWINGS

**Figure 1** is a graphical representation of nuclear factor (erythroid-derived-2)-like 2. Blk=normal, grn=inflamed, red=adenoma,blue=cancer
**Figure 2** is a graphical representation of SLIT and NTRK-like family, member 6 (left) and LINE1 type transposase domain containing 1 (right).
**Figure 3** is a graphical representation of collagen type XI, alpha 1.
**Figure 4** is an image of the immunohistochemical staining for MSLN in a normal tissue shows mild staining in the cytoplasm of the colonocytes.
**Figure 5** is an image of the immunohistochemical staining for MSLN in a representative cancer tissue shows moderate staining in the cytoplasm of the colonic epithelia.
**Figure 6** is an image of the immunohistochemical staining of MSLN in a moderately differentiated adenoma shows strong staining in the multilayered colonic epithelium.
**Figure 7** is a graphical representation depicting Probeset 205828_at Expression profile across 68 clinical specimens comprising 30 non-disease controls (black), 19 adenomas (red) and 19 adenocarcinoma (green).

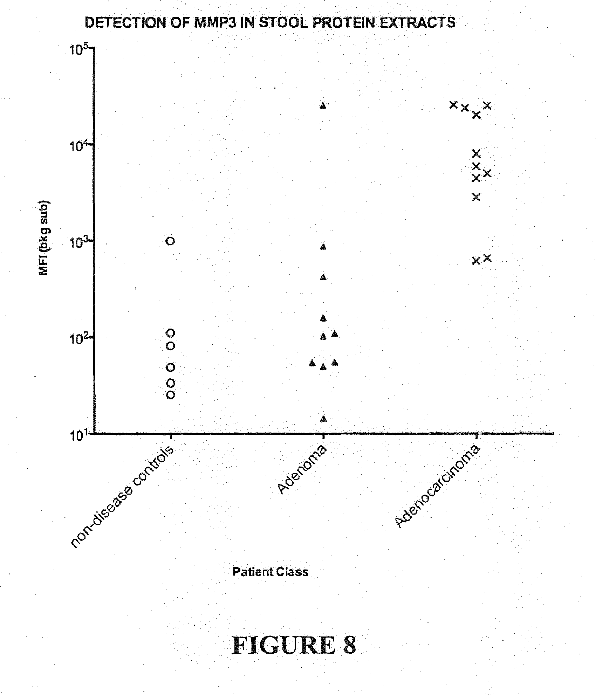

**Figure 8** is a graphical representation of the measurement of MMP3 expression in protein extract from 27 clinical stool specimens. Protein extraction was performed on 27 clinical stool specimens comprising 6 non-disease controls (circle), 10 adenoma (triangle) and 11 adenocarcinoma (cross) emulsified using a PBS pH 7.4 solution containing 0.05% Tween-20 and 1x Protease Inhibitor Cocktail (Roche). Proteins were further solubilised by 30 minutes incubation in an ultrasonic water bath. Solubilised proteins were isolated by centrifugation and endogenous levels of MMP3 in the resulting protein extracts were using measured using a commercially available Luminex bead-based suspension immunoassay as recommended by manufacturer (R&D Systems).

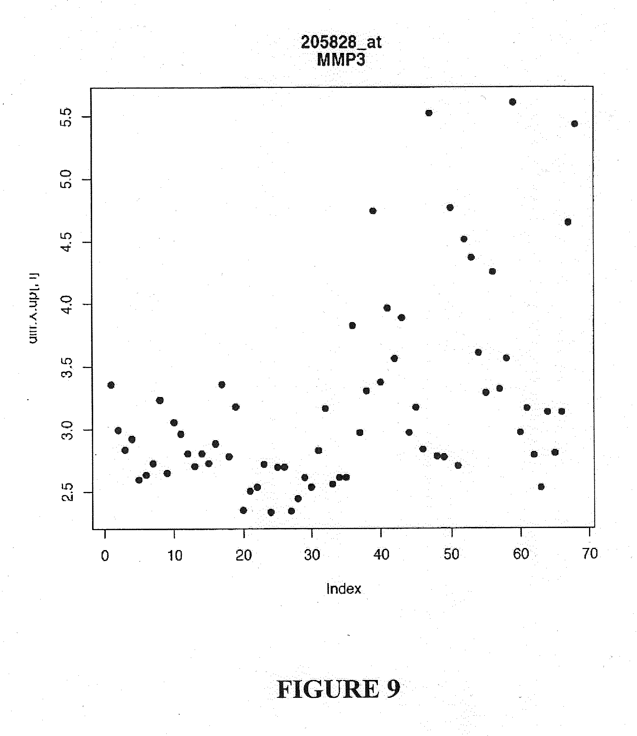

**Figure 9** is a graphical representation of Affymetrix probeset ID 205828 at which is known to hybrising to transcripts of the MMP3 gene depicting a gene expression profile in 68 clinical specimens comprising 30 non-disease controls (black), 19 adenomas (red) and 19 cancers (green).

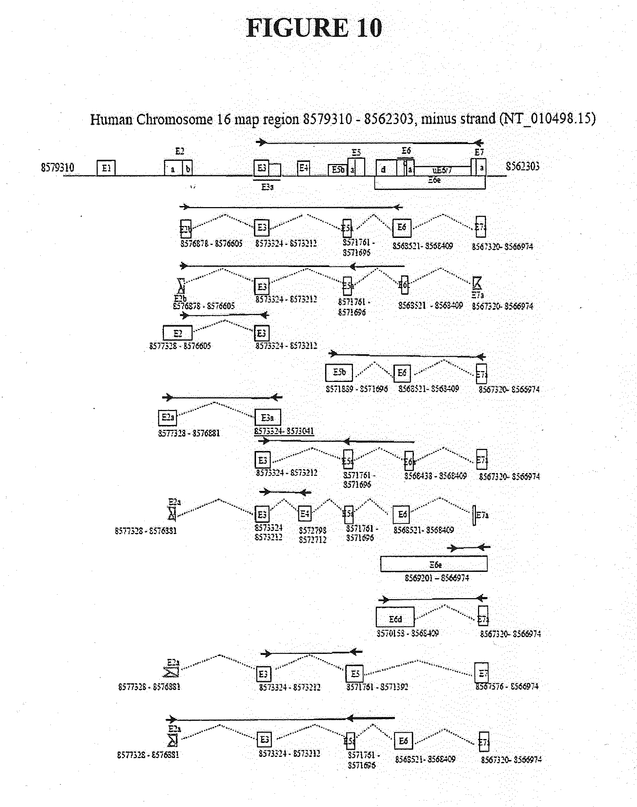

**Figure 10** is a schematic representation of predicted RNA variants derived from hCG_1815491. cDNA clones derived from map region 8579310 to 8562303 on human chromosome 16 were used to locate exon sequences. Arrows: Oligo nucleotide primer sets were designed to allow measurement of individual RNA variants by PCR. Primers covering splice junctions are shown as spanning intron sequences which is not included in the actual oligonucleotide primer sequence.

### DETAILED DESCRIPTION OF THE INVENTION

The present invention is predicated, in part, on the elucidation of gene expression profiles which characterise large intestine cellular populations in terms of their neoplastic state and, more particularly, whether they are malignant or pre-malignant. This finding has now facilitated the development of routine means of screening for the onset or predisposition to the onset of a large intestine neoplasm or characterising cellular populations derived from the large intestine based on screening for upregulation of the expression of these molecules, relative to control expression patterns and levels. To this end, in addition to assessing expression levels of the subject genes relative to normal or non-neoplastic levels, it has been determined that a proportion of these genes are expressed only in the diseased state, thereby facilitating the development of a simple qualitative test based on requiring assessment only relative to test background levels.

In accordance with the present invention, it has been determined that the genes detailed above are modulated, in terms of differential changes to their levels of expression, depending on whether the cell expressing that gene is neoplastic or not, It should be understood that reference to a gene "expression product" or "expression of a gene" is a reference to either a transcription product (such as primary RNA or mRNA) or a translation product such as protein. These genes and their expression products, whether they be RNA transcripts or encoded proteins, are collectively referred to as "neoplastic markers".

Accordingly, one aspect of the present disclosure is directed to a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 201328 at | 221577 x at | 205828 at |
| 201341_at | 221922_at | 205886_at |
| 201416_at | 60474_at | 205890_s_at |
| 201417_at | 222696_at | 205910_s_at |
| 201468_s_at | 223447_at | 205941_s_at |
| 201506_at | 223970_at | 206224_at |
| 201563_at | 225541_at | 206976_s_at |
| 201656_at | 225835_at | 207173_x_at |
| 201925_s_at | 226360_at | 207457_s_at |
| 201926_s_at | 227174_at | 208079_s_at |
| 202286_s_at | 227475_at | 208712_at |
| 202718_at | 228303_at | 209218_at |
| 202831_at | 228653_at | 209309_at |
| 202833_s_at | 228754_at | 209752_at |
| 202935_s_at | 228915_at | 209773_s_at |
| 202936_s_at | 229215_at | 209774_x_at |
| 203124_s_at | 231832_at | 209792_s_at |
| 203256_at | 231941_s_at | 209875_s_at |
| 203313_s_at | 232176_at | 209955_s_at |
| 203510_at | 232252_at | 210052_s_at |
| 203860_at | 232481_s_at | 210511_s_at |
| 203895_at | 234331_s_at | 210559_s_at |
| 203896_s_at | 235210_s_at | 210766_s_at |
| 203961_at | 235976_at | 211506_s_at |
| 203962_s_at | 236894_at | 212281_s_at |
| 204259_at | 238017_at | 212344_at |
| 204351_at | 238021_s_at | 212353_at |
| 204401_at | 238984_at | 212354_at |
| 204404_at | 241031_at | 213905_x_at |
| 204855_at | 200660_at | 214022_s_at |
| 204885_s_at | 200832_s_at | 214974_x_at |
| 205174_s_at | 200903_s_at | 215091_s_at |
| 205366_s_at | 201014_s_at | 217430_x_at |
| 205470_s_at | 201112_s_at | 217996_at |
| 205513_at | 201195_s_at | 218507_at |
| 205765_at | 201261_x_at | 218963_s_at |
| 205825_at | 201292_at | 218984_at |
| 205927_s_at | 201338_x_at | 219787_s_at |
| 205983_at | 201479_at | 219911_s_at |
| 206239_s_at | 201577_at | 221729_at |
| 206286_s_at | 201601_x_at | 221730_at |
| 207158_at | 201666_at | 221731_x_at |
| 207850_at | 202310_s_at | 221923_s_at |
| 209369_at | 202311_s_at | 37892_at |
| 210445_at | 202404_s_at | 222449_at |
| 210519_s_at | 202431_s_at | 222450_at |
| 211429_s_at | 202504_at | 222549_at |
| 212063_at | 202779_s_at | 222608_s_at |
| 212070_at | 202859_x_at | 223062_s_at |
| 212190_at | 202954_at | 224428_s_at |
| 212531_at | 202998_s_at | 224646_x_at |
| 212942_s_at | 203083_at | 224915_x_at |
| 213880_at | 203213_at | 225295_at |
| 213975_s_at | 203878_s_at | 225520_at |
| 214235_at | 204051_s_at | 225664_at |
| 214651_s_at | 204127_at | 225681_at |
| 217523_at | 204170_s_at | 225767_at |
| 217867_x_at | 204320_at | 225799_at |
| 218086_at | 204470_at | 225806_at |
| 218211_s_at | 204475_at | 226227_x_at |
| 218704_at | 204580_at | 226237_at |
| 218796_at | 204620_s_at | 226311_at |
| 218872_at | 204702_s_at | 226777_at |
| 219630_at | 205361_s_at | 226835_s_at |
| 219682_s_at | 205476_at | 227140_at |
| 219727_at | 205479_s_at | 229802_at |
| 219955_at | 205713_s_at | 231766_s_at |
| 219956_at | 205815_at | 232151_at; and/or |
| 200665_s_at | | |

**(ii)**

| | | | |
|---|---|---|---|
| CTHRC1 | | CDCA7 | LOXL2 |
| FOXQ1 | RNF43 | TDGF1 | AZGP1 |
| MMP1 | CCL20 | MTHFD1L | MYC |
| TCN1 | CTSE | ANLN | COMP |
| MMP7 | MSLN | H19 | AURKA |
| WDR72 | TIMP1 | FAP | PAICS |
| INHBA | PCSK1 | DACH1 | PUS7 |
| COL11A1 | CST1 | VCAN | ZNRF3 |
| GDF15 | BGN | SQLE | CCND1 |
| COL1A1 | AXIN2 | REG3A | CSEIL |
| LGR5 | MET | TESC | PFDN4 |
| DUSP27 | SOX9 | UBE2C | C20orf42 |
| SERPINB5 | TMEPAI | TMEM97 | SLC11A2 |
| ASCL2 | CDH11 | TRIM29 | NFE2L3 |
| SULF1 | MMP11 | KLK11 | CEL |
| SLC6A6 | CXCL1 | LY6G6D | NLF1 |
| TACSTD2 | QPCT | SLC7A5 | NPDC1 |
| NEBL | PDZK1IP1 | FABP6 | ENC1 |
| PCCA | CD55 | SLITRK6 | SERPINE2 |
| FLJ37644 | ECT2 | MLPH | UBE2S |
| KRT23 | COL12A1 | HOXA9 | TOP2A |
| CXCL3 | L1TD1 | TBX3 | CDC2 |
| MMP3 | WDR51B | BACE2 | RFC3 |
| SFRP4 | HOXB6 | GPX2 | LILRB1 |
| UBD | FAM84A | TPX2 | NPM1 |
| SCD | COL5A2 | KCNN4 | RDHE2 |
| DPEP1 | MMP12 | LOC541471 | PLAU |
| LCN2 | SORD | CYP3A5 | HSPH1 |
| PLCB4 | PSAT1 | ANXA3 | GTF3A |
| SPINK1 | CXCL5 | CYP3A5P2 | KLK10 |
| DUOX2 | IGFBP2 | C14orf94 | GPSM2 |
| SOX4 | TGIF1 | RP5-875H10.1 | NME1 |
| THBS2 | CXCL2 | RPL22L1 | MUC20 |
| CLDN1 | SLCO4A1 | APOBEC1 | RPESP |
| REG1B | CKS2 | HIG2 | RRM2 |
| CDH3 | PHLDA1 | COL10A1 | DKC1 |
| SPARC | SERPINA1 | AHCY | CD44 |
| | COL1A2 | IL8 | |

in a biological sample from said individual wherein a higher level of expression of the genes or transcripts of group (i) and/or group (ii) relative to control levels is indicative of a neoplastic large intestine cell or a cell predisposed to the onset of a neoplastic state.

Reference to "large intestine" should be understood as a reference to a cell derived from one of the six anatomical regions of the large intestine, which regions commence after the terminal region of the ileum, these being:
(i) the cecum;
(ii) the ascending colon;
(iii) the transverse colon;
(iv) the descending colon;
(v) the sigmoid colon; and
(vi) the rectum.

Reference to "neoplasm" should be understood as a reference to a lesion, tumour or other encapsulated or unencapsulated mass or other form of growth which comprises neoplastic cells. A "neoplastic cell" should be understood as a reference to a cell exhibiting abnormal growth. The term "growth" should be understood in its broadest sense and includes reference to proliferation. In this regard, an example of abnormal cell growth is the uncontrolled proliferation of a cell. Another example is failed apoptosis in a cell, thus prolonging its usual life span. The neoplastic cell may be a benign cell or a malignant cell. In a preferred embodiment, the subject neoplasm is an adenoma or an adenocarcinoma. Without limiting the present invention to any one theory or mode of action, an adenoma is generally a benign tumour of epithelial origin which is either derived from epithelial tissue or exhibits clearly defined epithelial structures. These structures may take on a glandular appearance. It can comprise a malignant cell population within the adenoma, such as occurs with the progression of a benign adenoma to a malignant adenocarcinoma.

Preferably, said neoplastic cell is an adenoma or adenocarcinoma and even more preferably a colorectal adenoma or adenocarcinoma.

Each of the genes and transcripts detailed in sub-paragraphs (i) and (ii), above, would be well known to the person of skill in the art, as would their encoded proteins. The identification of the expression products of these genes and transcripts as markers of neoplasia occurred by virtue of differential expression analysis using Affymetrix HGU133A or HGU133B gene chips. To this end, each gene chip is characterised by approximately 45,000 probe sets which detect the RNA transcribed from the genome. On average, approximately 11 probe pairs detect overlapping or consecutive regions of the RNA transcript. In general, the genes from which the RNA transcripts described herein are identified by the Affymetrix probesets are well known and characterised genes. However, to the extent that some of the probesets detect RNA transcripts which are not yet defined, these transcripts are indicated as "the gene, genes or transcripts detected by Affymetrix probe x". In some cases a number of genes and/or transcripts may be detectable by a single probeset. It should be understood, however, that this is not intended as a limitation as to how the expression level of the subject gene or transcript can be detected. In the first instance, it would be understood that the subject gene transcript is also detectable by other probesets which would be present on the Affymetrix gene chip. The reference to a single probesets is merely included as an identifier of the gene transcript of interest. In terms of actually screening for the transcript, however, one may utilise a probe or probeset directed to any region of the transcript and not just to the 3' terminal 600bp transcript region to which the Affymetrix probesets are often directed.

Reference to each of the genes and transcripts detailed above and their transcribed and translated expression products should therefore be understood as a reference to all forms of these molecules and to fragments or variants thereof. As would be appreciated by the person of skill in the art, some genes are known to exhibit allelic variation between individuals. Accordingly, the present invention should be understood to extend to such variants which, in terms of the present diagnostic applications, achieve the same outcome despite the fact that minor genetic variants between the actual nucleic acid sequences may exist between individuals or that within one individual there may exist two or more splice variants of one subject gene. The present invention should therefore be understood to extend to all forms of RNA (eg mRNA, primary RNA transcript, miRNA, etc), cDNA and peptide isoforms which arise from alternative splicing or any other mutation, polymorphic or allelic variation. It should also be understood to include reference to any subunit polypeptides such as precursor forms which may be generated, whether existing as a monomer, multimer, fusion protein or other complex.

For example, in one embodiment of the disclosure, the subject gene is CDH3. Analysis of the AceView Database reveals that there exist 12 CDH3 alternative mRNA transcripts. Nine are generated by alternative splicing while three are unspliced forms. In terms of the genes encompassed by the present invention, means for determining the existence of such variants and characterising same, are described in Example 6, To the extent that the genes of the present invention are described by reference to an Affymetrix probeset, Table 9 provides details of the nucleic acid sequence to which each probeset is directed. Based on this information, the skilled person could, as a matter of routine procedure, identify the gene in respect of which that sequence forms part. A typical protocol for doing this is also outlined in Example 6.

It should be understood that the "individual" who is the subject of testing may be any human or non-human mammal. Examples of non-human mammals includes primates, livestock animals (e.g. horses, cattle, sheep, pigs, donkeys), laboratory test animals (e.g. mice, rats, rabbits, guinea pigs), companion animals (e.g. dogs, cats) and captive wild animals (e.g. deer, foxes). Preferably the mammal is a human.

The method of the present invention is predicated on the comparison of the level of the neoplastic markers of a biological sample with the control levels of these markers. The "control level" may be either a "normal level", which is the level of marker expressed by a corresponding large intestine cell or cellular population which is not neoplastic, or the background level which is detectable in a negative control sample.

The normal (or "non-neoplastic") level may be determined using tissues derived from the same individual who is the subject of testing. However, it would be appreciated that this may be quite invasive for the individual concerned and it is therefore likely to be more convenient to analyse the test results relative to a standard result which reflects individual or collective results obtained from individuals other than the patient in issue. This latter form of analysis is in fact the preferred method of analysis since it enables the design of kits which require the collection and analysis of a single biological sample, being a test sample of interest. The standard results which provide the normal level may be calculated by any suitable means which would be well known to the person of skill in the art. For example, a population of normal tissues can be assessed in terms of the level of the neoplastic markers of the present invention, thereby providing a standard value or range of values against which all future test samples are analysed. It should also be understood that the normal level may be determined from the subjects of a specific cohort and for use with respect to test samples derived from that cohort. Accordingly, there may be determined a number of standard values or ranges which correspond to cohorts which differ in respect of characteristics such as age, gender, ethnicity or health status. Said "normal level" may be a discrete level or a range of levels. An increase in the expression level of the subject genes relative to normal levels is indicative of the tissue being neoplastic.

Without limiting the present invention to any one theory or mode of action, although each of the genes hereinbefore described is differentially expressed, either singly or in combination, as between neoplastic versus non-neoplastic cells of the large intestine, and is therefore diagnostic of the existence of a large intestine neoplasm, the expression of some of these genes was found to exhibit particularly significant levels of sensitivity, specificity and positive and negative predictive value. Accordingly, in a preferred embodiment one would screen for and assess the expression level of one or more of these genes. To this end, and without limiting the present disclosure to any one theory or mode of action, the following markers were determined to be expressed in neoplastic tissue at a level of 3, 4, 5 or 7 fold greater than non-neoplastic tissue when assessed by virtue of the method exemplified herein.

| **Fold Increase** | **Gene, genes or transcripts detected by Affymetrix Probe No:** | **Gene** |
|---|---|---|
| | 225681_at | CTHRC1 |
| 7 | 227140_at | |
| 5 | 227475_at | FOXQ1 |
| | 204475_at | MMP1 |
| | 20285_x_at | IL8 |
| | 202404_s_at | COL1A2 |
| | 205513_at | TCN1 |
| 4 | 204259_at | MMP7 |
| | 227174_at | WDR72 |
| | 210511_s_at | INHBA |
| | 37892_at | COL11A1 |
| | 221577_x_at | GDF15 |
| 3 | 202310_s_at | COL1A1 |
| | 213880_at | LGR5 |
| | 232252_at | DUSP27 |
| | 204855_at | SERPINB5 |
| | 229215_at | ASCL2 |
| | 212354_at | SULF1 |
| | 228754_at | SLC6A6 |
| | 202286_s_at | TACSTD2 |
| | 203962_s_at | NEBL |
| | 203860_at | PCCA |
| | 202935_s_at | FLJ37644 |
| | 218963_s_at | KRT23 |
| | 207850_at | CXCL3 |
| | 205828_at | MMP3 |
| | 204051_s_at | SFRP4 |
| | 205890_s_at | UBD |
| | 200832_s_at | SCD |
| | 205983_at | DPEP1 |
| | 212531_at | LCN2 |

There is therefore more particularly provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs: 225681_at; 227140_at; and/or
**(ii)** CTHRC1
in a biological sample from said individual wherein a higher level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In another embodiment, there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs: 227475_at, 204475_at, 202859_x_at, 202404_s_at; and/or
**(ii)** FOXQ1, MMP1, IL8, COL1A2
in a biological sample from said individual wherein a higher level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In yet another embodiment there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs: 205513_at, 204259_at, 227174_at, 210511_s_at, 37892_at; and/or
**(ii)** TCN1, MMP7, WDR72, INHBA, COL11A1
in a biological sample from said individual wherein a higher level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In still yet another preferred embodiment, there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 221577_x_at | 232252_at | 202310_s_at |
| 213880_at | 212354_at | 204855_at |
| 229215_at | 203962_s_at | 228754_at |
| 202286_s_at | 218963_s_at | 203860_at |
| 202935_s_at | 204051_s_at | 207850_at |
| 205828_at | 205983_at | 205890_s_at |
| 200832_s_at | | 212531_at; and/or |

**(ii)**

| | | |
|---|---|---|
| LGR5 | GDF15 | COL1A1 |
| ASCL2 | DUSP27 | SERPINB5 |
| TACSTD2 | SULF1 | SLC6A6 |
| FLJ37644 | NEBL | PCCA |
| MMP3 | KRT23 | CXCL3 |
| SCD | SFRP4 | UBD |
| | DPEP1 | LCN2 |

in a biological sample from said individual wherein a higher level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

According to these aspects of the present invention, said large intestine tissue is preferably colorectal tissue.

The detection method of the present invention can be performed on any suitable biological sample. To this end, reference to a "biological sample" should be understood as a reference to any sample of biological material derived from an animal such as, but not limited to, cellular material, biofluids (eg. blood), faeces, tissue biopsy specimens, surgical specimens or fluid which has been introduced into the body of an animal and subsequently removed (such as, for example, the solution retrieved from an enema wash). The biological sample which is tested according to the method of the present invention may be tested directly or may require some form of treatment prior to testing. For example, a biopsy or surgical sample may require homogenisation prior to testing or it may require sectioning for *in situ* testing of the qualitative expression levels of individual genes. Alternatively, a cell sample may require permeabilisation prior to testing. Further, to the extent that the biological sample is not in liquid form, (if such form is required for testing) it may require the addition of a reagent, such as a buffer, to mobilise the sample.

To the extent that the neoplastic marker gene expression product is present in a biological sample, the biological sample may be directly tested or else all or some of the nucleic acid or protein material present in the biological sample may be isolated prior to testing. In yet another example, the sample may be partially purified or otherwise enriched prior to analysis. For example, to the extent that a biological sample comprises a very diverse cell population, it may be desirable to enrich for a sub-population of particular interest. It is within the scope of the present invention for the target cell population or molecules derived therefrom to be treated prior to testing, for example, inactivation of live virus or being run on a gel. It should also be understood that the biological sample may be freshly harvested or it may have been stored (for example by freezing) prior to testing or otherwise treated prior to testing (such as by undergoing culturing).

The choice of what type of sample is most suitable for testing in accordance with the method disclosed herein will be dependent on the nature of the situation. Preferably, said sample is a faecal (stool) sample, enema wash, surgical resection, tissue biopsy or blood sample.

In a related aspect, it has been determined that certain of the markers hereinbefore defined are more indicative of adenoma development versus cancer development or vice versa. This is an extremely valuable finding since it enables one to more specifically characterise the likely nature of a neoplasm which is detected by virtue of the method of the present invention.

Accordingly, in a related aspect the present disclosure is directed to a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 201328_at | 205825_at | 219956_at |
| 201468_s_at | 205927_s_at | 221922_at |
| 201656_at | 206239_s_at | 223447_at |
| 201925_s_at | 207158_at | 223970_at |
| 201926_s_at | 210445_at | 225835_at |
| 202718_at | 210519_s_at | 226360_at |
| 202831_at | 211429_s_at | 228303_at |
| 202833_s_at | 212063_at | 228653_at |
| 203124_s_at | 213975_8_at | 228915_at |
| 203313_s_at | 214235_at | 231832_at |
| 203860_at | 214651_s_at | 231941_s_at |
| 203895_at | 217523_at | 232176_at |
| 203896_s_at | 217867_x_at | 232481_s_at |
| 204401_at | 218086_at | 234331_s_at |
| 204885_s_at | 218211_s_at | 235210_s_at |
| 205174_s_at | 218796_at | 235976_at |
| 205366_s_at | 219630_at | 236894_at |
| 205470_s_at | 219682_s_at | 238017_at |
| 205513_at | 219727_at | 238984_at |
| 205765_at | 219955_at; | 241031_at; and/or |

**(ii)**

| | | |
|---|---|---|
| APOBEC1 | HOXB6 | QPCT |
| BACE2 | IGFBP2 | RDHE2 |
| C20orf42 | ITGA6 | REG4 |
| CD44 | KCNN4 | RETNLB |
| CD55 | KLK11 | RP5-875H10.1 |
| CTSE | L1TD1 | RPESP |
| CYP3A5 | LILRB1 | SERPINA1 |
| CYP3A5P2 | MLPH | SLC11A2 |
| DACH1 | MSLN | SLC12A2 |
| DUOX2 | MUC20 | SLITRK6 |
| ETS2 | NLF1 | SPINK1 |
| FABP6 | NPDC1 | TBX3 |
| FAM84A | NQO1 | TCN1 |
| GALNT6 | PCCA | TGIF1 |
| GPSM2 | PCSK1 | WDR51B |
| GPX2 | PDZK1IP1 | ZNRF3 |
| HOXA9 | PLCB4 | |

in a biological sample from said individual wherein a higher level of expression of the genes or transcripts of group (i) and/or group (ii) relative to control levels is indicative of an adenoma cell or a cell predisposed to the onset of an adenoma state.

In another preferred embodiment of this aspect of the present disclosure there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 200660_at | 205476_at | 217430_x_at |
| 200665_s_at | 205479_s_at | 217996_at |
| 200832_s_at | 205713_s_at | 218507_at |
| 200903_s_at | 205815_at | 218963_s_at |
| 201014_s_at | 205828_at | 218984_at |
| 201112_s_at | 205886_at | 219787_s_at |
| 201195_s_at | 205890_s_at | 219911_s_at |
| 201261_x_at | 205910_s_at | 221729_at |
| 201292_at | 205941_s_at | 221730_at |
| 201338_x_at | 206224_at | 221731_x_at |
| 201479_at | 206976_s_at | 221923_s_at |
| 201577_at | 207173_x_at | 37892_at |
| 201601_x_at | 207457_s_at | 222449_at |
| 201666_at | 208079_s_at | 222450_at |
| 202310_s_at | 208712_at | 222549_at |
| 202311_s_at | 209218_at | 222608_s_at |
| 202403_s_at | 209309_at | 223062_s_at |
| 202404_s_at | 209752_at | 224428_s_at |
| 202431_s_at | 209773_s_at | 224646_x_at |
| 202504_at | 209774_x_at | 224915_x_at |
| 202779_s_at | 209792_s_at | 225295_at |
| 202859_x_at | 209875_s_at | 225520_at |
| 202954_at | 209955_s_at | 225664_at |
| 202998_s_at | 210052_s_at | 225681_at |
| 203083_at | 210511_s_at | 225767_at |
| 203213_at | 210559_s_at | 225799_at |
| 203878_s_at | 210766_s_at | 225806_at |
| 204051_s_at | 211506_s_at | 226227_x_at |
| 204127_at | 212281_s_at | 226237_at |
| 204170_s_at | 212344_at | 226311_at |
| 204320_at | 212353_at | 226777_at |
| 204470_at | 212354_at | 226835_s_at |
| 204475_at | 213905_x_at | 227140_at |
| 204580_at | 214022_s_at | 229802_at |
| 204620_s_at | 214974_x_at | 231766_s_at |
| 204702_s_at | 215091_s_at; | 232151_at; and/or |
| 205361_s_at | | |

**(ii)**

| | | |
|---|---|---|
| AHCY | DKC1 | PSAT1 |
| ANLN | ECT2 | PUS7 |
| AURKA | FAP | REG1A |
| AZGP1 | GTF3A | REG1B |
| BGN | H19 | REG3A |
| C14orf94 | HIG2 | RFC3 |
| C20orf199 | HSPH1 | RRM2 |
| CCL20 | IFITM1 | S100A11 |
| CCND1 | IL8 | SCD |
| CDC2 | INHBA | SFRP4 |
| CDCA7 | KLK10 | SLC39A10 |
| CDH11 | KRT23 | SLC7A5 |
| CEL | LOC541471 | SLC04A1 |
| CKS2 | LOXL2 | SPARC |
| CLDN1 | LY6G6D | SPP1 |
| COL10A1 | MMP1 | SQLE |
| COL11A1 | MMP11 | SULF1 |
| COL12A1 | MMP12 | THBS2 |
| COL1A1 | MMP3 | TIMP1 |
| COL1A2 | MTHFD1L | TMEM97 |
| COL5A2 | MYC | TMEPAI |
| COL8A1 | NFE2L3 | TOP2A |
| COMP | NME1 | TPX2 |
| CSE1L | NPM1 | TRIM29 |
| CST1 | PAICS | UBD |
| CTHRC1 | PFDN4 | UBE2C |
| CXCL1 | PHLDA1 | UBE2S |
| CXCL2 | PLAU | VCAN |
| CXCL5 | | |

in a biological sample from said individual wherein a higher level of expression of the genes or transcripts of group (i) and/or group (ii) relative to control levels is indicative of a cancer cell or a cell predisposed to the onset of a cancerous state.

According to these aspects, said control levels are preferably non-neoplastic levels and said large intestine tissue is colorectal tissue. Even more preferably, said biological sample is a stool sample or blood sample.

In a related aspect, it has been determined that a subpopulation of the markers of the present invention are not only expressed at levels higher than normal levels, their expression pattern is uniquely characterised by the fact that expression levels above that of background control levels are not detectable in non-neoplastic tissue. This determination has therefore enabled the development of qualitative screening systems which are simply designed to detect marker expression relative to a control background level. In accordance with this aspect of the present invention, said "control level" is therefore the "background level". Preferably, said background level is of the chosen testing methodology.

According to this aspect, there is therefore provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 202286_s_at | 235976_at | 209309_at |
| 204259_at | 236894_at | 211506_s_at |
| 204885_s_at | | 214974_x_at |
| 205174_s_at | 238984_at | 219787_s_at |
| 205825_at | 241031_at | 37892_at |
| 207850_at | 202311_s_at | 222608_s_at |
| 213880_at | 204320_at | 223062_s_at |
| 217523_at | 204475_at | 225806_at |
| 227174_at | 204702_s_at | 226237_at |
| 228915_at | 205910_s_at | 227140_at |
| 232252_at | 206224_at | 229802_at; and/or |

**(ii)**

| | | |
|---|---|---|
| MMP1 | PCSK1 | ANLN |
| MMP7 | CST1 | DACH1 |
| LGR5 | QPCT | COL11A1 |
| WDR72 | ECT2 | C14orf94 |
| COL11A1 | SLITRK6 | AZGP1 |
| COL1A1 | L1TD1 | REG4 |
| DUSP27 | KIAA1199 | NFE2L3 |
| NLF1 | PSAT1 | CEL |
| IL8 | CXCL5 | CD44 |
| TACSTD2 | CXCL3 | COL8A1 |
| MSLN | | |

in a biological sample from said individual wherein a higher level of expression of the genes or transcripts of group (i) and/or group (ii) relative to background levels is indicative of a neoplastic cell or a cell predisposed to the onset of a neoplastic state.

In a most preferred embodiment, said genes or transcripts are selected from:
**(i)** the gene, genes or transcripts detected by Affymetrix probeset IDs:

| | | |
|---|---|---|
| 227140_at | 227174_at | 241031_at |
| 204475_at | 37892_at | 211506_s_at |
| 204259_at | 202311_s_at | 202286_s_at |
| 213880_at | 232252_at | 226237_at; and/or |

**(ii)**

| | | |
|---|---|---|
| MMP1 | COL11A1 | IL8 |
| MMP7 | COL1A1 | TACSTD2 |
| LGR5 | DUSP27 | COL8A1 |
| WDR72 | NLF1 | |

Preferably, said neoplasm is an adenoma or an adenocarcinoma and said gastrointestinal tissue is colorectal tissue.

In yet another embodiment, it has been determined that a further subpopulation of these markers are more characteristic of adenoma development, while others are more characteristic of cancer development. Accordingly, there is provided a convenient means of qualitatively obtaining indicative information in relation to the characteristics of the subject neoplasm.

According to this embodiment there is provided a method of screening for the onset or predisposition to the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 204885_s_at | 217523_at | 236894_at |
| 205174_s_at | 228915_at | 238984_at |
| 205825_at | 235976_at | 241031_at; and/or |

**(ii)**

| | | |
|---|---|---|
| CD44 | MSLN | QPCT |
| DACH1 | NLF1 | REG4 |
| L1TD1 | PCSK1 | SLITRK6 |

in a biological sample from said individual wherein a higher level of expression of the genes or transcripts of group (i) and/or group (ii) relative to background levels is indicative of an adenoma cell or a cell predisposed to the onset of an adenoma state.

In yet still another preferred embodiment there is provided a method of screening for the onset or predisposition to the onset of a large intestine neoplasm in an individual, said method comprising measuring the level of expression of one or more genes or transcripts selected from:
**(i)** the gene or genes detected by Affymetrix probeset IDs:

| | | |
|---|---|---|
| 202311_s_at | 209309_at | 223062_s_at |
| 204320_at | 211506_s_at | 225806_at |
| 204475_at | 214974_x_at | 226237_at |
| 204702_s_at | 219787_s_at | 227140_at |
| 205910_s_at | 37892_at | 229802_at; and/or |
| 206224_at | 222608_s_at | |

**(ii)**

| | | |
|---|---|---|
| ANLN | COL1A1 | IL8 |
| AZGP1 | COL8A1 | MMP1 |
| C14orf94 | CST1 | NFE2L3 |
| CEL | CXCL5 | PSAT1 |
| COL11A1 | ECT2 | |

in a biological sample from said individual wherein a higher level of expression of the genes or transcripts of group (i) and/or group (ii) relative to background levels is indicative of a cancer cell or a cell predisposed to the onset of a cancerous state.

Preferably, said large intestine tissue is colorectal tissue.

More preferably, said biological sample is a blood sample or stool sample,

As detailed hereinbefore, the present invention is designed to screen for a neoplastic cell or cellular population, which is located in the large intestine. Accordingly, reference to "cell or cellular population" should be understood as a reference to an individual cell or a group of cells. Said group of cells may be a diffuse population of cells, a cell suspension, an encapsulated population of cells or a population of cells which take the form of tissue.

Reference to "expression" should be understood as a reference to the transcription and/or translation of a nucleic acid molecule. In this regard, the present invention is exemplified with respect to screening for neoplastic marker expression products taking the form of RNA transcripts (eg primary RNA or mRNA). Reference to "RNA" should be understood to encompass reference to any form of RNA, such as primary RNA or mRNA. Without limiting the present invention in any way, the modulation of gene transcription leading to increased or decreased RNA synthesis will also correlate with the translation of some of these RNA transcripts (such as mRNA) to produce a protein product. Accordingly, the present invention also extends to detection methodology which is directed to screening for modulated levels or patterns of the neoplastic marker protein products as an indicator of the neoplastic state of a cell or cellular population. Although one method is to screen for mRNA transcripts and/or the corresponding protein product, it should be understood that the present invention is not limited in this regard and extends to screening for any other form of neoplastic marker expression product such as, for example, a primary RNA transcript. It is well within the skill of the person of skill in the art to determine the most appropriate screening target for any given situation. To this end, the genes which are known to encode an expression product which is either secreted by the cell or membrane bound is detailed in the table, below. It would be appreciated that screening for neoplastic markers which are secreted or membrane bound may provide particular advantages in terms of the design of a diagnostic screening product.

| The gene or genes detected by Affymetrix probe Nos: | | | |
|---|---|---|---|
| 200600_at | 203961_at | 212077_at | 224724_at |
| 200832_s_at | 203962_s_at | 212281_s_at | 224915_x_at |
| 200903_s_at | 204006_s_at | 212344_at | 225295_at |
| 200974_at | 204122_at | 212353_at | 225520_at |
| 201058_s_at | 204127_at | 212354_at | 225541_at |
| 201069_at | 204170_s_at | 212942_s_at | 225664_at |
| 201105_at | 204320_at | 213125_at | 225710_at |
| 201112_s_at | 204351_at | 213524_s_at | 225767_at |
| 201195_s_at | 204401_at | 213869_x_at | 225799_at |
| 201328_at | 204404_at | 213880_at | 225806_at |
| 201341_at | 204702_s_at | 214022_5_at | 225835_at |
| 201416_at | 204885_5_at | 214235_at | 226227_x_at |
| 201417_at | 205361_s_at | 214651_s_at | 226311_at |
| 201426_s_at | 205366_s_at | 215049_x_at | 226360_at |
| 201468_s_at | 205547_s_at | 217523_at | 226694_at |
| 201479_at | 205765_at | 217762_s_at | 226777_at |
| 201563_at | 205825_at | 217763_s_at | 226835_s_at |
| 201601_x_at | 205890_s_at | 217764_s_at | 227099_s_at |
| 201616_s_at | 205927_s_at | 217867_x_at | 227140_at |
| 201656_at | 205941_s_at | 217996_at | 227174_at |
| 201667_at | 205983_at | 218086_at | 227475_at |
| 201925_s_at | 206286_s_at | 218211_s_at | 227566_at |
| 201926_s_at | 206976_s_at | 218559_s_at | 228303_at |
| 202237_at | 207158_at | 218704_at | 228653_at |
| 202238_s_at | 207173_x_at | 218796_at | 228754_at |
| 202286_s_at | 207191_s_at | 218872_at | 228915_at |
| 202431_s_at | 208079_s_at | 218963_s_at | 229215_at |
| 202450_s_at | 208712_at | 218984_at | 229802_at |
| 202504_at | 208782_at | 219630_at | 231766_s_at |
| 202620_s_at | 208788_at | 219682_s_at | 231832_at |
| 202779_s_at | 208850_s_at | 219727_at | 231879_at |
| 202831_at | 208851_s_at | 219787_s_at | 232151_at |
| 202878_s_at | 209156_s_at | 219911_s_at | 232176_at |
| 202917_s_at | 209218_at | 219955_at | 232252_at |
| 202935_s_at | 209369_at | 219956_at | 232481_s_at |
| 202936_s_at | 209596_at | 221011_s_at | 233555_s_at |
| 202954_at | 209773_s_at | 221922_at | 234331_s_at |
| 203124_s_at | 209955_s_at | 221923_s_at | 234994_at |
| 203213_at | 210052_s_at | 222449_at | 235210_s_at |
| 203256_at | 210445_at | 222450_at | 235976_at |
| 203313_s_at | 210519_s_at | 222549_at | 236894_at |
| 203382_s_at | 210559_s_at | 222608_s_at | 238017_at |
| 203645_s_at | 210766_s_at | 223062_s_at | |
| 203860_at | 211964_at | 223235_s_at | 241031_at |
| 203878_s_at | 211966_at | 224428_s_at | 37892_at |
| 203895_at | 212063_at | 224646_x_at | 60474_at |
| 203896_s_at | 212070_at | 224694_at | |
| ACTA2 | DUOX2 | LOC541471 | S100A11 |
| AHCY | DUSP27 | MAFB | S100A8 |
| ANLN | ECT2 | MLPH | S100P |
| ANTXR1 | ELOVL5 | MMP11 | SCD |
| ANXA3 | ENC1 | MMP2 | SLC11A2 |
| APOBEC1 | ETS2 | MSLN | SLC12A2 |
| APOE | FABP6 | MSN | SLC39A10 |
| ASCL2 | FAM84A | MTHFD1L | SLC6A6 |
| AURKA | FAP | MXRA5 | SLC7A5 |
| BACE2 | FCGR3B | MYC | SLCO4A1 |
| C14orf94 | FLJ37644 | MYL9 | SLITRK6 |
| C20orf199 | FOXQ1 | NEBL | SMOC2 |
| C20orf42 | FSTL1 | NFE2L3 | SORD |
| CALD1 | G0S2 | NLF1 | SOX4 |
| CCND1 | GALNT6 | NNMT | SOX9 |
| CD163 | GJA1 | NPDC1 | SQLE |
| CD44 | GPR56 | NPM1 | SULF1 |
| CD55 | GPSM2 | NQO1 | SULF2 |
| CD93 | GPX2 | OLFML2B | TACSTD2 |
| CDC2 | H19 | PALM2-AKAP2 | TAGLN |
| CDCA7 | | PCCA | TBX3 |
| CDH11 | HNT | PCSK1 | TDGF1 |
| CDH3 | HOXA9 | PDZK1IP1 | TESC |
| CKS2 | HOXB6 | PFDN4 | TGIF1 |
| CLDN1 | HSPH1 | PHLDA1 | THY1 |
| COL10A1 | IFITM1 | PLCB4 | TMEM97 |
| COL11A1 | ISLR | PLOD2 | TMEPAI |
| COL12A1 | ITGA6 | PSAT1 | TPX2 |
| COL4A2 | KCNN4 | PUS7 | TRIM29 |
| COL6A2 | KIAA1199 | RAB31 | TYROBP |
| CSE1L | KIAA1913 | RDHE2 | UBD |
| CTSE | KRT23 | RFC3 | UBE2C |
| CTSK | L1TD1 | RNF43 | UBE2S |
| CYP3A5 | LBH | RP5-875H10.1 | VIM |
| CYP3A5P2 | LGALS1 | RPESP | WDR51B |
| DACH1 | LGR5 | RPL22L1 | WDR72 |
| DKC1 | LOC387763 | RRM2 | ZNRF3 |
| DPEP1 | | | |

Reference to "nucleic acid molecule" should be understood as a reference to both deoxyribonucleic acid molecules and ribonucleic acid molecules and fragments thereof. The present invention therefore extends to both directly screening for mRNA levels in a biological sample or screening for the complementary cDNA which has been reverse-transcribed from an mRNA population of interest. It is well within the skill of the person of skill in the art to design methodology directed to screening for either DNA or RNA. As detailed above, the method of the present invention also extends to screening for the protein product translated from the subject mRNA.

Preferably, the level of gene expression is measured by reference to genes which encode a protein product and, more particularly, said level of expression is measured at the protein level. Accordingly, to the extent that the present invention is directed to screening for markers which are detailed in the preceding table, said screening is preferably directed to the encoded protein.

As detailed hereinbefore, it should be understood that although the present invention is exemplified with respect to the detection of expressed nucleic acid molecules (e.g. mRNA), it also encompasses methods of detection based on screening for the protein product of the subject genes. The present invention should also be understood to encompass methods of detection based on identifying both proteins and/or nucleic acid molecules in one or more biological samples. This may be of particular significance to the extent that some of the neoplastic markers of interest may correspond to genes or gene fragments which do not encode a protein product. Accordingly, to the extent that this occurs it would not be possible to test for a protein and the subject marker would have to be assessed on the basis of transcription expression profiles.

In terms of screening for the upregulation of expression of a marker it would also be well known to the person of skill in the art that changes which are detectable at the DNA level are indicative of changes to gene expression activity and therefore changes to expression product levels. Such changes include but are not limited to, changes to DNA methylation. Accordingly, reference herein to "screening the level of expression" and comparison of these "levels of expression" to control "levels of expression" should be understood as a reference to assessing DNA factors which are related to transcription, such as gene/DNA methylation patterns.

The term "protein" should be understood to encompass peptides, polypeptides and proteins (including protein fragments). The protein may be glycosylated or unglycosylated and/or may contain a range of other molecules fused, linked, bound or otherwise associated to the protein such as amino acids, lipids, carbohydrates or other peptides, polypeptides or proteins. Reference herein to a "protein" includes a protein comprising a sequence of amino acids as well as a protein associated with other molecules such as amino acids, lipids, carbohydrates or other peptides, polypeptides or proteins.

The proteins encoded by the neoplastic markers of the present invention may be in multimeric form meaning that two or more molecules are associated together. Where the same protein molecules are associated together, the complex is a homomultimer. An example of a homomultimer is a homodimer. Where at least one marker protein is associated with at least one non-marker protein, then the complex is a heteromultimer such as a heterodimer.

Reference to a "fragment" should be understood as a reference to a portion of the subject nucleic acid molecule or protein. This is particularly relevant with respect to screening for modulated RNA levels in stool samples since the subject RNA is likely to have been degraded or otherwise fragmented due to the environment of the gut. One may therefore actually be detecting fragments of the subject RNA molecule, which fragments are identified by virtue of the use of a suitably specific probe.

Reference to the "onset" of a neoplasm, such as adenoma or adenocarcinoma, should be understood as a reference to one or more cells of that individual exhibiting dysplasia. In this regard, the adenoma or adenocarcinoma may be well developed in that a mass of dysplastic cells has developed. Alternatively, the adenoma or adenocarcinoma may be at a very early stage in that only relatively few abnormal cell divisions have occurred at the time of diagnosis. The present invention also extends to the assessment of an individual's predisposition to the development of a neoplasm, such as adenocarcinoma.

Without limiting the present invention in any way, changed levels of the neoplastic markers may be indicative of that individual's predisposition to developing a neoplasia, such as the further development of adenocarcinoma or another adenocarcinoma.

In yet another related aspect of the present invention, markers have been identified which enable the characterisation of neoplastic tissue of the large intestine in terms of whether it is an adenoma or a cancer. This development now provides a simple yet accurate means of characterising tissue using means other than the traditional methods which are currently utilised.

According to this aspect of the present invention, there is provided a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 200884_at | 214234_s_at | 226248_s_at |
| 203240_at | 214235_at | 226302_at |
| 203963_at | 214433_s_at | 227676_at |
| 204508_s_at | 215125_s_at | 227719_at |
| 204607_at | 215867_x_at | 227725_at |
| 204811_s_at | 217109_at | 228232_s_at |
| 204895_x_at | 217110_s_at | 229070_at |
| 204897_at | 218211_s_at | 231832_at |
| 205259_at | 219543_at | 232176_at |
| 205765_at | 219955_at | 232481_s_at |
| 205927_s_at | 221841_s_at | 235976_at |
| 208063_s_at | 221874_at | 236894_at |
| 208937_s_at | 223969_s_at | 237521_x_at |
| 210107_at | 223970_at | 242601_at |
| 213106_at; and/or | | |

**(ii)**

| | | |
|---|---|---|
| CLCA1 | CTSE | ATP8B1 |
| FCGBP | C6orf105 | CACNA2D2 |
| HMGCS2 | CKB | KLF4 |
| RETNLB | ATP8A1 | CYP3A5P2 |
| L1TD1 | MUC4 | CAPN9 |
| SLITRK6 | UGT1A1 | NR3C2 |
| VSIG2 | SELENBP1 | PBLD |
| LOC253012 | PTGER4 | CA12 |
| ST6GALNAC1 | MLPH | WDR51B |
| ID1 | KIAA1324 | FAM3D |
| CYP3A5 | | |

in said cell or cellular population wherein a higher level of expression of the genes or transcripts of group (i) and/or group (ii) relative to a gastrointestinal cancer cell level is indicative of an adenoma cell or a cell predisposed to the onset of an adenoma state.

In another aspect there is provided a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 200600_at | 204006_s_at | 213428_s_at |
| 200665_s_at | 204051_s_at | 213524_s_at |
| 200832_s_at | 204122_at | 213869_x_at |
| 200974_at | 204320_at | 213905_x_at |
| 200986_at | 204475_at | 214247_s_at |
| 201058_s_at | 204620_s_at | 215049_x_at |
| 201069_at | 205479_s_at | 215076_s_at |
| 201105_at | 205547_s_at | 215646_s_at |
| 201141_at | 205828_at | 216442_x_at |
| 201147_s_at | 207173_x_at | 217430_x_at |
| 201150_s_at | 207191_s_at | 217762_s_at |
| 201162_at | 208747_s_at | 217763_s_at |
| 201163_s_at | 208782_at | 217764_s_at |
| 201185_at | 208788_at | 218468_s_at |
| 201261_x_at | 208850_s_at | 218469_at |
| 201289_at | 208851_s_at | 218559_s_at |
| 201426_s_at | 209101_at | 218638_s_at |
| 201438_at | 209156_s_at | 219087_at |
| 201616_s_at | 209218_at | 221011_s_at |
| 201645_at | 209395_at | 221729_at |
| 201667_at | 209396_s_at | 221730_at |
| 201744_s_at | 209596_at | 221731_x_at |
| 201792_at | 209875_s_at | 37892_at |
| 201842_s_at | 209955_s_at | 223122_s_at |
| 201852_x_at | 210095_s_at | 223235_s_at |
| 201859_at | 210495_x_at | 224560_at |
| 201893_x_at | 210511_s_at | 224694_at |
| 202237_at | 210764_s_at | 224724_at |
| 202238_s_at | 210809_s_at | 225664_at |
| 202283_at | 211161_s_at | 225681_at |
| 202291_s_at | 211571_s_at | 225710_at |
| 202310_s_at | 211719_x_at | 225799_at |
| 202311_s_at | 211813_x_at | 226237_at |
| 202403_s_at | 211896_s_at | 226311_at |
| 202404_s_at | 211959_at | 226694_at |
| 202450_s_at | 211964_at | 226777_at |
| 202620_s_at | 211966_at | 226930_at |
| 202766_s_at | 211980_at | 227099_s_at |
| 202859_x_at | 211981_at | 227140_at |
| 202878_s_at | 212077_at | 227566_at |
| 202917_s_at | 212344_at | 229218_at |
| 202998_s_at | 212353_at | 229802_at |
| 203083_at | 212354_at | 231579_s_at |
| 203325_s_at | 212464_s_at | 231766_s_at |
| 203382_s_at | 212488_at | 231879_at |
| 203477_at | 212489_at | 232458_at |
| 203570_at | 212667_at | 233555_s_at |
| 203645_s_at | 213125_at | 234994_at |
| 203878_s_at; and/or | | |

**(ii)**

| | | |
|---|---|---|
| COL1A2 | LGALS1 | SRGN |
| CTHRC1 | ELOVL5 | LBH |
| FN1 | MGP | CTGF |
| POSTN | MMP2 | TNC |
| SPP1 | LOXL2 | G0S2 |
| MMP1 | MYL9 | SQLE |
| SPARC | DCN | EFEMP1 |
| LUM | CALD1 | APOE |
| GREM1 | FBN1 | MSN |
| IL8 | MMP3 | IGFBP3 |
| IGFBP5 | IGFBP7 | SERPINF1 |
| SFRP2 | FSTL1 | ISLR |
| SULF1 | COL4A2 | HNT |
| ASPN | VCAN | COL5A1 |
| COL6A3 | SMOC2 | OLFML2B |
| COL8A1 | HTRA1 | KIAA1913 |
| COL12A1 | CYR61 | PALM2-AKAP2 |
| COL5A2 | FAP | SERPING1 |
| CDH11 | VIM | TYROBP |
| THBS2 | TIMP2 | ACTA2 |
| COL15A1 | SCD | COL3A1 |
| COL11A1 | TIMP3 | PLOD2 |
| S100A8 | AEBP1 | MMP11 |
| FNDC1 | GJA1 | CD163 |
| SFRP4 | NNMT | FCGR3B |
| INHBA | COL1A1 | PLAU |
| COL6A2 | SULF2 | MAFB |
| ANTXR1 | COL6A1 | LOC541471 |
| GPNMB | SPON2 | LOC387763 |
| BGN | CTSK | CHI3L1 |
| TAGLN | MXRA5 | THY1 |
| COL4A1 | C1S | LOXL1 |
| RAB31 | DKK3 | CD93 |

in a biological sample from said individual wherein a higher level of expression of the genes or transcripts of group (i) and/or group (ii) relative to a gastrointestinal adenoma cell level is indicative of a cancer or a cell predisposed to the onset of a cancerous state.

Preferably, said gastrointestinal tissue is colorectal tissue.

Reference to an "adenoma control level" or "cancer control level" should be understood as a reference to the level of said gene expression in a population of adenoma or cancer gastrointestinal cells, respectively. As discussed hereinbefore in relation to "normal levels", the subject level may be a discrete level or a range of levels. Accordingly, the definition of "adenoma control level" or "cancer control level" should be understood to have a corresponding definition to "normal level", albeit in the context of the expression of genes by a neoplastic population of large intestine cells.

In terms of this aspect of the present invention, the subject analysis is performed on a population of neoplastic cells. These cells may be derived in any manner, such as sloughed of neoplastic cells which have been collected via an enema wash or from a gastrointestinal sample, such as a stool sample. Alternatively, the subject cells may have been obtained via a biopsy or other surgical technique.

Without limiting this aspect of the invention in any way, several of the markers of this aspect of the present disclosure have been determined to be expressed at particularly significant levels above those of neoplastic cells. For example, increased expression levels of 3- and 5-fold have been observed in respect of the following markers, when assessed by the method exemplified herein, which are indicative of gastrointestinal adenomas.

| **Fold Increase** | **Gene or genes detected by Affymetrix Probe No:** | **Gene** |
|---|---|---|
| 5 | 210107_at | CLCA1 |
| 3 | 203240_at | FCGBP |
| | 204607_at | HMGCS2 |
| | 223969_s_at | RETNLB |
| | 219955_at | L1TD1 |
| | 232481_s_at | SLITRK6 |
| | 228232_s_at | VSIG2 |
| | 242601_at | LOC253012 |
| | 227725_at | ST6GALNAC1 |

In another example, increased expression levels of between 3- and 9-fold have been observed in respect of the following markers which are indicative of gastrointestinal cancers, when assessed by the method herein exemplified:

| **Fold Increase** | **Gene or genes detected by Affymetrix Probe No:** | **Gene** |
|---|---|---|
| 9 | 202404_s_at | COL1A2 |
| 8 | 225681_at | CTHRC1 |
| 7 | 212464_s_at | FN1 |
| | 210809_s_at | POSTN |
| 6 | 209875_s_at | SPP1 |
| 5 | 227140_st | MMP1 |
| | 204475_at | |
| 4 | 200665_s_at | SPARC |
| | 201744_s_at | LUM |
| | 218468_s_at | GREM1 |
| | 202859_x_at | IL8 |
| | 211959_at | IGFBP5 |
| 3 | 223122_s_at | SFRP2 |
| | 212353_at | SULF1 |
| | 219087 at | ASPN |
| | 201438_at | COL6A3 |
| | 226237_at | COL8A1 |
| | 225664_at | COL12A1 |
| | 221730_at | COL5A2 |
| | 207173_x_at | CDH11 |
| | 203083_at | THBS2 |
| | 203477_at | COL15A1 |
| | 37892_at | COL11A1 |
| | 202917_s_at | S100A8 |
| | 226930_at | FNDC1 |
| | 204051_s_at | SFRP4 |
| | 210511_s_at | INHBA |
| | 209156_s_at | COL6A2 |
| | 224694_at | ANTXR1 |
| | 201141_at | GPNMB |
| | 213905_x_at | BGN |
| | 205547_s_at | TAGLN |

According to this embodiment, there is therefore provided a method of characterising a neoplastic cell or cellular population, which cell or cellular population is derived from the large intestine of an individual, said method comprising measuring the level of expression of one or more genes or transcripts selected from:
**(i)** the gene or genes detected by Affymetrix probeset IDs: 210107_at; and/or
**(ii)** CLCA1
in a biological sample from said individual wherein a higher level of expression of the genes or transcripts of group (i) and/or group (ii) relative to a gastrointestinal cancer control level is indicative of an adenoma cell or a cell predisposed to the onset of an adenoma state.

In another embodiment, there is provided a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 203240_at | 219955_at | 242601_at |
| 204607_at | 232481_s_at | 227725_at |
| 223969_s_at | 228232_s_at; and/or | |

**(ii)**

| | | |
|---|---|---|
| FCGBP | L1TD1 | LOC253012 |
| HMGCS2 | SLITRK6 | ST6GALNAC1 |
| RETNLB | VSIG2 | |

in a biological sample from said individual wherein a higher level of expression of the genes or transcripts of group (i) and/or group (ii) relative to a gastrointestinal cancer control level is indicative of an adenoma cell or a cell predisposed to the onset of an adenoma state.

Preferably, said gastrointestinal tissue is colorectal tissue.

Still more preferably, said biological sample is a tissue sample.

In another preferred embodiment the present disclosure is directed to a method of characterising a cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 202404_s_at | 210809_s_at | 227140_at |
| 225681_at | 209875_s_at | 204475_at |
| 212464_s_at; | and/or | |

**(ii)**

| | | |
|---|---|---|
| COL1A2 | FN1 | SPP1 |
| CTHRC 1 | POSTN | MMP1 |

in a biological sample from said individual wherein a higher level of expression of the genes or transcripts of group (i) and/or (ii) relative to a gastrointestinal adenoma control level is indicative of a cancer or a cell predisposed to the onset of a cancerous state.

In yet another preferred embodiment the present disclosure is directed to a method of characterising a cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 200665_s_at | 218468_s_at | 211959_at |
| 201744_s_at | 202859_x_at; and/or | |

**(ii)**

| | | |
|---|---|---|
| SPARC | GREM1 | IGFBP5 |
| LUM | IL8 | |

in a biological sample from said individual wherein a higher level of expression of the genes or transcripts of group (i) and/or (ii) relative to a gastrointestinal adenoma control level is indicative of a cancer or a cell predisposed to the onset of a cancerous state.

In still yet another preferred embodiment the present disclosure is directed to a method of characterising a cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 223122_s_at | 207173_x_at | 210511_s_at |
| 212353_at | 203083_at | 209156_s_at |
| 219087_at | 203477_at | 224694_at |
| 201438_at | 37892_at | 201141_at |
| 226237_at | 202917_s_at | 213905_x_at |
| 225664_at | 226930_at | 205547 _s_at |
| 221730_at | 204051_s_at; | and/or |

**(ii)**

| | | |
|---|---|---|
| SFRP2 | CDH11 | INHBA |
| SULF1 | THBS2 | COL6A2 |
| ASPN | COL15A1 | ANTXR1 |
| COL6A3 | COL11A1 | GPNMB |
| COL8A1 | S100A8 | BGN |
| COL12A1 | FNDC1 | TAGLN |
| COL5A2 | SFRP4 | |

in a biological sample from said individual wherein a higher level of expression of the genes or transcripts of group (i) and/or (ii) relative to a gastrointestinal adenoma control level is indicative of a cancer or a cell predisposed to the onset of a cancerous state.

Preferably, said gastrointestinal tissue is colorectal tissue.

Even more preferably, said biological sample is a tissue sample.

In still another related aspect it has been determined that a subset of the markers of this aspect of the present invention are useful as qualitative markers of neoplastic tissue characterisation in that these markers, if detectable above background levels in neoplastic tissue are indicative of cancerous tissue.

According to this aspect, the present invention provides a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | |
|---|---|
| 235976_at | 236894_at; and/or |

**(ii)**

| | |
|---|---|
| SLITRK6 | L1TD1 |

in a biological sample from said individual wherein a higher level of expression of the genes or transcripts of group (i) and/or (ii) relative to neoplastic tissue background levels is indicative of an adenoma cell or a cell predisposed to the onset of an adenoma state.

In another aspect the present disclosure provides a method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 202311_s_at | 209396_s_at | 226237_at |
| 204320_at | 215646_s_at | 227140_at |
| 204475_at | 37892_at | 229802_at |
| 209395_at; and/or | | |

**(ii)**

| | | |
|---|---|---|
| COL1A1 | VCAN | CHI3L1 |
| MMP 1 | COL8A1 | COL11A1 |

in a biological sample from said individual wherein a higher level of expression of the genes or transcripts of group (i) and/or (ii) relative to neoplastic tissue background neoplastic cell levels is indicative of a cancer or a cell predisposed to the onset of a cancerous state.

Preferably, said gastrointestinal tissue is colorectal tissue.

Still more preferably, said biological sample is a tissue sample.

In a most preferred embodiment, the methods of the present invention are preferably directed to screening for proteins encoded by the markers of the present invention.

Although the preferred method is to detect the expression products of the neoplastic markers for the purpose of diagnosing neoplasia development or predisposition thereto, the detection of converse changes in the levels of said markers may be desired under certain circumstances, for example, to monitor the effectiveness of therapeutic or prophylactic treatment directed to modulating a neoplastic condition, such as adenoma or adenocarcinoma development. For example, where elevated levels of the subject markers indicate that an individual has developed a condition characterised by adenoma or adenocarcinoma development, for example, screening for a decrease in the levels of these markers subsequently to the onset of a therapeutic regime may be utilised to indicate reversal or other form of improvement of the subject individual's condition.

The method of the present invention is therefore useful as a one-time test or as an on-going monitor of those individuals thought to be at risk of neoplasia development or as a monitor of the effectiveness of therapeutic or prophylactic treatment regimes directed to inhibiting or otherwise slowing neoplasia development. In these situations, mapping the modulation of neoplastic marker expression levels in any one or more classes of biological samples is a valuable indicator of the status of an individual or the effectiveness of a therapeutic or prophylactic regime which is currently in use. Accordingly, the method of the present invention should be understood to extend to monitoring for increases or decreases in marker expression levels in an individual relative to their normal level (as hereinbefore defined), background control levels, cancer levels, adenoma levels or relative to one or more earlier marker expression levels determined from a biological sample of said individual.

Means of testing for the subject expressed neoplasm markers in a biological sample can be achieved by any suitable method, which would be well known to the person of skill in the art, such as but not limited to:
(i) *In vivo* detection.
   Molecular Imaging may be used following administration of imaging probes or reagents capable of disclosing altered expression of the markers in the intestinal tissues.
   Molecular imaging (Moore et a/., BBA, 1402:239-249, 1988; Weissleder et al., Nature Medicine 6:351-355, 2000) is the *in vivo* imaging of molecular expression that correlates with the macro-features currently visualized using "classical" diagnostic imaging techniques such as X-Ray, computed tomography (CT), MRI, Positron Emission Tomography (PET) or endoscopy.
(ii) Detection of up-regulation of RNA expression in the cells by Fluorescent *In Situ* Hybridization (FISH), or in extracts from the cells by technologies such as Quantitative Reverse Transcriptase Polymerase Chain Reaction (QRTPCR) or Flow cytometric qualification of competitive RT-PCR products (Wedemeyer et al., Clinical Chemistry 48:9 1398-1405, 2002).
(iii) Assessment of expression profiles of RNA, for example by array technologies (Alon et al., Proc. Natl. Acad. Sci. USA: 96, 6745-6750, June 1999).
   A "microarray" is a linear or multi-dimensional array of preferably discrete regions, each having a defined area, formed on the surface of a solid support. The density of the discrete regions on a microarray is determined by the total numbers of target polynucleotides to be detected on the surface of a single solid phase support. As used herein, a DNA microarray is an array of oligonucleotide probes placed onto a chip or other surfaces used to detect complementary oligonucleotides from a complex nucleic acid mixture. Since the position of each particular group of probes in the array is known, the identities of the target polynucleotides can be determined based on their binding to a particular position in the microarray.
   Recent developments in DNA microarray technology make it possible to conduct a large scale assay of a plurality of target nucleic acid molecules on a single solid phase support. U.S. Pat. No. 5,837,832 (Chee et al.) and related patent applications describe immobilizing an array of oligonucleotide probes for hybridization and detection of specific nucleic acid sequences in a sample. Target polynucleotides of interest isolated from a tissue of interest are hybridized to the DNA chip and the specific sequences detected based on the target polynucleotides' preference and degree of hybridization at discrete probe locations. One important use of arrays is in the analysis of differential gene expression, where the profile of expression of genes in different cells or tissues, often a tissue of interest and a control tissue, is compared and any differences in gene expression among the respective tissues are identified. Such information is useful for the identification of the types of genes expressed in a particular tissue type and diagnosis of conditions based on the expression profile.
   In one example, RNA from the sample of interest is subjected to reverse transcription to obtain labelled cDNA. See U.S. Pat. No. 6,410,229 (Lockhart et al.) The cDNA is then hybridized to oligonucleotides or cDNAs of known sequence arrayed on a chip or other surface in a known order. In another example, the RNA is isolated from a biological sample and hybridised to a chip on which are anchored cDNA probes. The location of the oligonucleotide to which the labelled cDNA hybridizes provides sequence information on the cDNA, while the amount of labelled hybridized RNA or cDNA provides an estimate of the relative representation of the RNA or cDNA of interest. See Schena, et al. Science 270:467-470 (1995). For example, use of a cDNA microarray to analyze gene expression patterns in human cancer is described by DeRisi, et al. (Nature Genetics 14:457-460 (1996)).
   In a preferred embodiment, nucleic acid probes corresponding to the subject nucleic acids are made. The nucleic acid probes attached to the microarray are designed to be substantially complementary to the nucleic acids of the biological sample such that specific hybridization of the target sequence and the probes of the present invention occurs. This complementarity need not be perfect, in that there may be any number of base pair mismatches that will interfere with hybridization between the target sequence and the single stranded nucleic acids of the present invention, It is expected that the overall homology of the genes at the nucleotide level probably will be about 40% or greater, probably about 60% or greater, and even more probably about 80% or greater; and in addition that there will be corresponding contiguous sequences of about 8-12 nucleotides or longer. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions, particularly high stringency conditions.
   A nucleic acid probe is generally single stranded but can be partly single and partly double stranded. The strandedness of the probe is dictated by the structure, composition, and properties of the target sequence. In general, the oligonucleotide probes range from about 6, 8, 10, 12, 15, 20, 30 to about 100 bases long, with from about 10 to about 80 bases being preferred, and from about 15 to about 40 bases being particularly preferred. That is, generally entire genes are rarely used as probes. In some embodiments, much longer nucleic acids can be used, up to hundreds of bases. The probes are sufficiently specific to hybridize to a complementary template sequence under conditions known by those of skill in the art. The number of mismatches between the probe's sequences and their complementary template (target) sequences to which they hybridize during hybridization generally do not exceed 15%, usually do not exceed 10% and preferably do not exceed 5%, as-determined by BLAST (default settings).
   Oligonucleotide probes can include the naturally-occurring heterocyclic bases normally found in nucleic acids (uracil, cytosine, thymine, adenine and guanine), as well as modified bases and base analogues. Any modified base or base analogue compatible with hybridization of the probe to a target sequence is useful in the practice of the invention. The sugar or glycoside portion of the probe can comprise deoxyribose, ribose, and/or modified forms of these sugars, such as, for example, 2'-O-alkyl ribose. In a preferred embodiment, the sugar moiety is 2'-deoxyribose; however, any sugar moiety that is compatible with the ability of the probe to hybridize to a target sequence can be used.
   In one embodiment, the nucleoside units of the probe are linked by a phosphodiester backbone, as is well known in the art. In additional embodiments, internucleotide linkages can include any linkage known to one of skill in the art that is compatible with specific hybridization of the probe including, but not limited to phosphorothioate, methylphosphonate, sulfamate (e.g., U.S. Pat. No. 5,470,967) and polyamide (i.e., peptide nucleic acids). Peptide nucleic acids are described in Nielsen et al. (1991) Science 254: 1497-1500, U.S. Pat. No. 5,714,331, and Nielsen (1999) Curr. Opin. Biotechnol. 10:71-75.
   In certain embodiments, the probe can be a chimeric molecule; i.e., can comprise more than one type of base or sugar subunit, and/or the linkages can be of more than one type within the same primer. The probe can comprise a moiety to facilitate hybridization to its target sequence, as are known in the art, for example, intercalators and/or minor groove binders. Variations of the bases, sugars, and internucleoside backbone, as well as the presence of any pendant group on the probe, will be compatible with the ability of the probe to bind, in a sequence-specific fashion, with its target sequence. A large number of structural modifications, are possible within these bounds. Advantageously, the probes according to the present invention may have structural characteristics such that they allow the signal amplification, such structural characteristics being, for example, branched DNA probes as those described by Urdea et al. (Nucleic Acids Symp. Ser., 24:197-200 (1991)) or in the European Patent No. EP-0225,807. Moreover, synthetic methods for preparing the various heterocyclic bases, sugars, nucleosides and nucleotides that form the probe, and preparation of oligonucleotides of specific predetermined sequence, are well-developed and known in the art. A preferred method for oligonucleotide synthesis is based on the teaching of U.S. Pat. No. 5,419,966.
   Multiple probes may be designed for a particular target nucleic acid to account for polymorphism and/or secondary structure in the target nucleic acid, redundancy of data and the like. In some embodiments, where more than one probe per sequence is used, either overlapping probes or probes to different sections of a single target gene are used. That is, two, three, four or more probes, are used to build in a redundancy for a particular target. The probes can be overlapping (i.e. have some sequence in common), or are specific for distinct sequences of a gene. When multiple target polynucleotides are to be detected according to the present invention, each probe or probe group corresponding to a particular target polynucleotide is situated in a discrete area of the microarray.
   Probes may be in solution, such as in wells or on the surface of a micro-array, or attached to a solid support. Examples of solid support materials that can be used include a plastic, a ceramic, a metal, a resin, a gel and a membrane. Useful types of solid supports include plates, beads, magnetic material, microbeads, hybridization chips, membranes, crystals, ceramics and self-assembling monolayers. One example comprises a two-dimensional or three-dimensional matrix, such as a gel or hybridization chip with multiple probe binding sites (Pevzner et al., J. Biomol. Struc. & Dyn. 9:399-410, 1991; Maskos and Southern, Nuc. Acids Res. 20:1679-84, 1992). Hybridization chips can be used to construct very large probe arrays that are subsequently hybridized with a target nucleic acid. Analysis of the hybridization pattern of the chip can assist in the identification of the target nucleotide sequence. Patterns can be manually or computer analyzed, but it is clear that positional sequencing by hybridization lends itself to computer analysis and automation. In another example, one may use an Affymetrix chip on a solid phase structural support in combination with a fluorescent bead based approach. In yet another example, one may utilise a cDNA microarray. In this regard, the oligonucleotides described by Lockkart *et al.* (i.e. Affymetrix synthesis probes *in situ* on the solid phase) are particularly preferred, that is, photolithography.
   As will be appreciated by those in the art, nucleic acids can be attached or immobilized to a solid support in a wide variety of ways. By "immobilized" herein is meant the association or binding between the nucleic acid probe and the solid support is sufficient to be stable under the conditions of binding, washing, analysis, and removal. The binding can be covalent or non-covalent. By "non-covalent binding" and grammatical equivalents herein is meant one or more of either electrostatic, hydrophilic, and hydrophobic interactions. Included in non-covalent binding is the covalent attachment of a molecule, such as streptavidin, to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.
   Nucleic acid probes may be attached to the solid support by covalent binding such as by conjugation with a coupling agent or by covalent or non-covalent binding such as electrostatic interactions, hydrogen bonds or antibody-antigen coupling, or by combinations thereof. Typical coupling agents include biotin/avidin, biotin/streptavidin, Staphylococcus aureus protein A/IgG antibody F_{c} fragment, and streptavidin/protein A chimeras (T. Sano and C. R. Cantor, Bio/Technology 9:1378-81 (1991)), or derivatives or combinations of these agents. Nucleic acids may be attached to the solid support by a photocleavable bond, an electrostatic bond, a disulfide bond, a peptide bond, a diester bond or a combination of these sorts of bonds. The array may also be attached to the solid support by a selectively releasable bond such as 4,4'-dimethoxytrityl or its derivative, Derivatives which have been found to be useful include 3 or 4 [bis-(4-methoxyphenyl)]-methyl-benzoic acid, N-succinimidyl-3 or 4 [bis-(4-methoxyphenyl)]-methyl-benzoic acid, N-succinimidyl-3 or 4 [bis-(4-methoxyphenyl)]-hydroxymethyl-benzoic acid, N-succinimidyl-3 or 4 [bis-(4-methoxyphenyl)]-chloromethyl-benzoic acid, and salts of these acids.
   In general, the probes are attached to the microarray in a wide variety of ways, as will be appreciated by those in the art. As described herein, the nucleic acids can either be synthesized first, with subsequent attachment to the microarray, or can be directly synthesized on the microarray.
   The microarray comprises a suitable solid substrate. By "substrate" or "solid support" or other grammatical equivalents herein is meant any material that can be modified to contain discrete individual sites appropriate for the attachment or association of the nucleic acid probes and is amenable to at least one detection method. The solid phase support used in the present invention can be of any solid material and structures suitable for supporting nucleotide hybridization and synthesis. Preferably, the solid phase support comprises at least one substantially rigid surface on which the primers can be immobilized and the reverse transcriptase reaction performed. The substrates with which the polynucleotide microarray elements are stably associated and may be fabricated from a variety of materials, including plastics, ceramics, metals, acrylamide, cellulose, nitrocellulose, glass, polystyrene, polyethylene vinyl acetate, polypropylene, polymethacrylate, polyethylene, polyethylene oxide, polysilicates, polycarbonates, Teflon, fluorocarbons, nylon, silicon rubber, polyanhydrides, polyglycolic acid, polylactic acid, polyorthoesters, polypropylfumerate, collagen, glycosaminoglycans, and polyamino acids. Substrates may be two-dimensional or three-dimensional in form, such as gels, membranes, thin films, glasses, plates, cylinders, beads, magnetic beads, optical fibers, woven fibers, etc. A preferred form of array is a three-dimensional array. A preferred three-dimensional array is a collection of tagged beads. Each tagged bead has different primers attached to it. Tags are detectable by signalling means such as color (Luminex, Illumina) and electromagnetic field (Pharmaseq) and signals on tagged beads can even be remotely detected (e.g., using optical fibers). The size of the solid support can be any of the standard microarray sizes, useful for DNA microarray technology, and the size may be tailored to fit the particular machine being used to conduct a reaction of the invention. In general, the substrates allow optical detection and do not appreciably fluoresce.
   In one embodiment, the surface of the microarray and the probe may be derivatized with chemical functional groups for subsequent attachment of the two. Thus, for example, the microarray is derivatized with a chemical functional group including, but not limited to, amino groups, carboxy groups, oxo groups and thiol groups, with amino groups being particularly preferred. Using these functional groups, the probes can be attached using functional groups on the probes. For example, nucleic acids containing amino groups can be attached to surfaces comprising amino groups, for example using linkers as are known in the art; for example, homo-or hetero-bifunctional linkers as are well known. In addition, in some cases, additional linkers, such as alkyl groups (including substituted and heteroalkyl groups) may be used.
   In this embodiment, the oligonucleotides are synthesized as is known in the art, and then attached to the surface of the solid support. As will be appreciated by those skilled in the art, either the 5' or 3' terminus may be attached to the solid support, or attachment may be via an internal nucleoside. In an additional embodiment, the immobilization to the solid support may be very strong, yet non-covalent. For example, biotinylated oligonucleotides can be made, which bind to surfaces covalently coated with streptavidin, resulting in attachment.
   The arrays may be produced according to any convenient methodology, such as preforming the polynucleotide microarray elements and then stably associating them with the surface. Alternatively, the oligonucleotides may be synthesized on the surface, as is known in the art. A number of different array configurations and methods for their production are known to those of skill in the art and disclosed in WO 95/25116 and WO 95/35505 (photolithographic techniques), U.S. Pat. No. 5,445,934 (in situ synthesis by photolithography), U.S. Pat. No. 5,384,261 (in situ synthesis by mechanically directed flow paths); and U.S. Pat. No. 5,700,637 (synthesis by spotting, printing or coupling). Another method for coupling DNA to beads uses specific ligands attached to the end of the DNA to link to ligand-binding molecules attached to a bead. Possible ligand-binding partner pairs include biotin-avidin/streptavidin, or various antibody/antigen pairs such as digoxygenin-antidigoxygenin antibody (Smith et al., Science 258:1122-1126 (1992)). Covalent chemical attachment of DNA to the support can be accomplished by using standard coupling agents to link the 5'-phosphate on the DNA to coated microspheres through a phosphoamidate bond. Methods for immobilization of oligonucleotides to solid-state substrates are well established. See Pease et al., Proc. Natl. Acad. Sci. USA 91(11):5022-5026 (1994). A preferred method of attaching oligonucleotides to solid-state substrates is described by Guo et al., Nucleic Acids Res. 22:5456-5465 (1994). Immobilization can be accomplished either by in situ DNA synthesis (Maskos and Southern, *supra*) or by covalent attachment of chemically synthesized oligonucleotides (Guo *et al., supra*) in combination with robotic arraying technologies.
   In addition to the solid-phase technology represented by microarray arrays, gene expression can also be quantified using liquid-phase assays. One such system is kinetic polymerase chain reaction (PCR). Kinetic PCR allows for the simultaneous amplification and quantification of specific nucleic acid sequences. The specificity is derived from synthetic oligonucleotide primers designed to preferentially adhere to single-stranded nucleic acid sequences bracketing the target site. This pair of oligonucleotide primers form specific, non-covalently bound complexes on each strand of the target sequence. These complexes facilitate in vitro transcription of doublestranded DNA in opposite orientations. Temperature cycling of the reaction mixture creates a continuous cycle of primer binding, transcription, and re-melting of the nucleic acid to individual strands. The result is an exponential increase of the target dsDNA product. This product can be quantified in real time either through the use of an intercalating dye or a sequence specific probe. SYBR(r) Green 1, is an example of an intercalating dye, that preferentially binds to dsDNA resulting in a concomitant increase in the fluorescent signal. Sequence specific probes, such as used with TaqMan technology, consist of a fluorochrome and a quenching molecule covalently bound to opposite ends of an oligonucleotide. The probe is designed to selectively bind the target DNA sequence between the two primers. When the DNA strands are synthesized during the PCR reaction, the fluorochrome is cleaved from the probe by the exonuclease activity of the polymerase resulting in signal dequenching. The probe signalling method can be more specific than the intercalating dye method, but in each case, signal strength is proportional to the dsDNA product produced. Each type of quantification method can be used in multi-well liquid phase arrays with each well representing primers and/or probes specific to nucleic acid sequences of interest. When used with messenger RNA preparations of tissues or cell lines, an array of probe/primer reactions can simultaneously quantify the expression of multiple gene products of interest. See Germer et al., Genome Res. 10:258-266 (2000); Heid et al., Genome Res. 6:986-994 (1996).
(iv) Measurement of altered neoplastic marker protein levels in cell extracts, for example by immunoassay.
   Testing for proteinaceous neoplastic marker expression product in a biological sample can be performed by any one of a number of suitable methods which are well known to those skilled in the art. Examples of suitable methods include, but are not limited to, antibody screening of tissue sections, biopsy specimens or bodily fluid samples.
   To the extent that antibody based methods of diagnosis are used, the presence of the marker protein may be determined in a number of ways such as by Western blotting, ELISA or flow cytometry procedures. These, of course, include both single-site and two-site or "sandwich" assays of the non-competitive types, as well as in the traditional competitive binding assays. These assays also include direct binding of a labelled antibody to a target.
   Sandwich assays are among the most useful and commonly used assays. A number of variations of the sandwich assay technique exist, and all are intended to be encompassed by the present invention. Briefly, in a typical forward assay, an unlabelled antibody is immobilized on a solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample. Variations on the forward assay include a simultaneous assay, in which both sample and labelled antibody are added simultaneously to the bound antibody. These techniques are well known to those skilled in the art, including any minor variations as will be readily apparent.
   In the typical forward sandwich assay, a first antibody having specificity for the marker or antigenic parts thereof, is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking, covalently binding or physically adsorbing, the polymer-antibody complex is washed in preparation for the test sample. An aliquot of the sample to be tested is then added to the solid phase complex and incubated for a period of time sufficient (e.g. 2-40 minutes) and under suitable conditions (e.g. 25°C) to allow binding of any subunit present in the antibody. Following the incubation period, the antibody subunit solid phase is washed and dried and incubated with a second antibody specific for a portion of the antigen. The second antibody is linked to a reporter molecule which is used to indicate the binding of the second antibody to the antigen.
   An alternative method involves immobilizing the target molecules in the biological sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.
   By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.
   In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample. "Reporter molecule" also extends to use of cell agglutination or inhibition of agglutination such as red blood cells on latex beads, and the like.
   Alternately, fluorescent compounds, such as fluorecein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic color visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength the fluorescence observed indicates the presence of the hapten of interest. Immunofluorescence and EIA techniques are both very well established in the art and are particularly preferred for the present method. However, other reporter molecules, such as radioisotope, chemiluminescent or bioluminescent molecules, may also be employed.
(v) Without limiting the present invention to any one theory or mode of action, during development gene expression is regulated by processes that alter the availability of genes for expression in different cell lineages without any alteration in gene sequence, and these states can be inherited through a cell division - a process called epigenetic inheritance. Epigenetic inheritance is determined by a combination of DNA methylation (modification of cytosine to give 5-methyl cytosine, 5meC) and by modifications of the histone chromosomal proteins that package DNA. Thus methylation of DNA at CpG sites and modifications such as deacetylation of histone H3 on lysine 9, and methylation on lysine 9 or 27 are associated with inactive chromatin, while the converse state of a lack of DNA methylation, acetylation of lysine 9 of histone H3 is associated with open chromatin and active gene expression. In cancer, this epigenetic regulation of gene expression is frequently found to be disrupted (Esteller & Herman, 2000; Jones & Baylin, 2002). Genes such as tumour suppressor or metastasis suppressor genes are often found to be silenced by DNA methylation, while other genes may be hypomethylated and inappropriately expressed. Thus, among genes that elevated or inappropriate expression in cancer, this in some instances is characterised by a loss of methylation of the promoter or regulatory region of the gene.
   A variety of methods are available for detection of aberrantly methylated DNA of a specific gene, even in the presence of a large excess of normal DNA (Clark 2007). Thus, elevated expression of certain genes may be detected through detection of the presence of hypomethylated sequences in tissue, bodily fluid or other patient samples.
   Epigenetic alterations and chromatin changes in cancer are also evident in the altered association of modified histones with specific genes(Esteller, 2007); for example activated genes are often found associated with histone H3 that is acetylated on lysine 9 and methylated on lysine 4. The use of antibodies targeted to altered histones allows for the isolation of DNA associated with particular chromatin states and has potential use in cancer diagnosis.
(vi) Determining altered expression of protein neoplastic markers on the cell surface, for example by immunohistochemistry.
(vii) Determining altered protein expression based on any suitable functional test, enzymatic test or immunological test in addition to those detailed in points (iv) and (v) above.

A person of ordinary skill in the art could determine, as a matter of routine procedure, the appropriateness of applying a given method to a particular type of biological sample.

Without limiting the present invention in any way, and as detailed above, gene expression levels can be measured by a variety of methods known in the art. For example, gene transcription or translation products can be measured. Gene transcription products, i.e., RNA, can be measured, for example, by hybridization assays, run-off assays., Northern blots, or other methods known in the art.

Hybridization assays generally involve the use of oligonucleotide probes that hybridize to the single-stranded RNA transcription products. Thus, the oligonucleotide probes are complementary to the transcribed RNA expression product. Typically, a sequence-specific probe can be directed to hybridize to RNA or cDNA. A "nucleic acid probe", as used herein, can be a DNA probe or an RNA probe that hybridizes to a complementary sequence. One of skill in the art would know how to design such a probe such that sequence specific hybridization will occur. One of skill in the art will further know how to quantify the amount of sequence specific hybridization as a measure of the amount of gene expression for the gene was transcribed to produce the specific RNA.

The hybridization sample is maintained under conditions that are sufficient to allow specific hybridization of the nucleic acid probe to a specific gene expression product. "Specific hybridization", as used herein, indicates near exact hybridization (e.g., with few if any mismatches). Specific hybridization can be performed under high stringency conditions or moderate stringency conditions. In one embodiment, the hybridization conditions for specific hybridization are high stringency. For example, certain high stringency conditions can be used to distinguish perfectly complementary nucleic acids from those of less complementarity. "High stringency conditions", "moderate stringency conditions" and "low stringency conditions" for nucleic acid hybridizations are explained on pages 2.10.1-2.10.16 and pages 6.3.1-6.3.6 in Current Protocols in Molecular Biology (Ausubel, F. et al., "Current Protocols in Molecular Biology", John Wiley & Sons, (1998). The exact conditions that determine the stringency of hybridization depend not only on ionic strength (e.g., 0.2.times.SSC, 0.1.times.SSC), temperature (e.g., room temperature, 42°C., 68°C.) and the concentration of destabilizing agents such as formamide or denaturing agents such as SDS, but also on factors such as the length of the nucleic acid sequence, base composition, percent mismatch between hybridizing sequences and the frequency of occurrence of subsets of that sequence within other non-identical sequences. Thus, equivalent conditions can be determined by varying one or more of these parameters while maintaining a similar degree of identity or similarity between the two nucleic acid molecules. Typically, conditions are used such that sequences at least about 60%, at least about 70%, at least about 80%, at least about 90% or at least about 95% or more identical to each other remain hybridized to one another. By varying hybridization conditions from a level of stringency at which no hybridization occurs to a level at which hybridization is first observed, conditions that will allow a given sequence to hybridize (e.g., selectively) with the most complementary sequences in the sample can be determined.

Exemplary conditions that describe the determination of wash conditions for moderate or low stringency conditions are described in Kraus, M. and Aaronson, S., 1991. Methods Enzymol., 200:546-556; and in, Ausubel et al., Current Protocols in Molecular Biology, John Wiley & Sons, (1998). Washing is the step in which conditions are usually set so as to determine a minimum level of complementarity of the hybrids. Generally, starting from the lowest temperature at which only homologous hybridization occurs, each °C. by which the final wash temperature is reduced (holding SSC concentration constant) allows an increase by 1% in the maximum mismatch percentage among the sequences that hybridize. Generally, doubling the concentration of SSC results in an increase in Tₘ of about 17°C. Using these guidelines, the wash temperature can be determined empirically for high, moderate or low stringency, depending on the level of mismatch sought. For example, a low stringency wash can comprise washing in a solution containing 0.2.times.SSC/0.1% SDS for 10 minutes at room temperature; a moderate stringency wash can comprise washing in a pre-warmed solution (42°C) solution containing 0.2.times.SSC/0.1% SDS for 15 minutes at 42°C.; and a high stringency wash can comprise washing in pre-warmed (68°C.) solution containing 0.1.times.SSC/0.1% SDS for 15 minutes at 68°C. Furthermore, washes can be performed repeatedly or sequentially to obtain a desired result as known in the art. Equivalent conditions can be determined by varying one or more of the parameters given as an example, as known in the art, while maintaining a similar degree of complementarity between the target nucleic acid molecule and the primer or probe used (e.g., the sequence to be hybridized).

An aspect related to the present invention provides a molecular array, which array comprises a plurality of:
(i) nucleic acid molecules comprising a nucleotide sequence corresponding to any one or more of the neoplastic marker genes hereinbefore described or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (iv) is indicative of the neoplastic state of a cell or cellular subpopulation derived from the large intestine.

Preferably, said percent identity is at least 85%, 90%, 91%, 92%, 93%, 94%, 95%, 96%, 97%, 98% or 99%.

Low stringency includes and encompasses from at least about 1% v/v to at least about 15% v/v formamide and from at least about 1M to at least about 2M salt for hybridisation, and at least about 1M to at least about 2M salt for washing conditions. Alternative stringency conditions may be applied where necessary, such as medium stringency, which includes and encompasses from at least about 16% v/v at least about 30% v/v formamide and from at least about 0.5M to at least about 0.9M salt for hybridisation, and at least about 0.5M to at least about 0.9M salt for washing conditions, or high stringency, which includes and encompasses from at least about 31% v/v to at least about 50% v/v formamide and from at least about 0.01M to at least about 0.15M salt for hybridisation, and at least about 0.01M to at least about 0.15M salt for washing conditions. In general, washing is carried out at Tₘ = 69.3 + 0.41 (G + C) % [19] = -12°C. However, the Tₘ of a duplex DNA decreases by 1°C with every increase of 1% in the number of mismatched based pairs (Bonner et al (1973) J. Mol. Biol. 81:123).

Preferably, the subject probes are designed to bind to the nucleic acid or protein to which they are directed with a level of specificity which minimises the incidence of non-specific reactivity. However, it would be appreciated that it may not be possible to eliminate all potential cross-reactivity or non-specific reactivity, this being an inherent limitation of any probe based system.

In terms of the probes which are used to detect the subject proteins, they may take any suitable form including antibodies and aptamers.

A library or array of nucleic acid or protein probes provides rich and highly valuable information. Further, two or more arrays or profiles (information obtained from use of an array) of such sequences are useful tools for comparing a test set of results with a reference, such as another sample or stored calibrator. In using an array, individual probes typically are immobilized at separate locations and allowed to react for binding reactions. Primers associated with assembled sets of markers are useful for either preparing libraries of sequences or directly detecting markers from other biological samples.

A library (or array, when referring to physically separated nucleic acids corresponding to at least some sequences in a library) of gene markers exhibits highly desirable properties. These properties are associated with specific conditions, and may be characterized as regulatory profiles. A profile, as termed here refers to a set of members that provides diagnostic information of the tissue from which the markers were originally derived. A profile in many instances comprises a series of spots on an array made from deposited sequences.

A characteristic patient profile is generally prepared by use of an array. An array profile may be compared with one or more other array profiles or other reference profiles. The comparative results can provide rich information pertaining to disease states, developmental state, receptiveness to therapy and other information about the patient.

Another aspect of the present invention relates to a diagnostic kit for assaying biological samples comprising an agent for detecting one or more neoplastic markers and reagents useful for facilitating the detection by said agent. Further means may also be included, for example, to receive a biological sample. The agent may be any suitable detecting molecule,

The present invention is further described by the following non-limiting examples:

### EXAMPLE 1

### Methods and Materials

### Affymetrix GeneChip data

Gene expression profiling data and accompanying clinical data was purchased from GeneLogic Inc (Gaithersburg, MD USA). For each tissue analyzed, oligonucleotide microarray data for 44,928 probesets (Affymetrix HGU133A & HGU133B, combined), experimental and clinical descriptors, and digitally archived microscopy images of histological preparations were received, A quality control analysis was performed to remove arrays not meeting essential quality control measures as defined by the manufacturer.

Transcript expression levels were calculated by both Microarray Suite (MAS) 5.0 (Affymetrix) and the Robust Multichip Average (RMA) normalization techniques (Affymetrix. GeneChip expression data analysis fundamentals. Affymetrix, Santa Clara, CA USA, 2001; Hubbell et al. Bioinformatics, 18:1585-1592, 2002; Irizarry et al. Nucleic Acid Research, 31, 2003)MAS normalized data was used for performing standard quality control routines and the final data set was normalized with RMA for all subsequent analyses.

### Univariate differential expression

Differentially expressed gene transcripts were identified using a moderated t-test implemented in the limma library downloaded from the Bioconductor repository for R. (G. K. Smyth. Statistical Applications in Genetics and Molecular Biology, 3(1):Article 3, 2004; G K Smyth. Bioinformatics and Computational Biology Solutions using R and Bioconductor. Springer, New York, 2005). Significance estimates (p-values) were corrected to adjust for multiple hypothesis testing using the Bonferonni correction.

### Tissue specific expression patterns

To construct a filter for hypothetically 'turned on' gene expression the mean expression level for all 44,928 probesets across the full range of 454 tissues was first estimated. To estimate an expression on/off threshold, the 44,928 mean values were ranked and the expression value equivalent to the 30th percentile across the dataset calculated. This arbitrary threshold was chosen because it was theorized that the majority of transcripts (and presumably more than 30%) in a given specimen should be transcriptionally silenced. Thus this threshold represents a conservative upper bound for what is estimated as non-specific, or background, signal.

### Gene symbol annotations

To map Affymetrix probeset names to official gene symbols the annotation metadata available from Bioconductor was used. hgu133plus2 library version 1.16.0, which was assembled using Entrez Gene data downloaded on 15 March 2007, was used.

### Estimates of performance characteristics

Diagnostic utility for each table of markers shown herein was estimated including: sensitivity, specificity, positive predictive value, negative predictive value, likelihood ratio positive, likelihood ratio negative. These estimates were calculated in the same data used to discover the markers and will therefore potentially overestimate the performance characteristics in future tissue samples. To improve the generalisabilty of the estimates a modified jackknife resampling technique was used to calculate a less biased value for each characteristic.

### Results

A range of univariate statistical tests were applied on Affymetrix oligonucleotide microarray data to reveal human genes that could be used to discriminate colorectal neoplastic tissues from non-neoplastic tissues. There were further identified a number of gene transcripts that appear to be useful for differentiating colorectal adenomas from colorectal carcinoma. Also identified were a subset of these transcripts that may have particular diagnostic utility because due to the protein products being either secreted or displayed on the cell surface of epithelial cells. Finally, there were identified a further subset of transcripts expressed specifically in neoplastic tissues and at low- or near-background levels in non-neoplastic tissues.

### Genes differentially expressed in neoplastic tissues

From a total GeneChip set of 44,928 probesets it was determined that over 11,000 probesets were differentially expressed by moderated t-test using the limma package in BioConductor (G. K. Smyth, 2004 *supra*) employing conservative (Bonferroni) multiple test correction. When this list was further filtered to include only those probesets demonstrating a 2-fold or greater mean expression change between the neoplastic and non-neoplastic tissues, 206 probesets were found to be expressed *higher* in neoplasias relative to normals.

These 205 probesets were annotated using the most recent metadata and annotation packages available for the chips. The 205 overexpressed probesets were mapped to 174 gene symbols,

| Δ-expression | ProbeSet ID | Gene Symbol Maps |
|---|---|---|
| UP | 205 | 157 |

### Hypothetical markers specific for colorectal neoplasia

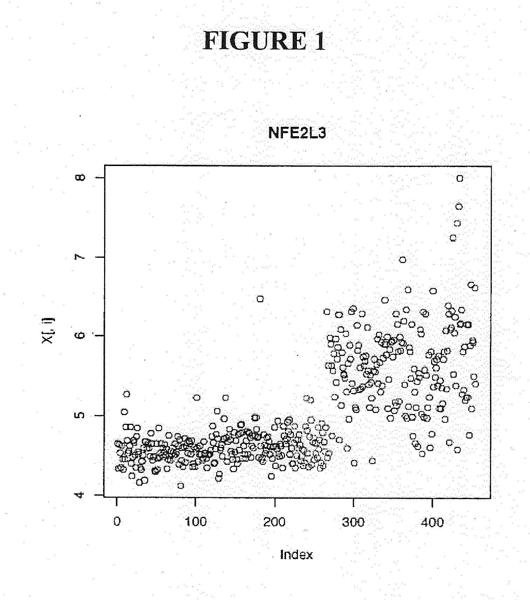

While differential gene expression patterns are useful for diagnostic purposes, this project also seeks to identify diagnostic proteins shed into the lumen of the gut by neoplastic colorectal epithelia. To discover candidate proteins the list of differentially expressed transcripts were filtered with a selection criteria aimed at identifying markers specifically expressed in colorectal neoplasia tissues. This filter criteria is based on a theoretical assumption that most genes on the GeneChip will be turned 'off' and that any microarray signals for such 'off' transcripts will reflect technical assay background and non-specific oligonucleotide binding. Accordingly, to select genes specifically expressed in neoplastic tumours (i.e. 'on') the non-neoplastic signals were compared with a hypothetical background signal threshold from across all genes on the chip. By design, all transcripts in the candidate pool from which the 'on' transcripts are chosen are at least two fold overexpressed in the diseased tissues. Combined, it is hypothesized that these criteria yield the subset of differentially expressed genes that are specifically expressed in neoplasia. The expression profile for a representative 'on' transcript is shown in Figure 1.

### Genes differentially expressed between adenomas and cancer tissues

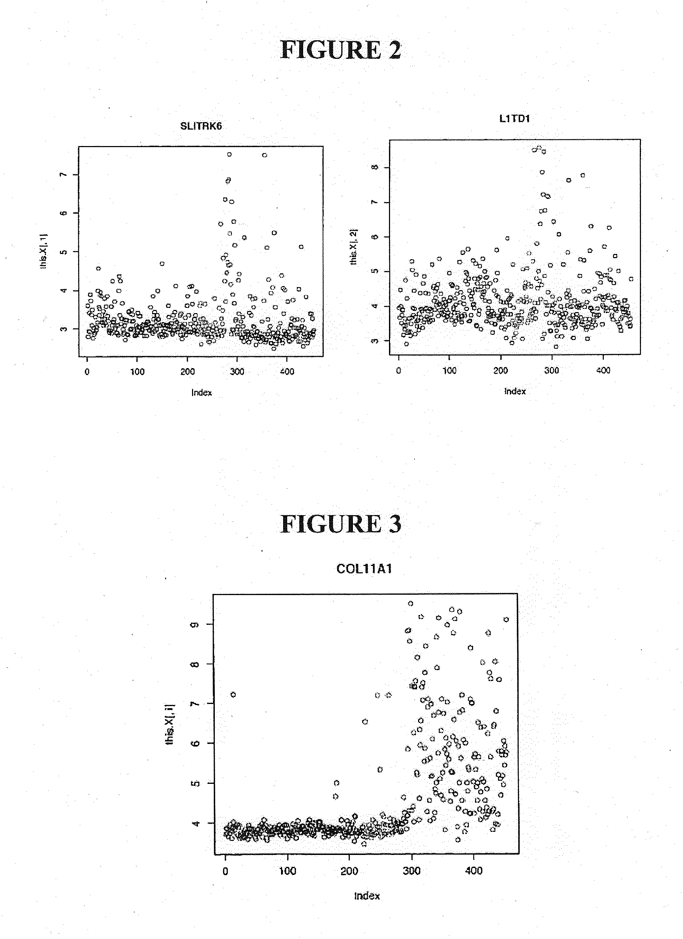

There were 33 transcripts observed that were differentially expressed at least two-fold higher in adenoma tissues relative to cancer tissues. In particular, there were identified several transcripts that exhibit an expression pattern specific for adenomas, including SLITRK6 and L1TD1, shown in Figure 2.

Further, there were also identified cancer specific transcripts. The expression profile of one such transcript, COL11A1 is shown in Figure 3.

### EXAMPLE 2

### Probesets Elevated In Neoplasia

Differential expression analysis was applied to Affymetrix gene chip data measuring RNA concentration in 454 colorectal tissues including 161 adenocarcinoma specimens, 29 adenoma specimens, 42 colitis specimens and 222 non-diseased tissues. Using conservative corrections for multiple hypothesis testing, it was determined that over 25% of the 44,928 probesets measured in each tissue experiment were differentially expressed between the 190 neoplasia specimens and 264 non-neoplasia controls. To identify robust biomarkers for colorectal neoplasia the list of putative probeset biomarkers were further filtered to include only those probesets shown to be expressed at least 2-fold higher in neoplastic vs. non-neoplastic tissues.

205 probesets hybridising to approximately 157 putative genes were observed to be expressed at a statistically significant higher level in neoplastic tissues relative to non-neoplastic controls.

### Validation/Hypothesis testing

To validate these discovery results the hybridisation of 199 candidate probesets were measured against RNA extracts from 68 clinical specimens comprising 19 adenomas, 19 adenocarcinomas, and 30 non-diseased controls using a custom-designed 'Adenoma Gene Chip'. Six (6) probesets were not tested as they were not included on the custom design. It was confirmed that 186/199 (88%) of the target probesets or probesets which also hybridise to the target locus were likewise differentially expressed (P < 0.05) in these independently-derived tissues. The results of testing these probesets in 68 independently collected clinical specimens is shown in Table 1.

We further tested the 142 of the 157 unique gene loci to which the 205 probesets are understood to hybridise. We note the remaining 15 gene symbols were not represented in the validation data. We observed that 133 of 142 gene symbols were represented in the validation data by at least one differentially expressed probeset and many symbols included multiple probesets against regions across the putative locus. A complete list of probesets that bind to target loci is shown in Table 2.

### Conclusion

The candidate probesets shown in Tables 1 and 2 are differentially expressed in neoplastic colorectal tissues compared to non-neoplastic controls.

### EXAMPLE 3

### Probesets Demonstrating A Neoplasia-Specific Profile

During analysis of the data, a novel expression profile was observed between neoplastic and non-neoplastic phenotypes. It was hypothesized that a subset of quantitatively differentially expressed probesets are furthermore qualitatively differentially expressed. Such probesets show evidence of a neoplasia-specific gene expression profile, i.e. these probesets appear to be expressed above background levels in neoplastic tissues only. This observation and the resulting hypothesis are based on two principles:
1. That the majority of human transcripts that are present on a genome-wide GeneChip (e.g. U133) will not likely be expressed in the colorectal mucosa; and
2. That microarray binding intensity for such 'off' probesets to labelled cRNA will reflect technical assay background, i.e. non-specific oligonucleotide binding.

To generate a list of neoplasia specific probesets the non-neoplastic intensity of differentially expressed probesets was compared with a hypothetical background signal threshold from across all probesets on the chip. Bydesign, all probesets in the candidate pool from which the 'on' transcripts are chosen are at least two fold over-expressed in the diseased tissues. Combined, these criteria yield the subset of differentially expressed transcript species that are specifically expressed in neoplasia.

### Validation/Hypothesis Testing

The custom gene chip design precludes testing the hypothetically neoplasia-specific probesets using the same principles as used for discovery. In particular, the custom gene chip (by design) does not contain a large pool of probesets anticipated to hybridise to hypothetically 'off'/'non-transcribed' gene transcripts. This is because the custom gene chip design is biased toward differentially expressed transcripts in colorectal neoplastic tissues.

The usual differential expression testing (limma) was therefore to these candidate probesets for neoplasia-specific transcripts. Of the 33 probesets on the custom gene chip, 32 probesets (or probesets which bind to the same locus) were differentially expressed between the 38 neoplastic tissues (adenoma & cancer) and non-neoplastic controls. The results of these validation experiments is shown in Table 3.

All probesets which are known to hybridise to the gene loci to which the 33 probesets claimed herein were tested. Of the 32 putative gene loci targeted by the probesets, 29 were present in the validation data. Twenty-eight (28) of these 29 gene symbols demonstrated at least one hybridising probeset which was differentially expressed in the neoplastic tissues. Results for these experiments, including all probesets that bind to each target locus in a differentially expressed manner are shown in Table 4.

### EXAMPLE 4

### Probesets Useful For Characterizing Neoplastic Tissues

Differential expression analysis was applied to Affymetrix gene chip data measuring RNA concentration in neoplastic tissues including 161 adenocarcinoma specimens and 29 adenoma specimens. It was observed that 43 probesets hybridizing to approximately 33 putative gene symbols were expressed higher (P < 0.05) in adenoma tissues relative to cancer tissues. Conversely, 145 probesets (104 gene symbols) were identified to be expressed higher in cancer relative to adenomas.

### Validation/Hypothesis testing

188 (43 + 145) of these probesets were then measured in a set of independent clinical specimens including 19 adenoma tissues and 19 cancer tissues. It was confirmed that 158 (30 + 128) of the target probesets (or probesets against the same gene locus) were likewise differentially expressed (P < 0.05) in these independently-derived tissues. Probesets elevated in adenoma and cancers relative to each other are shown in Table 5 and Table 6 respectively.

It was further observed that 137 (33+104) gene loci are diagnostically useful for discriminating colorectal adenomas and cancers relative to each phenotype. The validation data included probesets designed to hybridise to 128 of these candidate gene symbols. It was observed that 21 of the 31 genes elevated in adenomas relative to cancers were likewise differentially expressed by at least one probeset. Of the 97 gene symbols elevated in cancer relative to adenoma it was confirmed that 89 gene symbols demonstrated at least one probeset in the validation data to be likewise differentially expressed. The validation testing of the adenoma and cancer elevated gene loci is shown in Table 7 and Table 8, respectively.

### Conclusion

It was concluded that the candidate probesets shown in FIXME are differentially expressed between adenomatous and adenocarcinoma tissues and thus useful for distinguishing these tissues. Gene transcripts that hybridise to these probesets are thus diagnostically informative in a clinical setting to classify such neoplastic tissues.

### EXAMPLE 5

### Materials And Methods For Examples 2 To 3

Gene expression profiling data measured in 454 colorectal tissue specimens including neoplastic, normal and non-neoplastic disease controls was purchased from GeneLogic Inc (Gaithersburg, MD USA). For each tissue specimen an Affymetrix (Santa Clara, CA USA) oligonucleotide microarray data totalling 44,928 probesets (HGU133A & HGU133B, combined), experimental and clinical descriptors, and digitally archived microscopy images of histological preparations was received. Prior to applying discovery methods to these data extensive quality control methods were carried out, including statistical exploration, review of clinical records for consistency and histopathology audit of a random sample of arrays. Microarrays that did not meet acceptable quality criteria were removed from the analysis.

### Hypothesis testing

Candidate transcription biomarkers were tested using a custom oligonucleotide microarray of 25-mer oligonucleotide probesets designed to hybridise to candidate RNA transcripts identified during discovery. Differential expression hypotheses were tested using RNA extracts derived from independently collected clinical samples comprising 30 normal colorectal tissues, 19 colorectal adenoma tissues, and 19 colorectal adenocarcinoma tissues. Each RNA extract was confirmed to meet strict quality control criteria.

### Colorectal tissue specimens

All tissues used for hypothesis testing were obtained from a tertiary referral hospital tissue bank in metropolitan Adelaide, Australia (Repatriation General Hospital and Flinders Medical Centre). Access to the tissue bank for this research was approved by the Research and Ethics Committee of the Repatriation General Hospital and the Ethics Committee of Flinders Medical Centre. Informed patient consent was received for each tissue studied.

Following surgical resection, specimens were placed in a sterile receptacle and collected from theatre. The time from operative resection to collection from theatre was variable but not more than 30 minutes. Samples, approximately 125mm3 (5x5x5mm) in size, were taken from the macroscopically normal tissue as far from pathology as possible, defined both by colonic region as well as by distance either proximal or distal to the pathology. Tissues were placed in cryovials, then immediately immersed in liquid nitrogen and stored at -150C until processing.

### RNA extraction

RNA extractions were performed using Trizol(R)reagent (Invitrogen, Carlsbad, CA, USA) as per manufacturer's instructions. Each sample was homogenised in 300µL of Trizol reagent using a modified Dremel drill and sterilised disposable pestles. An additional 200µL of Trizol reagent was added to the homogenate and samples were incubated at RT for 10 minutes. 100µL of chloroform was then added, samples were shaken vortexed for 15 seconds, and incubated at RT for 3 further minutes. The aqueous phase containing target RNA was obtained by centrifugation at 12,000 rpm for 15 min, 40C. RNA was then precipitated by incubating samples at RT for 10 min with 250µL of isopropanol. Purified RNA precipitate was collected by centrifugation at 12,000 rpm for 10 minutes, 40C and supernatants were discarded. Pellets were then washed with 1mL 75% ethanol, followed by vortexing and centrifugation at 7,500g for 8 min, 40C. Finally, pellets were air-dried for 5 min and resuspended in 80µL of RNase free water. To improve subsequent solubility samples were incubated at 55°C for 10 min. RNA was quantified by measuring the optical density at A260/280 nm. RNA quality was assessed by electrophoresis on a 1.2% agarose formaldehyde gel.

### Gene Chip processing

To test hypotheses related to biomarker candidates for colorectal neoplasia RNA extracts were assayed using a custom GeneChip designed in collaboration with Affymetrix (Santa Clara, CA USA). These custom GeneChips were processed using the standard Affymetrix protocol developed for the HU Gene ST 1.0 array described in (Affy:WTAssay).

### Statistical software and data processing

The R statistics environment R and BioConductor libraries (BioConductor, www.bioconductor.org) (BIOC) were used for most analyses. To map probeset IDs to gene symbol on the Custom GeneChip, hgu133plus2 library version 2.2.0, which was assembled using Entrez Gene data downloaded on Apr 18 12:30:55 2008 (BIOC) was used.

### Hypothesis testing of differentially expressed biomarkers

To assess differential expression between tissue classes, the Student's t test for equal means between two samples or the robust variant provided by the limma library (Smyth)(limma) was used. The impact of false discovery due to multiple hypothesis testing was mitigated by applying a Bonferroni adjustment to P values in the discovery process (MHT:Bonf). For hypotheses testing the slightly less conservative multiple hypothesis testing correction of Benjamini & Hochberg, which aims to control the false discovery rate of solutions(MHT:BH) was applied.

### Discovery of tissue-specific gene expression patterns

Discovery methods using gene expression data often yield numerous candidates, many of which are not suitable for commercial products because they involve subtle gene expression differences that would be difficult to detect in laboratory practice. Pepe *et al.* note that the 'ideal' biomarker is detectable in tumor tissue but not detectable (at all) in non-tumour tissue (Pepe:biomarker:development.) To bias toward candidates that meet this criterion, an analysis method was developed that aims to enrich the candidates for biomarkers whose qualitative absence or presence measurement is diagnostic for the phenotype of interest. This method attempts to select candidates that show a prototypical 'turned-on' or 'turned-off' pattern relative to an estimate of the background/noise expression across the chip. Such RNA transcripts are more likely to correlate with downstream translated proteins with diagnostic potential or to predict upstream genomic changes (e.g. methylation status) that can be used diagnostically. This focus on qualitative rather than quantitative outcomes may simplify the product development process for such biomarkers.

The method is based on the assumption that the pool of extracted RNA species in any given tissue (e.g. colorectal mucosae) will specifically bind to a relatively small subset of the full set of probesets on a GeneChip designed to measure the whole genome. On this assumption, it is estimated that most probesets on a full human gene chip will not exhibit specific, high-intensity signals.

This observation is utilised to approximate the background or 'non-specific binding' across the chip by choosing a theoretical level equal to the value of e.g. lowest 30% quantile of the ranked mean values. This quantile can be arbitrarily set to some level below which a reasonable assumption is made that the signals do not represent above-background RNA binding. Finally, this background estimate is used as a threshold to estimate the 'OFF' probesets in an experiment for, say, the non-neoplastic tissue specimens.

Conversely, probesets which are 1) expressed above this theoretical threshold level and 2) at differentially higher levels in the tumour specimens may be a tumour specific candidate biomarker. In this case the concept of 'fold-change' thresholds can also be conveniently applied to further emphasize the concept of absolute expression increases in a putatively 'ON' probeset.

Given the assumption of low background binding for a sizeable fraction of the measured probesets, this method was only used in the large GeneLogic data and discovery. To construct a filter for hypothetically 'turned on' biomarkers in the GeneLogic discovery data, the mean expression level for all 44,928 probesets was first estimated across the full range of 454 tissues. The 44,928 mean values were then ranked and the expression value equivalent to the 25th percentile across the dataset calculated. This arbitrary threshold was chosen because it was theorized that the majority of transcripts (and presumably more than 25%) in a given specimen should exhibit low concentration which effectively transcriptional silence. Thus this threshold represents a conservative upper bound for what is estimated as non-specific, or background, expression.

### EXAMPLE 6

### Determine Gene Identity of a Nucleic Acid Sequence of Interest which is Define by an Affymetrix Probeset

*BLAST the sequence of interest using online available Basic Local Alignment Search Tools [BLAST]. e.g. NCBI*/*BLAST*
(http://blast.ncbi.nlm.nih.gov/Blast.cgi)
(a) Select "Human" in BLAST ASSEMBLED GENOMES on the web page http://blast.ncbi.nlm.nih.gov/Blast.cgi
(b) Leave the default settings, i.e.:
   - Database: Genome (all assemblies)
   - Program: megaBLAST: compare highly related nucleotide sequences
   - Optional parameters: Expect: 0.01, Filter: default, Descriptions: 100, Alignments: 100
(c) Copy/Paste Sequence into the "BLAST" window
(d) Click "Begin Search"
(e) Click "View Report"

### Assessment of the Open BLAST Search Results

Multiple significant sequence alignments may be identified when "blasting" the sequence.

### Identify gene nomenclature of the identified sequence match

(a) Click the link to one of the identified hits
(b) The new page will schematically depict the position of the hit on one chromosome. It will be apparent which gene is hit.
(c) Retrieve the "hit" sequence clicking on the link
(d) Do a search for the gene in the provided "search" window. This provides the gene nucleotide coordinates for the gene.

### Determine promiscuity of Sequence

(a) Open the NCBI/BLAST tool, (http://blast.ncbi.nlm.nih.gov/Blast.cgi)
(b) Click on "nucleotide Blast" under "basic BLAST"
(c) Copy/paste the sequence of interest into the "Query Sequence" window
(d) Click "Blast".

### Assessment of the nBLAST Search Results of the Sequence

(a) The nBLAST exercise with the Sequence may result in multiple Blast hits of which some accession entry numbers are listed in "Description".
(b) These hits should be reviewed.

### Determine location of the Sequence in the Gene

The Ensembl database is an online database, which produces and maintains automatic annotation selected eukaryotic genomes (www.ensembl.org/index.html)

### Identify location of the Sequence in the Gene

(a) Set "Search" to Homo Sapiens, Type "the gene name" in the provided Search Field Ensemble.org/index.html)
(b) Click "Go"
(c) Click the "vega protein_coding Gene: OTTHUMG000000144184" link to get an annotation report
(d) Click on "Gene DAS Report" to retrieve information regarding Alternative splice site database: Type "the gene name" in search field
   - Click on "the gene entry"
   - Scroll down to "evidence"
   - Review alternative splice sites
   - Click "Confirmed intron/exons" to get a list of coordinates for the exons & introns.

### Alternative splicing and/or transcription

The AceView Database provides curated and non-redundant sequence representation of all public mRNA sequences. The database is available through NCBI: http://www.ncbi.nlm.nih.gov/IEB/Research/Acembly/

### Further investigation of the Gene mRNA transcripts

(a) Type "the gene name" into the provided "search" field
(b) Click "Go"
(c) The following information is available from the resulting entry in AceView:
   - The number of cDNA clones from which the gene is constructed (ie originated-from experimental work involving isolation of mRNA)
   - The mRNAs predicted to be produced by the gene
   - The existence of non-overlapping alternative exons and validated alternative polyadenylation sites
   - The existence of truncations
   - The possibility of regulated alternate expression
   - Introns recorded as participating in alternatively splicing of the gene
(d) Classic splice site motives

### Application of Method to LOC643911/hCG_1815491

### Materials and Methods

### Extraction of RNA

RNA extractions were performed using Trizol(R) reagent (Invitrogen, Carlsbad, CA, USA) as per manufacturer's instructions. Each sample was homogenised in 300µL of Trizol reagent using a modified dremel drill and sterilised disposable pestles. Additional 200µL of Trizol reagent was added to the homogenate and samples were incubated at RT for 10 minutes. 100µL of chloroform was then added, samples were shaken vortexed for 15 seconds, and incubated at RT for 3 further minutes. The aqueous phase containg target RNA was obtained by centrifugation at 12,000 rpm for 15 min, 40° C. RNA was then precipitated by incubating samples at RT for 10 min with 250µL of isopropanol. Purified RNA precipitate was collected by centrifugation at 12,000 rpm for 10 minutes, 40° C and supernatants were discarded. Pellets were then washed with 1mL 75% ethanol, followed by vortexing and centrifugation at 7,500g for 8 min, 40° C . Finally, pellets were air-dried for 5 min and resuspended in 80µL of RNase free water, To improve subsequent solubility samples were incubated at 55° C for 10 min. RNA was quantified by measuring the optical density at A260/280 nm. RNA quality was assessed by electrophoresis on a 1.2% agarose formaldehyde gel.

### Gene Chip processing

RNA samples to analyze on Human Exon 1.0 ST GeneChips were processed using the Affymetrix WT target labeling and control kit (part# 900652) following the protocol described in (Affymetrix 2007 P/N 701880 Rev.4). Briefly: First cycle cDNA was synthesized from 100ng ribosomal reduced RNA using random hexamer primers tagged with T7 promoter sequence and SuperScript II (Invitrogen, Carlsbad CA), this was followed by DNA Polymerase I synthesis of the second strand cDNA. Anti-sense cRNA was then synthesized using T7 polymerase. Second cycle sense cDNA was then synthesised using SuperScript II, dNTP+ dUTP, and random hexamers to produce sense strand cDNA incorporating uracil. This single stranded uracil containing cDNA was then fragmented using a combination of uracil DNA glycosylase (UDG) and apurinic/ apyrimidinic endonuclease1 (APE 1). Finally the DNA was biotin labelled using terminal deoxynucleotidyl transferase (TdT) and the Affymetrix proprietary DNA Labeling reagent. Hybridization to the arrays was carried out at 45°C for 16-18hours.

Washing and staining of the hybridized GeneChips was carried out using the Affymetrix Fluidics Station 450 and scanned with the Affymetrix Scanner 3000 following recommended protocols.

### SYBR green based Quantitative Real Time-PCR

Quantitative real time polymerase chain reaction was performed on RNA isolated from clinical samples for the amplification and detection of the various hCG_1815491 transcripts.

Firstly cDNA was synthesized from 2ug of total RNA using the Applied Biosystems High Capacity Reverse transcription Kit (P/N 4368814). After synthesis the reaction was diluted 1:2 with water to obtain a final volume of 40ul and 1ul of this diluted cDNA used in subsequent PCR reactions.

PCR was performed in a 25ul volume using 12.5ul Promega 2x PCR master mix (P/N M7502), 1.5ul 5uM forward primer, 1.5ul 5uM reverse primer, 7.875ul water, 0.625ul of a 1:3000 dilution of 10,000x stock of SYBR green 1 pure dye (Invitrogen P/N S7567), and lul of cDNA.

Cycling conditions for amplification were 95° for 2minutes xl cycle, 95° for 15 seconds and 60° for 1 minute x40 cycles. The amplification reactions were performed in a Corbett Research Rotor-Gene RG3000 or a Roche LightCycler480 real-time PCR machine. When the Roche LightCycler480 real-time PCR machine was used for amplification the reaction volume was reduced to 10ul and performed in a 384 well plate but the relative ratios between all the components remained the same. Final results were calculated using the ΔΔCt method with the expression levels of the various hCG_1815491 transcripts being calculated relative to the expression level of the endogenous house keeping gene HPRT.

### End-point PCR

End point PCR was performed on RNA isolated from clinical samples for the various hCG_1815491 transcripts. Conditions were identical to those described for the SYBR green assay above but with the SYBR green dye being replaced with water. The amplification reactions were performed in a MJ Research PTC-200 thermal cycler. 2.5µl of the amplified products were analysed on 2% agarose E-gel (Invitrogen) along with a 100-base pair DNA Ladder Marker.

### Results

The nucleotide structure and expression levels of transcripts related to hCG_1815491 was analysed based on the identification of diagnostic utility of Affymetrix probesets 238021_s_at and 238022_at from the gene chip analysis,

The gene hCG_1815491 is currently represented in NCBI as a single RefSeq sequence, XM_93911. The RefSeq sequence of hCG_1815491 is based on 89 GenBank accessions from 83 cDNA clones, Prior to March 2006, these clones were predicted to represent two overlapping genes, LOC388279 and LOC650242 (the latter also known as LOC643911). In March 2006, the human genome database was filtered against clone rearrangements, co-aligned with the genome and clustered in a minimal non-redundant way. As a result, LOC388272 and LOC650242 were merged into one gene named hCG_1815491 (earlier references to hCG_1815491 are: LOC388279, LOC643911, LOC650242, XM_944116, AF275804, XM_373688).

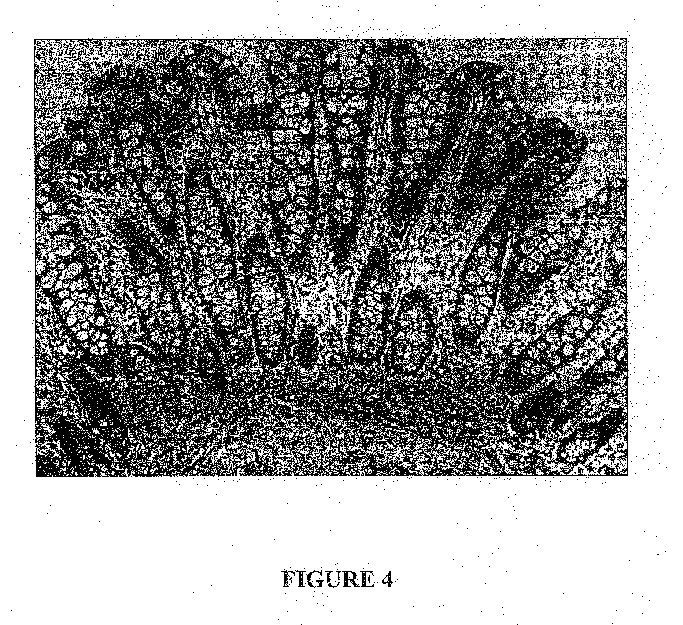

It has been determined that the Ref Sequence, which is defined by the genomic coordinates 8579310 to 8562303 on human chromosome 16 as defined by the NCBI contig reference NT_010498.15 | Hs16_10655, NCBI 36 March 2006 genome encompasses hCG_1815491. The 10 predicted RNA variants derived from this gene have been aligned with the genomic nucleotide sequence residing in the map region 8579310 to 8562303. This alignment analysis revealed the existence of at least 6 exons of which several are alternatively spliced. The identified exons are in contrast to the just 4 exons specified in the NCBI hCG_1815491 RefSeq XM_93911. Two additional putative exons were also identified in the Ref Sequence by examination of included probesets on Affymetrix Genechip HuGene Exon 1.0 that target nucleotide sequences embedded in the Ref Sequence. The identified and expanded exon-intron structure of hCG_1815491 have been used to design specific oligonucleotide primers, which allowed measurement of the expression of RNA variants generated from the Ref Sequence by using PCR-based methodology (Figure 4)

### EXAMPLE 7

Immunohistochemistry is a useful method for evaluating changes in local expression of up or down-regulated markers in human tissue.

### Materials and Methods:

Four micrometre sections were incubated in a universal decloaking buffer for 75 minutes at 80µL to expose masked epitopes. Protein expression was determined using an antibody targeting the C-terminal domain of Mesothelin (MSLN) on colonic biopsies from 30 patients (10 normals, 10 cancers, 10 adenomas). Antibodies were applied for one hour at room temperature. After washing, sections were incubated with polymeric horse- radish peroxidase. Antibody localization was visualized using 3'3' diaminobenzidine.

### Result:

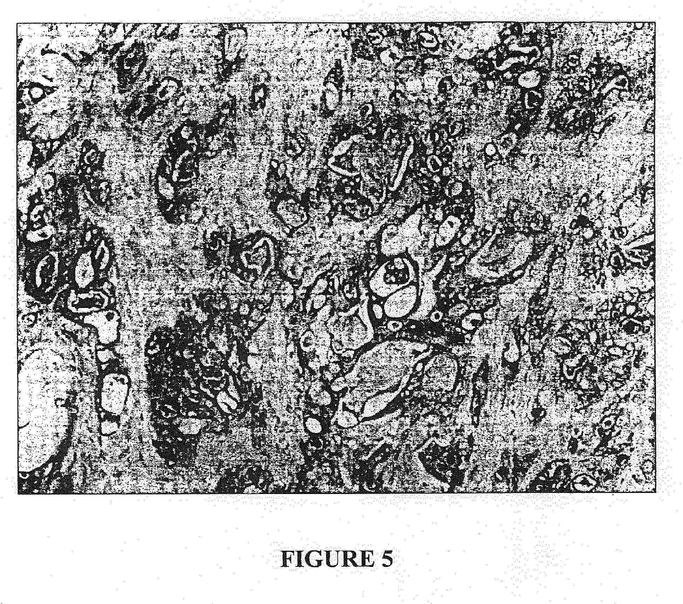

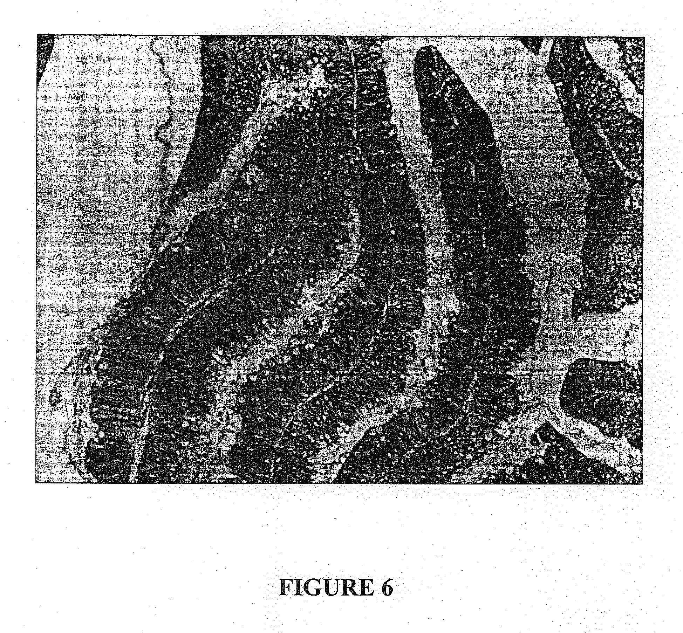

There was a marked upregulation of MSLN in the adenoma and cancer tissues compared to the normal controls. The normal tissues showed mild staining for MSLN in the cytoplasm of the colonic epithelium but the cancer and particularly the adenomas tissues shows significant upregulation of MSLN in their multilayered epithelium. This upregulation was observed in all 10 adenomas tissue and in 9 out of the 10 cancer tissues. These patterns of staining are illustrated in Figures 4, 5 and 6.

### Conclusion:

Elevated expression of MSLN has been detected in colon neoplasia, confirming the upregulation observed in the mRNA expression data and verifying the diagnostic utility of both the MSLN mRNA and protein for detection of colorectal neoplasia.

### EXAMPLE 8

### EVIDENCE OF MMP3 PROTEIN EXPRESSION IN STOOLS OF PATIENTS WITH COLORECTAL NEOPLASIA.

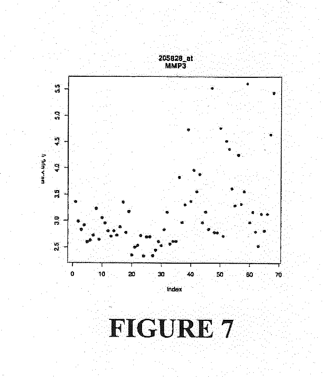

Affymetrix probeset designated 205828_at was identified to be expressed higher in 190 neoplastic tissue specimens relative to 264 non-neoplastic specimens. The probeset 205828_at hybridizes to RNA transcribed from the gene encoding Matrix Metalloproteinase 3 (MMP3) NM_002422. The differential expression profile of probeset 205828_at was further demonstrated by profiling RNA collected from 68 independent clinical specimens comprising 19 adenomas, 19 adenocarcinomas and 30 non-disease controls, Figure 7.

### Materials and Methods

A commercially available bead-suspension immunoassay targeting the protein MMP3 was purchased from R&D Systems (MMP Kit reagents LMP000 and LMP513) to measure MMP3 concentration in stools of human patients diagnosed with colorectal neoplasia. Proteins were extracted from stool specimens using a phosphate buffered saline wash from 6 non-disease controls, 10 adenoma and 11 adenocarcinoma subjects. The resulting protein extracts were analyzed using the Luminex bead-based suspension MMP3 assay as recommended by manufacturer.

### Results

An elevated endogenous expression of MMP3 was observed in stool specimens from patients diagnosed with colon adenomas or adenocarcinomas relative to non-neoplastic controls (Figure 8).

### Conclusion

Measurement of MMP3 protein in bodily fluids such as stool samples is useful for diagnosing colorectal neoplasia.

### TABLES

Probeset designations include both HG-133plus2 probeset IDs and Human Gene 1.0ST array probe ids. The latter can be conveniently mapped to Transcript Cluster ID using the Human Gene 1.0ST probe tab file provided by Affymetrix (http://www.affymetrix.com/Auth/analysis/downloads/na22/wtgene/HuGene-1_0-st-v1.probe.tab.zip). Using publicly available software such as NetAffx (provided by Affymetrix), the Transcript Cluster ID may be further mapped to gene symbol, chromosomal location, etc.

### Table 1.

Probesets demonstrated to be expressed higher in neoplastic tissues relative to non-neoplastic controls. TargetPS: Affymetrix HG-U133plus2 probeset id; Symbol: putative gene symbol corresponding to target probeset id - multiple symbol names indicate the possibility of probeset hybridisation to multiple gene targets; Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of neoplasia vs. non-neoplasia; Sens-Spec: Estimate of diagnostic performance corresponding to the ROC curve point demonstrating equal sensitivity and specificity; CI (95): 95% confidence interval of sensitivity and specificity estimates.

### Table 2.

Evidence of multiple probesets which correspond to gene symbols claimed herein exhibiting RNA concentration differences between neoplasia and non-neoplastic controls. Symbol: gene symbol; ValidPS_UP: Affymetrix probeset IDs demonstrating statistically significant overexpression in neoplastic RNA extracts relative to non-neoplastic controls. Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of neoplasia vs. non-neoplasia; Sens-Spec: Estimate of diagnostic performance corresponding to the ROC curve point demonstrating equal sensitivity and specificity; CI (95): 95% confidence interval of sensitivity and specificity estimates.

### Example 2

### Table 3.

Probesets which demonstrate a qualitatively (in addition to quantitative) elevated profile in neoplastic tissues relative to non-neoplastic controls. TargetPS: Affymetrix HG-U133plus2 probeset id; Symbol: putative gene symbol corresponding to target probeset id - multiple symbol names indicate the possibility of probeset hybridisation to multiple gene targets; Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of neoplasia vs. non-neoplasia; Sens-Spec: Estimate of diagnostic performance corresponding to the ROC curve point demonstrating equal sensitivity and specificity; CI (95): 95% confidence interval of sensitivity and specificity estimates.

### Table 4.

Evidence of multiple probesets which correspond to gene symbols claimed herein exhibiting qualitative changes in RNA concentration in neoplastic tissues. Symbol: gene symbol; ValidPS_UP: Affymetrix probeset IDs demonstrating statistically significant overexpression in neoplastic RNA extracts relative to non-neoplastic controls. Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of neoplasia vs. non-neoplasia; Sens-Spec: Estimate of diagnostic performance corresponding to the ROC curve point demonstrating equal sensitivity and specificity; CI (95): 95% confidence interval of sensitivity and specificity estimates.

### Table 5

Probesets demonstrated to be expressed higher in adenoma tissues relative to cancer tissues. TargetPS: Affymetrix HG-U133plus2 probeset id; Symbol: putative gene symbol corresponding to target probeset id - multiple symbol names indicate the possibility of probeset hybridisation to multiple gene targets; Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of adenomas vs. cancers; Sens-Spec: Estimate of diagnostic performance corresponding to the ROC curve point demonstrating equal sensitivity and specificity; CI (95): 95% confidence interval of sensitivity and specificity estimates.

### Table 6

Probesets demonstrated to be expressed higher in cancer tissues relative to adenoma tissues, TargetPS: Affymetrix HG-U133plus2 probeset id; Symbol: putative gene symbol corresponding to target probeset id - multiple symbol names indicate the possibility of probeset hybridisation to multiple gene targets; Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of cancer tissues vs. adenoma tissues; Sens-Spec: Estimate of diagnostic performance corresponding to the ROC curve point demonstrating equal sensitivity and specificity; CI (95): 95% confidence interval of sensitivity and specificity estimates.

### Table 7

Evidence of multiple probesets which correspond to gene symbols claimed herein exhibiting RNA concentration differences between adenoma and cancer tissues. Symbol: gene symbol ; ValidPS_UP: Affymetrix probeset IDs demonstrating statistically significant overexpression in neoplastic RNA extracts relative to non-neoplastic controls. Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of adenoma tissues vs. cancer tissues; Sens-Spec: Estimate of diagnostic performance corresponding to the ROC curve point demonstrating equal sensitivity and specificity; CI (95): 95% confidence interval of sensitivity and specificity estimates.

### Table 8

Evidence of multiple probesets which correspond to gene symbols claimed herein exhibiting RNA concentration differences between cancer and adenoma tissues. Symbol: gene symbol ; ValidPS_UP: Affymetrix probeset IDs demonstrating statistically significant overexpression in neoplastic RNA extracts relative to non-neoplastic controls. Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of cancer tissues vs. adenoma tissues; Sens-Spec: Estimate of diagnostic performance corresponding to the ROC curve point demonstrating equal sensitivity and specificity; CI (95): 95% confidence interval of sensitivity and specificity estimates.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

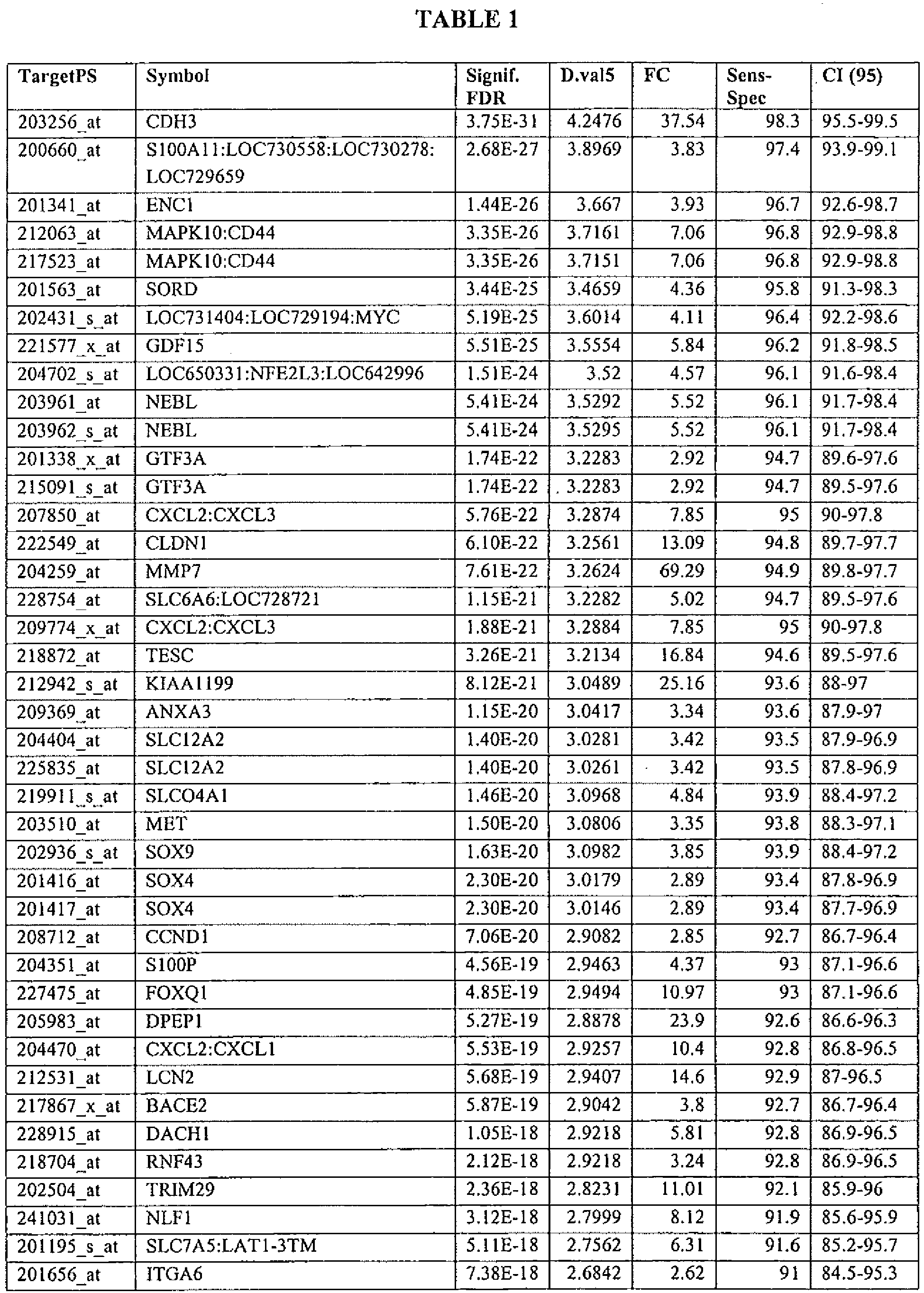

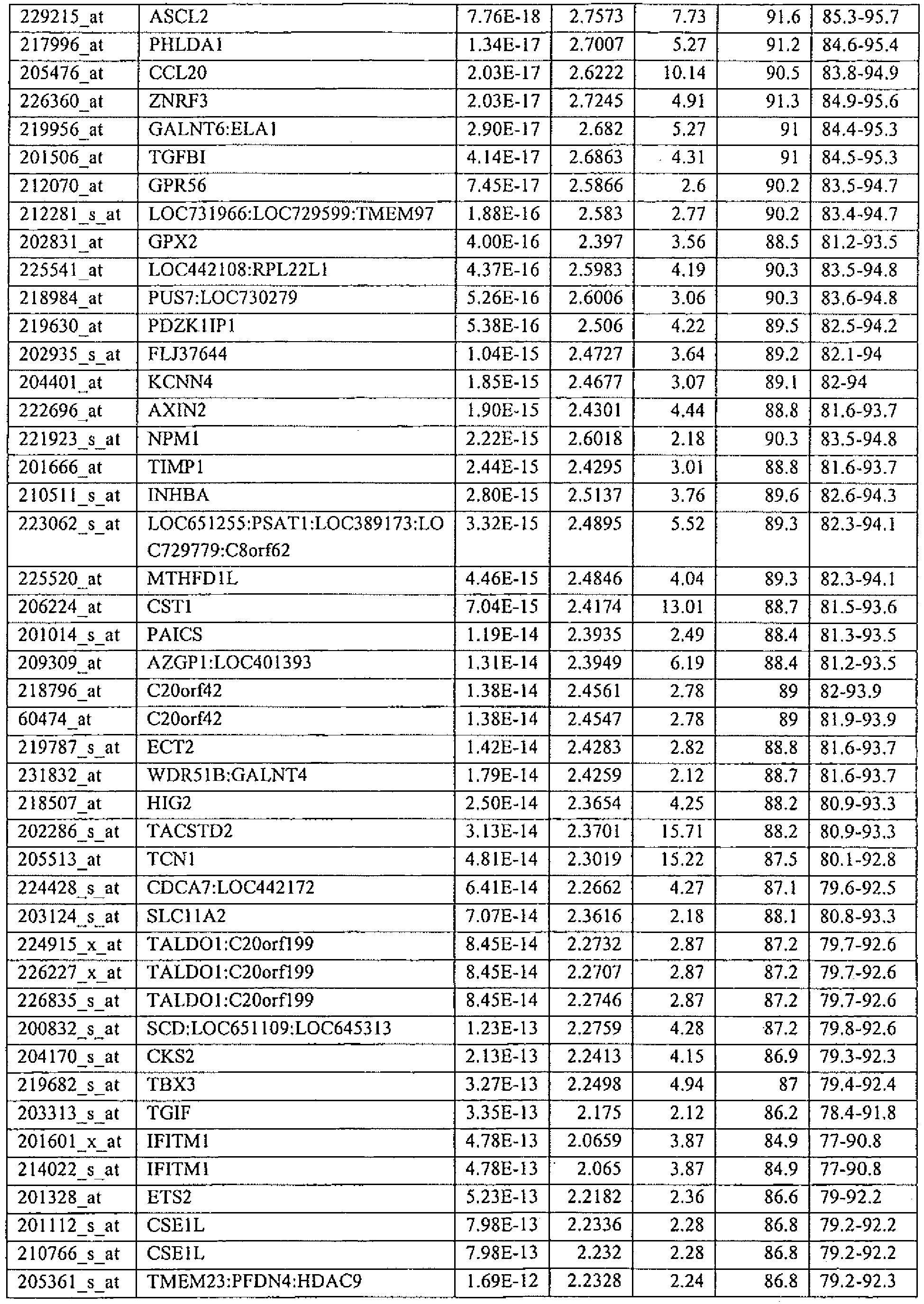

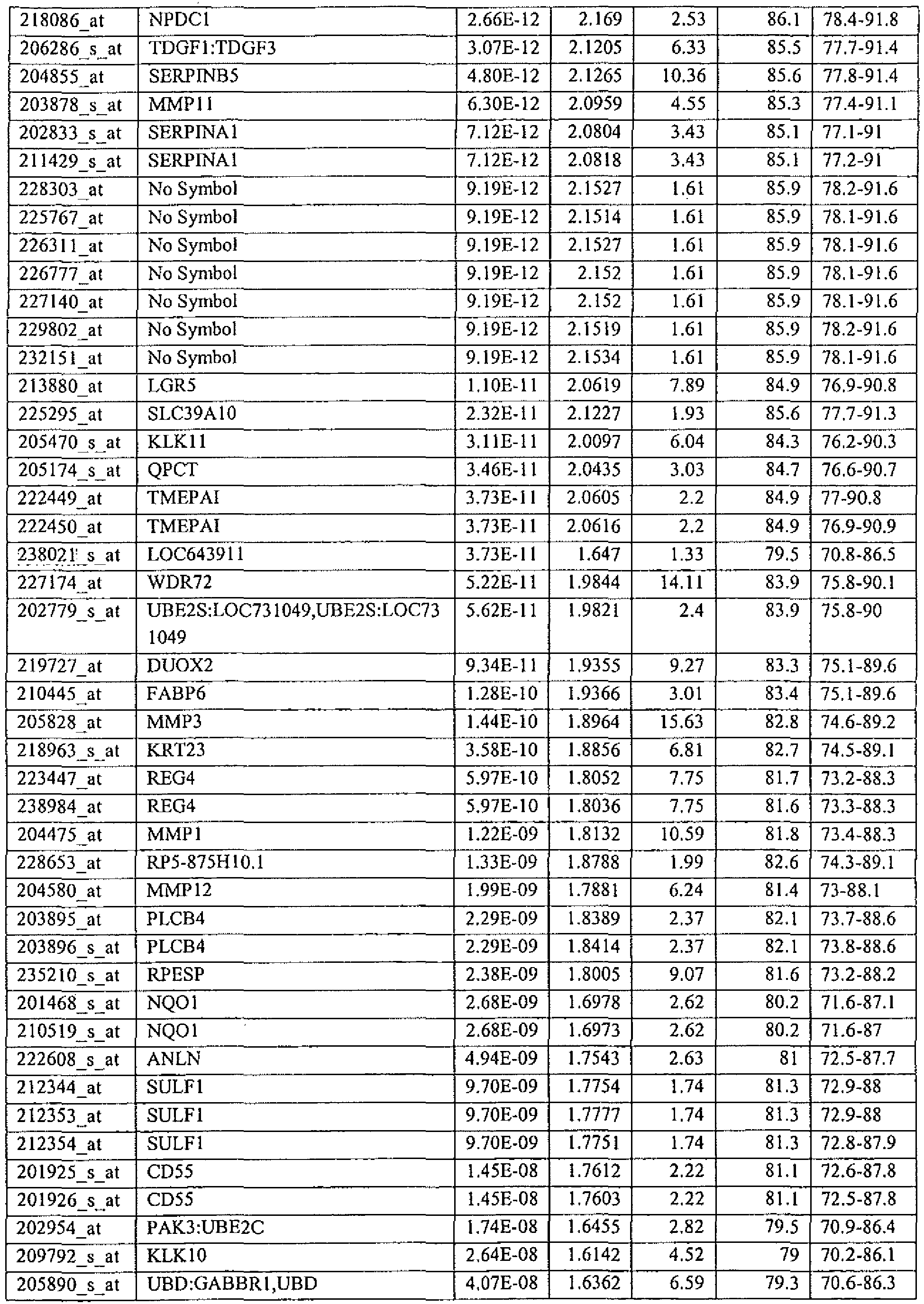

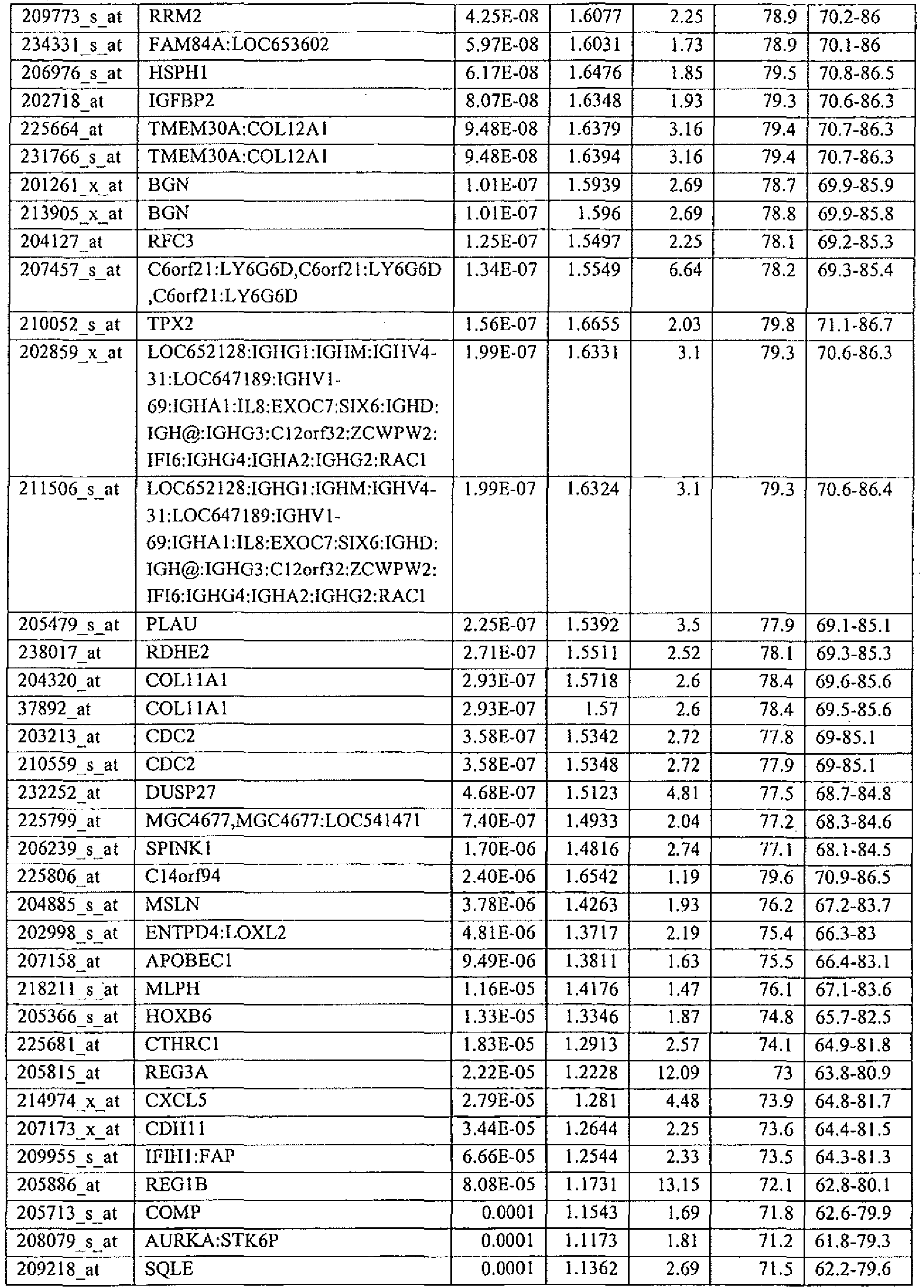

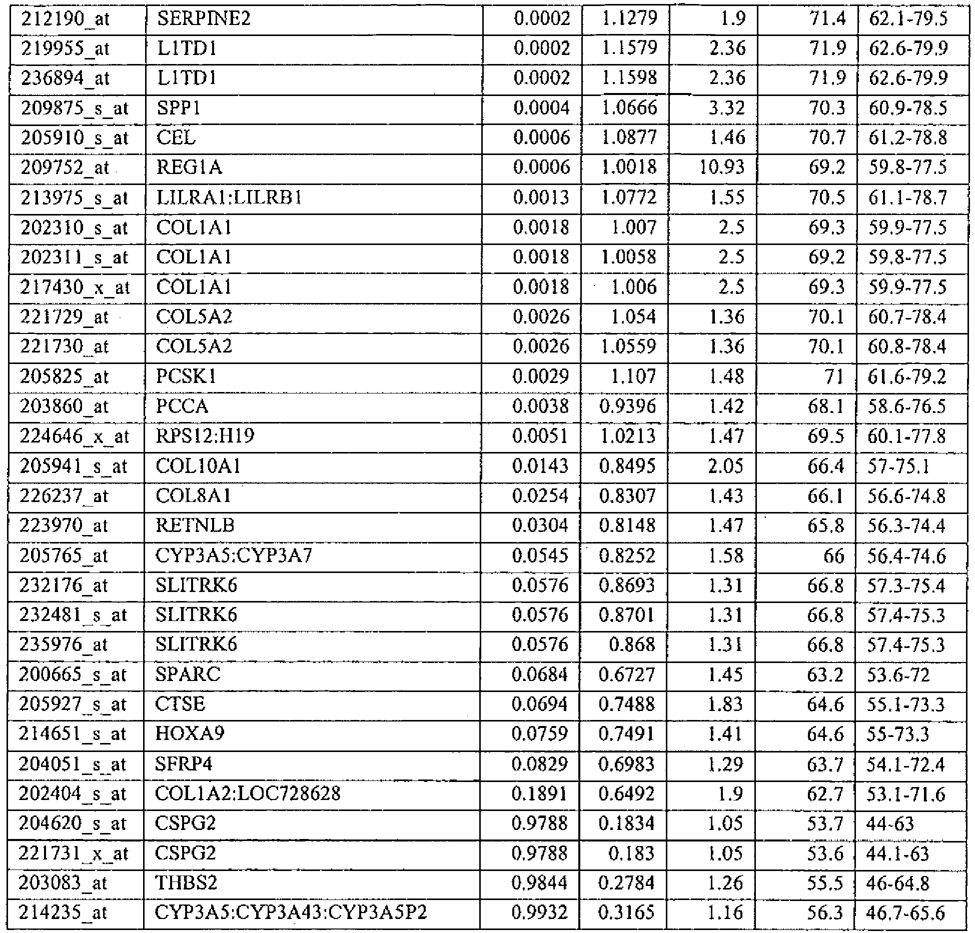

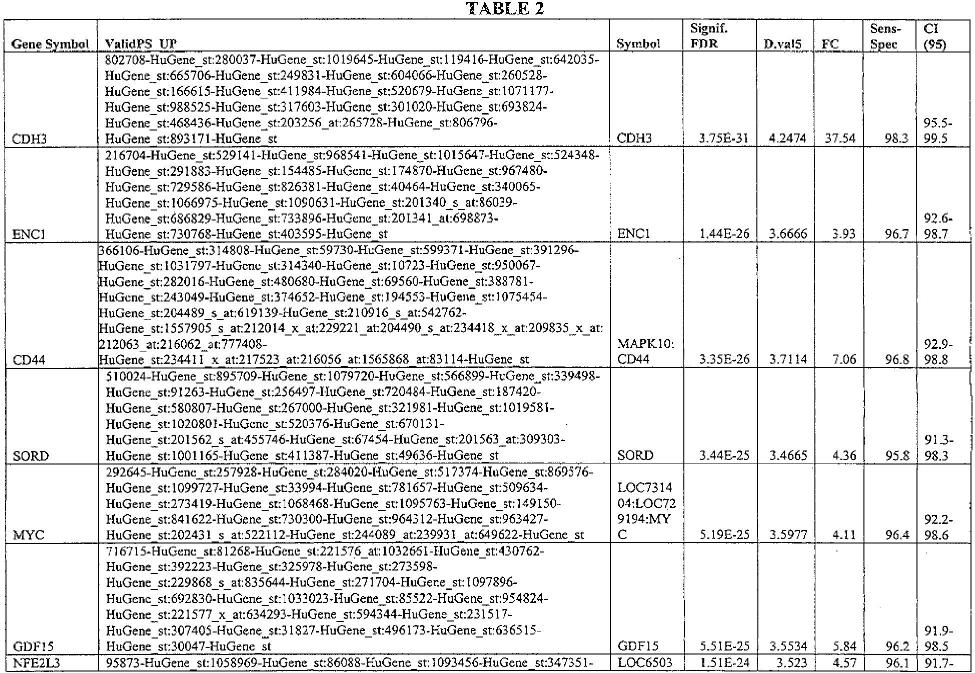

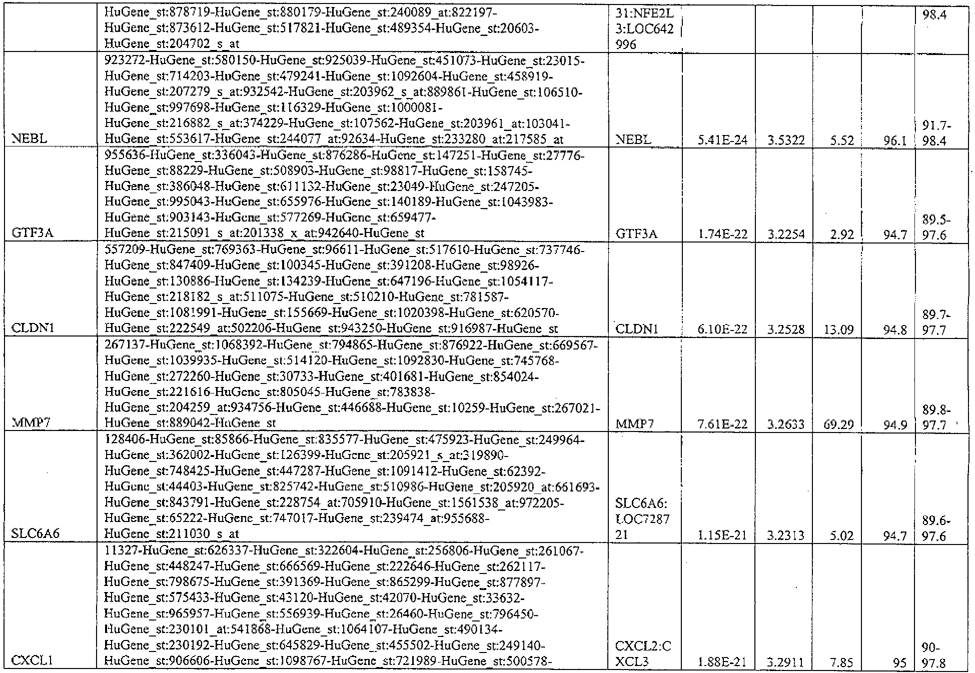

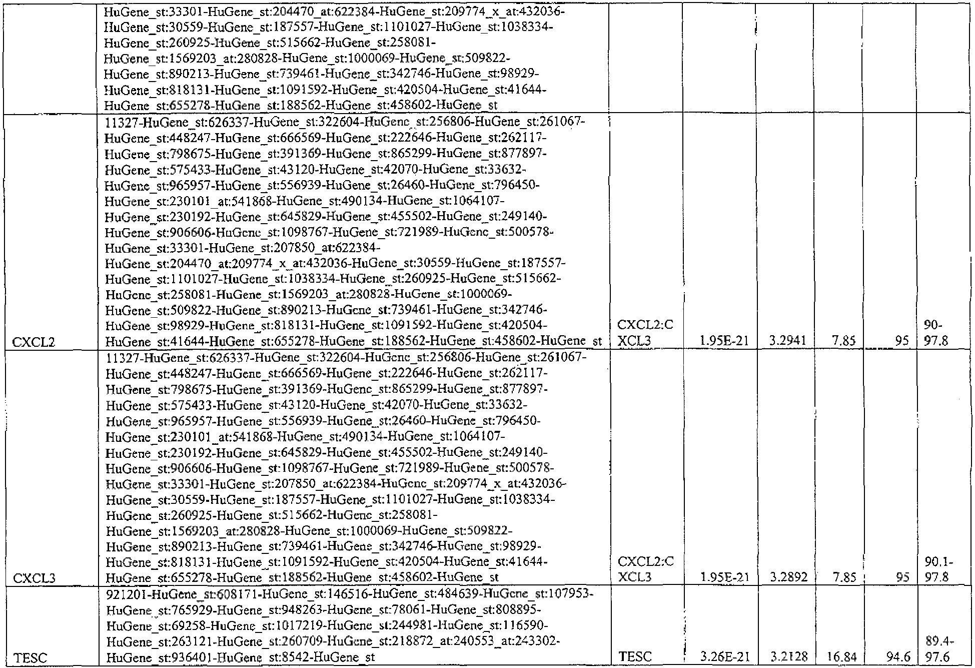

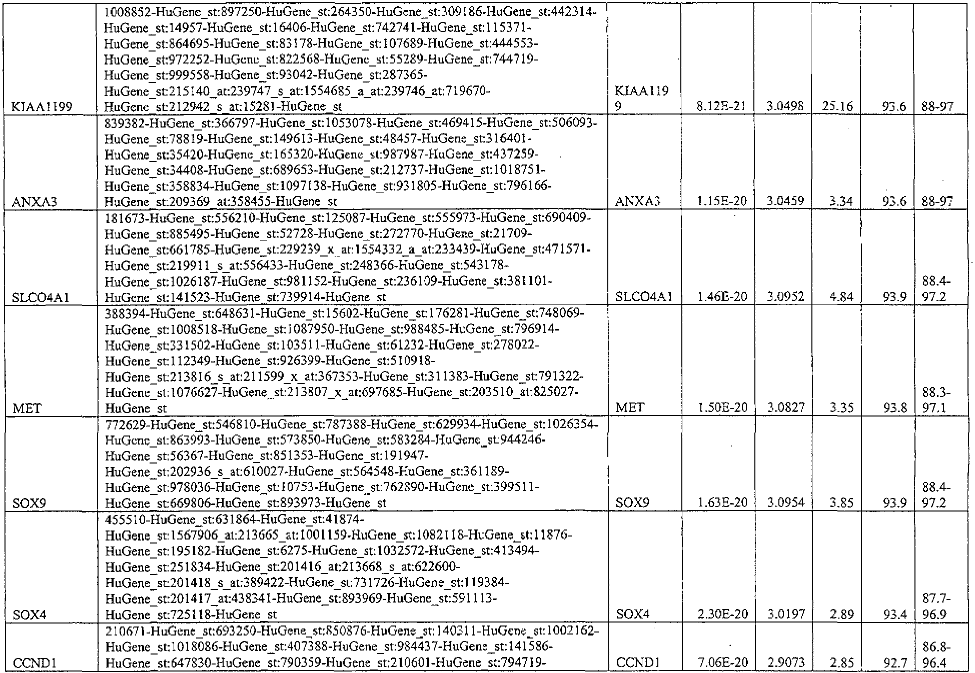

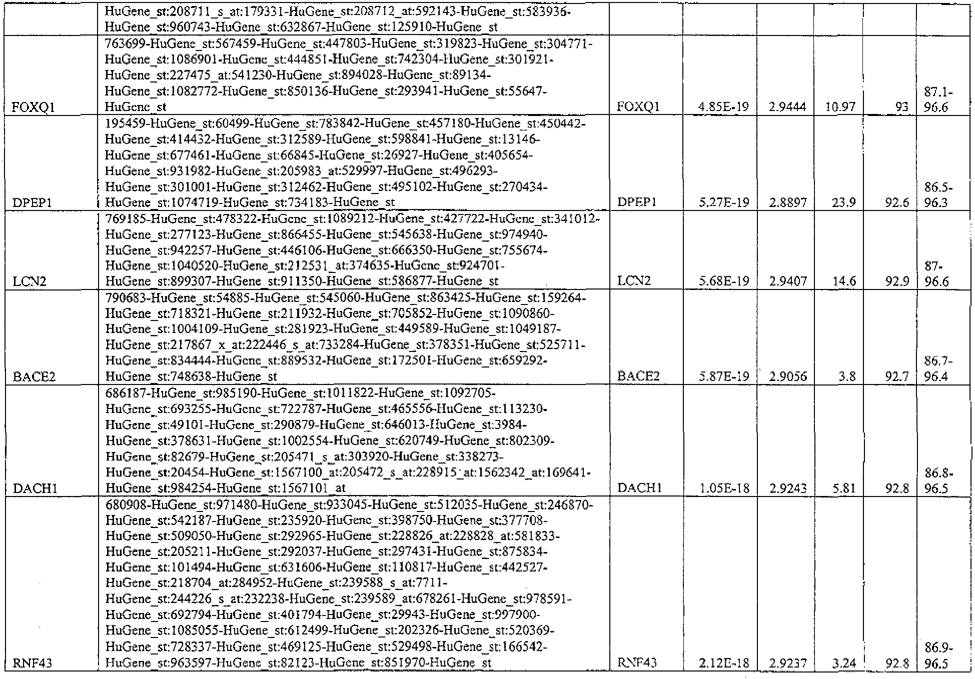

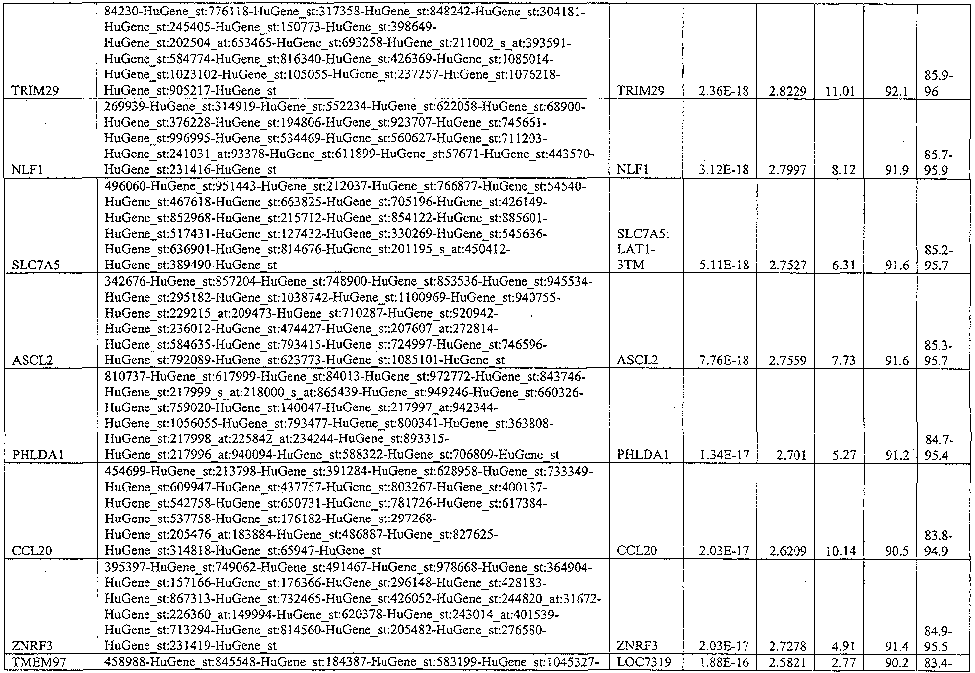

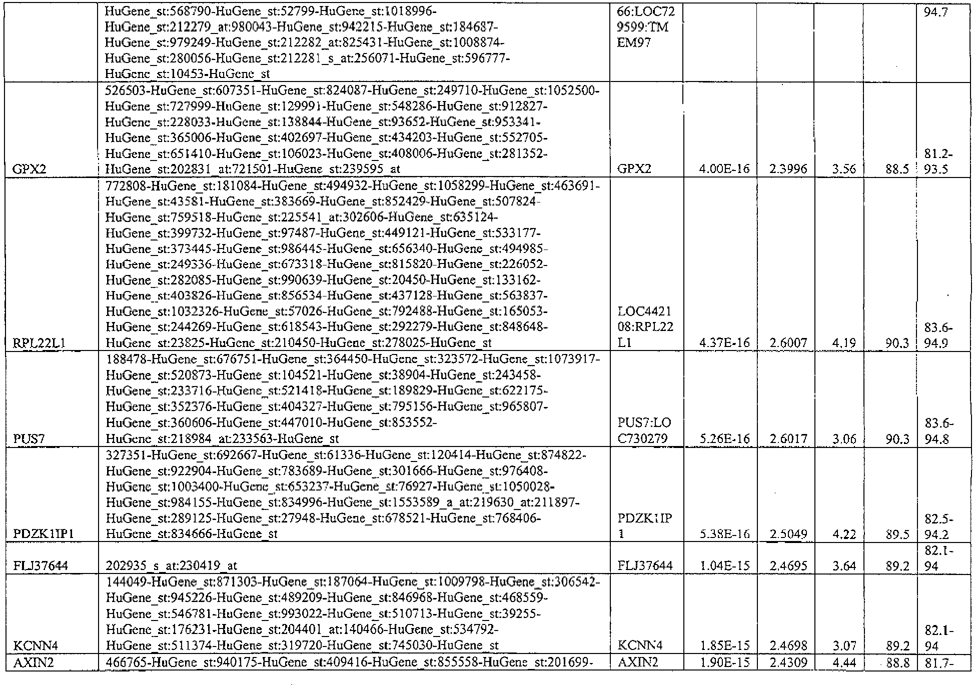

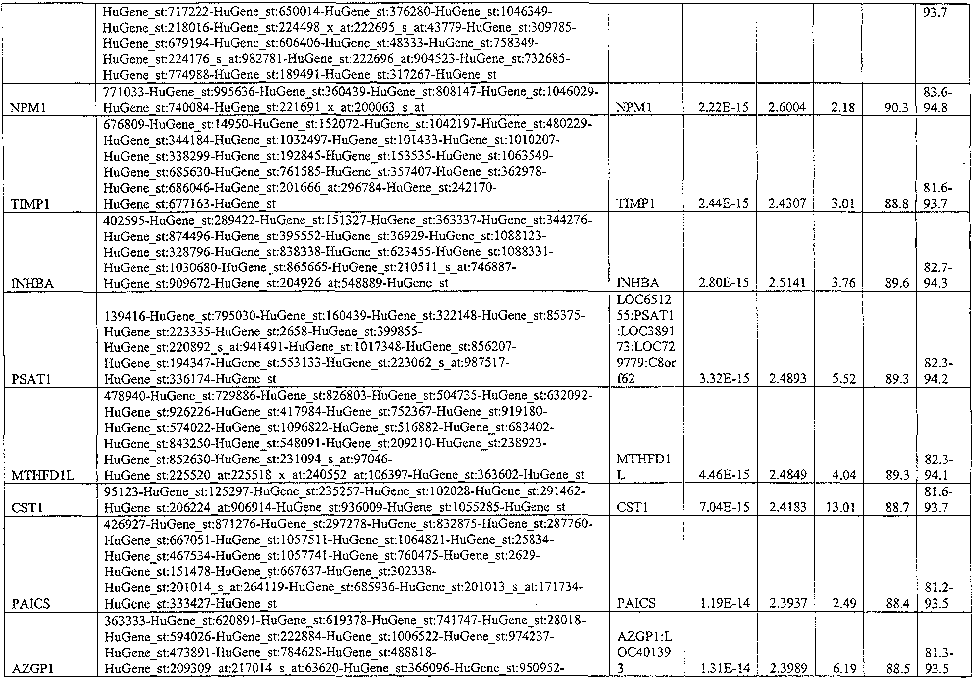

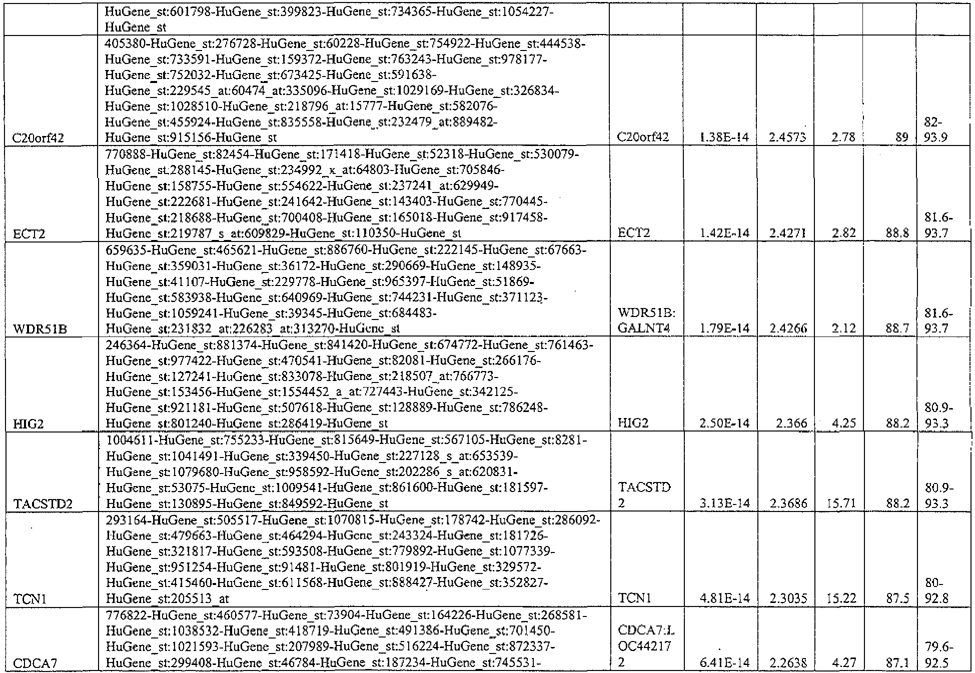

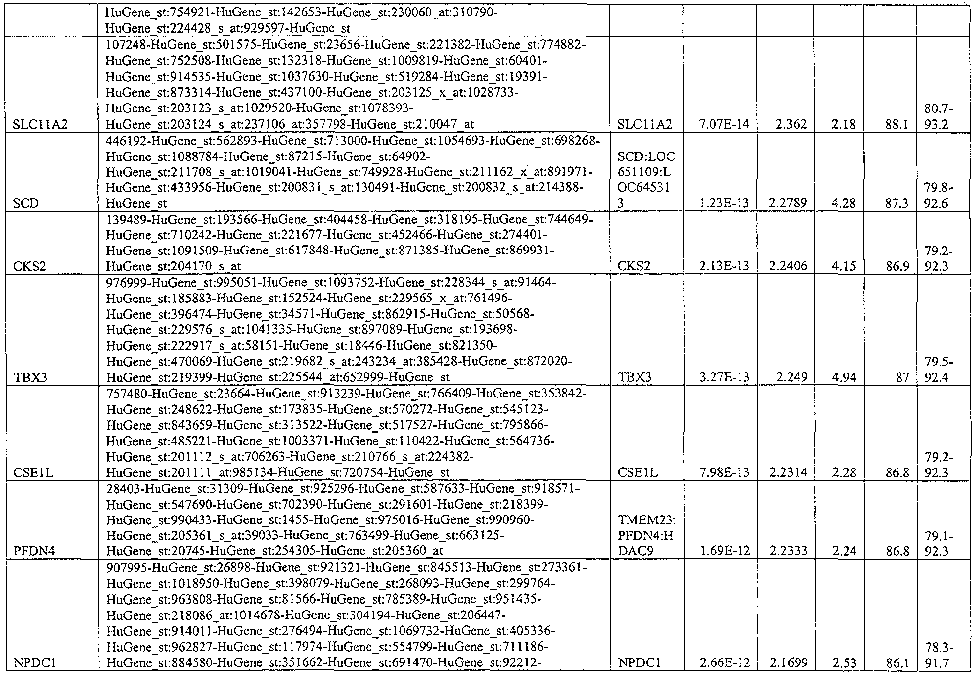

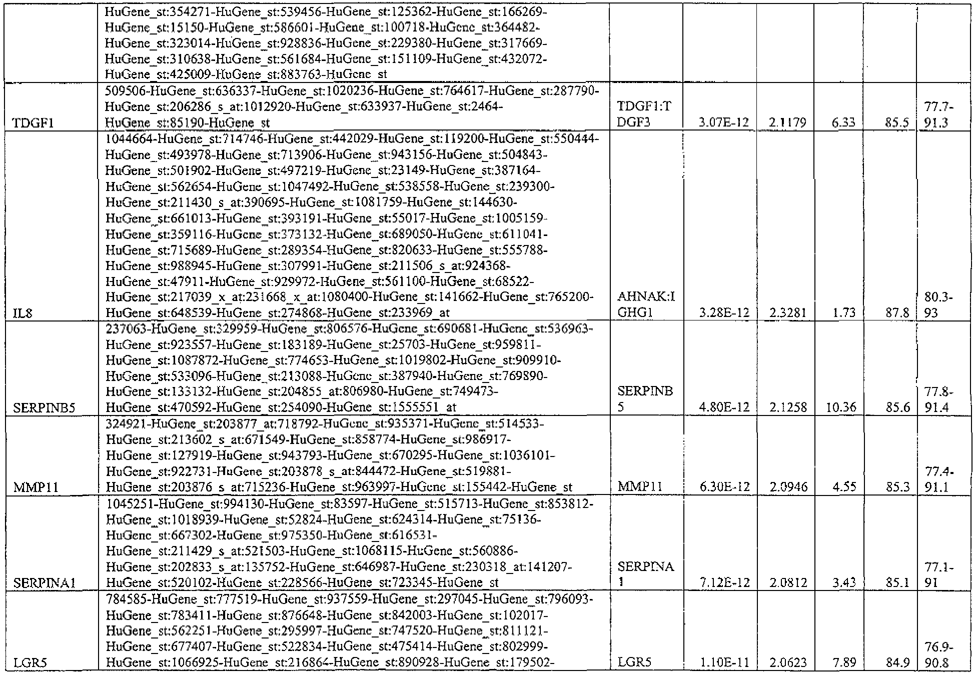

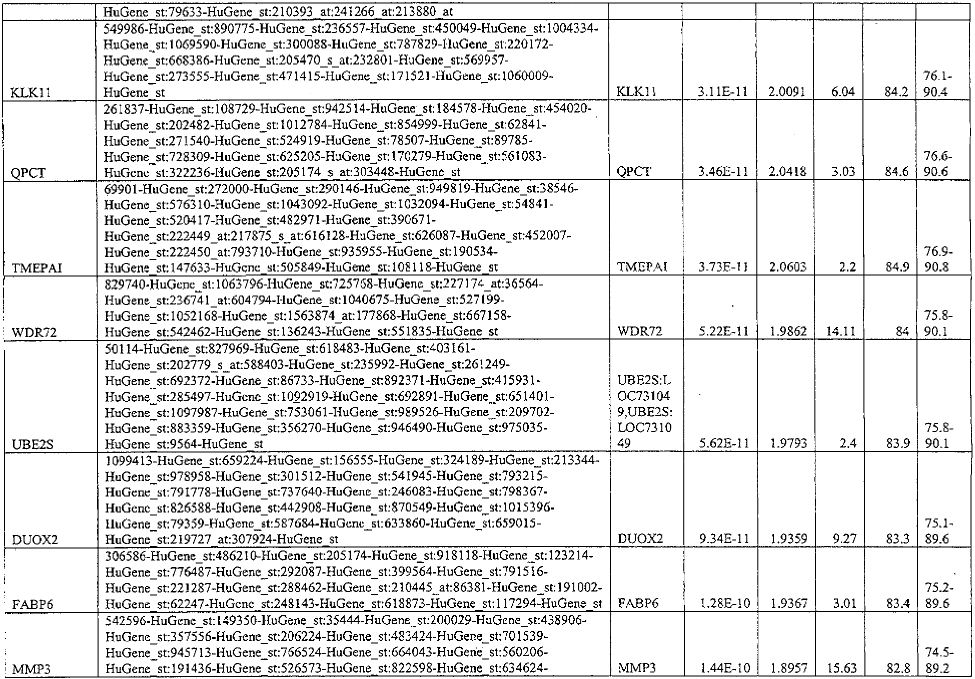

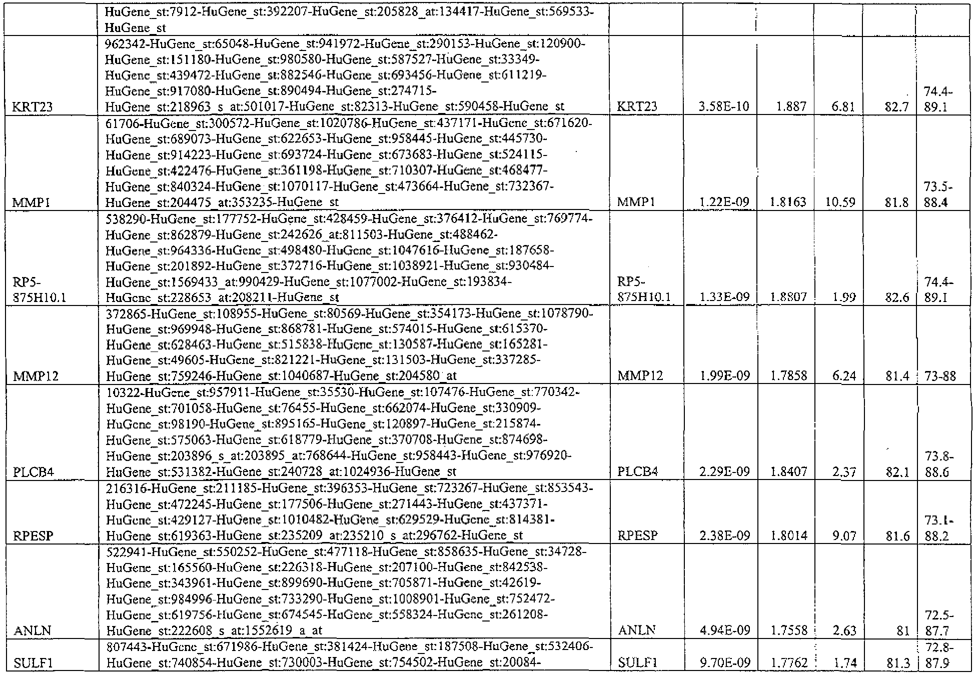

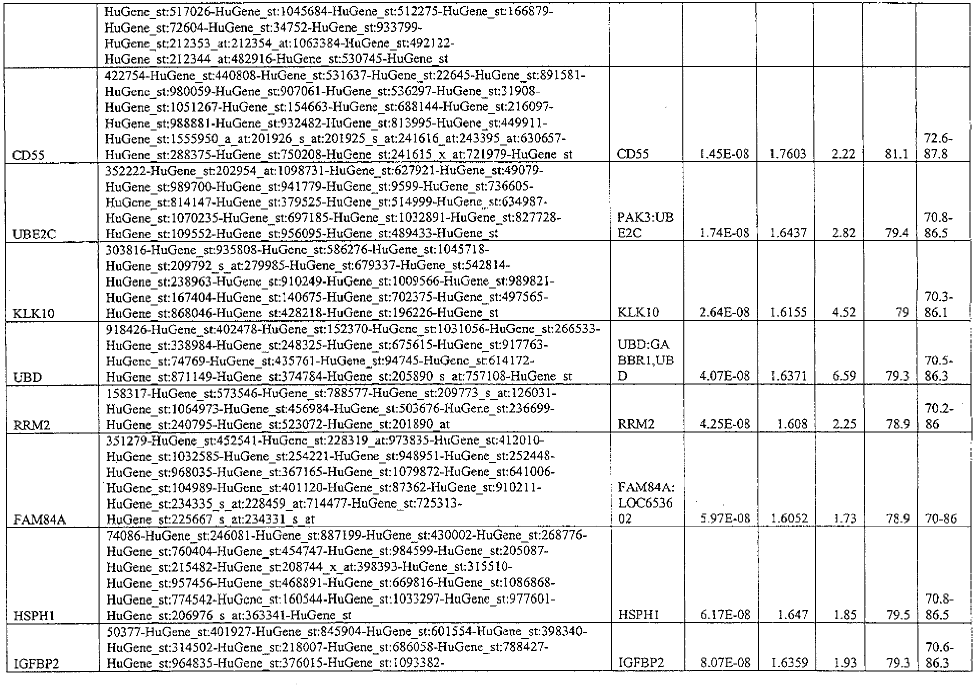

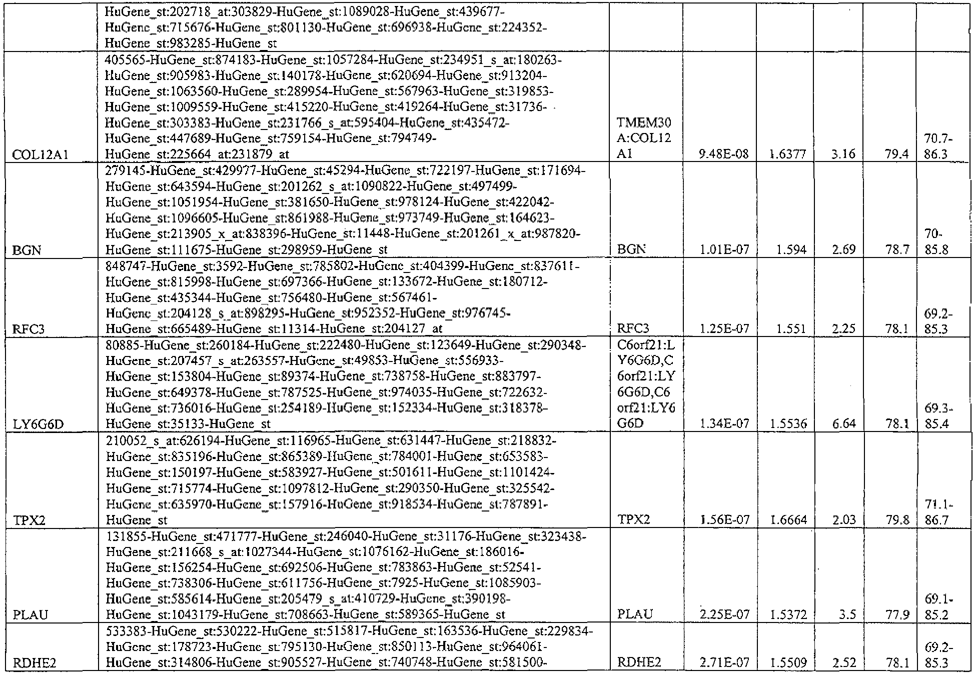

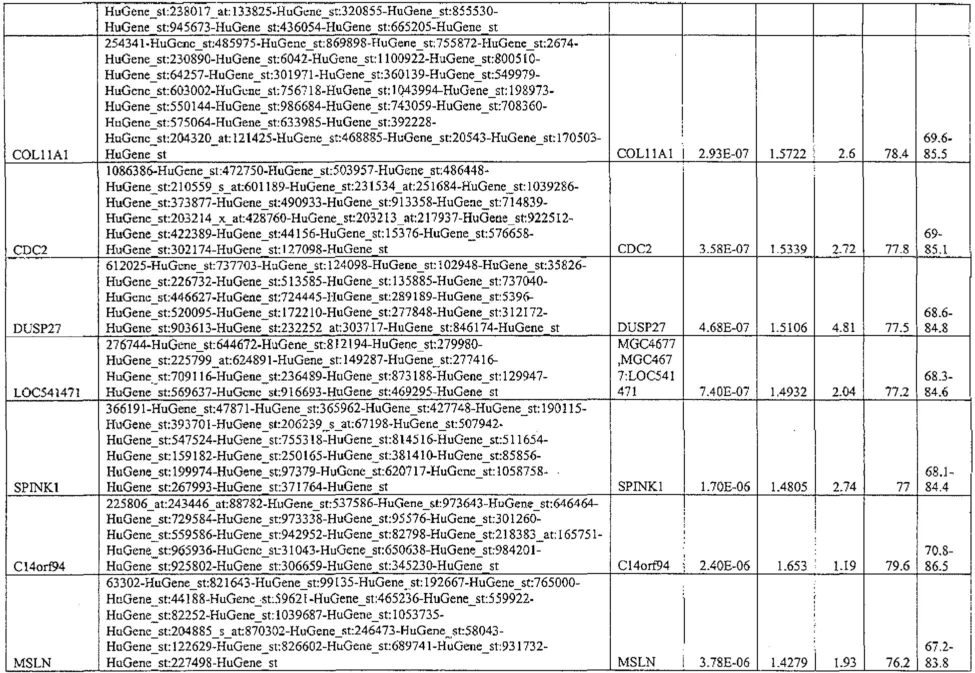

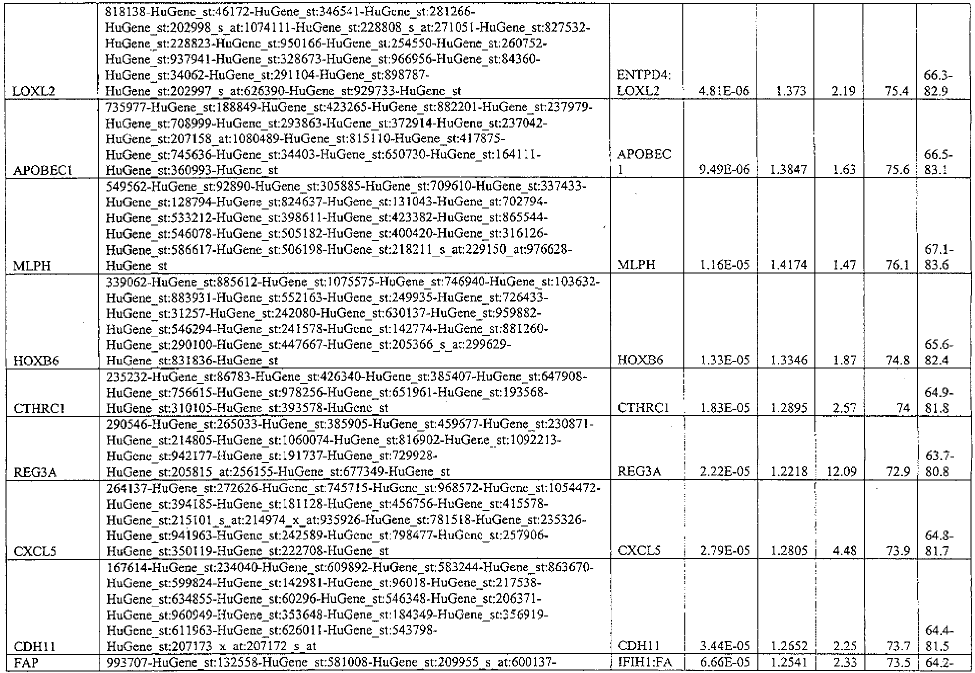

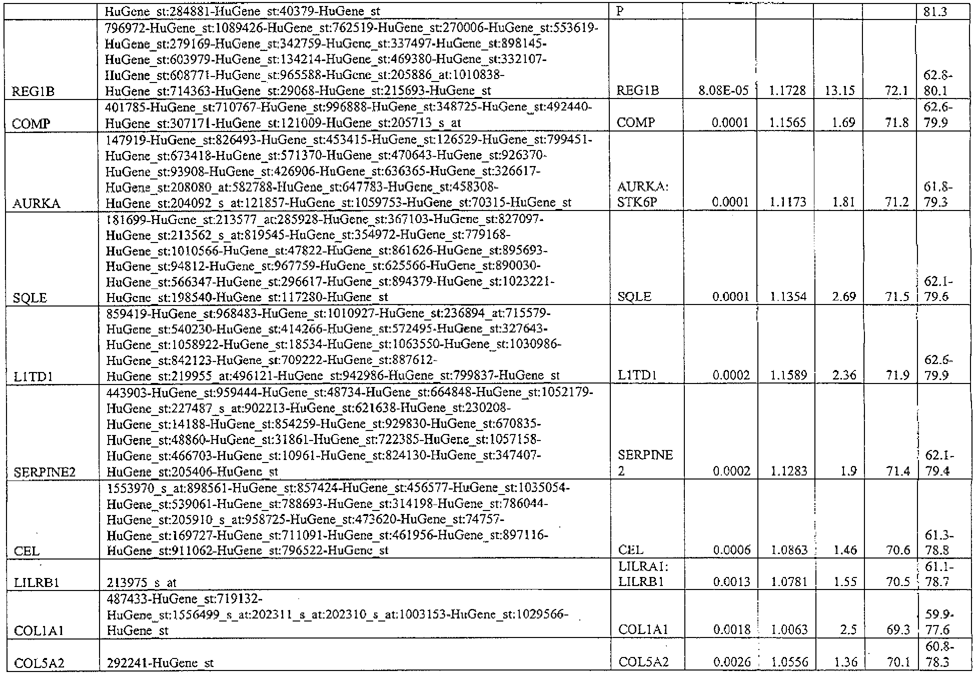

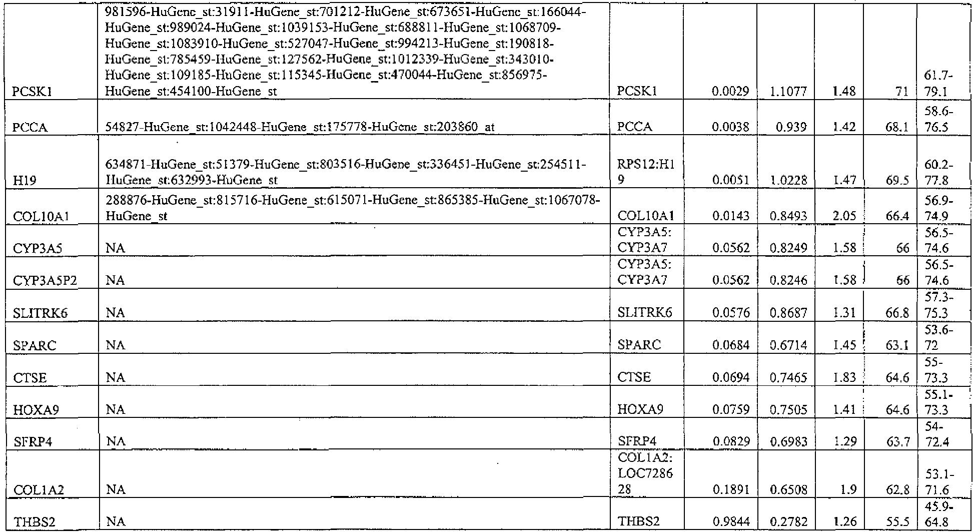

**TABLE 3**

| TargetPS | Symbol | Signif. FDR | D.val5 | FC | Sens-Spec | CI (95) |
|---|---|---|---|---|---|---|
| 217523_at | MAPK10:CD44 | 3.35E-26 | 3.7166 | 7.06 | 96.8 | 92.9-98,8 |
| 204702_s_at | LOC650331:NFE2L3:LOC642996 | 1.51E-24 | 3.521 | 4.57 | 96.1 | 91.7-98.4 |
| 207850_at | CXCL2:CXCL3 | 5.76E-22 | 3.2893 | 7.85 | 95 | 90-97.8 |
| 204259_at | MMP7 | 7.61E-22 | 3.2634 | 69.29 | 94.9 | 89.8-97.7 |
| 228915_at | DACH1 | 1.05E-18 | 2.9232 | 5.81 | 92.8 | 86.9-96.5 |
| 241031_at | NLF1 | 3.12E-18 | 2.8001 | 8.12 | 91.9 | 85.7-95.9 |
| 223062_s_at | LOC651255:PSAT1:LOC389173:LOC7297 79:C8orf62 | 3.32E-15 | 2.4882 | 5.52 | 89.3 | 82.3-94.2 |
| 206224_at | CST1 | 7.04E-15 | 2.4165 | 13.01 | 88.7 | 81.5-93.6 |
| 209309 at | AZGP1 :LOC401393 | 1.31E-14 | 2.3986 | 6.19 | 88.5 | 81.3-93.5 |
| 219787_s_at | ECT2 | 1.42E-14 | 2.4245 | 2.82 | 88.7 | 81.6-93.7 |
| 202286_s_at | TACSTD2 | 3.13E-14 | 2.3676 | 15.71 | 88.2 | 80.9-93.3 |
| 227140_at | No Symbol | 9.19E-12 | 2.1522 | 1.61 | 85.9 | 78.2-91.6 |
| 229802_at | No Symbol | 9.19E-12 | 2.1536 | 1.61 | 85.9 | 78.2-91.6 |
| 213880_at | LGR5 | 1.10E-11 | 2.0621 | 7.89 | 84.9 | 76.9-90.8 |
| 205174_s_at | QPCT | 3.46E-11 | 2.0422 | 3.03 | 84.6 | 76.7-90.6 |
| 238021_s_at | LOC643911 | 3.73E-11 | 1.6459 | 1.33 | 79.5 | 70.8-86.5 |
| 227174_at | WDR72 | 5.22E-11 | 1.9854 | 14.11 | 84 | 75.8-90.1 |
| 238984_at | REG4 | 5.97E-10 | 1.8033 | 7.75 | 81.6 | 73.2-88.3 |
| 204475_at | MMP1 | 1.22E-09 | 1.8138 | 10.59 | 81.8 | 73.4-88.4 |
| 222608_s_at | ANLN | 4.94E-09 | 1.7541 | 2.63 | 81 | 72.4-87.7 |
| 211506_s_at | LOC652128:IGHG1:IGHM:IGHV4-31:LOC647189:IGHV1-69:IGHA1:IL8:EXOC7:SIX6:IGHD:IGH@: IGHG3:C12orf32:ZCWPW2:IFI6:IGHG4:I GHA2:IGHG2:RAC1 | 1.99E-07 | 1.6343 | 3.1 | 79.3 | 70.6-86.3 |
| 204320_at | COL11A1 | 2.93E-07 | 1.5718 | 2.6 | 78.4 | 69.6-85.6 |
| 37892_at | COL11A1 | 2.93E-07 | 1.5707 | 2.6 | 78.4 | 69.6-85.6 |
| 232252_at | DUSP27 | 4.68E-07 | 1.5112 | 4.81 | 77.5 | 68.6-84.8 |
| 225806_at | C14orf94 | 2.40E-06 | 1.6519 | 1.19 | 79.6 | 70.9-86.6 |
| 204885_s_at | MSLN | 3.78E-06 | 1.4266 | 1.93 | 76.2 | 67.2-83.7 |
| 214974_x_at | CXCL5 | 2.79E-05 | 1.2818 | 4.48 | 73.9 | 64.7-81.6 |
| 236894_at | L1TD1 | 0.0002 | 1.1596 | 2.36 | 71.9 | 62.6-79.9 |
| 205910_s_at | CEL | 0.0006 | 1.0869 | 1.46 | 70.7 | 61.3-78.8 |
| 202311_s_at | COL1A1 | 0.0018 | 1.0054 | 2.5 | 69.2 | 59.9-77.6 |
| 205825_at | PCSK1 | 0.0029 | 1.1086 | 1.48 | 71 | 61.7-79.1 |
| 226237_at | COL8A1 | 0.0254 | 0.8303 | 1.43 | 66.1 | 56.6-74.7 |
| 235976_at | SLITRK6 | 0.0576 | 0.8689 | 1.31 | 66.8 | 57.2-75.3 |

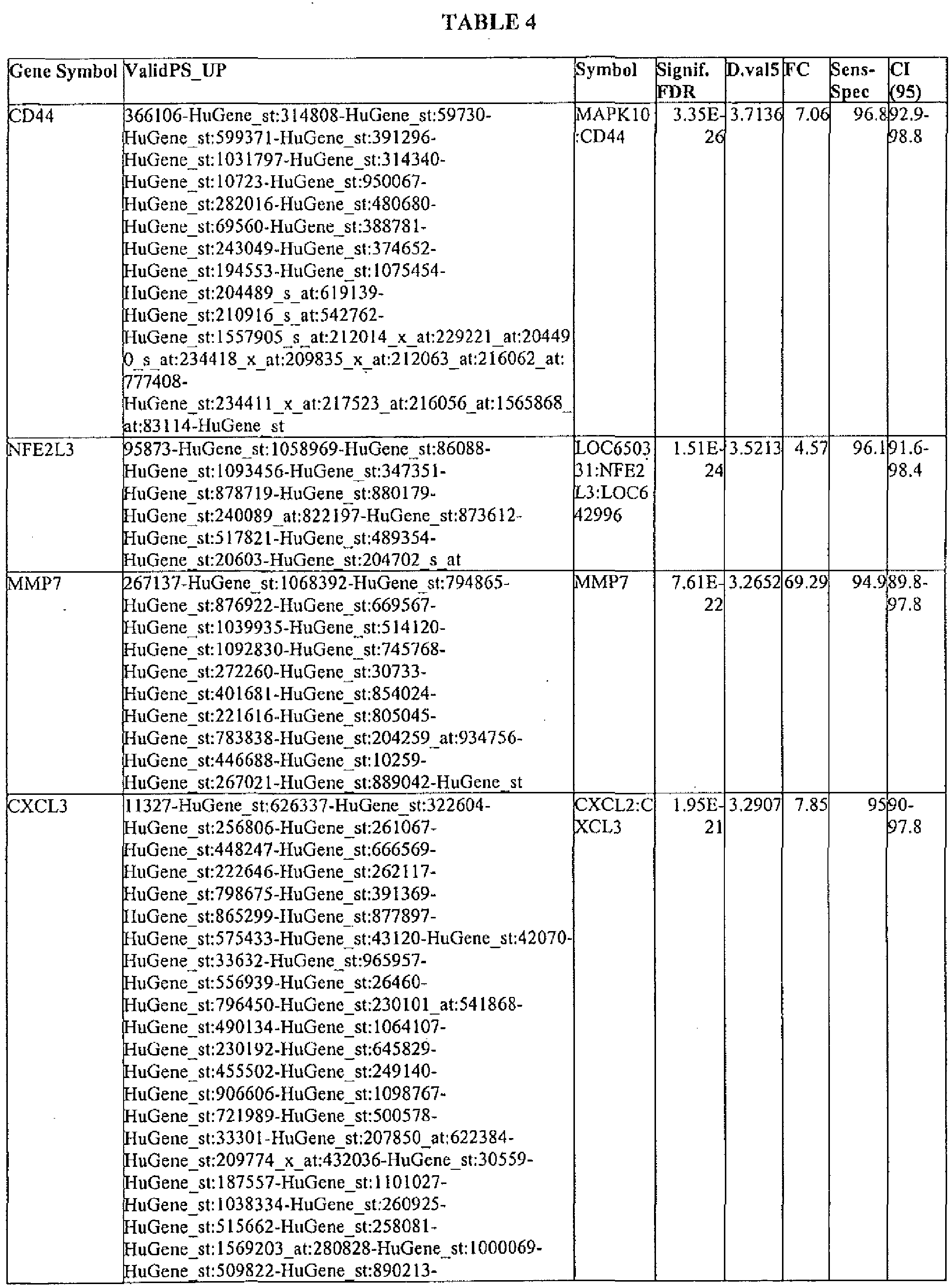

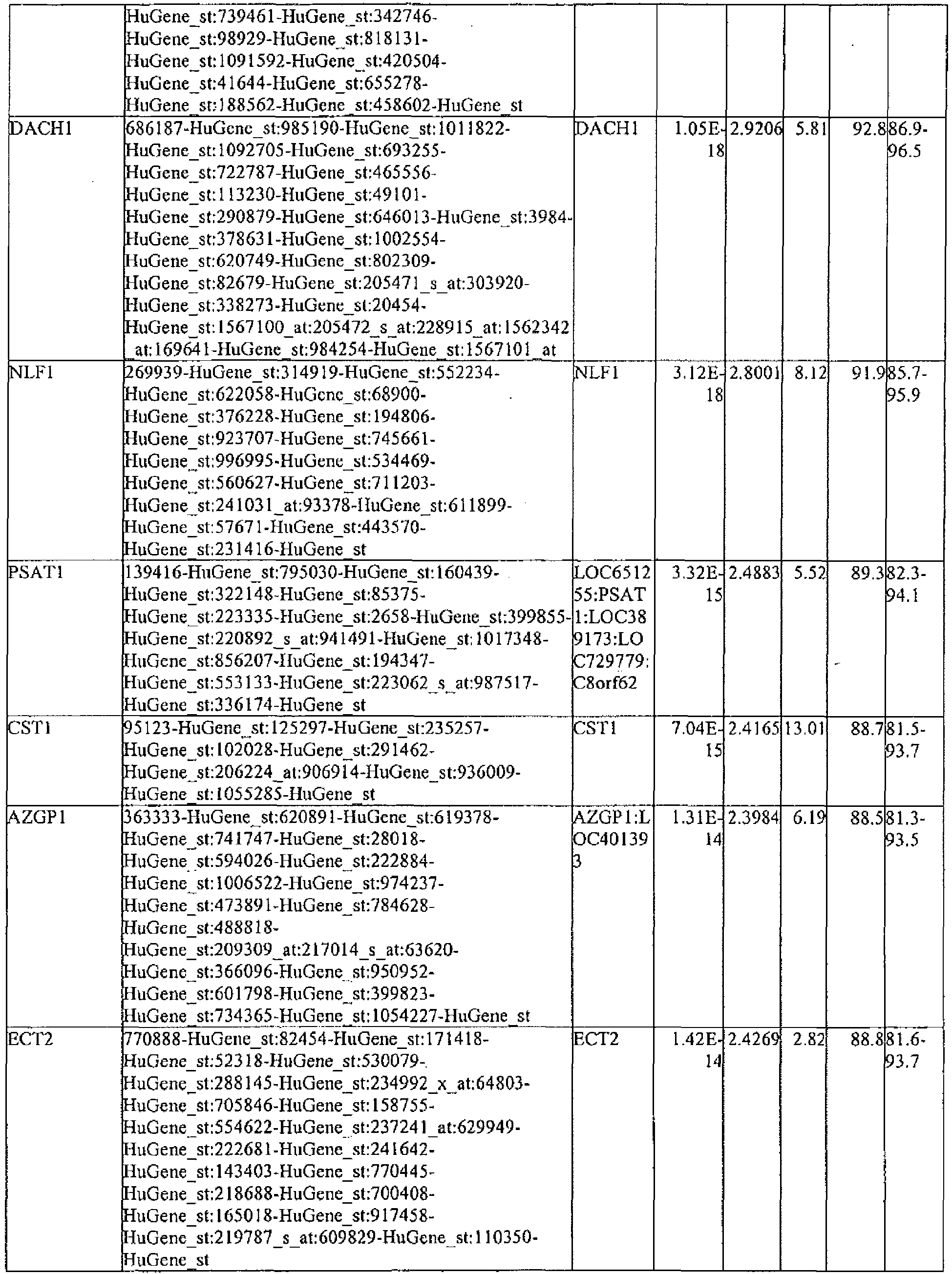

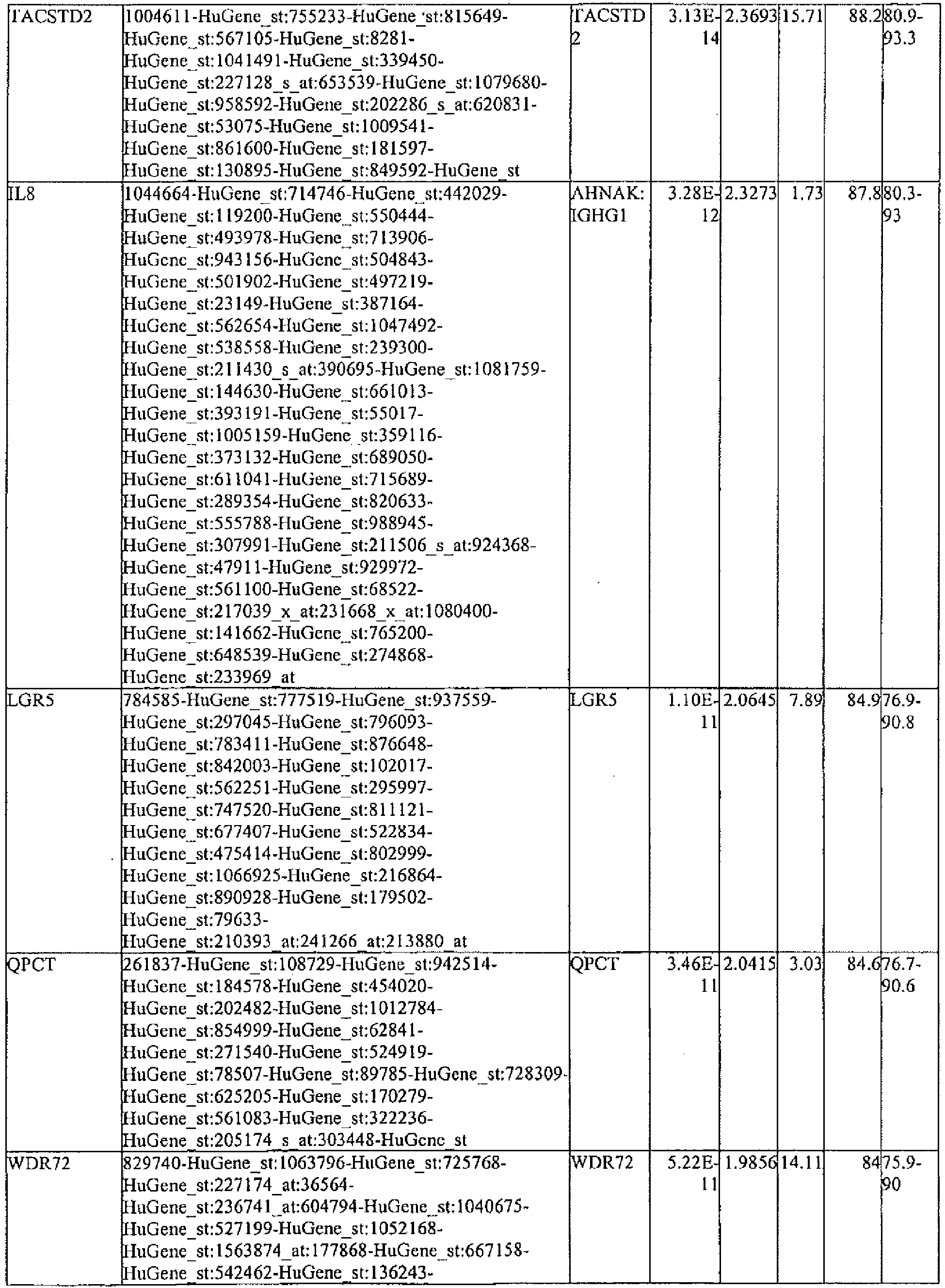

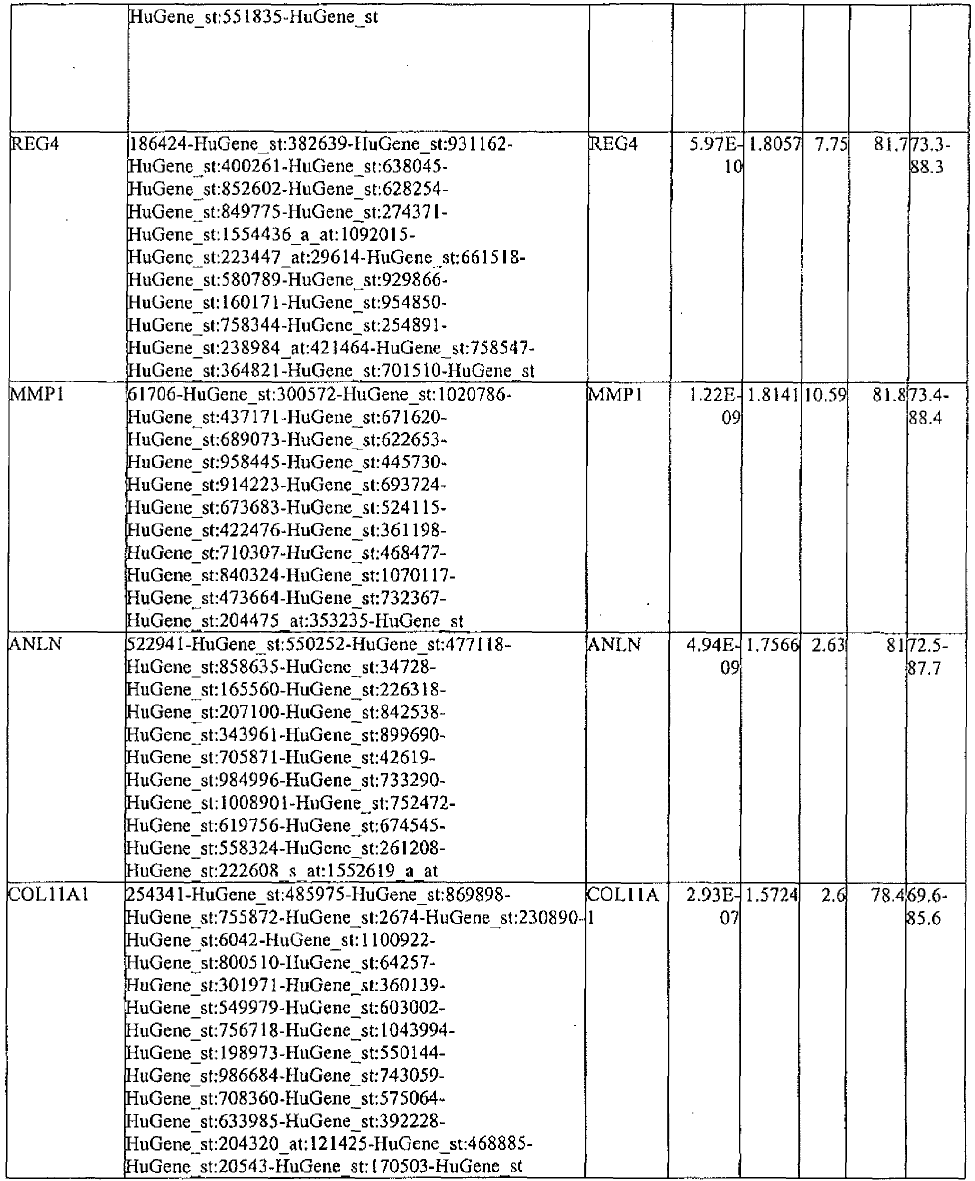

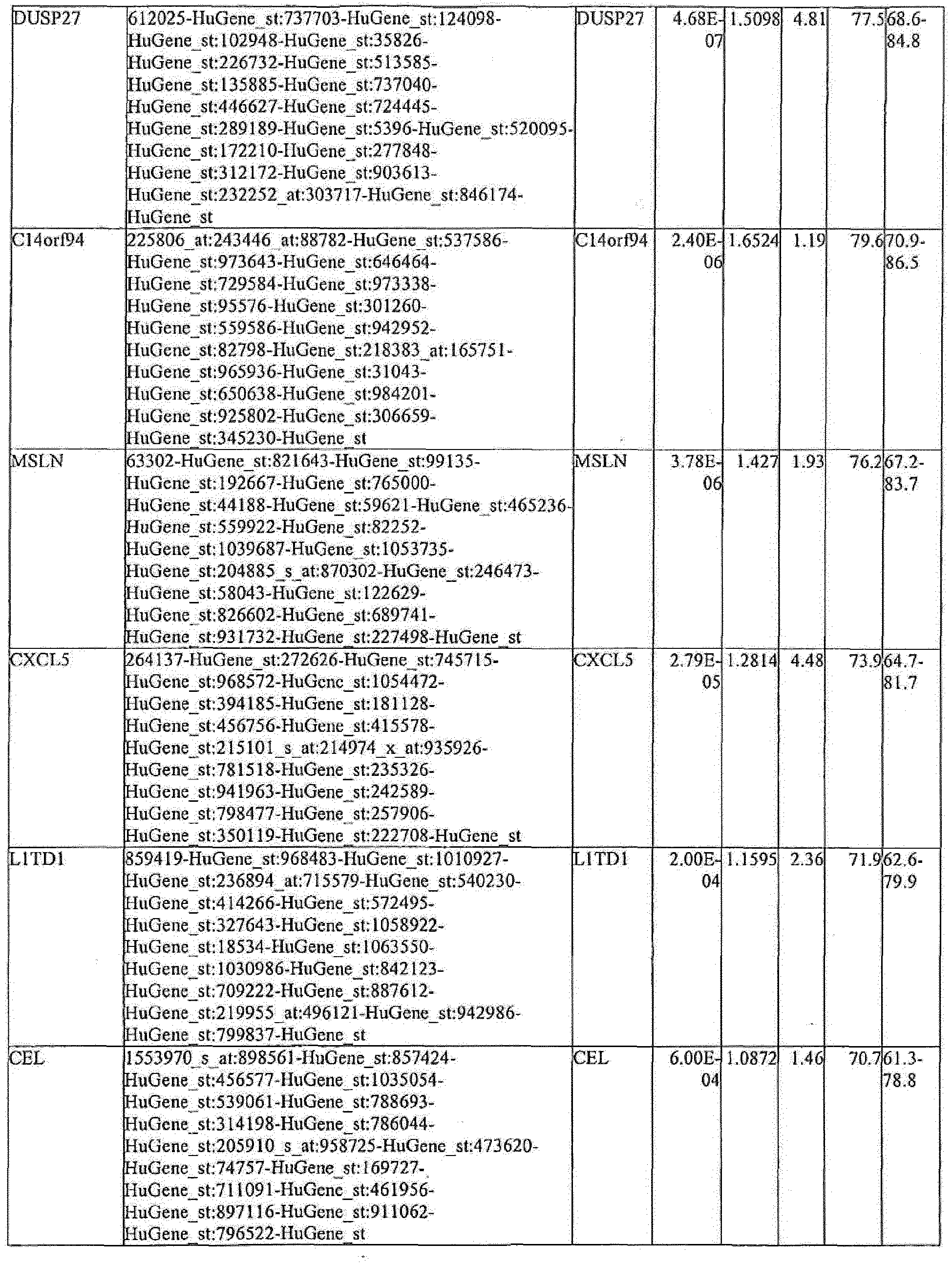

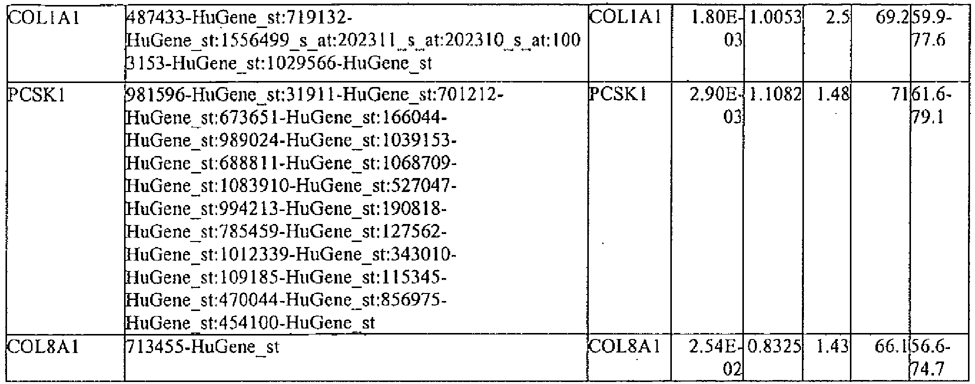

**TABLE 5**

| TargetPS | Symbol | Signif. FDR | D.val5 | FC | Sens-Spec | CI (95) |
|---|---|---|---|---|---|---|
| 213106_at | ATP8A1 | 6.60E-08 | 2.2884 | 3.11 | 87.4 | 76.9-94.1 |
| 210107_at | CLCA1 | 3.19E-07 | 2.1573 | 9.98 | 86 | 75.2-93.1 |
| 204811_s_at | CACNA2D2 | 3.61E-05 | 1.659 | 4.41 | 79.7 | 67.7-88.5 |
| 223969_s_at | RETNLB | 4.3968E-05 | 1.6581 | 11.64 | 79.6 | 67.7-88.5 |
| 223970_at | RETNLB | 4.3968E-05 | 1.6576 | 11.64 | 79.6 | 67.8-88.5 |
| 228232_s_at | VSIG2 | 0.0001 | 1.5718 | 2.4 | 78.4 | 66.3-87.6 |
| 242601_at | LOC253012 | 0.0001 | 1.5777 | 5.28 | 78.5 | 66.5-87.6 |
| 227719_at | No Symbol | 0.0002 | 1.642 | 1.98 | 79.4 | 67.4-88.3 |
| 237521_x_at | No Symbol | 0.0002 | 1.6433 | 1.98 | 79.4 | 67.5-88.3 |
| 203240_at | FCGBP | 3.00E-04 | 1.5005 | 3.07 | 77.3 | 65.2-86.7 |
| 204897_at | PTGER4 | 6.00E-04 | 1.4732 | 1.55 | 76.9 | 64.7-86.4 |
| 227676_at | FAM3D | 0.001 | 1.2706 | 2.15 | 73.7 | 61.3-83.8 |
| 205765 at | CYP3A5 | 1.20E-03 | 1.5279 | 1.8 | 77.8 | 65.6-87.1 |
| 232176_at | SLITRK6 | 0.0016 | 1.3618 | 5.96 | 75.2 | 62.8-85 |
| 232481_s_at | SLITRK6 | 0.0016 | 1.3607 | 5.96 | 75.2 | 62.9-85 |
| 235976_at | SLITRK6 | 0.0016 | 1.3648 | 5.96 | 75.3 | 62.9-84.9 |
| 221874_at | KIAA1324 | 3.80E-03 | 1.325 | 2.1 | 74.6 | 62.2-84.5 |
| 226248_s_at | KIAA1324 | 0.0038 | 1.3246 | 2.1 | 74.6 | 62.2-84.6 |
| 204607_at | HMGCS2 | 5.20E-03 | 1.2354 | 3.59 | 73.2 | 60.7-83.3 |
| 203963_at | CA12 | 6.50E-03 | 1.1828 | 1.86 | 72.3 | 59.7-82.6 |
| 204508_s_at | CA12 | 6.50E-03 | 1.1861 | 1.86 | 72.3 | 59.8-82.6 |
| 215867_x_at | CA12 | 6.50E-03 | 1.1858 | 1.86 | 72.3 | 59.7-82.6 |
| 227725_at | ST6GALNAC1 | 0.0068 | 1.1419 | 1.76 | 71.6 | 59-82 |
| 200884_at | CKB | 7.30E-03 | 1.1481 | 2.25 | 71.7 | 59.1-82.1 |
| 219955_at | L1TD1 | 7.70E-03 | 1.2502 | 3.27 | 73.4 | 60.9-83.5 |
| 236894_at | L1TD1 | 0.0077 | 1.2465 | 3.27 | 73.3 | 60.9-83.5 |
| 205259_at | NR3C2 | 1.29E-02 | 1.1933 | 1.63 | 72.5 | 60.1-82.8 |
| 218211_s_at | MLPH | 1.38E-02 | 1.2269 | 1.59 | 73 | 60.5-83.2 |
| 214234_s_at | CYP3A5P2 | 1.94E-02 | 1.098 | 1.7 | 70.8 | 58.1-81.3 |
| 214235_at | CYP3A5P2 | 1.94E-02 | 1.0976 | 1.7 | 70.8 | 58.2-81.3 |
| 204895_x_at | MUC4:TAF5L:LOC650855:L OC645744 | 4.53E-02 | 1.1998 | 1.31 | 72.6 | 60.1-82.8 |
| 217109_at | MUC4:TAF5L:LOC650855:L OC645744 | 4.53E-02 | 1.2028 | 1.31 | 72.6 | 60.2-82.8 |
| 217110_s_at | MUC4:TAF5L:LOC650855:L OC645744 | 4.53E-02 | 1.198 | 1.31 | 72.5 | 60-82.8 |
| 221841_s_at | KLF4 | 7.31E-02 | 1.0009 | 1.58 | 69.2 | 56.5-79.9 |
| 215125_s_at | UGT1A9 | 7.37E-02 | 1.0664 | 1.28 | 70.3 | 57.6-80.9 |
| 208063_s_at | CAPN9 | 7.40E-02 | 0.998 | 1.84 | 69.1 | 56.4-79.9 |
| 214433_s_at | SELENBP1 | 9.02E-02 | 0.9783 | 2 | 68.8 | 56-79.6 |
| 226302_at | ATP8B1 | 0.093 | 1.039 | 1.4 | 69.8 | 57.2-80.5 |
| 231832_at | WDR51B | 0.1284 | 1.0008 | 1.28 | 69.2 | 56,5-79.9 |
| 219543_at | PBLD | 1.66E-01 | 0.8734 | 1.89 | 66.9 | 54.2-77.9 |
| 208937_s_at | ID1 | 2.34E-01 | 0.7639 | 1.66 | 64.9 | 52.1-76.2 |
| 205927_s_at | CTSE | 2.81E-01 | 0.7393 | 3.23 | 64.4 | 51.6-75.8 |
| 229070_at | C6orf105 | 0.7794 | 0.7027 | 1.37 | 63.7 | 50.9-75.2 |

**TABLE 6**

| **TargetPS** | **Symbol** | **Signif. FDR** | **D.val5** | **FC** | **Sens-Spec** | **CI (95)** |
|---|---|---|---|---|---|---|
| 200665_s_at | SPARC | 3.05E-09 | 2.3771 | 3.24 | 88.3 | 78-94.6 |
| 212667_at | SPARC | 3.0479E-09 | 2.3752 | 3.24 | 88.3 | 78.1-94.6 |
| 211964_at | COL4A2 | 5.5315E-09 | 2.4316 | 2.87 | 88.8 | 78.8-95 |
| 211966_at | COL4A2 | 5.5315E-09 | 2.4321 | 2.87 | 88.8 | 78.8-95 |
| 211980_at | COL4A1 | 1.243E-08 | 2.3031 | 3.03 | 87.5 | 77.2-94.2 |
| 211981_at | COL4A1 | 1.243E-08 | 2.3045 | 3.03 | 87.5 | 77.2-94.2 |
| 218638_s_at | SPON2 | 1.9471E-08 | 2.3321 | 3.38 | 87.8 | 77.5-94.3 |
| 221729_at | COL5A2 | 5.7658E-08 | 2.2145 | 3.31 | 86.6 | 76-93.5 |
| 221730_at | COL5A2 | 5.7658E-08 | 2.2138 | 3.31 | 86.6 | 75.9-93.6 |
| 208782_at | FSTL1 | 7.8434E-08 | 2.228 | 2.64 | 86.7 | 76.1-93.7 |
| 201261_x_at | BGN | 9.6881E-08 | 2.2253 | 2.99 | 86.7 | 76-93.6 |
| 213905_x_at | BGN | 9.6881E-08 | 2.2232 | 2.99 | 86.7 | 76.1-93.6 |
| 209955_s_at | IFIH1:FAP | 1.358E-07 | 2.2261 | 4.88 | 86.7 | 76.2-93.6 |
| 226237_at | COL8A1 | 3.6053E-07 | 2.1456 | 3.09 | 85.8 | 75-93 |
| 202310_s_at | COL1A1 | 5.44E-07 | 2.0121 | 3.29 | 84.3 | 73-92 |
| 202311_s_at | COL1A1 | 5.44E-07 | 2.0131 | 3.29 | 84.3 | 73.1-92 |
| 217430_x_at | COL1A1 | 5.4426E-07 | 2.015 | 3.29 | 84.3 | 73.2-92 |
| 201438_at | COL6A3 | 5.7026E-07 | 2.0286 | 2.73 | 84.5 | 73.5-92.1 |
| 202403_s_at | COL1A2:LOC728628 | 8.85E-07 | 2.0609 | 2.62 | 84.9 | 73.9-92.4 |
| 202404_s_at | COL1A2:LOC728628 | 8.85E-07 | 2.0621 | 2.62 | 84.9 | 73.7-92.4 |
| 229218_at | COL1A2:LOC728628 | 8.8466E-07 | 2.0618 | 2.62 | 84.9 | 73.9-92.4 |
| 208850_s_at | THY1 | 1.0326E-06 | 1.9159 | 3.34 | 83.1 | 71.8-91.1 |
| 208851_s_at | THY1 | 1.0326E-06 | 1.915 | 3.34 | 83.1 | 71.7-91.1 |
| 213869_x_at | THY1 | 1.0326E-06 | 1.9132 | 3.34 | 83.1 | 71.7-91.1 |
| 203477_at | COL15A1 | 1.0611E-06 | 2.0121 | 3.37 | 84.3 | 73.2-91.9 |
| 201616_s_at | CALD1 | 1.11E-06 | 2.1136 | 1.7 | 85.5 | 74.6-92.8 |
| 212077_at | CALD1 | 1.1059E-06 | 2.1131 | 1.7 | 85.5 | 74.5-92.8 |
| 201162_at | IGFBP7 | 1.1574E-06 | 2.0016 | 1.91 | 84.2 | 72.9-91.8 |
| 201163_s_at | IGFBP7 | 1.16E-06 | 1.999 | 1.91 | 84.1 | 73-91.9 |
| 210511_s_at | INHBA | 1.1943E-06 | 2.0027 | 3.38 | 84.2 | 73-91.9 |
| 225664_at | TMEM30A:COL12A1 | 1.2492E-06 | 2.0313 | 4.16 | 84.5 | 73.4-92.1 |
| 231766_s_at | TMEM30A:COL12A1 | 1.2492E-06 | 2.0303 | 4.16 | 84.5 | 73.4-92.1 |
| 231879 at | TMEM30A:COL12A1 | 1.2492E-06 | 2.0325 | 4.16 | 84.5 | 73.5-92.1 |
| 203325_s_at | COL5A1 | 1.6076E-06 | 1.934 | 2.75 | 83.3 | 72.1-91.3 |
| 212488_at | COL5A1 | 1.6076E-06 | 1.9342 | 2.75 | 83.3 | 72.1-91.3 |
| 212489_at | COL5A1 | 1.6076E-06 | 1.9339 | 2.75 | 83.3 | 72-91.2 |
| 203083_at | THBS2 | 2.3382E-06 | 1.9081 | 6.5 | 83 | 71.6-91 |
| 202291_s_at | MGP:C12orf46 | 2.50E-06 | 1.9993 | 2.62 | 84.1 | 73-91.9 |
| 201645_at | TNC | 3.74E-06 | 1.9608 | 2.14 | 83.7 | 72.5-91.5 |
| 201852_x_at | COL3A1 | 9.29E-06 | 1.8472 | 2.32 | 82.2 | 70.7-90.5 |
| 211161_s_at | COL3A1 | 9.2906E-06 | 1.8501 | 2.32 | 82.3 | 70.7-90.5 |
| 215076_s_at | COL3A1 | 9.2906E-06 | 1.8495 | 2.32 | 82.2 | 70.7-90.5 |
| 232458_at | COL3A1 | 9.2906E-06 | 1.8473 | 2.32 | 82.2 | 70.7-90.5 |
| 212344_at | SULF1 | 1.4436E-05 | 1.7528 | 2.84 | 81 | 69.3-89.5 |
| 212353_at | SULF1 | 1.4436E-05 | 1.7555 | 2.84 | 81 | 69.2-89.5 |
| 212354_at | SULF1 | 1.4436E-05 | 1.7521 | 2.84 | 80.9 | 69.3-89.5 |
| 201185_at | HTRA1 | 1.7178E-05 | 1.7346 | 2.29 | 80.7 | 68.9-89.3 |
| 202998_s_at | ENTPD4:LOXL2 | 1.99E-05 | 1.7754 | 2.45 | 81.3 | 69.6-89.7 |
| 224724_at | SULF2 | 2.0766E-05 | 1.6837 | 2.25 | 80 | 68.2-88.7 |
| 233555_s_at | SULF2 | 2.0766E-05 | 1.6795 | 2.25 | 79.9 | 68-88.7 |
| 201069_at | MMP2 | 2.5409E-05 | 1.7345 | 3.02 | 80.7 | 69-89.3 |
| 20H47_s_at | TIMP3 | 2.62E-05 | 1.7776 | 2.56 | 81.3 | 69.6-89.7 |
| 201150_s_at | TIMP3 | 2.62E-05 | 1.7774 | 2.56 | 81.3 | 69.6-89.7 |
| 209156_s_at | COL6A2 | 2.9551E-05 | 1.7373 | 1.67 | 80.7 | 69-89.4 |
| 227099_s_at | LOC387763 | 0.00003052 | 1.8968 | 1.43 | 82.9 | 71.4-90.9 |
| 214247_s_at | DKK3 | 3.1838E-05 | 1.7434 | 1.86 | 80.8 | 69.1-89.5 |
| 202450_s_at | CTSK | 3.98E-05 | 1.7153 | 2.16 | 80.4 | 68.7-89.1 |
| 225799_at | MGC4677,MGC4677:LOC5414 71 | 4.1139E-05 | 1.8003 | 1.45 | 81.6 | 70-90 |
| 209395_at | CHI3L1 :MYBPH | 5.1515E-05 | 1.6358 | 3.06 | 79.3 | 67.4-88.2 |
| 209396_s_at | CHI3L1:MYBPH | 5.1515E-05 | 1.6352 | 3.06 | 79.3 | 67.4-88.2 |
| 202878_s_at | CD93 | 5.19E-05 | 1.6674 | 2.36 | 79.8 | 67.9-88.6 |
| 204320_at | COL11A1 | 5.3391E-05 | 1.7594 | 2.55 | 81 | 69.3-89.6 |
| 37892_at | COL11A1 | 5.3391E-05 | 1.7556 | 2.55 | 81 | 69.3-89.6 |
| 221011_s_at | LBH | 6.3299E-05 | 1.6771 | 1.9 | 79.9 | 68.1-88.7 |
| 213125_at | OLFML2B | 6.6557E-05 | 1.7057 | 1.97 | 80.3 | 68.5-89 |
| 204475_at | MMP1 | 7.0616E-05 | 1.6759 | 3.91 | 79.9 | 68-88.8 |
| 226694_at | AKAP2:PALM2:PALM2-AKAP2 | 7.4206E-05 | 1.7231 | 1.58 | 80.6 | 68.8-89.2 |
| 202766_s_at | FBN1 | 0.0001 | 1.5984 | 1.48 | 78.8 | 66.7-87.9 |
| 205828_at | MMP3 | 0.0001 | 1.6223 | 6.68 | 79.1 | 67.3-88.1 |
| 207191_s_at | ISLR | 0.0001 | 1.6448 | 1.87 | 79.5 | 67.5-88.4 |
| 213428_s_at | COL6A1 | 0.0001 | 1.7301 | 1.58 | 80.6 | 68.8-89.3 |
| 226930_at | FNDC1 | 0.0001 | 1.521 | 3.14 | 77.7 | 65.6-87 |
| 201792_at | AEBP1 | 0.0002 | 1.4772 | 2.11 | 77 | 64.8-86.5 |
| 203878_s_at | MMP11 | 0.0002 | 1.6971 | 1.28 | 80.2 | 68.4-88.9 |
| 211959_at | IGFBP5 | 0.0002 | 1.5662 | 3.96 | 78.3 | 66.3-87.5 |
| 225710_at | No Symbol | 0.0002 | 1.6426 | 1.98 | 79.4 | 67.5-88.4 |
| 226311_at | No Symbol | 0.0002 | 1.6436 | 1.98 | 79.4 | 67.5-88.3 |
| 226777_at | No Symbol | 0.0002 | 1.645 | 1.98 | 79.5 | 67.5-88.4 |
| 227140_at | No Symbol | 0.0002 | 1.6427 | 1.98 | 79.4 | 67.6-88.3 |
| 229802_at | No Symbol | 0.0002 | 1.6421 | 1.98 | 79.4 | 67.5-88.3 |
| 205479_s_at | PLAU | 0.0003 | 1.484 | 2.11 | 77.1 | 64.9-86.5 |
| 210495_x_at | FN1 | 0.0003 | 1.494 | 2.87 | 77.2 | 65.1-86.6 |
| 211719_x_at | FN1 | 0.0003 | 1.4894 | 2.87 | 77.2 | 65.1-86.7 |
| 212464_s_at | PN1 | 0.0003 | 1.493 | 2.87 | 77.2 | 65-86.7 |
| 216442_x_at | FN1 | 0.0003 | 1.4935 | 2.87 | 77.2 | 65-86.6 |
| 217762_s_at | RAB31 | 0.0003 | 1.4864 | 2.08 | 77.1 | 65-86.6 |
| 217763_s_at | RAB31 | 0.0003 | 1.4848 | 2.08 | 77.1 | 64.9-86.6 |
| 217764_s_at | RAB31 | 0.0003 | 1.4882 | 2.08 | 77.2 | 65-86.5 |
| 225681_at | CTHRC1 | 0.0003 | 1.4588 | 3.41 | 76.7 | 64.5-86.3 |
| 201105_at | LGALS1 | 0.0004 | 1.4844 | 1.61 | 77.1 | 64.9-86.6 |
| 208788_at | ELOVL5 | 0.0004 | 1.5418 | 1.86 | 78 | 65.8-87.2 |
| 224694_at | ANTXR1 | 0.0005 | 1.52 | 2 | 77.6 | 65.5-86.9 |
| 200974_at | ACTA2 | 0.0006 | 1.4251 | 1.75 | 76.2 | 64-85.8 |
| 210095_s_at | IGFBP3 | 0.0006 | 1.5538 | 1.38 | 78.1 | 66-87.4 |
| 201426_s_at | VIM | 8.00E-04 | 1.4819 | 1.48 | 77.1 | 64.9-86.4 |
| 219087_at | ASPN | 0.0009 | 1.4046 | 2.75 | 75.9 | 63.6-85.5 |
| 227566_at | HNT | 0.001 | 1.4215 | 2.26 | 76.1 | 64-85.7 |
| 201667_at | GJA1 | 130E-03 | 1.4084 | 1.63 | 75.9 | 63.7-85.6 |
| 200600_at | MSN | 1.40E-03 | 1.4942 | 1.36 | 77.2 | 65-86.7 |
| 204051_s_at | SFRP4 | 0.0015 | 1.4056 | 2.2 | 75.9 | 63.6-85.6 |
| 209101_at | CTGF | 0.0015 | 1.3331 | 1.78 | 74.7 | 62.4-84.6 |
| 204620_s_at | CSPG2 | 0.0016 | 1.21 | 1.38 | 72.7 | 60.3-83 |
| 211571_s_at | CSPG2 | 0.0016 | 1.2123 | 1.38 | 72.8 | 60.1-82.9 |
| 215646_s_at | CSPG2 | 0.0016 | 1.2095 | 1.38 | 72.7 | 60.3-83 |
| 221731_x_at | CSPG2 | 0.0016 | 1.2115 | 1.38 | 72.8 | 60.3-83 |
| 204006_s_at | FCGR3B | 0.0026 | 1.2308 | 2.14 | 73.1 | 60.6-83.2 |
| 203570_at | LOXL1 | 0.0027 | 1.4048 | 1.33 | 75.9 | 63.7-85.6 |
| 201744_s_at | LUM | 2.90E-03 | 1.3418 | 2.18 | 74.9 | 62.5-84.7 |
| 202283_at | SERPINF1 | 3.10E-03 | 1.2121 | 1.76 | 72.8 | 60.2-83 |
| 209596_at | MXRA5 | 0.0034 | 1.2974 | 1.62 | 74.2 | 61.7-84.1 |
| 210809_s_at | POSTN | 0.005 | 1.3011 | 2.58 | 74.2 | 61.8-84.2 |
| 205547_s_at | TAGLN | 0.0051 | 1.1829 | 2.19 | 72.3 | 59.7-82.6 |
| 202237_at | NNMT | 5.40E-03 | 1.223 | 1.44 | 73 | 60.4-83.1 |
| 202238_s_at | NNMT | 5.40E-03 | 1.2259 | 1.44 | 73 | 60.5-83.1 |
| 218468_s_at | GREM1 | 0.007 | 1.3017 | 1.31 | 74.2 | 61.8-84.2 |
| 218469_at | GREM1 | 0.007 | 1.3027 | 1.31 | 74.3 | 61.9-84.2 |
| 208747_s_at | C1S | 0.0088 | 1.3575 | 1.77 | 75.1 | 62.9-85 |
| 224560_at | TIMP2 | 0.0097 | 1.1629 | 1.72 | 72 | 59.4-82.3 |
| 231579_s_at | TIMP2 | 0.0097 | 1.1641 | 1.72 | 72 | 59.5-82.4 |
| 209875_s_at | SPP1 | 0.0109 | 1.0673 | 3.91 | 70.3 | 57.6-80.9 |
| 202859_x_at | IL8 | 0.0112 | 1.3238 | 4.54 | 74.6 | 62.2-84.5 |
| 200832_s_at | SCD:LOC651109:LOC645313 | 1.27E-02 | 1.0749 | 2.33 | 70.5 | 57.9-81.1 |
| 201058_s_at | MYL9 | 0.0131 | 1.1765 | 1.37 | 72.2 | 59.6-82.5 |
| 203645_s_at | CD163 | 0.0146 | 1.1655 | 2.19 | 72 | 59.4-82.3 |
| 215049_x_at | CD163 | 0.0146 | 1.1664 | 2.19 | 72 | 59.4-82.3 |
| 202917_s_at | S100A8 | 1.48E-02 | 1.0979 | 3.17 | 70.8 | 58.3-81.4 |
| 201289_at | CYR61 | 0.015 | 1.0919 | 1.69 | 70.7 | 58.1-81.2 |
| 210764_s_at | CYR61 | 0.015 | 1.0938 | 1.69 | 70.8 | 58.2-81.2 |
| 218559_s_at | MAFB | 0.0182 | 1.162 | 1.48 | 71.9 | 59.3-82.3 |
| 203382_s_at | APOE | 0.0285 | 1.1124 | 1.43 | 71.1 | 58.5-81.6 |
| 201893_x_at | DCN | 3.64E-02 | 1.0821 | 1.29 | 70.6 | 58-81.1 |
| 211813_x_at | DCN | 0.0364 | 1.0838 | 1.29 | 70.6 | 57.8-81.1 |
| 211896_s_at | DCN | 0.0364 | 1.0814 | 1.29 | 70.6 | 58-81.1 |
| 213524_s_at | G0S2 | 0.0383 | 0.994 | 1.53 | 69 | 56.3-79.8 |
| 207173_x_at | CDH11 | 0.0384 | 1.0121 | 1.98 | 69.4 | 56.6-80.1 |
| 209218_at | SQLE | 0.041 | 1.0162 | 2.15 | 69.4 | 56.6-80.1 |
| 201141_at | GPNMB | 0.0623 | 1.0165 | 1.52 | 69.4 | 56.7-80.1 |
| 201859_at | PRG1 | 0.0634 | 0.798 | 1.34 | 65.5 | 52.6-76.7 |
| 234994_at | KIAA1913 | 0.1061 | 0.9579 | 1.2 | 68.4 | 55.7-79.3 |
| 223122_s_at | SFRP2 | 0.1336 | 0.968 | 1.24 | 68.6 | 55.9-79.4 |
| 223235_s_at | SMOC2 | 0.2175 | 0.6547 | 2.06 | 62.8 | 50-74.4 |
| 200986_at | SERPING1 | 0.2636 | 0.7831 | 1.26 | 65.2 | 52.4-76.4 |
| 201842_s_at | EFEMP1 | 4.91E-01 | 0.6821 | 1.27 | 63.3 | 50.5-74.8 |
| 204122_at | TYROBP | 0.4923 | 0.7066 | 1.23 | 63.8 | 51.1-75.3 |
| 202620_s_at | PLOD2 | 5.15E-01 | 0.7784 | 1.51 | 65.1 | 52.3-76.4 |

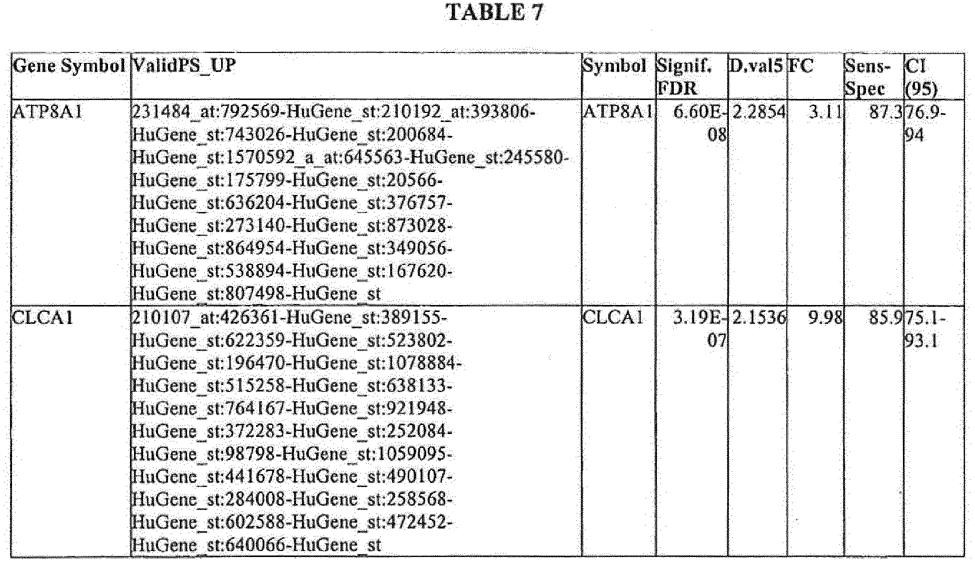

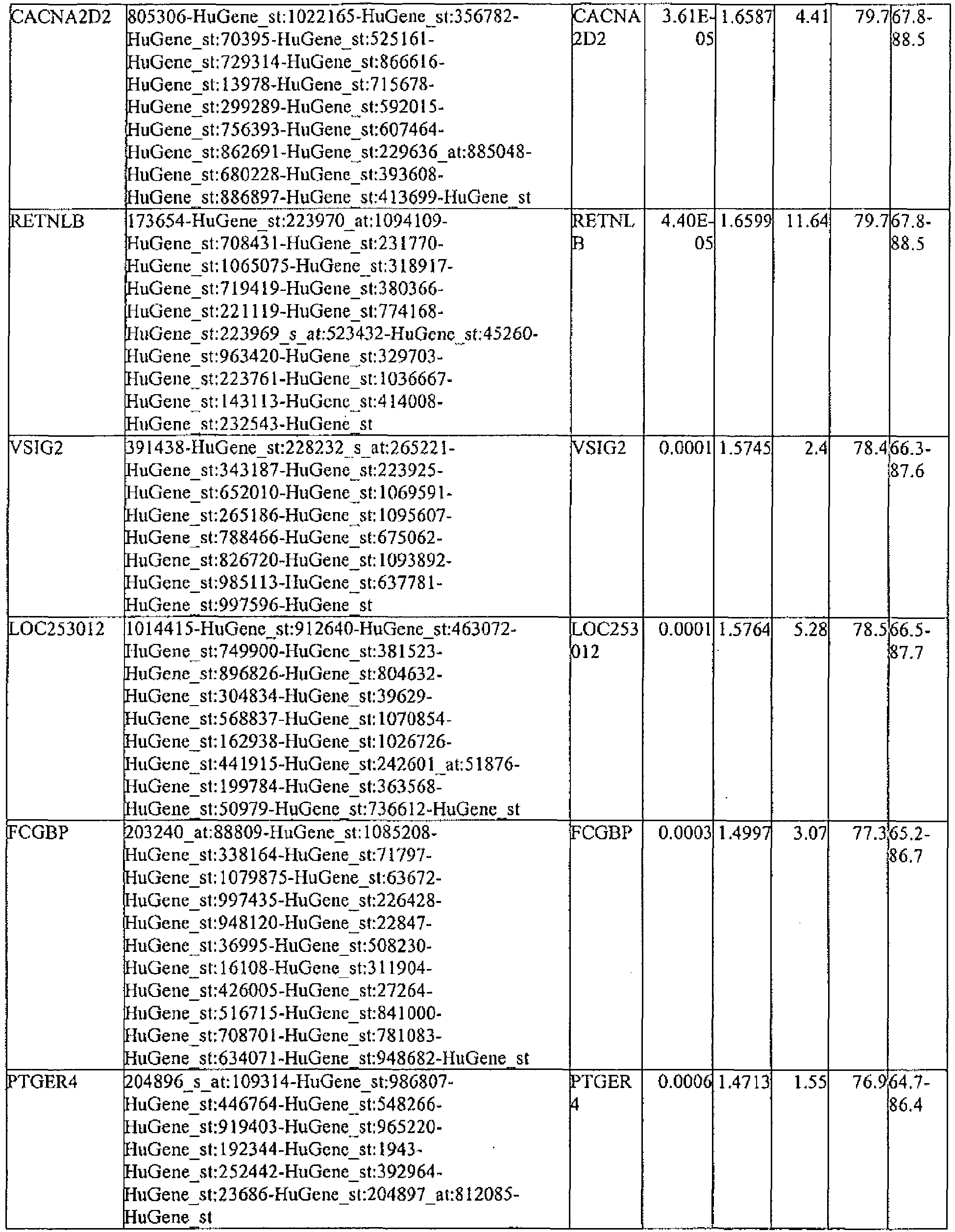

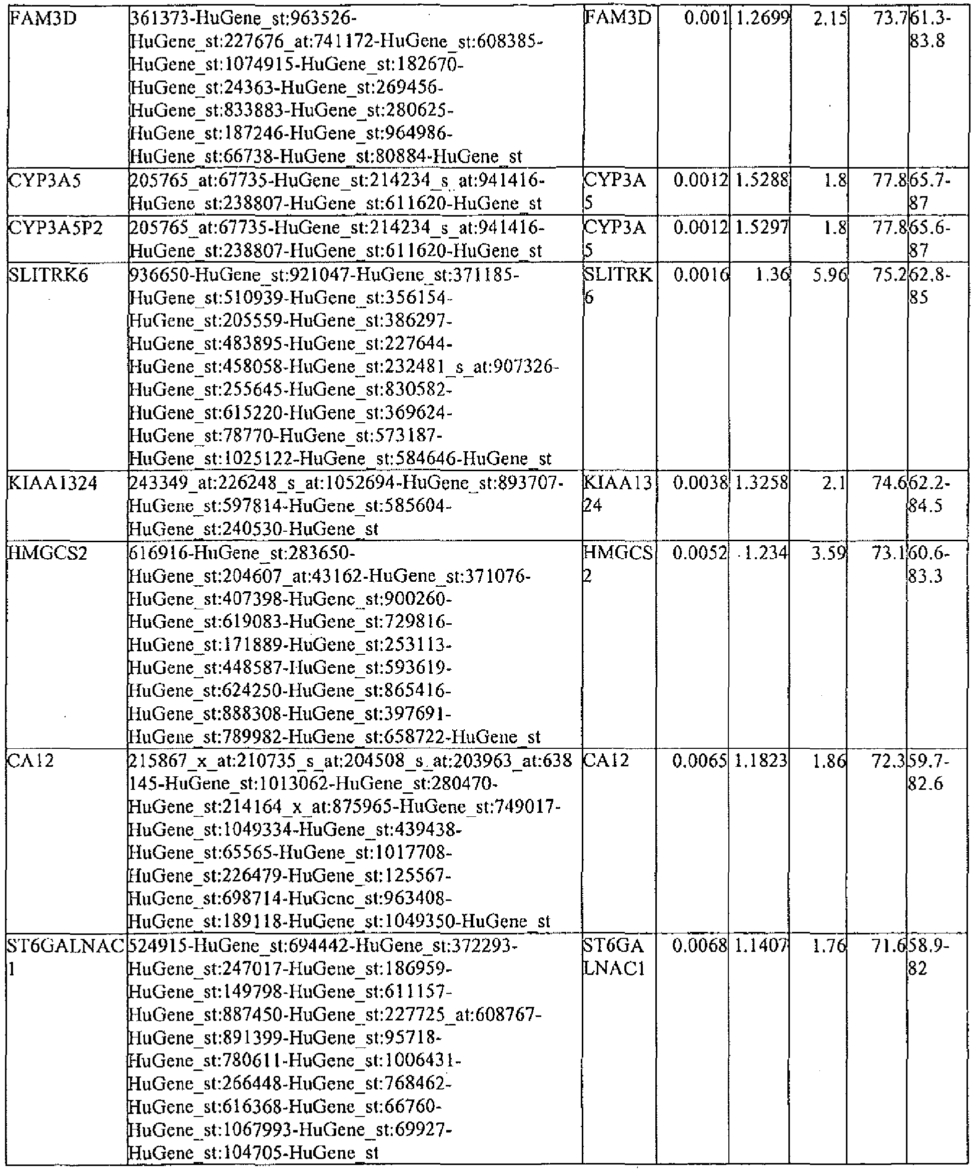

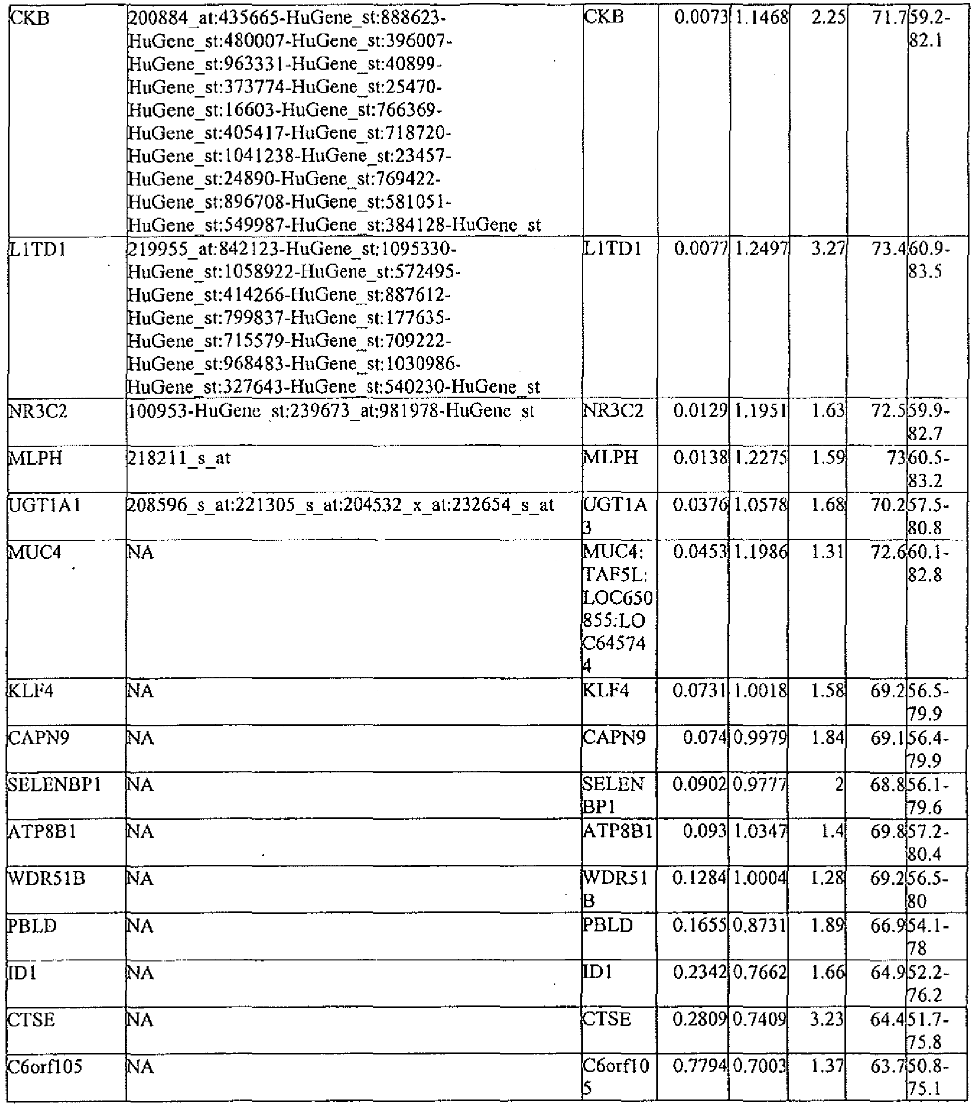

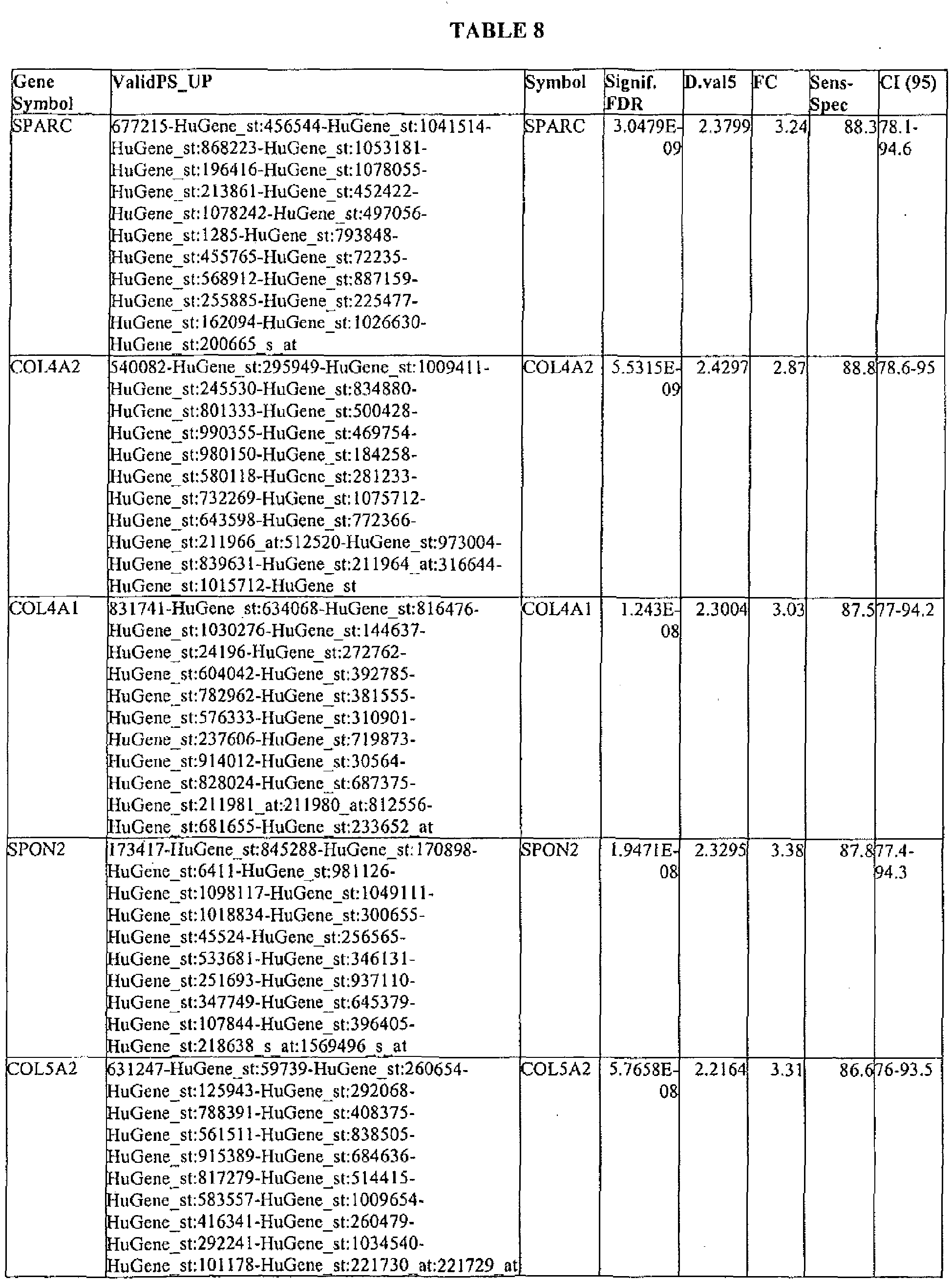

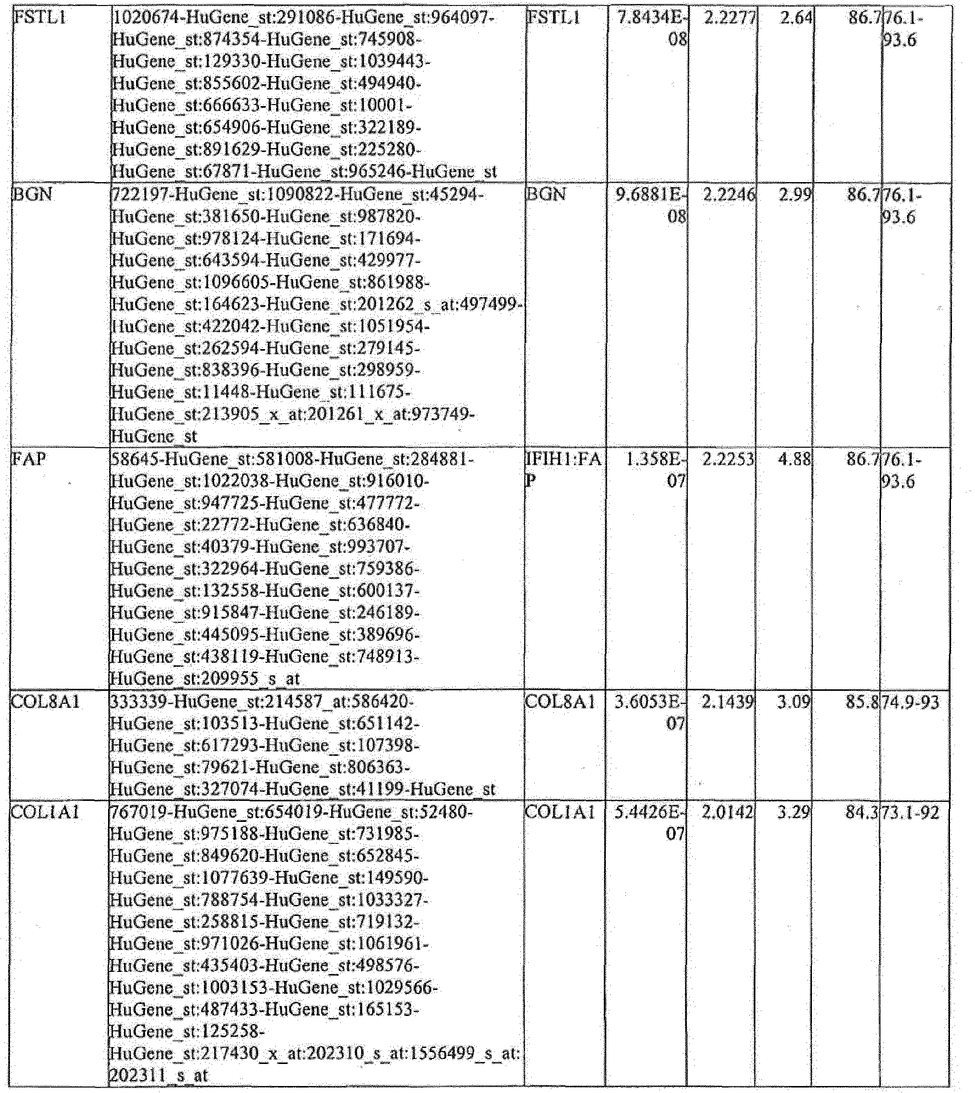

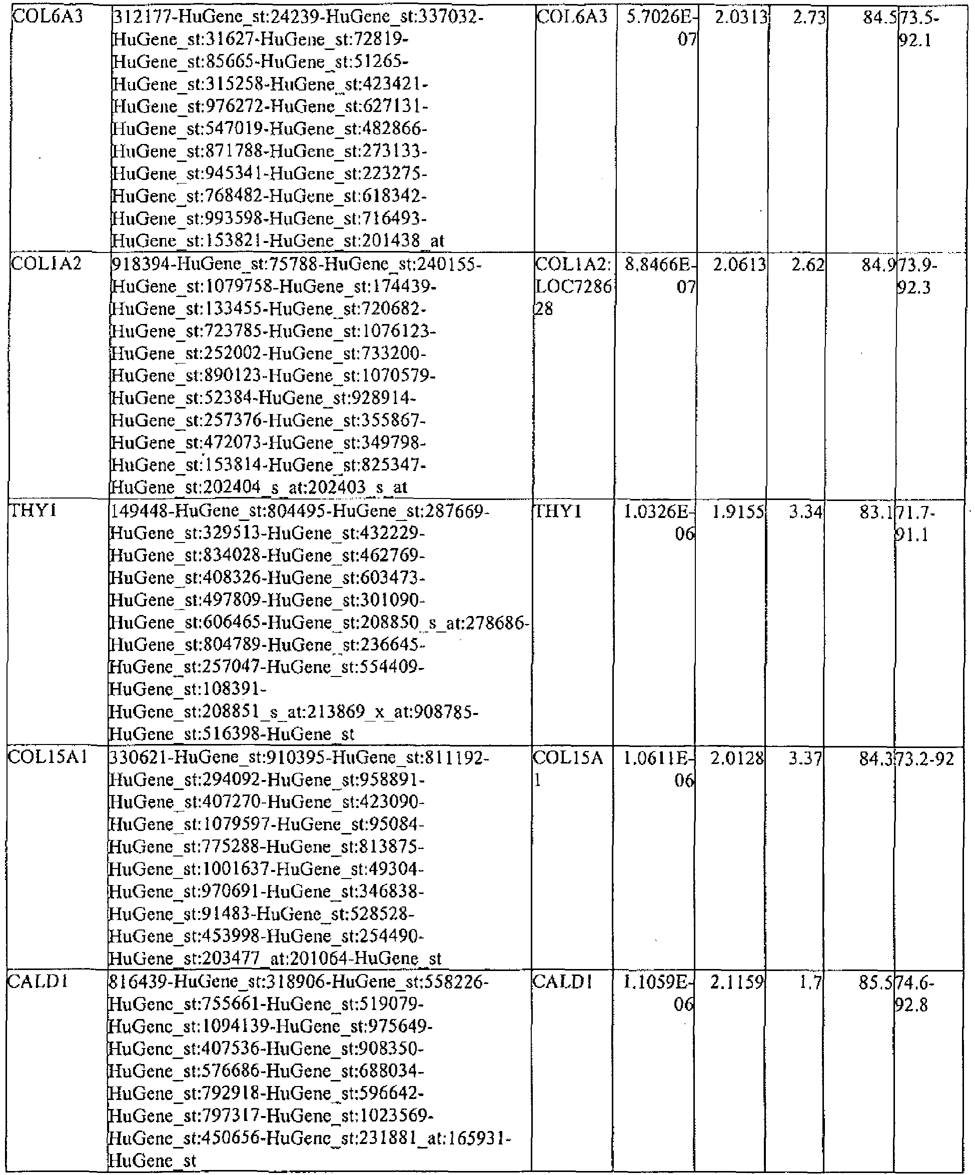

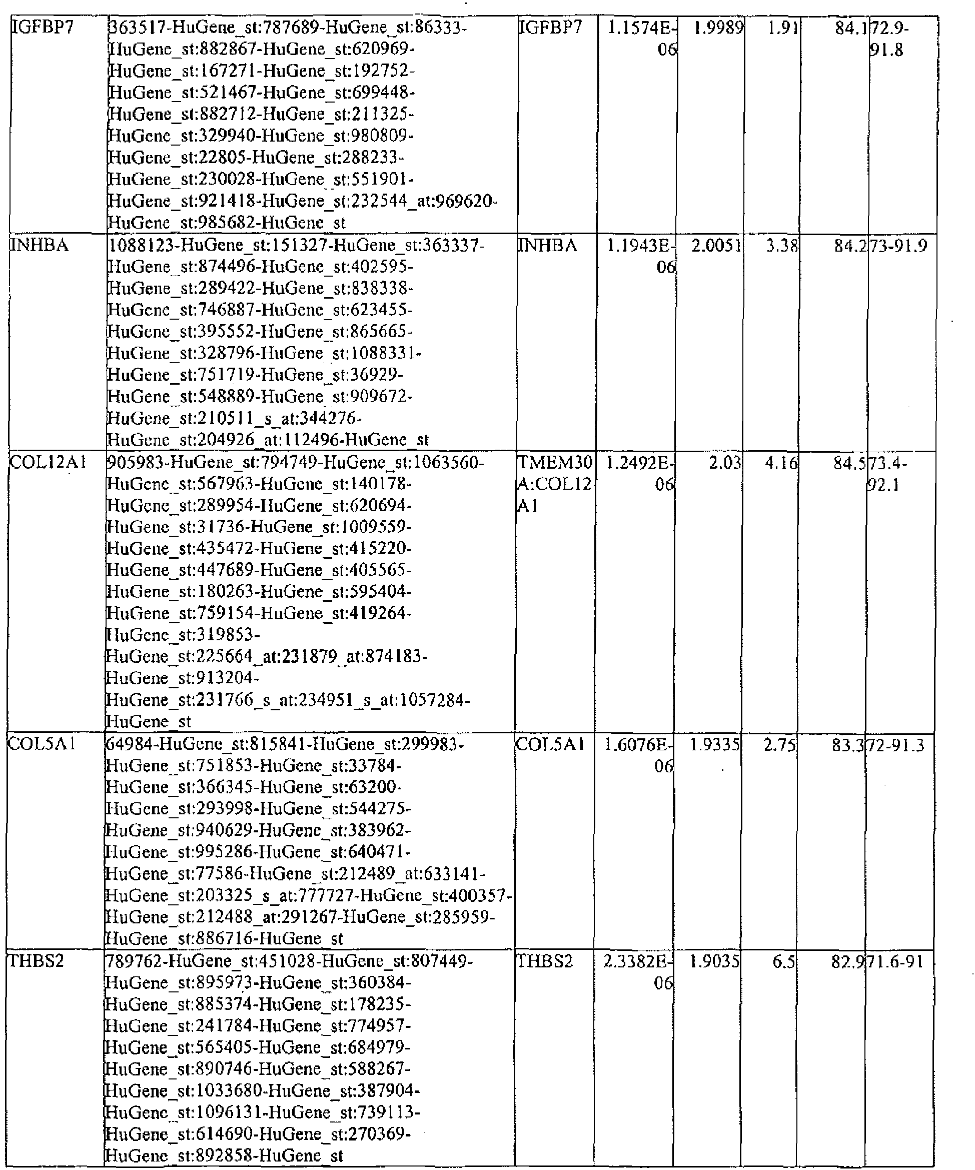

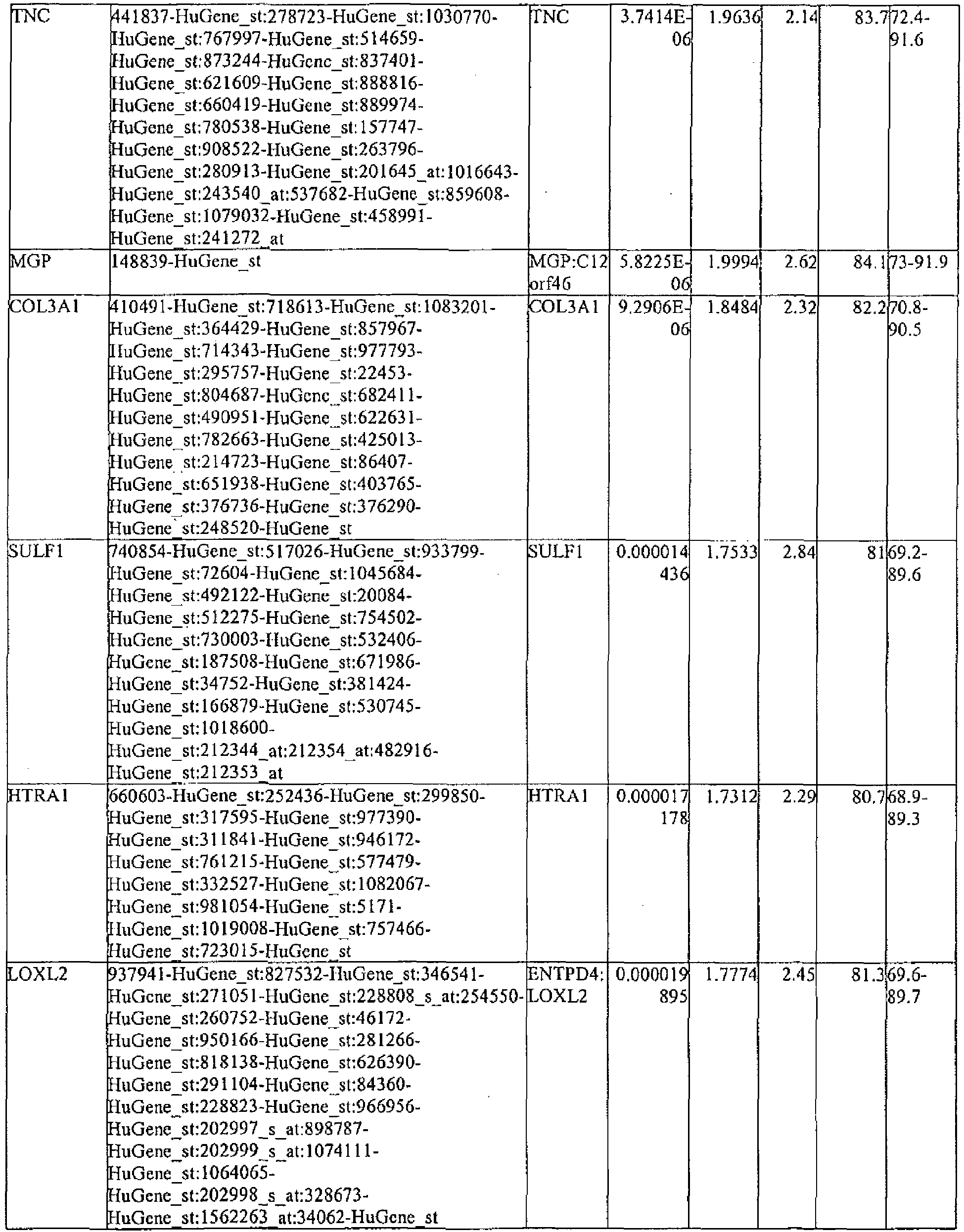

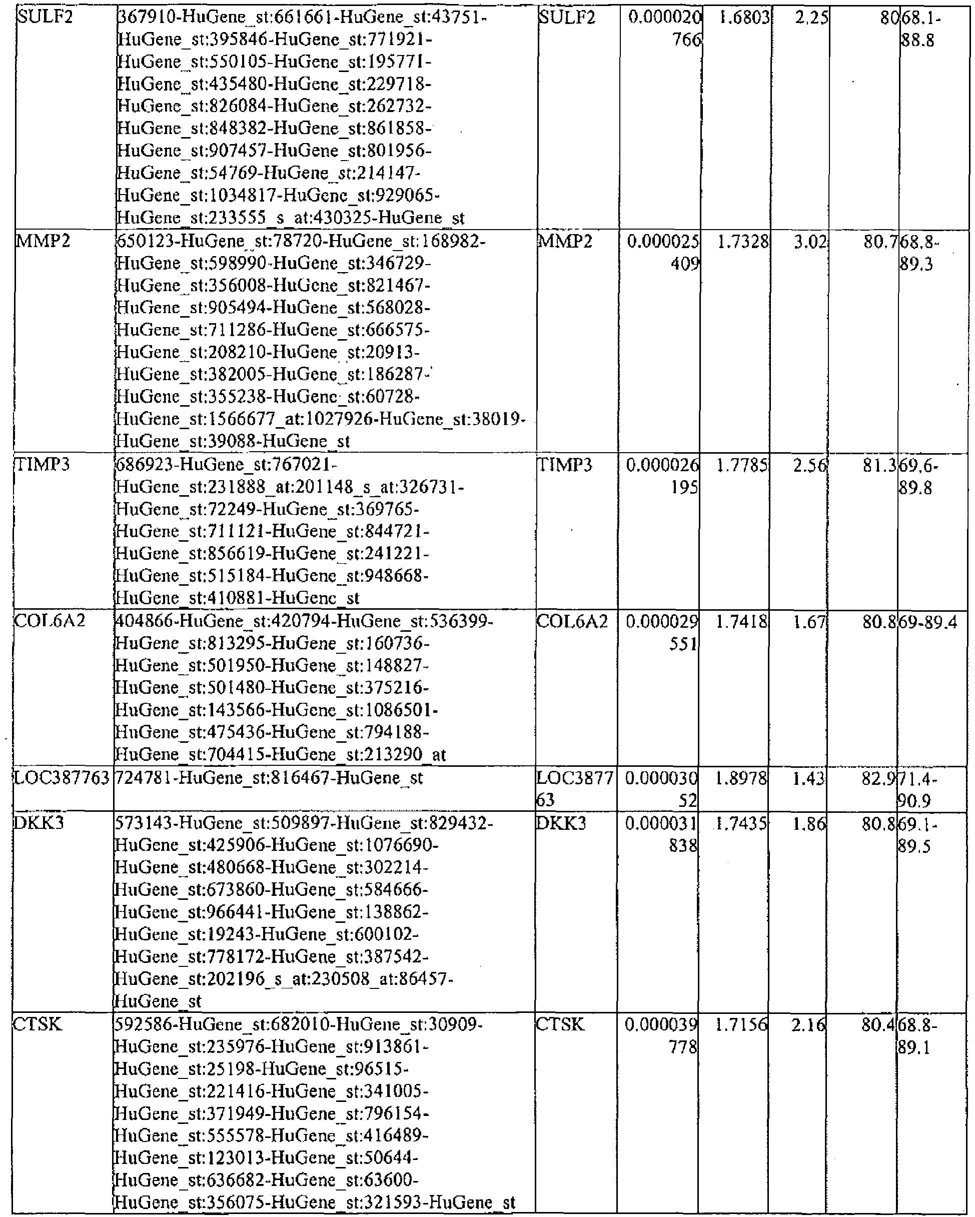

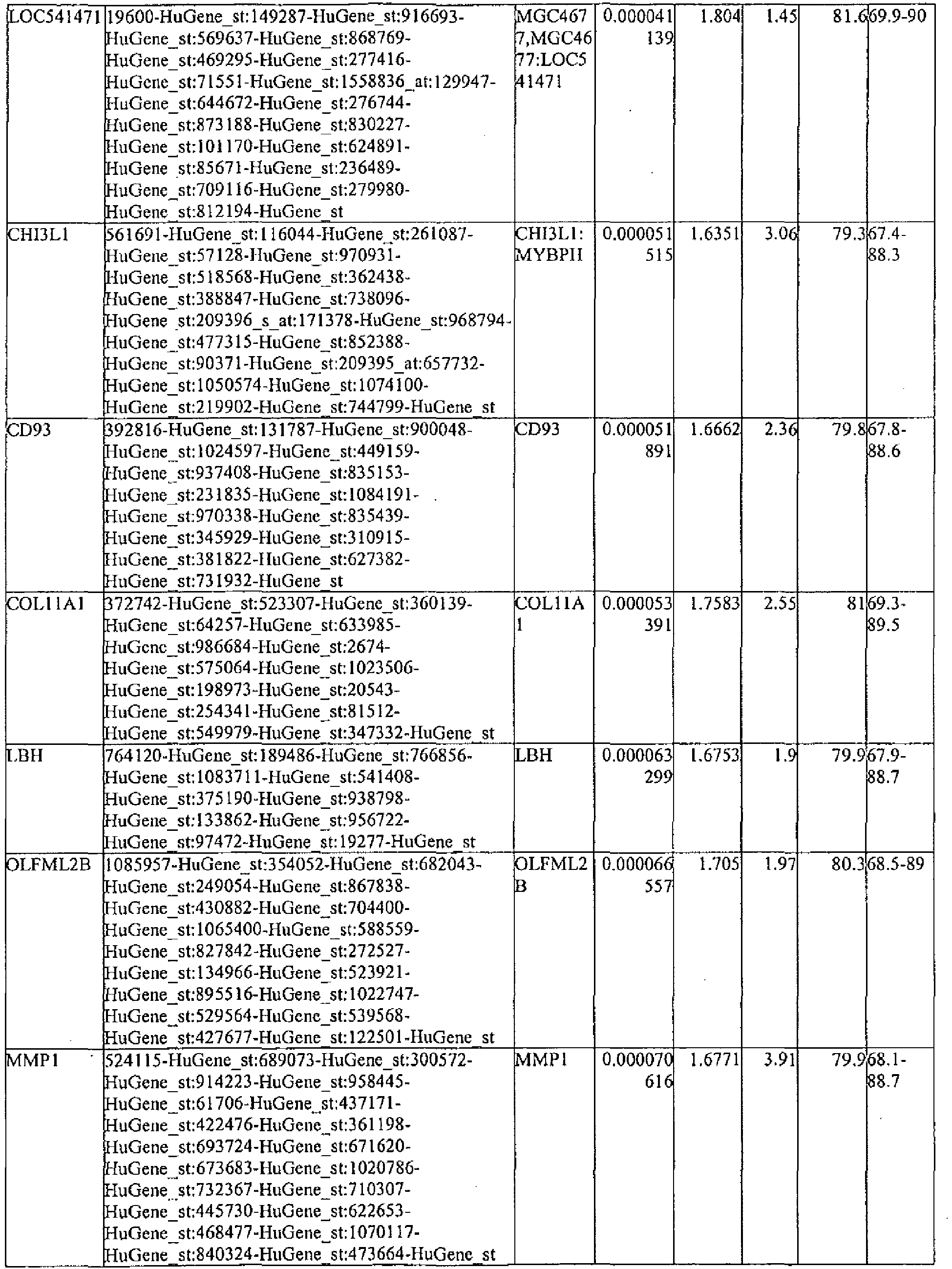

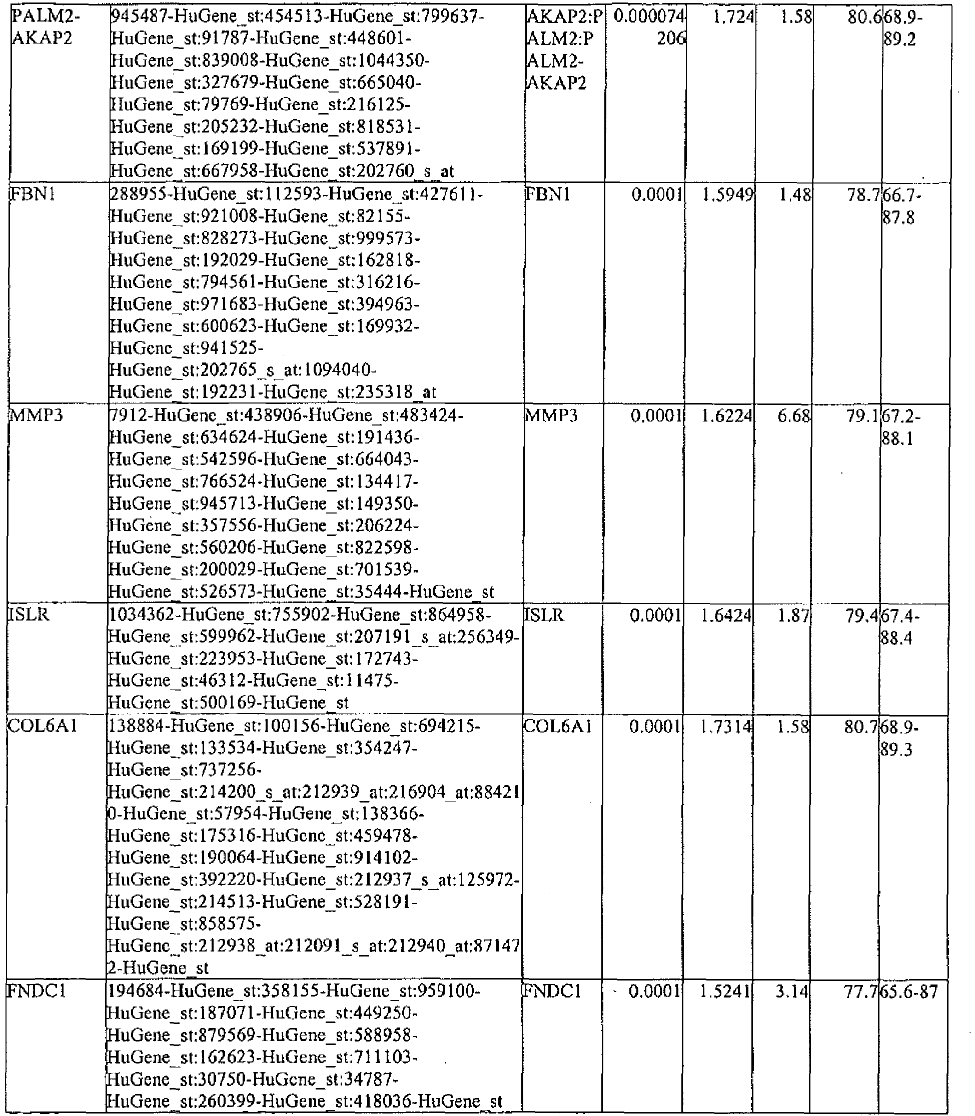

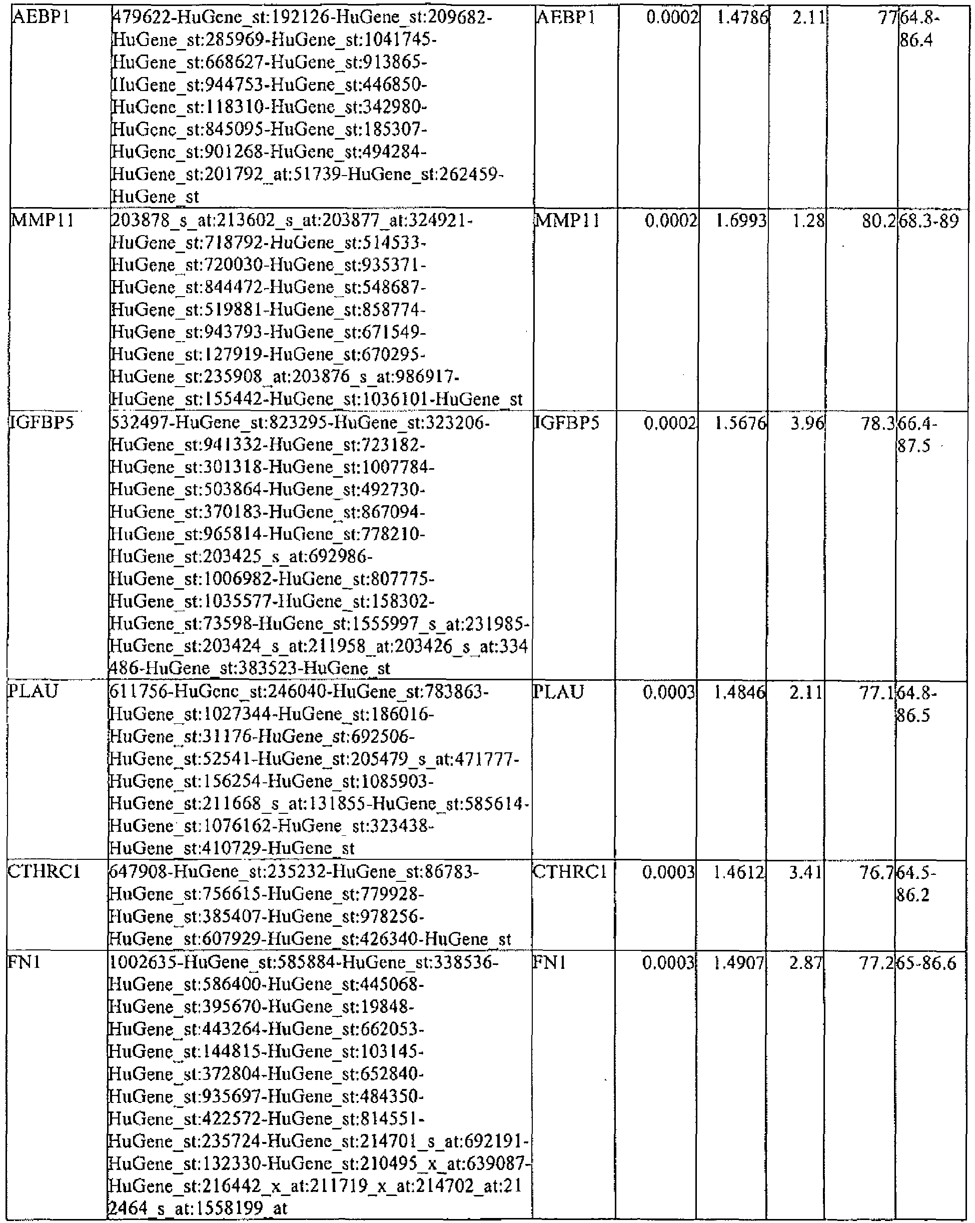

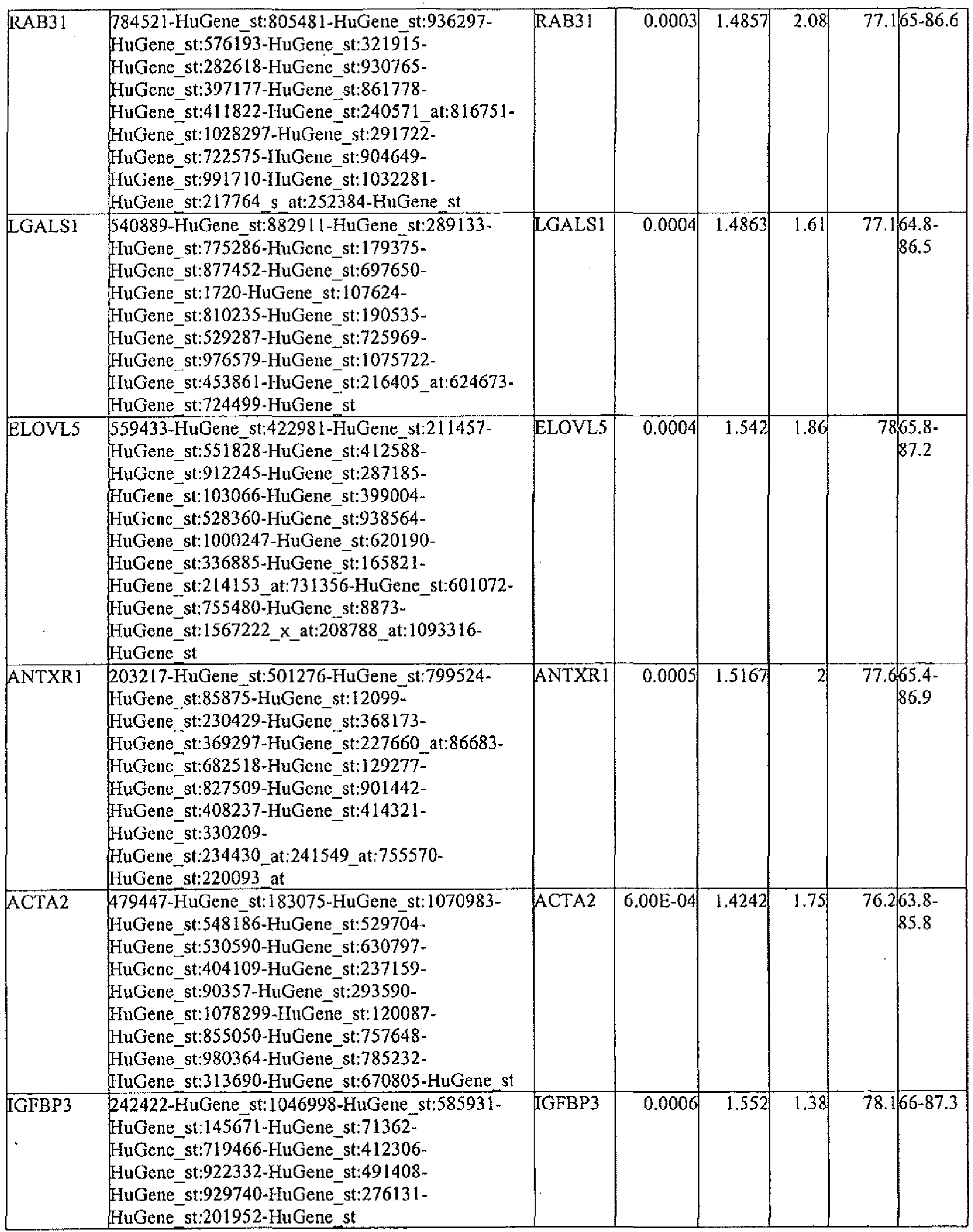

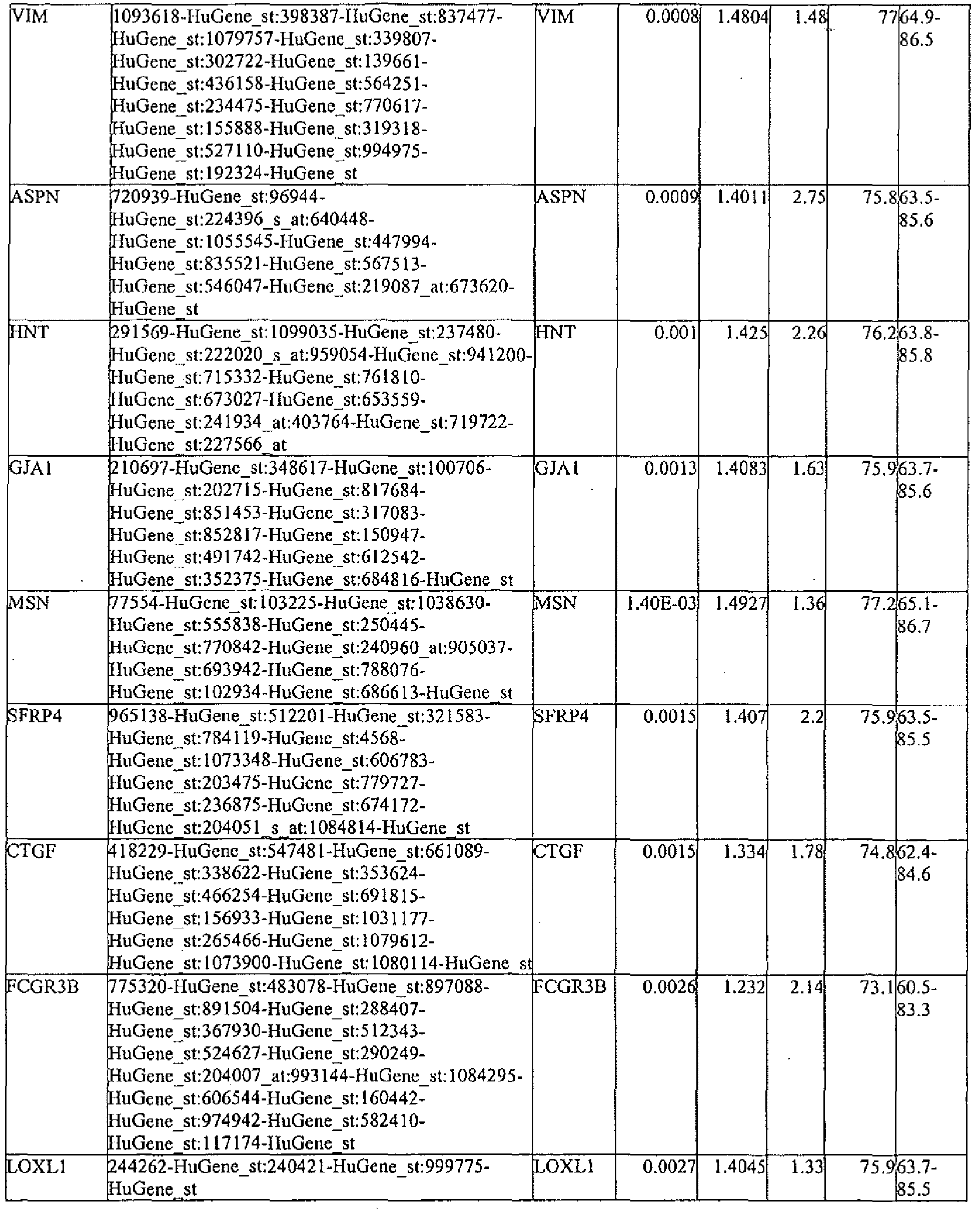

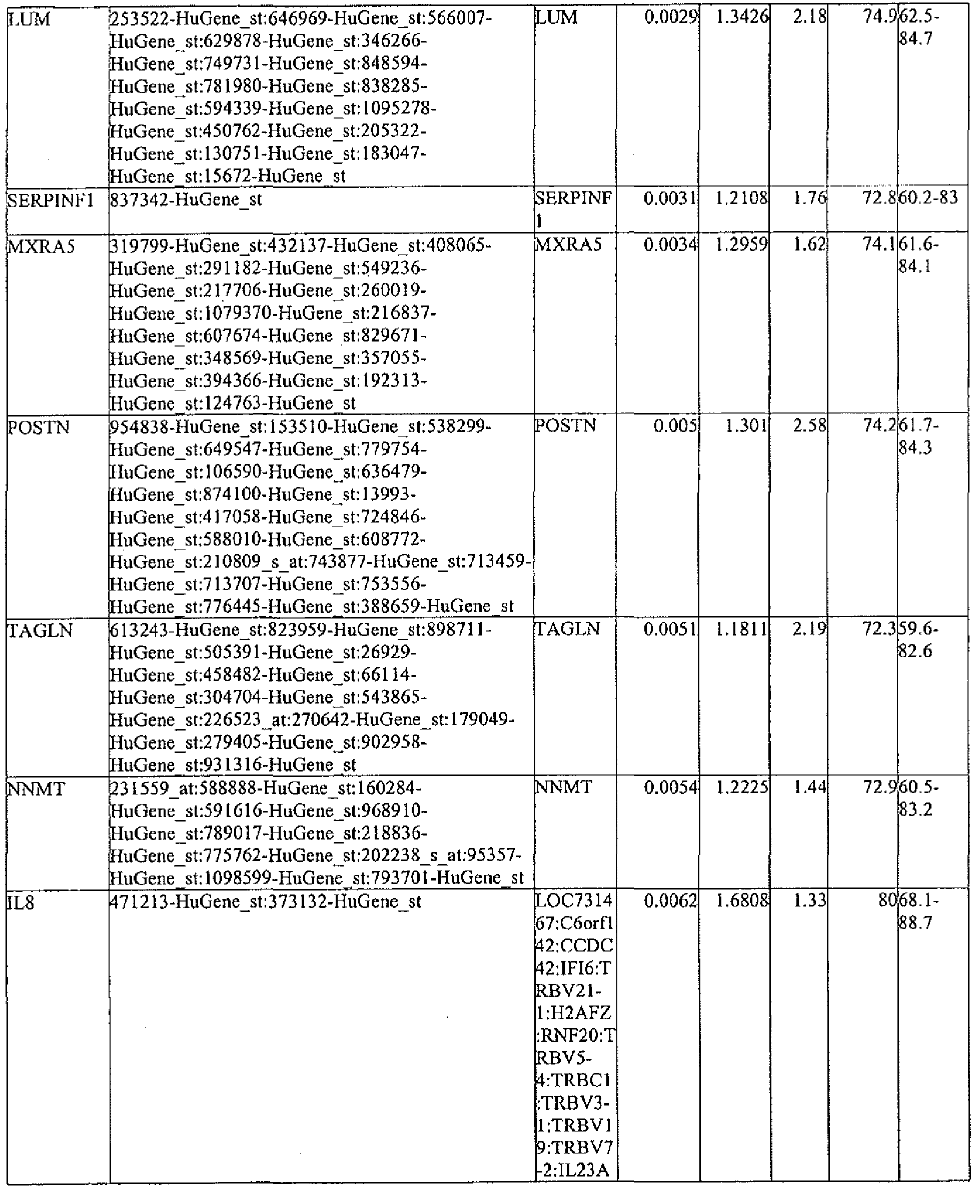

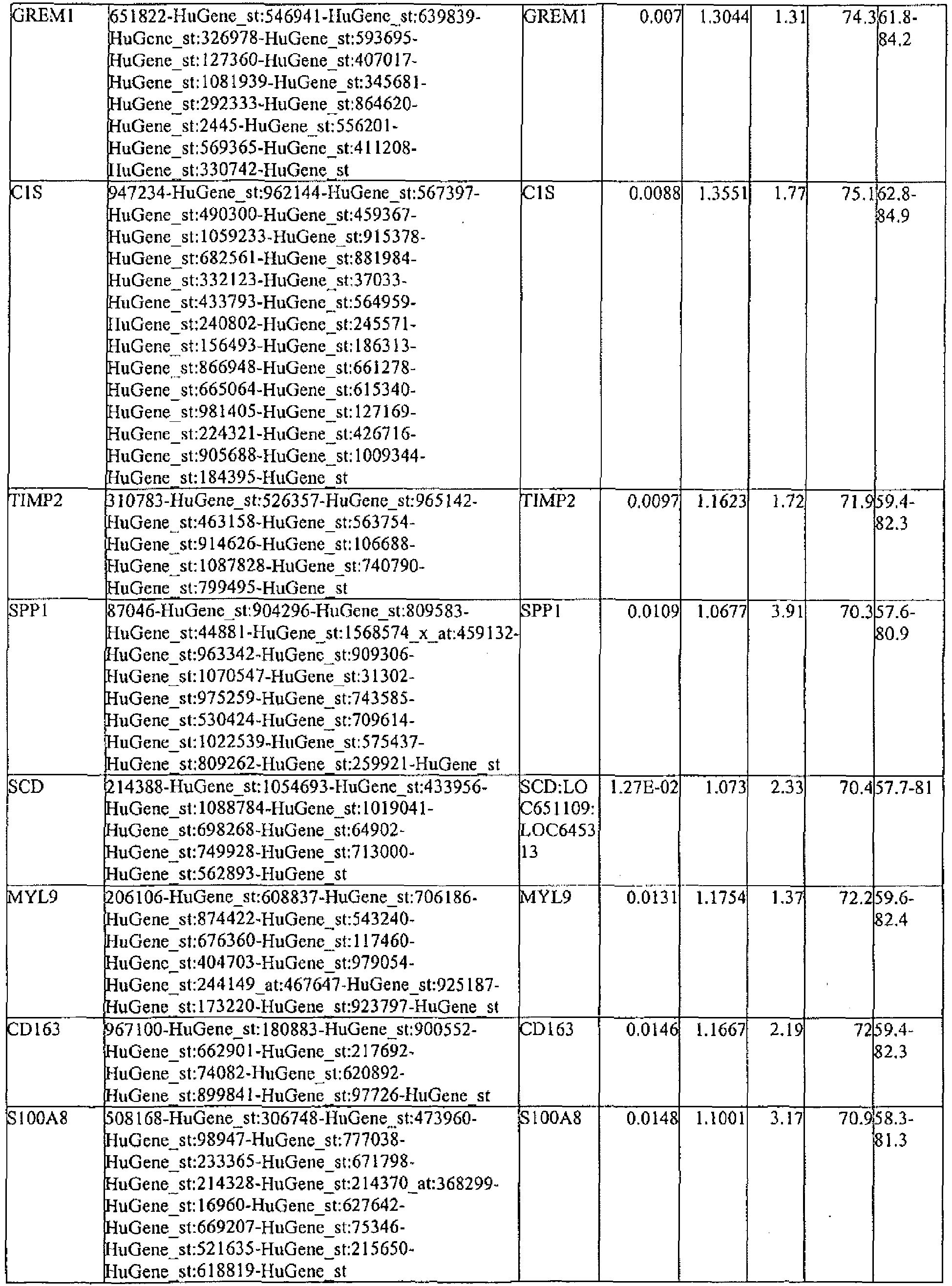

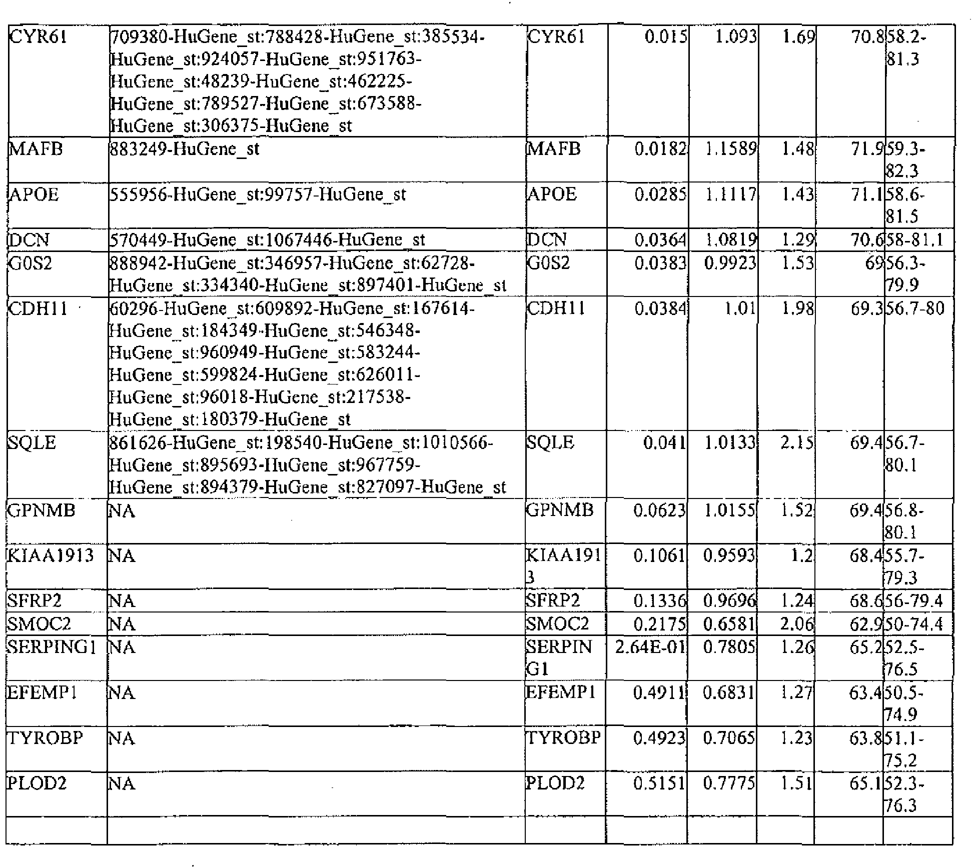

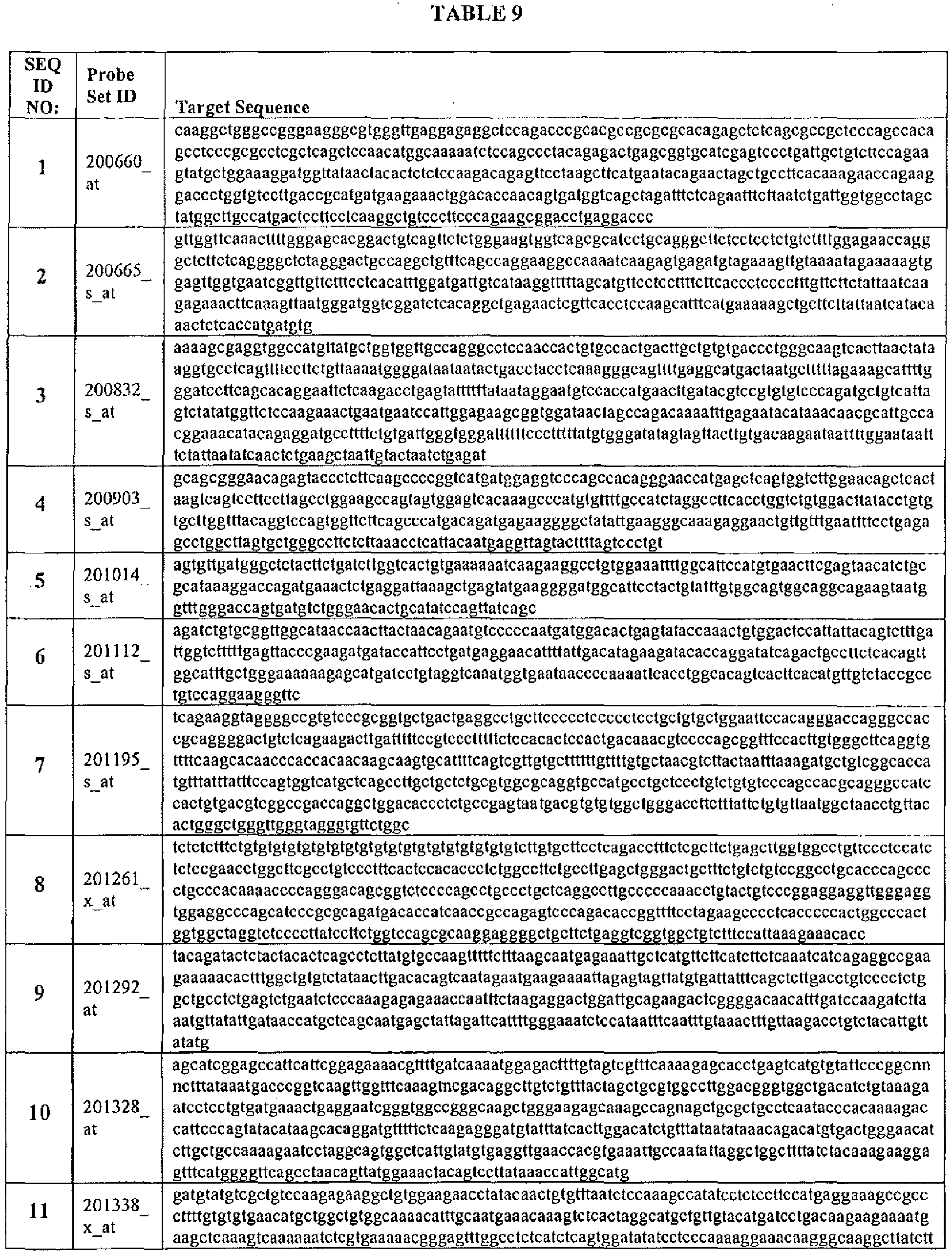

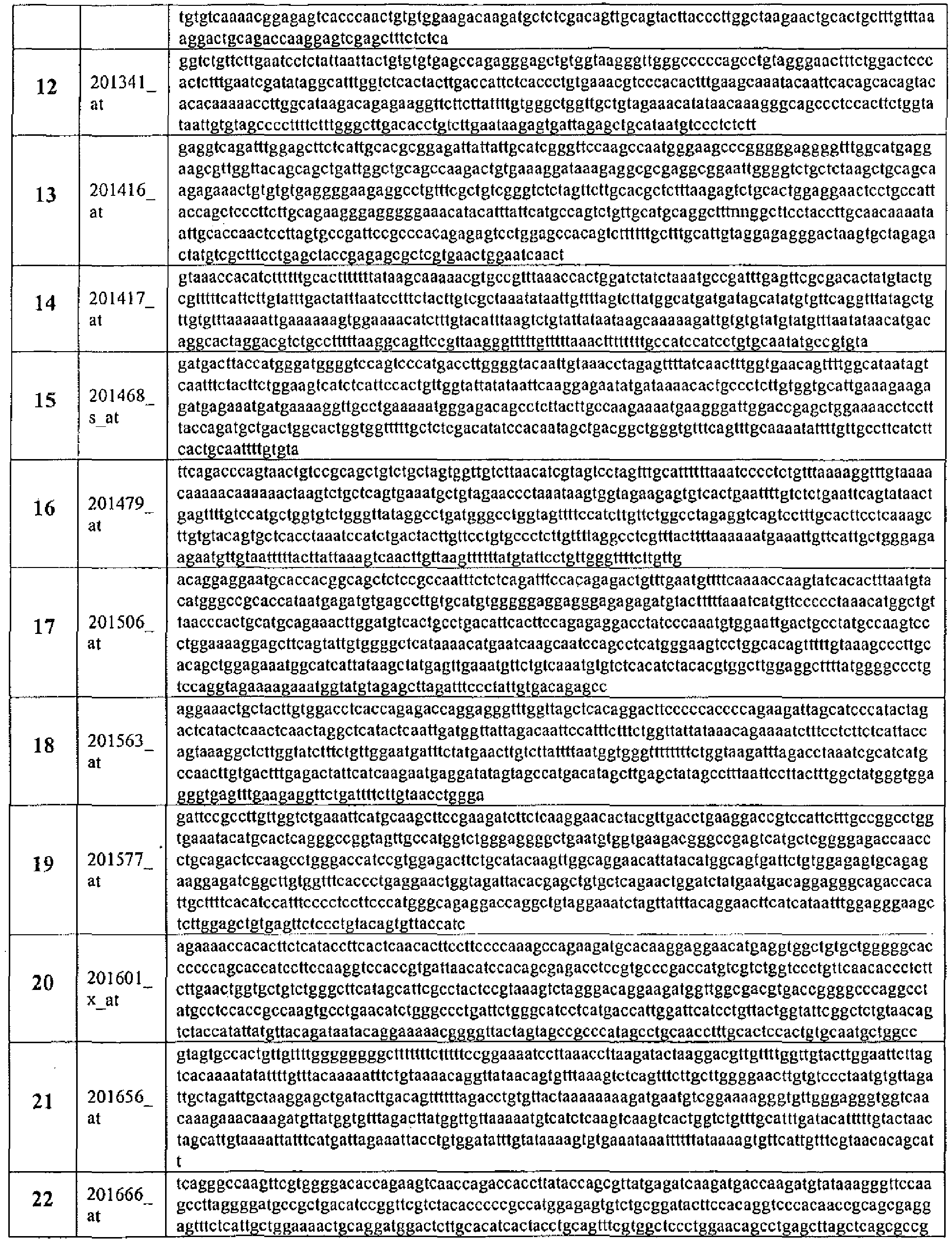

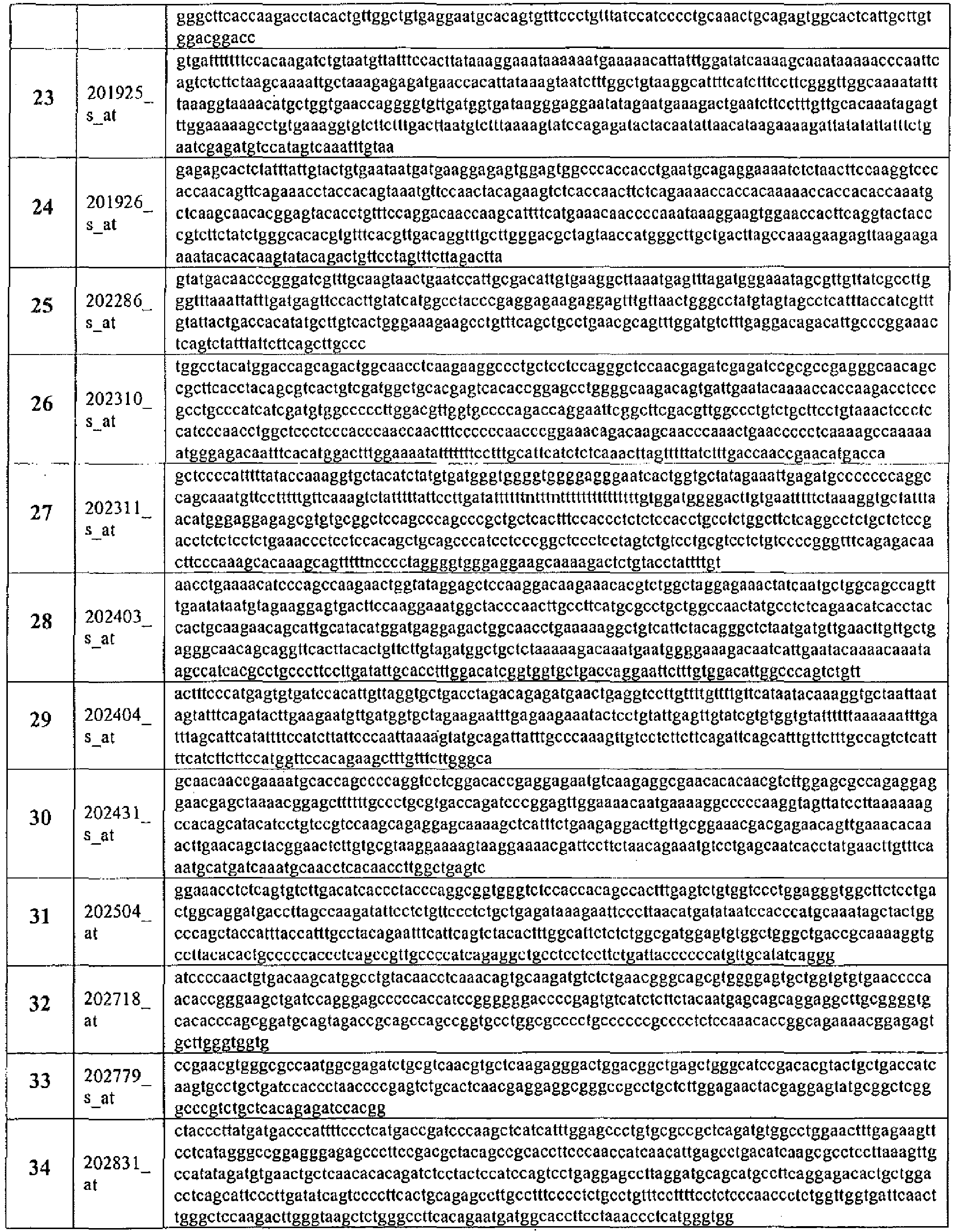

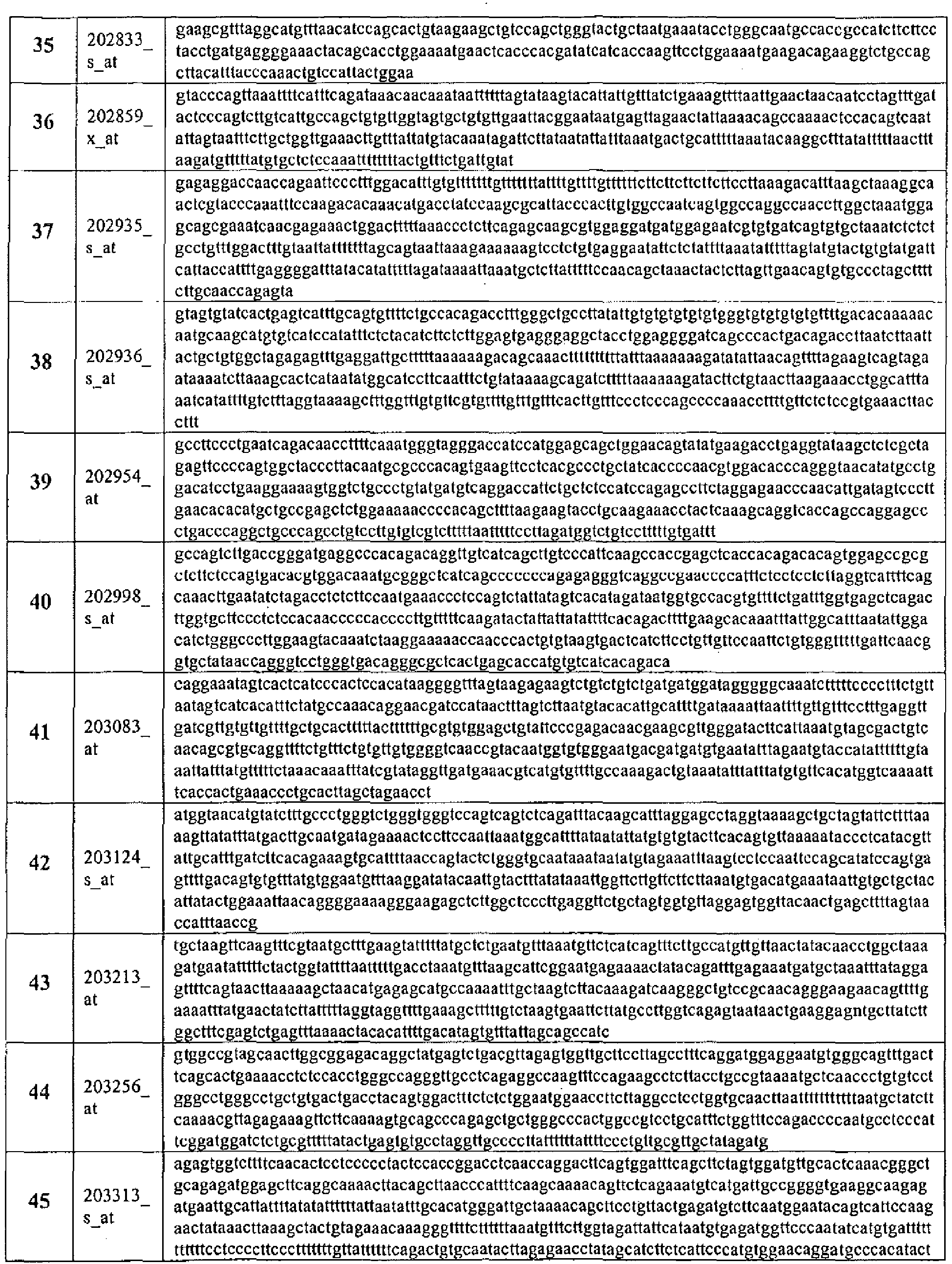

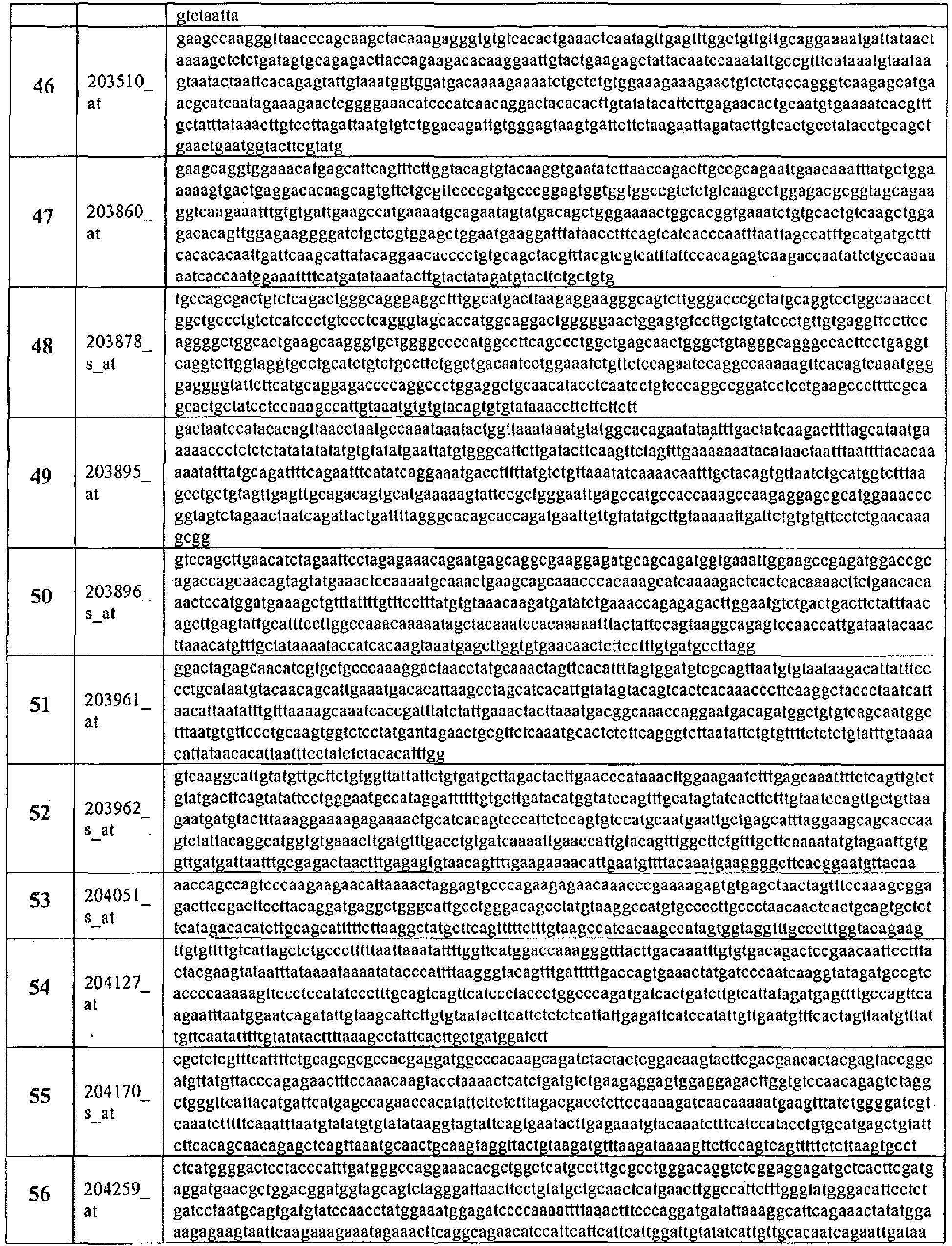

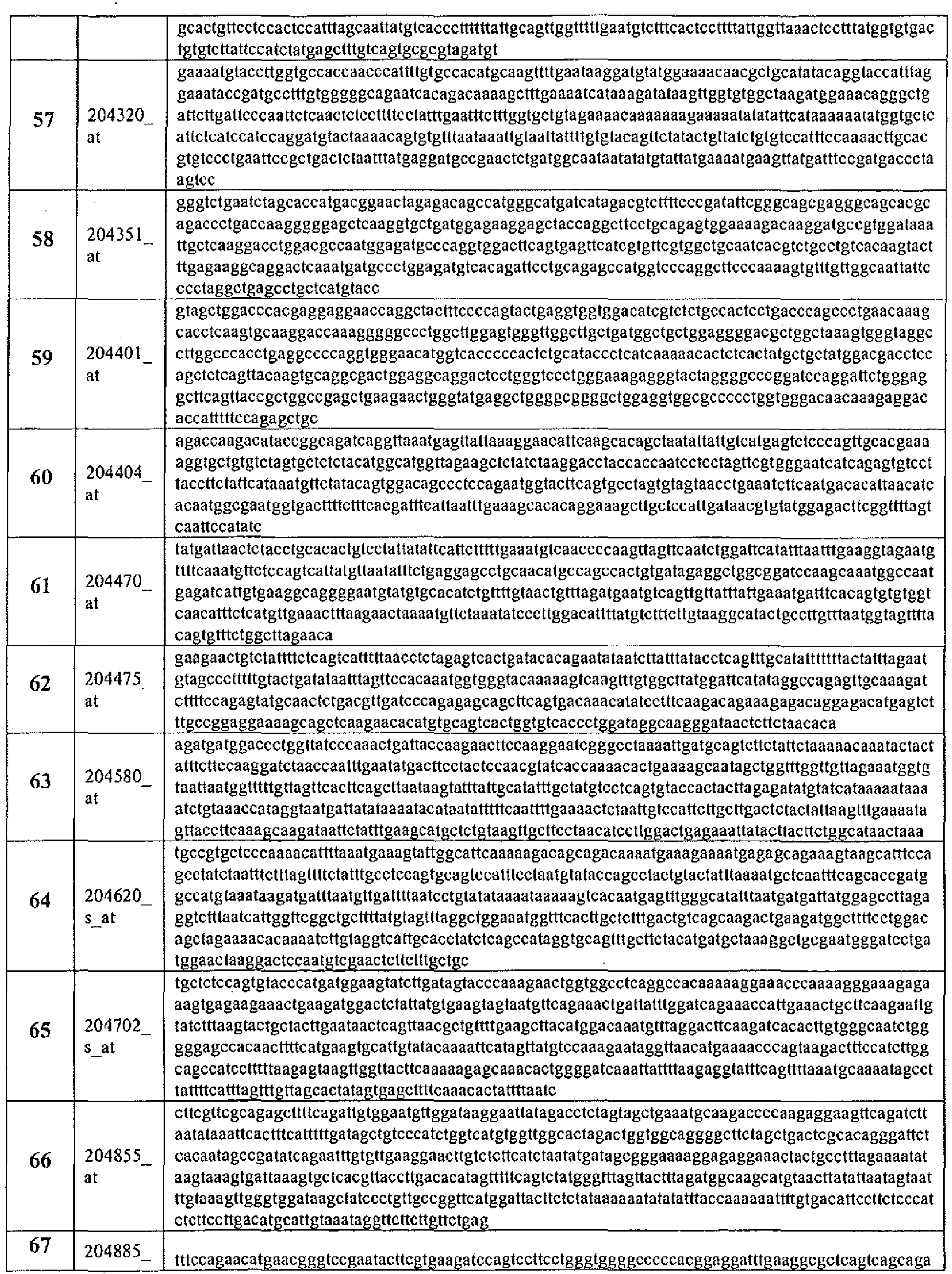

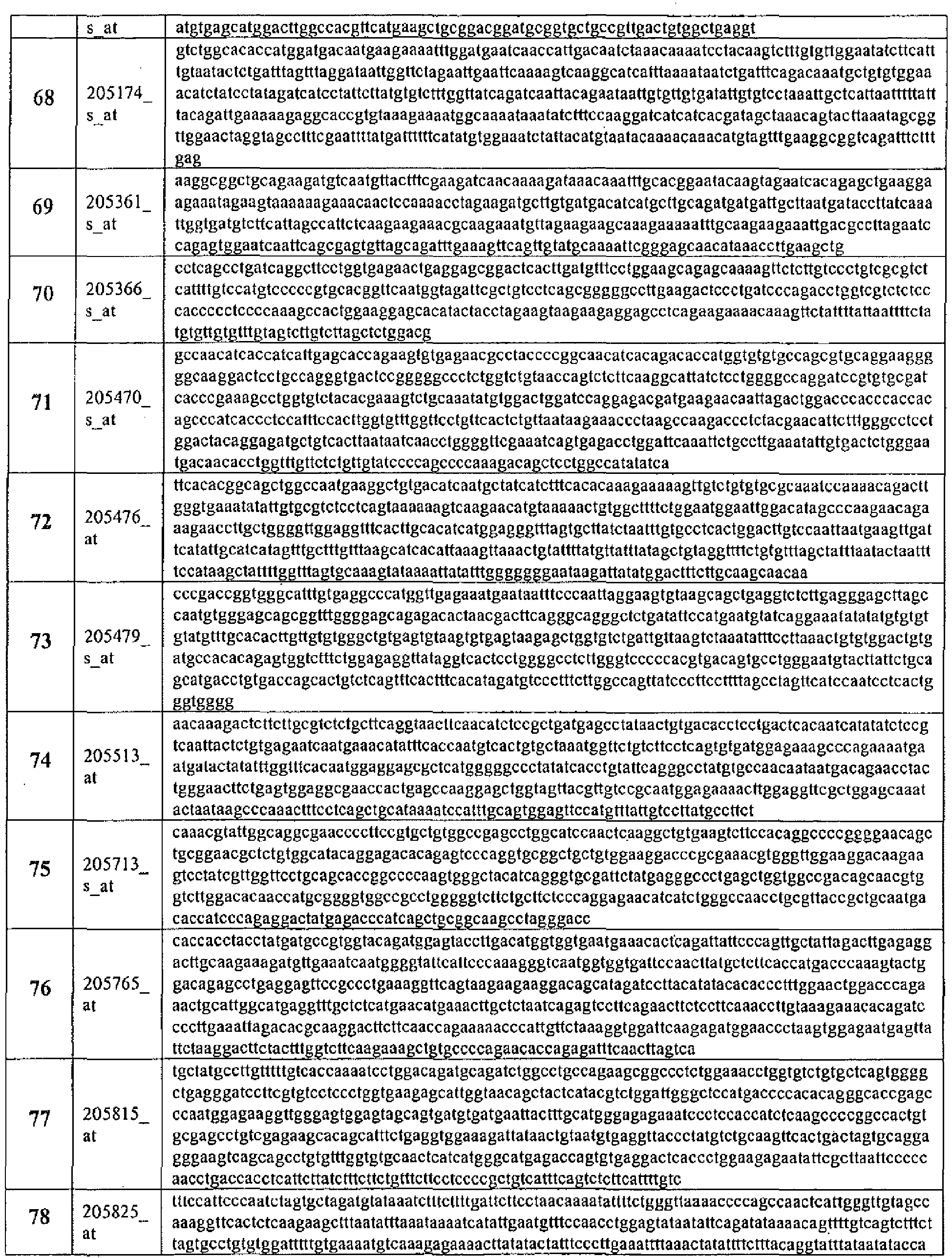

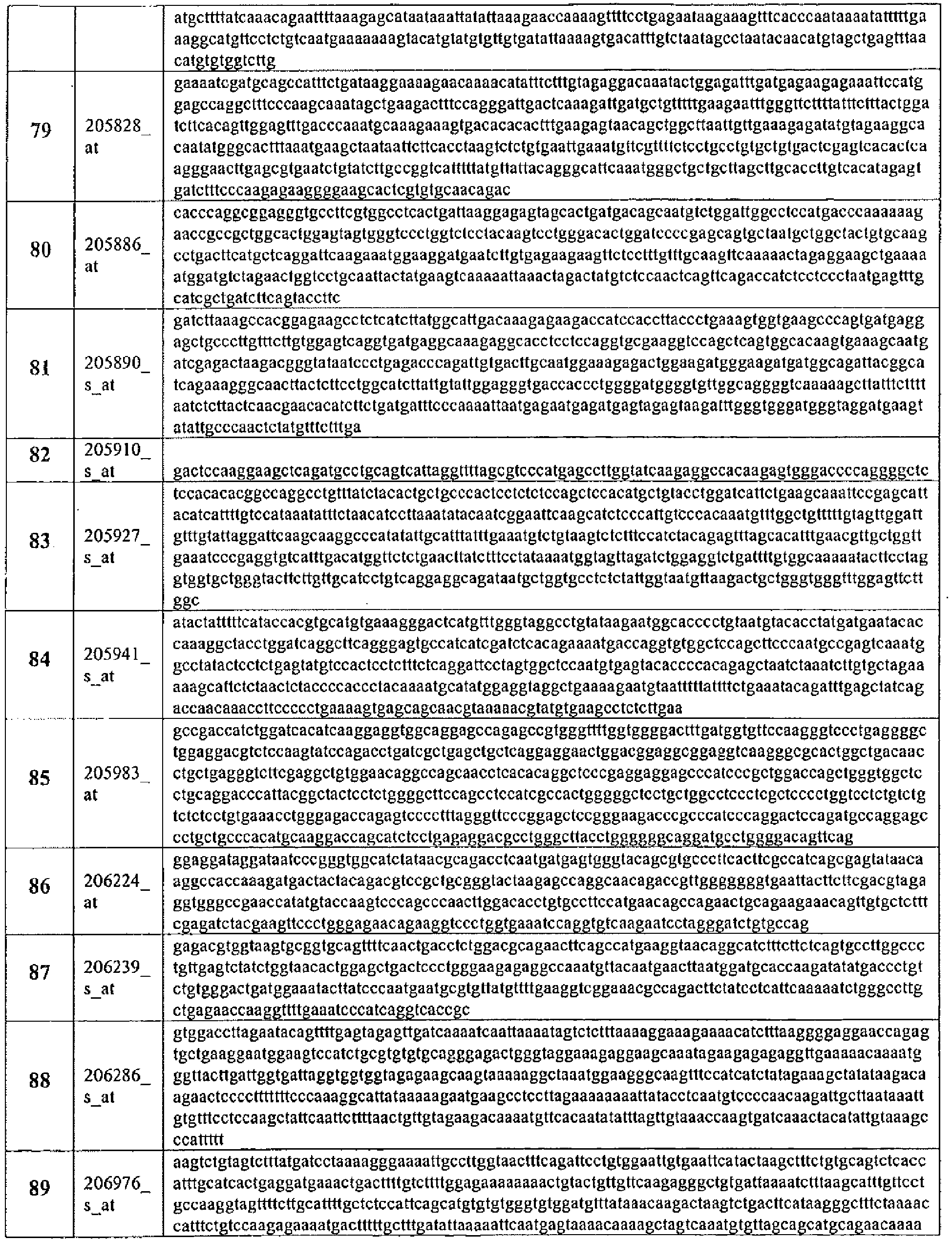

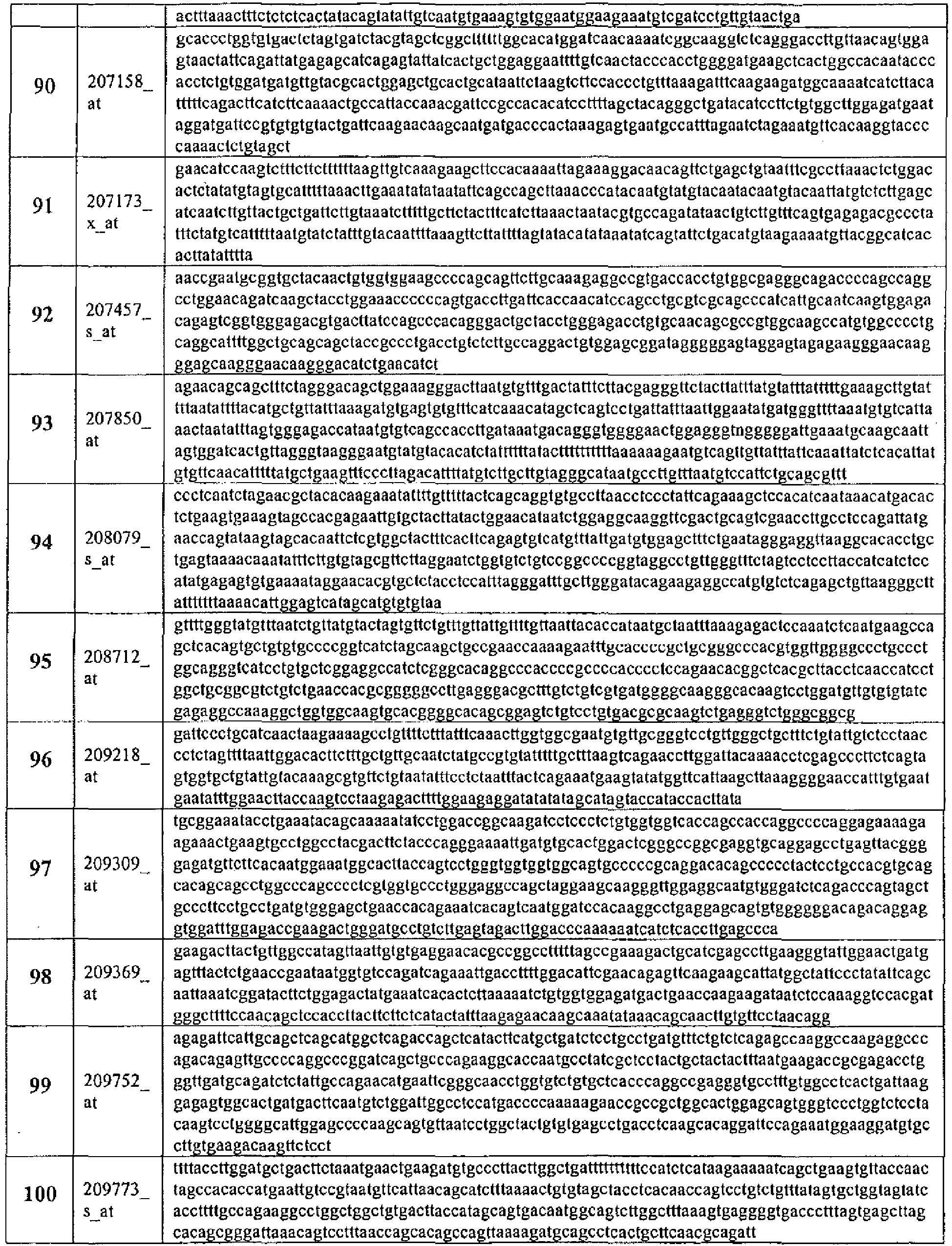

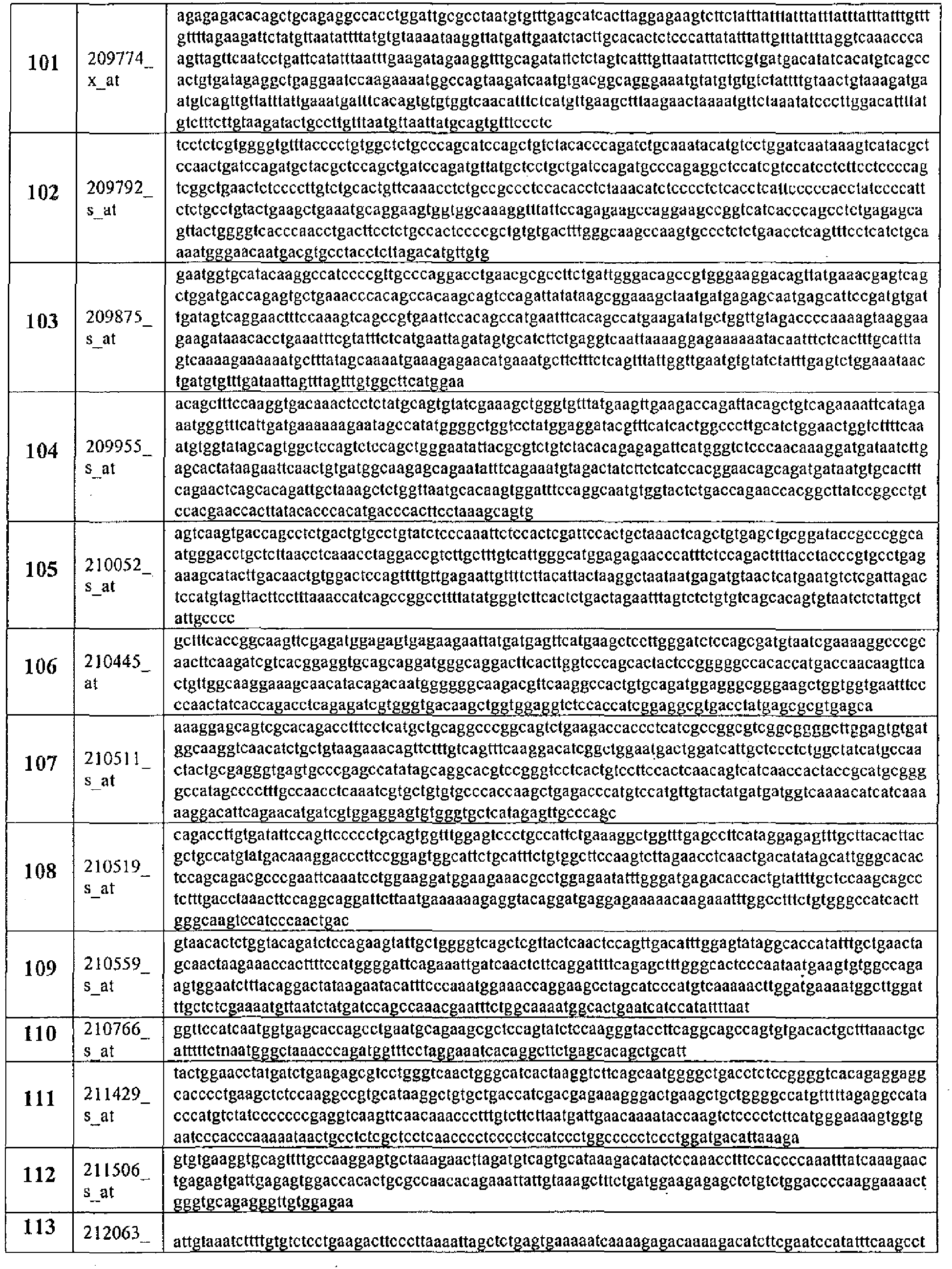

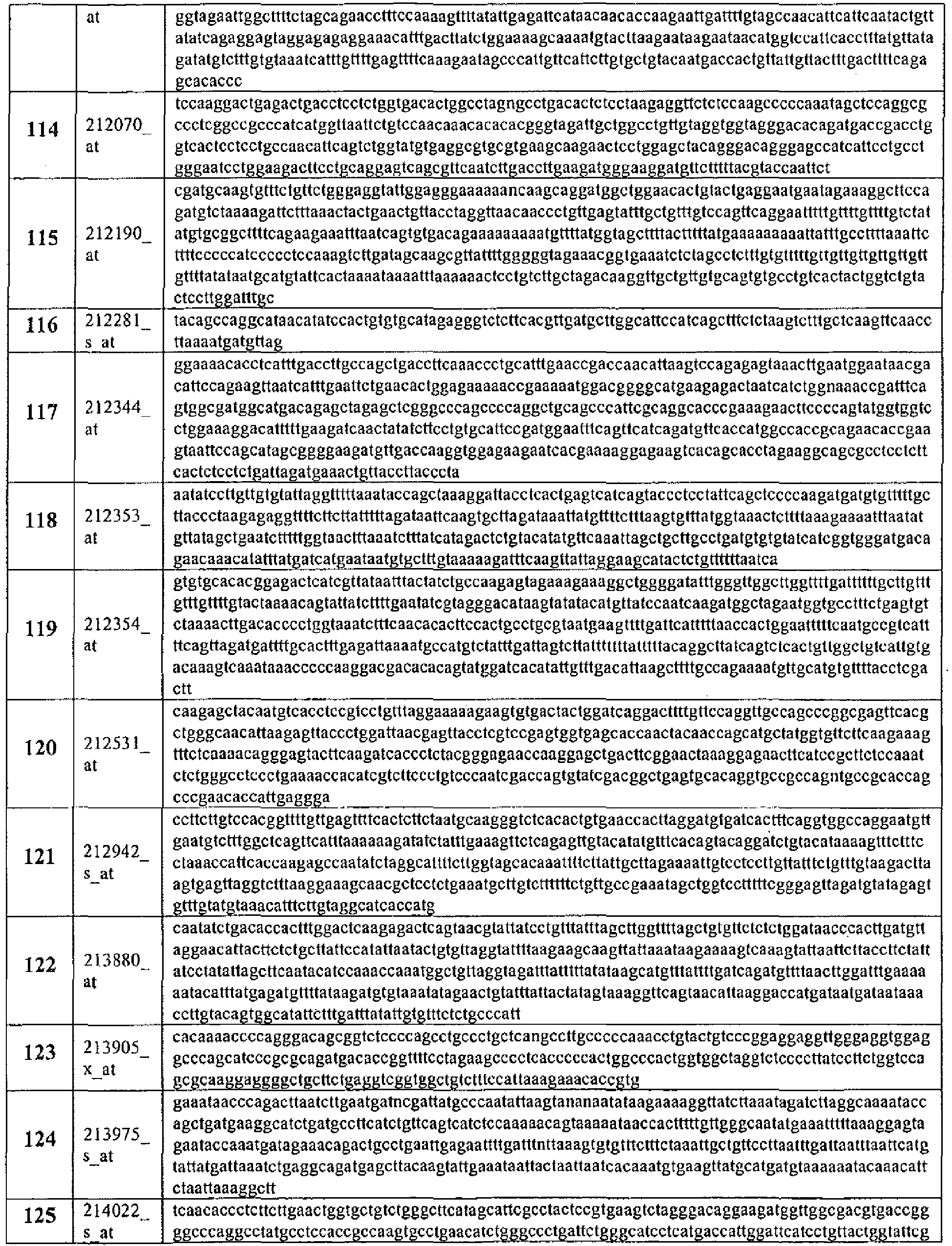

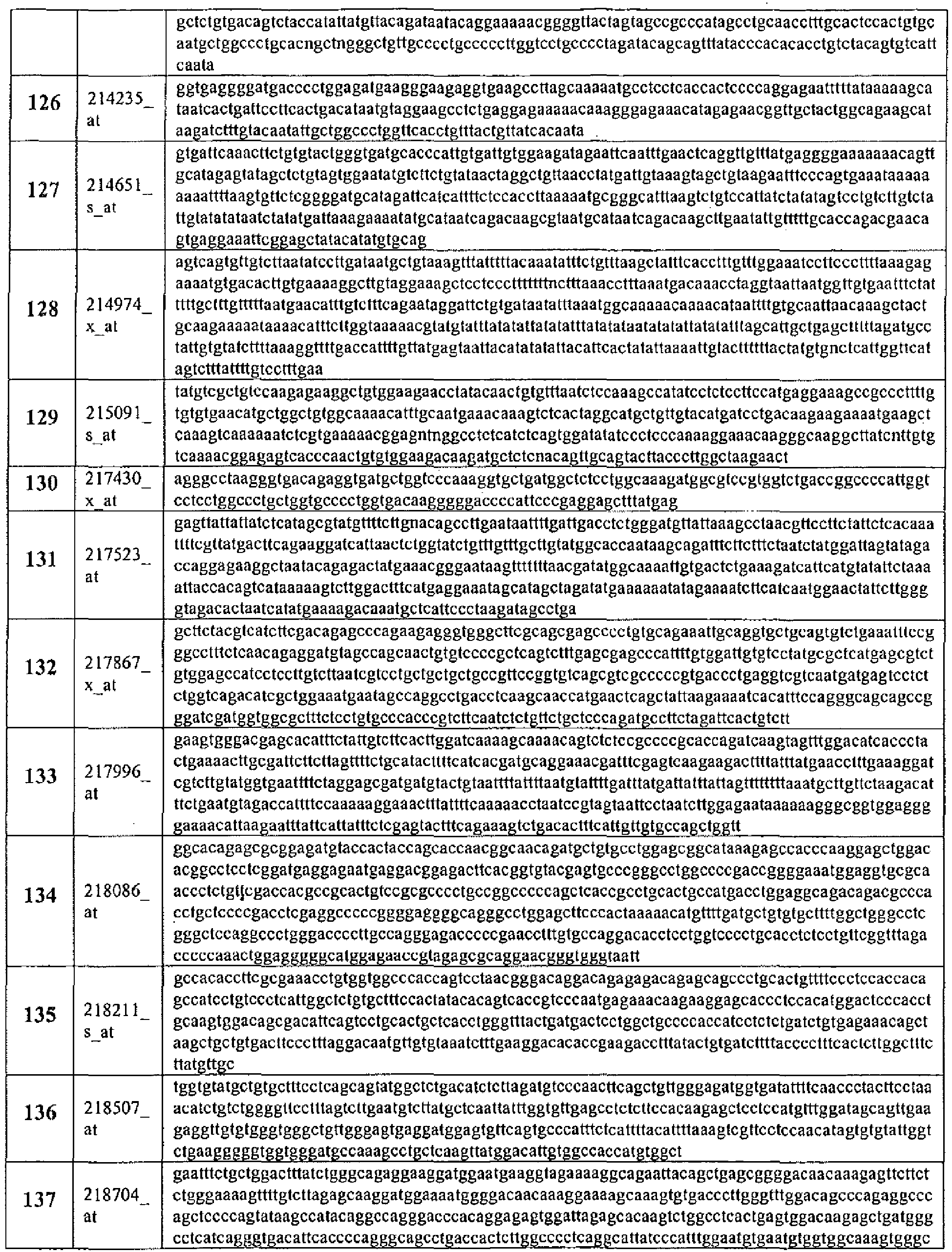

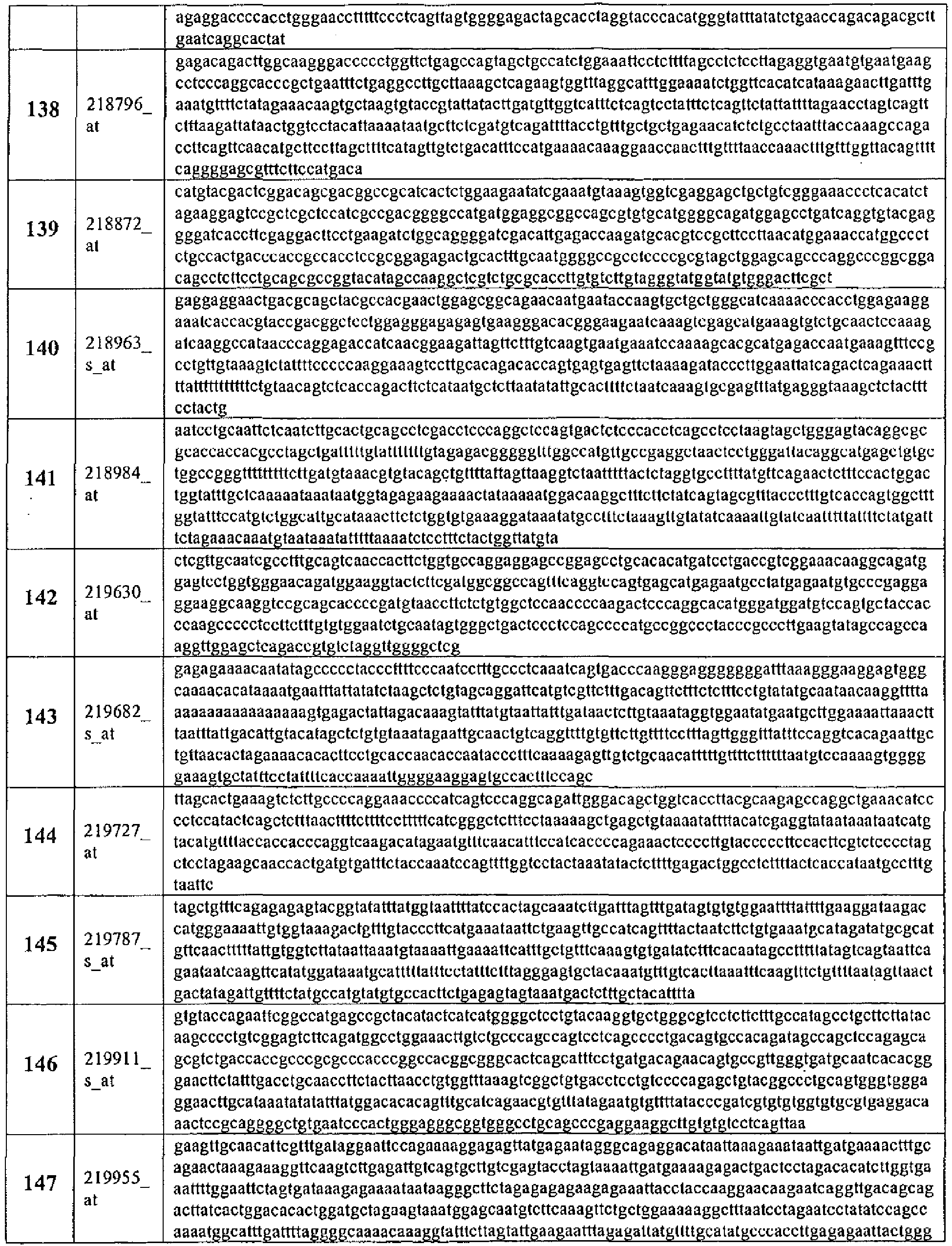

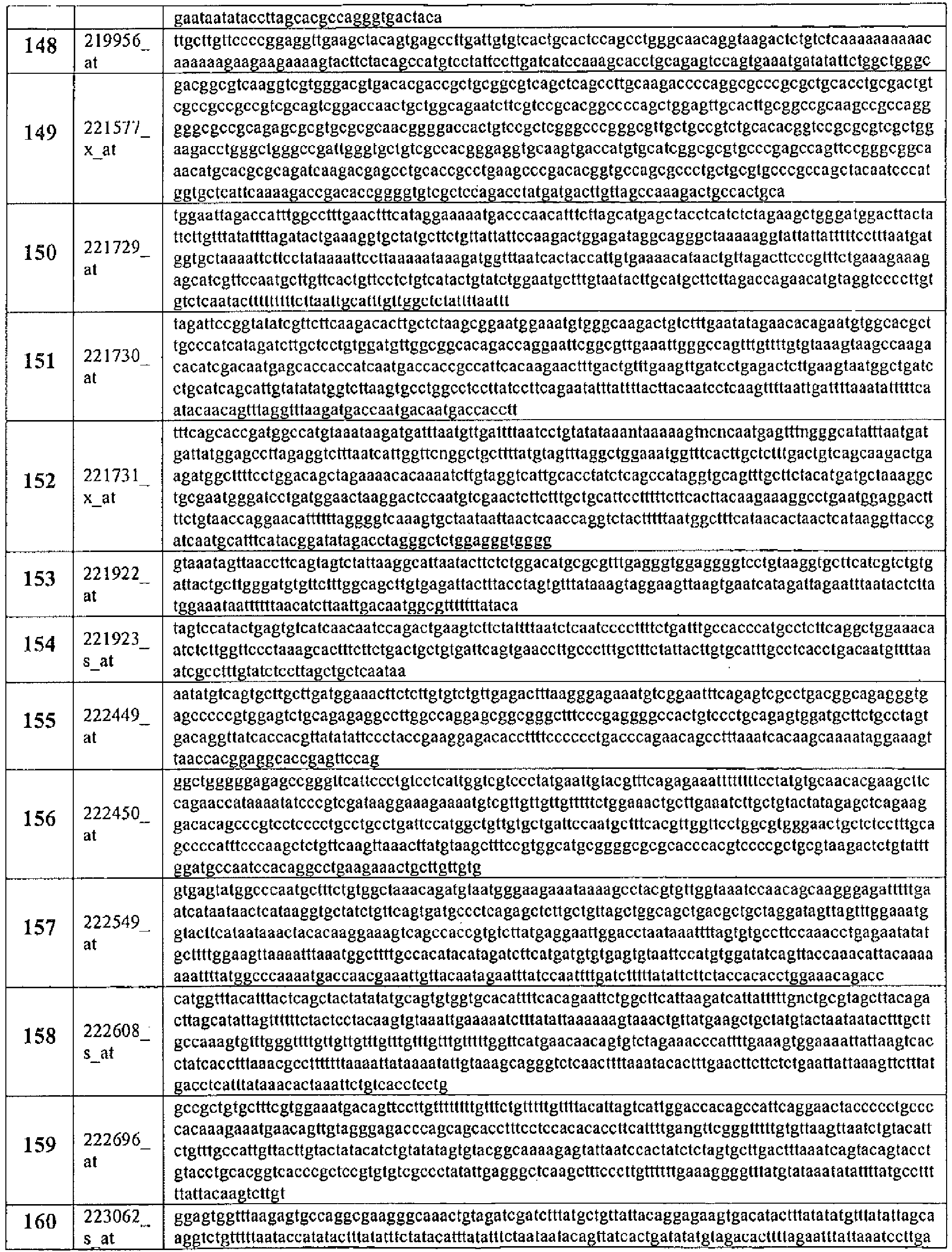

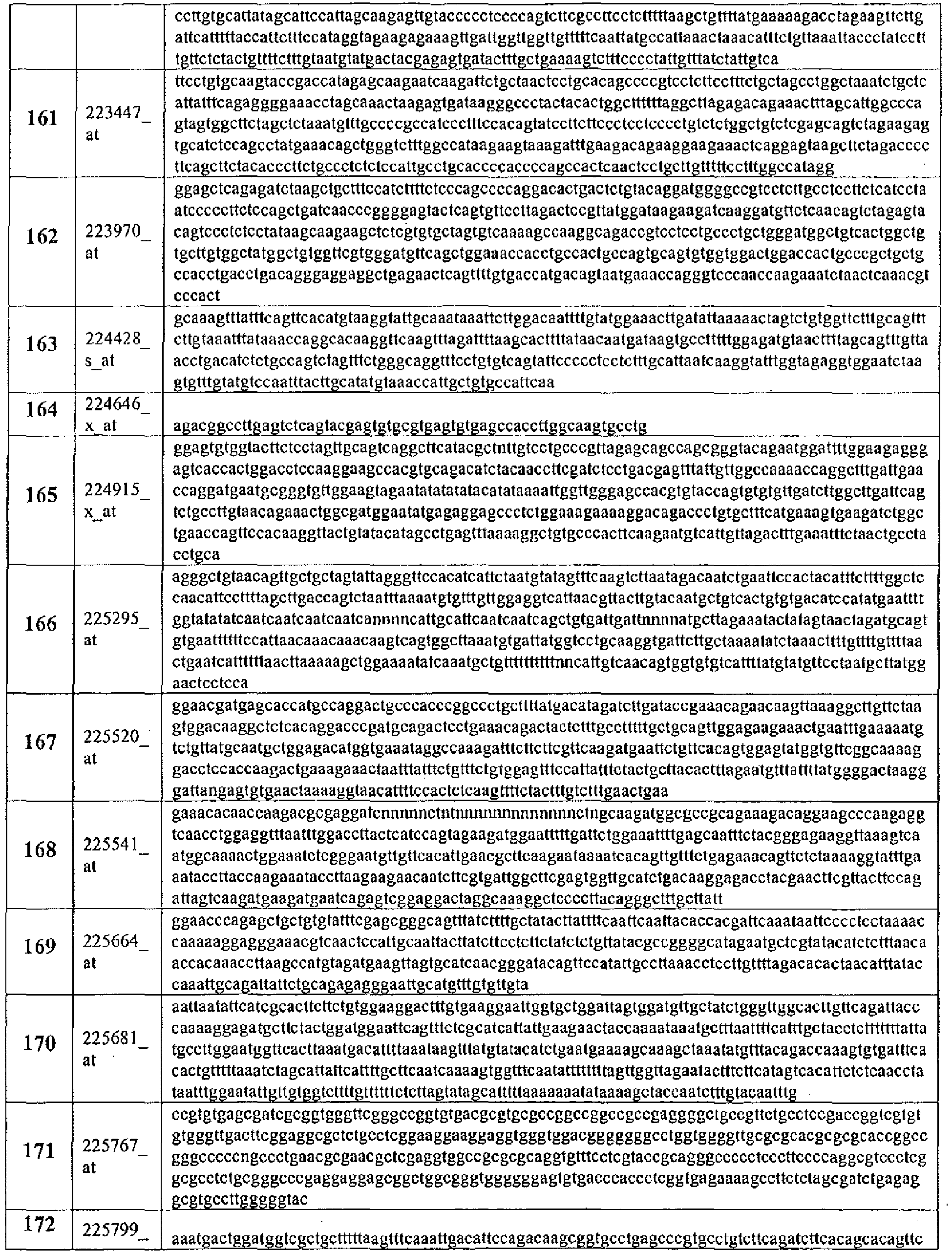

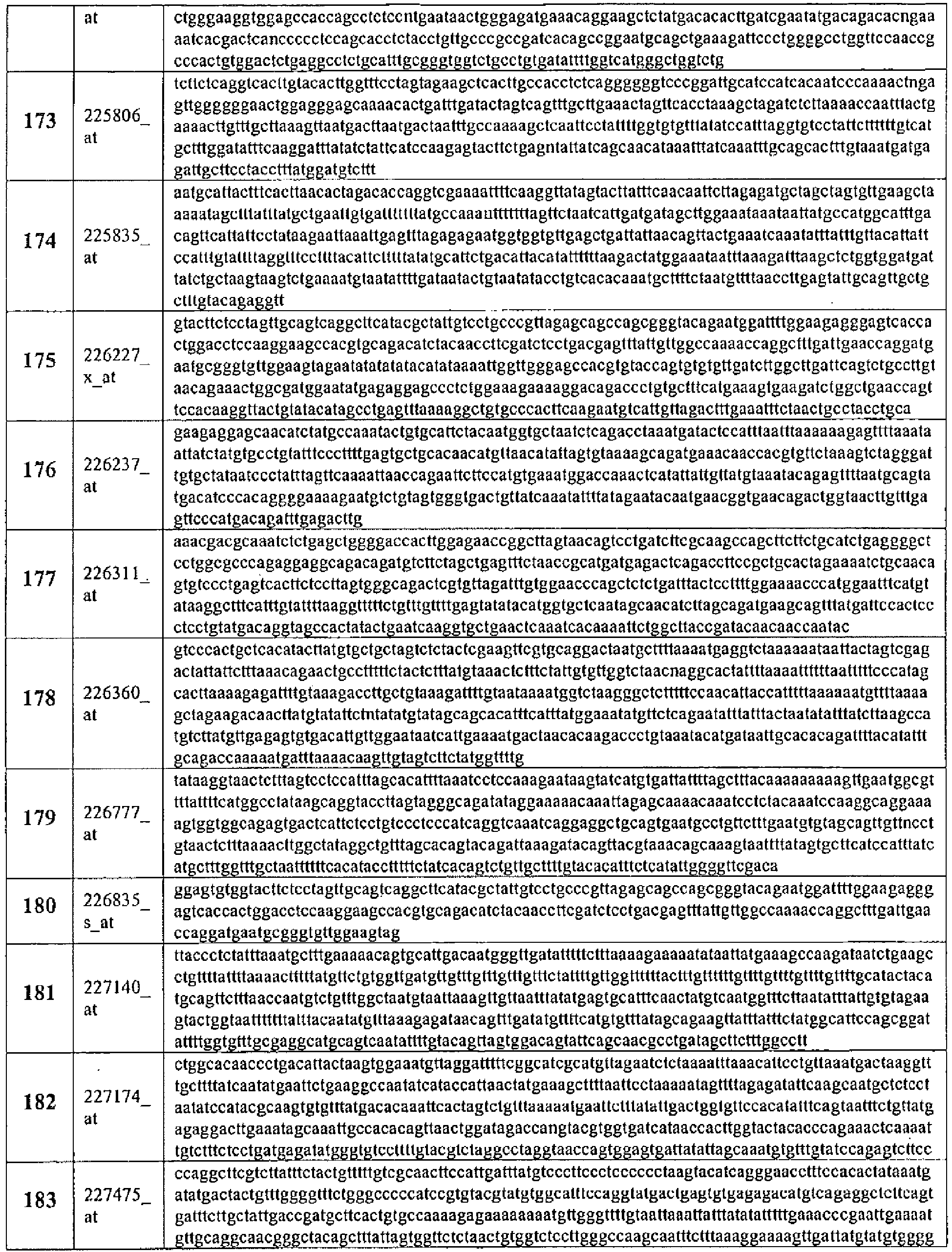

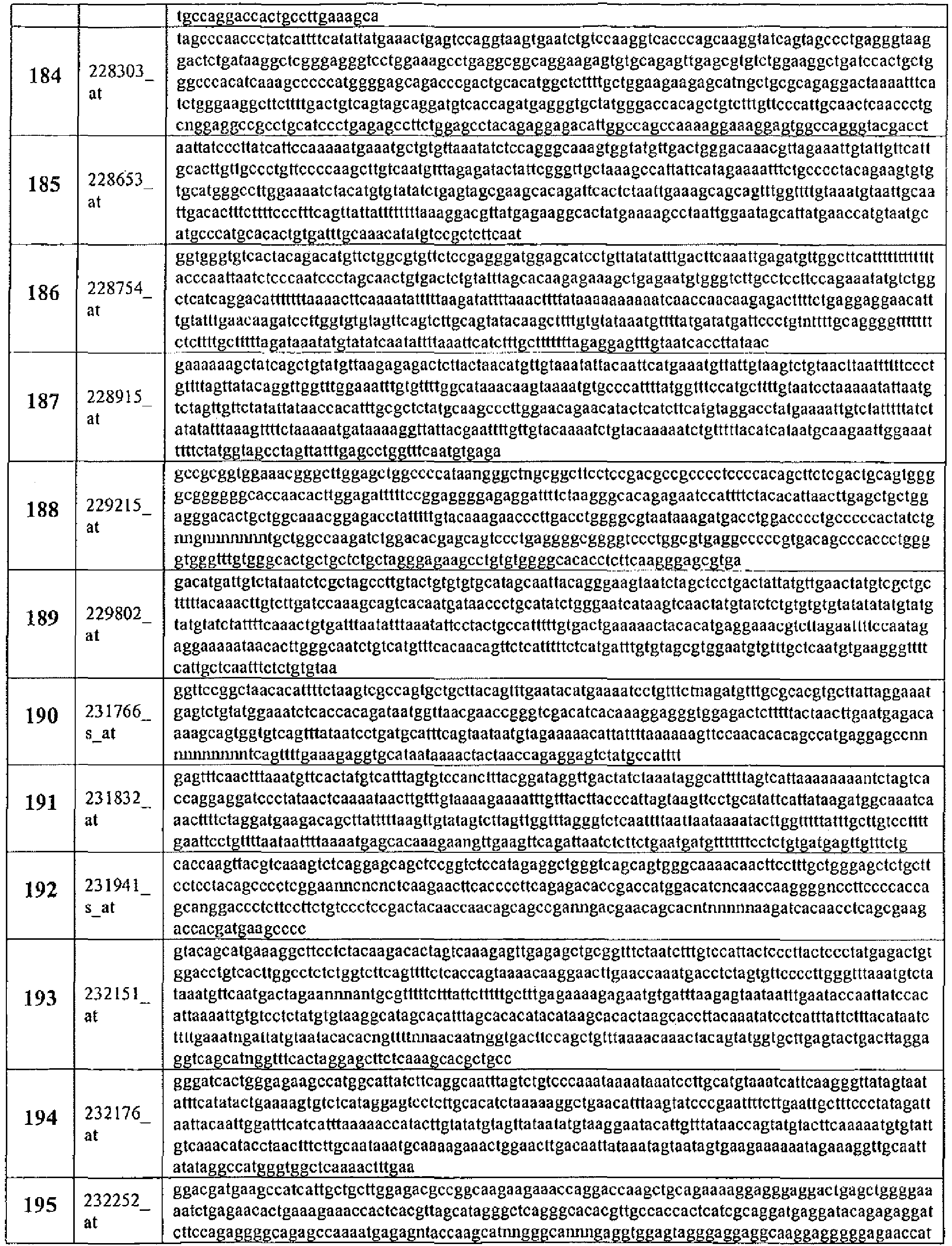

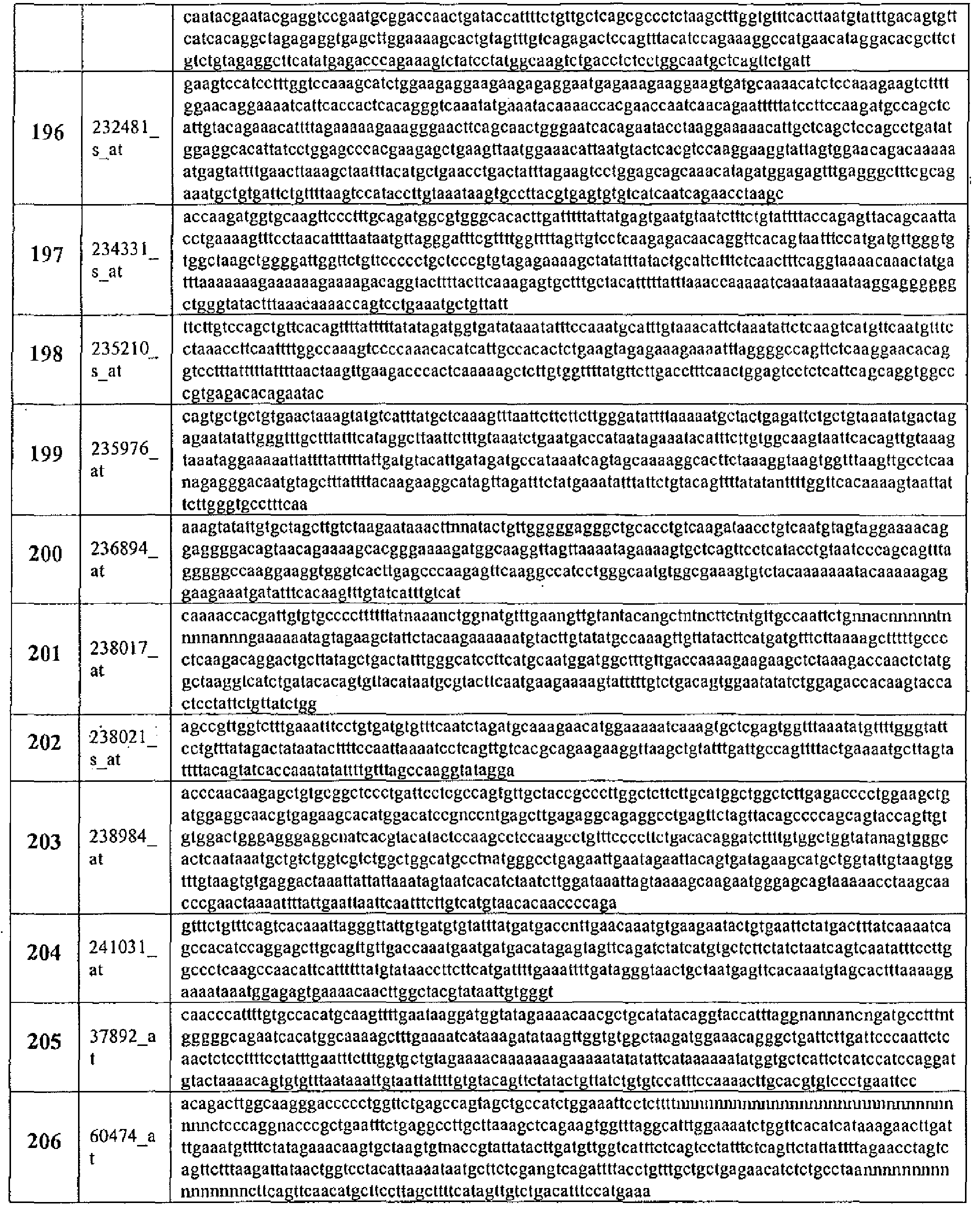

### BIBLIOGRAPHY

Affymetrix. GeneChip expression data analysis fundamentals. Affymetrix, Santa Clara, CA USA, 2001.
Alon et al., Proc. Natl. Acad. Sci. USA: 96, 6745-6750, June 1999
Ausubel, F. et al., "Current Protocols in Molecular Biology", John Wiley & Sons, (1998)
Bonner et al (1973) J. Mol. Biol. 81:123
DeRisi, et al., Nature Genetics 14:457-460 (1996)
Germer et al., Genome Res. 10:258-266 (2000)
Guo et al., Nucleic Acids Res. 22:5456-5465 (1994)
Heid et al., Genome Res. 6:986-994 (1996)
Hubbell E.W., W. M. Liu, and R. Mei. Robust estimators for expression analysis. Bioinformatics, 18:1585-1592, 2002.
Irizarry R.W., B. M. Bolstad, F. Collin, L. M. Cope, B. Hobbs, and T. P. Speed. Summaries of affymetrix genechip probe level data. Nucleic Acid Research, 31, 2003.
Kraus, M. and Aaronson, S., 1991. Methods Enzymol., 200:546-556
Maskos and Southern, Nuc. Acids Res. 20:1679-84, 1992
Moore et al., BBA, 1402:239-249, 1988
Nielsen (1999) Curr. Opin. Biotechnol. 10:71-75
Nielsen et al. (1991) Science 254: 1497-1500
Pease et al., Proc. Natl. Acad. Sci. USA 91(11):5022-5026 (1994)
Pevzner et al., J. Biomol. Struc. & Dyn. 9:399-410, 1991
Schena, et al. Science 270:467-470 (1995)
Smith et al., Science 258:1122-1126 (1992)
Smyth G.K. Bioinformatics and Computational Biology Solutions using R and Bioconductor. Springer, New York, 2005.
Smyth G.K. Linear models and empirical bayes methods for assessing differential expression in microarray experiments. Statistical Applications in Genetics and Molecular Biology, 3(1):Article 3, 2004.
T. Sano and C. R. Cantor, Bio/Technology 9:1378-81 (1991)
Urdea et al., Nucleic Acids Symp. Ser., 24:197-200 (1991)
Wedemeyer et al., Clinical Chemistry 48:9 1398-1405, 2002)
Weissleder et al., Nature Medicine 6:351-355, 2000

The disclosure may be described by reference to the following numbered paragraphs:-
1. A method of screening for the onset or predisposition to the onset of a large intestine neoplasm or monitoring the progress of a neoplasm in an individual, said method comprising measuring the level of expression of one or more genes or transcripts selected from:
**(i)** the gene, genes or transcripts detected by Affymetrix probeset IDs:

| | | |
|---|---|---|
| 201328_at | 221577_x_at | 205828_at |
| 201341_at | 221922_at | 205886_at |
| 201416_at | 60474_at | 205890_s_at |
| 201417_at | 222696_at | 205910_s_at |
| 201468_s_at | 223447_at | 205941_s_at |
| 201506_at | 223970_at | 206224_at |
| 201563_at | 225541_at | 206976_s_at |
| 201656_at | 225835_at | 207173_x_at |
| 201925_s_at | 226360_at | 207457_s_at |
| 201926_s_at | 227174_at | 208079_s_at |
| 202286_s_at | 227475_at | 208712_at |
| 202718_at | 228303_at | 209218_at |
| 202831_at | 228653_at | 209309_at |
| 202833_s_at | 228754_at | 209752_at |
| 202935_s_at | 228915_at | 209773_s_at |
| 202936_s_at | 229215_at | 209774_x_at |
| 203124_s_at | 231832_at | 209792_s_at |
| 203256_at | 231941_s_at | 209875_s_at |
| 203313_s_at | 232176_at | 209955_s_at |
| 203510_at | 232252_at | 210052_s_at |
| 203860_at | 232481_s_at | 210511_s_at |
| 203895_at | 234331_s_at | 210559_s_at |
| 203896_s_at | 235210_s_at | 210766_s_at |
| 203961_at | 235976_at | 211506_s_at |
| 203962_s_at | 236894_at | 212281_s_at |
| 204259_at | 238017_at | 212344_at |
| 204351_at | | 212353_at |
| 204401_at | 238984_at | 212354_at |
| 204404_at | 241031_at | 213905_x_at |
| 204855_at | 200660_at | 214022_s_at |
| 204885_s_at | 200832_s_at | 214974_x_at |
| 205174_s_at | 200903_s_at | 215091_s_at |
| 205366 s_at | 201014_s_at | 217430_x_at |
| 205470_s_at | 201112_s_at | 217996_at |
| 205513_at | 201195_s_at | 218507_at |
| 205765_at | 201261_x_at | 218963_s_at |
| 205825_at | 201292_at | 218984_at |
| 205927_s_at | 201338_x_at | 219787_s_at |
| 205983_at | 201479_at | 219911_s_at |
| 206239_s_at | 201577_at | 221729_at |
| 206286_s_at | 201601_x_at | 221730_at |
| 207158_at | 201666_at | 221731_x_at |
| 207850_at | 202310_s_at | 221923_s_at |
| 209369_at | 202311_s_at | 37892_at |
| 210445_at | 202404_s_at | 222449_at |
| 210519_s_at | 202431_s_at | 222450_at |
| 211429_s_at | 202504_at | 222549_at |
| 212063_at | 202779_s_at | 222608_s_at |
| 212070_at | 202859_x_at | 223062_s_at |
| 212190_at | 202954_at | 224428_s_at |
| 212531_at | 202998_s_at | 224646_x_at |
| 212942_s_at | 203083_at | 224915_x_at |
| 213880_at | 203213_at | 225295_at |
| 213975_s_at | 203878_s_at | 225520_at |
| 214235_at | 204051_s_at | 225664_at |
| 214651_s_at | 204127_at | 225681_at |
| 217523_at | 204170_s_at | 225767_at |
| 217867_x_at | 204320_at | 225799_at |
| 218086_at | 204470_at | 225806_at |
| 218211_s_at | 204475_at | 226227_x_at |
| 218704_at | 204580 at | 226237_at |
| 218796_at | 204620_s_at | 226311_at |
| 218872_at | 204702_s_at | 226777_at |
| 219630_at | 205361_s_at | 226835_s_at |
| 219682_s_at | 205476_at | 227140_at |
| 219727_at | 205479_s_at | 229802_at |
| 219955_at | 205713_s_at | 231766_s_at |
| 219956_at | 205815_at | 232151_at; and/or |
| 200665_s_at | | |

**(ii)**

| | | | |
|---|---|---|---|
| KIAA1199 | | CDCA7 | LOXL2 |
| FOXQ1 | RNF43 | TDGF1 | AZGP1 |
| MMP1 | CCL20 | MTHFDIL | MYC |
| TCN1 | CTSE | ANLN | COMP |
| MMP7 | MSLN | H19 | AURKA |
| WDR72 | TIMP1 | FAP | PAICS |
| INHBA | PCSK1 | DACH1 | PUS7 |
| COL11A1 | CST1 | VCAN | ZNRF3 |
| GDF15 | BGN | SQLE | CCND1 |
| CTHRC1 | AXIN2 | REG3A | CSE1L |
| COL1A1 | MET | TESC | PFDN4 |
| LGR5 | SOX9 | UBE2C | C20orf42 |
| DUSP27 | TMEPAI | TMEM97 | SLC11A2 |
| SERPINB5 | CDH11 | TRIM29 | NFE2L3 |
| ASCL2 | MMP11 | KLK11 | CEL |
| SULF1 | CXCL1 | LY6G6D | NLF1 |
| SLC6A6 | QPCT | SLC7A5 | NPDC1 |
| TACSTD2 | PDZK1IP1 | FABP6 | ENC1 |
| NEBL | CD55 | SLITRK6 | SERPINE2 |
| PCCA | ECT2 | MLPH | UBE2S |
| FLJ37644 | COL12A1 | HOXA9 | TOP2A |
| KRT23 | L1TD1 | TBX3 | CDC2 |
| CXCL3 | WDR51B | BACE2 | RFC3 |
| MMP3 | HOXB6 | GPX2 | LILRB1 |
| SFRP4 | FAM84A | TPX2 | NPM1 |
| UBD | COL5A2 | KCNN4 | RDHE2 |
| SCD | MMP12 | LOC541471 | PLAU |
| DPEP1 | SORD | CYP3A5 | HSPH1 |
| LCN2 | PSAT1 | ANXA3 | GTF3A |
| PLCB4 | CXCL5 | CYP3A5P2 | KLK10 |
| SPINK1 | IGFBP2 | C14orf94 | GPSM2 |
| DUOX2 | TGIF1 | RP5-875H10.1 | NME1 |
| SOX4 | CXCL2 | RPL22L1 | MUC20 |
| THBS2 | SLCO4A1 | APOBEC1 | RPESP |
| CLDN1 | CKS2 | HIG2 | RRM2 |
| REGIB | PHLDA1 | COL10A1 | DKC1 |
| CDH3 | SERPINA1 | AHCY | CD44 |
| SPARC | COL1A2 | IL8 | |

in a biological sample from said individual wherein a higher level of expression of the genes or transcripts of group (i) and/or group (ii) relative to control levels is indicative of a neoplastic large intestine cell or a cell predisposed to the onset of a neoplastic state.
2. The method according to paragraph 1 wherein said one or more genes or transcripts are selected from:
**(i)** the gene, genes or transcripts detected by Affymetrix probeset IDs: 225681_at; 227140_at; and/or
**(ii)** CTHRC1.

3. The method according to paragraph 1 wherein said one or more genes or transcripts are selected from:
**(i)** the gene, genes or transcripts detected by Affymetrix probeset IDs: 227475_at, 204475_at, 202859_x_at, 202404_s_at; and/or
**(ii)** FOXQ1, MMP1, IL8, COL1A2.

4. The method according to paragraph 1 wherein said one or more genes or transcripts are selected from:
**(i)** the gene, genes or transcripts detected by Affymetrix probeset IDs: 205513_at, 204259_at, 227174_at, 210511_s_at, 37892_at; and/or
**(ii)** TCN1, MMP7, WDR72, INHBA, COL11A1.

5. The method according to paragraph 1, wherein said one or more genes or transcripts are selected from:
**(i)** the gene, genes or transcripts detected by Affymetrix probeset IDs:

| | | |
|---|---|---|
| 221577_x_at | 212942_s_at | 202310_s_at |
| 213880_at | 232252_at | 204855_at |
| 229215_at | 212354_at | 228754_at |
| 202286_s_at | 203962_s_at | 203860_at |
| 202935_s_at | 218963_s_at | 207850_at |
| 205828_at | 204051_s_at | 205890_s_at |
| 200832_s_at | 205983_at | 212531_at; and/or |

**(ii)**

| | | |
|---|---|---|
| KIAA1199 | GDF15 | COL1A1 |
| LGR5 | DUSP27 | SERPINB5 |
| ASCL2 | SULF1 | SLC6A6 |
| TACSTD2 | NEBL | PCCA |
| FLJ37644 | KRT23 | CXCL3 |
| MMP3 | SFRP4 | UBD |
| SCD | DPEP1 | LCN2. |

6. The method according to any one of paragraphs 1-5 wherein said control level is a non-neoplastic level.
7. The method according to any one of paragraphs 1-5 wherein said neoplastic cell is an adenoma or an adenocarcinoma.
8. The method according to any one of paragraphs 1 to 3 wherein said neoplasm is an adenoma and said one or more genes or transcripts are selected from:
**(i)** the gene, genes or transcripts detected by Affymetrix probeset IDs:

| | | |
|---|---|---|
| 201328_at | 205825_at | 219956_at |
| 201468_s_at | 205927_s_at | 221922_at |
| 201656_at | 206239_s_at | 223447_at |
| 201925_s_at | 207158_at | 223970_at |
| 201926_s_at | 210445_at | 225835_at |
| 202718_at | 210519_s_at | 226360_at |
| 202831_at | 211429_s_at | 228303_at |
| 202833_s_at | 212063_at | 228653_at |
| 203124_s_at | 213975_s_at | 228915_at |
| 203313_s_at | 214235_at | 231832_at |
| 203860_at | 214651_s_at | 231941_s_at |
| 203895_at | 217523_at | 232176_at |
| 203896_s_at | 217867_x_at | 232481_s_at |
| 204401_at | 218086_at | 234331_s_at |
| 204885_s_at | 218211_s_at | 235210_s_at |
| 205174_s_at | 218796_at | 235976_at |
| 205366_s_at | 219630_at | 236894_at |
| 205470_s_at | 219682_s_at | 238017_at |
| 205513_at | 219727_at | 238984_at |
| 205765_at | 219955_at; | 241031_at; and/or |

**(ii)**

| | | |
|---|---|---|
| APOBEC1 | HOXB6 | QPCT |
| BACE2 | IGFBP2 | RDHE2 |
| C20orf42 | ITGA6 | REG4 |
| CD44 | KCNN4 | RETNLB |
| CD55 | KLK11 | RP5-875H10.1 |
| CTSE | L1TD1 | RPESP |
| CYP3A5 | LILRB1 | SERPINA1 |
| CYP3A5P2 | MLPH | SLC11A2 |
| DACH1 | MSLN | SLC12A2 |
| DUOX2 | MUC20 | SLITRK6 |
| ETS2 | NLF1 | SPINK1 |
| FABP6 | NPDC1 | TBX3 |
| FAM84A | NQO1 | TCN1 |
| GALNT6 | PCCA | TGIF1 |
| GPSM2 | PCSK1 | WDR51B |
| GPX2 | PDZK1IP1 | ZNRF3 |
| HOXA9 | PLCB4. | |

9. The method according to paragraph 1 wherein said neoplasm is a cancer and said one or more genes or transcripts are selected from:
**(i)** the gene, genes or transcripts detected by Affymetrix probeset IDs:

| | | |
|---|---|---|
| 200660_at | 205476_at | 217430_x_at |
| 200665_s_at | 205479_s_at | 217996_at |
| 200832_s_at | 205713_s_at | 218507_at |
| 200903_s_at | 205815_at | 218963_s_at |
| 201014_s_at | 205828_at | 218984_at |
| 201112 _s_at | 205886_at | 219787_s_at |
| 201195_s_at | 205890_s_at | 219911_s_at |
| 201261_x_at | 205910_s_at | 221729_at |
| 201292_at | 205941_s_at | 221730_at |
| 201338_x_at | 206224_at | 221731_x_at |
| 201479_at | 206976_s_at | 221923_s_at |
| 201577_at | 207173_x_at | 37892_at |
| 201601_x_at | 207457_s_at | 222449_at |
| 201666_at | 208079_s_at | 222450_at |
| 202310_s_at | 208712_at | 222549_at |
| 202311_s_at | 209218_at | 222608_s_at |
| 202403_s_at | 209309_at | 223062_s_at |
| 202404_s_at | 209752_at | 224428_s_at |
| 202431_s_at | 209773_s_at | 224646_x_at |
| 202504_at | 209774_x_at | 224915_x_at |
| 202779_s_at | 209792_s_at | 225295_at |
| 202859_x_at | 209875_s_at | 225520_at |
| 202954_at | 209955_s_at | 225664_at |
| 202998_s_at | 210052_s_at | 225681_at |
| 203083_at | 210511_s_at | 225767_at |
| 203213_at | 210559_s_at | 225799_at |
| 203878_s_at | 210766_s_at | 225806_at |
| 204051_s_at | 211506_s_at | 226227_x_at |
| 204127_at | 212281_s_at | 226237_at |
| 204170_s_at | 212344_at | 226311_at |
| 204320_at | 21353_at | 226777_at |
| 204470_at | 212354_at | 226835_s_at |
| 204475_at | 213905_x_at | 227140_at |
| 204580 at | 214022_s_at | 229802_at |
| 204620_s_at | 214974_x_at | 231766_s_at |
| 204702_s_at | 215091_s_at; | 232151_at; and/or |
| 205361_s_at | | |

**(ii)**

| | | |
|---|---|---|
| AHCY | DKC1 | PSAT1 |
| ANLN | ECT2 | PUS7 |
| AURKA | FAP | REG1A |
| AZGP1 | GTF3A | REG1B |
| BGN | H19 | REG3A |
| C14orf94 | HIG2 | RFC3 |
| C20orf199 | HSPH1 | RRM2 |
| CCL20 | IFITM1 | S100A11 |
| CCND1 | IL8 | SCD |
| CDC2 | INHBA | SFRP4 |
| CDCA7 | KLK10 | SLC39A10 |
| CDH11 | KRT23 | SLC7A5 |
| CEL | LOC541471 | SLCO4A1 |
| CKS2 | LOXL2 | SPARC |
| CLDN1 | LY6G6D | SPP1 |
| COL10A1 | MMP1 | SQLE |
| COL11A1 | MMP11 | SULF1 |
| COL12A1 | MMP12 | THBS2 |
| COL1A1 | MMP3 | TIMP1 |
| COL1A2 | MTHFD1L | TMEM97 |
| COL5A2 | MYC | TMEPAI |
| COL8A1 | NFE2L3 | TOP2A |
| COMP | NME1 | TPX2 |
| CSEIL | NPM1 | TRIM29 |
| CST1 | PAICS | UBD |
| CTHRC1 | PFDN4 | UBE2C |
| CXCL1 | PHLDA1 | UBE2S |
| CXCL2 | PLAU | VCAN |
| CXCL5. | | |

10. A method of screening for the onset of a large intestine neoplasm in an individual, said method comprising measuring the level of expression of one or more genes or transcripts selected from:
**(i)** the gene, genes or transcripts detected by Affymetrix probeset IDs:

| | | |
|---|---|---|
| 202286_s_at | 235976_at | 209309_at |
| 204259_at | 236894_at | 211506_s_at |
| 204885_s_at | | 214974_x_at |
| 205174_s_at | 238984_at | 219787_s_at |
| 205825_at | 241031_at | 37892_at |
| 207850_at | 202311_s_at | 222608_s_at |
| 213880_at | 204320_at | 223062_s_at |
| 217523_at | 204475_at | 225806_at |
| 227174_at | 204702_s_at | 226237_at |
| 228915_at | 205910_s_at | 227140_at |
| 232252 at | 206224 at | 229802_at; and/or |

**(ii)**

| | | |
|---|---|---|
| MMP1 | PCSK1 | ANLN |
| MMP7 | CST1 | DACH1 |
| LGR5 | QPCT | COL11A1 |
| WDR72 | ECT2 | C14orf94 |
| COL11A1 | SLITRK6 | AZGP1 |
| COL1A1 | LITD1 | REG4 |
| DUSP27 | KIAA1199 | NFE2L3 |
| NLF1 | PSAT1 | CEL |
| IL8 | CXCL5 | CD44 |
| TACSTD2 | CXCL3 | COL8A1 |
| MSLN | | |

in a biological sample from said individual wherein a higher level of expression of the genes or transcripts of group (i) and/or group (ii) relative to background levels is indicative of a neoplastic cell or a cell predisposed to the onset of a neoplastic state.
11. The method according to paragraph 10 wherein said neoplastic cell is an adenoma or adenocarcinoma.
12. The method according to any one of paragraphs 10 to 11 wherein said neoplasm is an adenoma and said one or more genes or transcripts are selected from:
**(i)** the gene, genes or transcripts detected by Affymetrix probeset IDs: 204885_s_at, 217523_at, 236894_at, 205174_s_at, 228915_at, 238984_at, 205825_at, 235976_at, 241031_at; and/or
**(ii)** CD44, MSLN, QPCT, DACH1, NLF1, REG4, L1TD1, PCSK1, SLITRK6.

13. The method according to any one of paragraphs 13 to 15 wherein said neoplasm is a cancer and said one or more genes or transcripts are selected from:
**(i)** the gene or genes detected by Affymetrix probeset IDs:

| | | |
|---|---|---|
| 202311_s_at | 209309_at | 223062_s_at |
| 204320_at | 211506_s_at | 225806_at |
| 204475_at | 214974_x_at | 226237_at |
| 204702_s_at | 219787_s_at | 227140_at |
| 205910_s_at | 37892_at | 229802_at; and/or |
| 206224_at | 222608_s_at | |

**(ii)**

| | | |
|---|---|---|
| ANLN | COL1A1 | IL8 |
| AZGP1 | COL8A1 | MMP1 |
| C14orf94 | CST1 | NFE2L3 |
| CEL | CXCL5 | PSAT1 |
| COL11A1 | ECT2. | |

14. The method according to any one of paragraphs 10-13 wherein said background level is the background of the testing methodology.
15. A method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 200884_at | 214234_s_at | 226248_s_at |
| 203240_at | 214235_at | 226302_at |
| 203963_at | 214433_s_at | 227676_at |
| 204508_s_at | 215125_s_at | 227719_at |
| 20460_at | 215867_x_at | 227725_at |
| 204811_s_at | 217109_at | 228232_s_at |
| 204895_x_at | 217110_s_at | 229070_at |
| 204897_at | 218211_s_at | 231832_at |
| 205259_at | 219543_at | 232176_at |
| 205765_at | 219955_at | 232481_s_at |
| 205927_s_at | 221841_s_at | 235976_at |
| 208063_s_at | 221874_at | 236894_at |
| 208937_s_at | 223969_s_at | 237521_x_at |
| 210107_at | 223970_at | 242601_at |
| 213106_at; and/or | | |

**(ii)**

| | | |
|---|---|---|
| CLCA1 | CTSE | ATP8B1 |
| FCGBP | C6orf105 | CACNA2D2 |
| HMGCS2 | CKB | KLF4 |
| RETNLB | ATP8A1 | CYP3A5P2 |
| LITD1 | MUC4 | CAPN9 |
| SLITRK6 | UGT1A1 | NR3C2 |
| VSIG2 | SELENBP1 | PBLD |
| LOC253012 | PTGER4 | CA12 |
| ST6GALNAC1 | MLPH | WDR51B |
| ID1 | KIAA1324 | FAM3D |
| CYP3A5 | | |

in said cell or cellular population wherein a higher level of expression of the genes or transcripts of group (i) and/or group (ii) relative to a gastrointestinal cancer cell level is indicative of an adenoma cell or a cell predisposed to the onset of an adenoma state.
16. A method of characterising a neoplastic cell or cellular population, which cell or cellular population is derived from the large intestine of an individual, said method comprising measuring the level of expression of one or more genes or transcripts selected from:
**(i)** the gene, genes or transcripts detected by Affymetrix probeset IDs:

| | | |
|---|---|---|
| 200600_at at | 204006_s_at | 213428_s_at |
| 200665_s_at | 204051_s_at | 213524_s_at |
| 200832_s_at | 204122_at | 213869_x_at |
| 200974_at | 204320_at | 213905_x_at |
| 200986_at | 204475_at | 214247_s_at |
| 201058_s_at | 204620_s_at | 215049_x_at |
| 201069_at | 205479_s_at | 215076_s_at |
| 201105_at | 205547_s_at | 215646_ s_at |
| 201141_at | 205828_at | 216442_ x_at |
| 201147_s_at | 207173_x_at | 217430_x_at |
| 201150_s_at | 207191_s_at | 217762_s_at |
| 201162_at | 208747_s_at | 217763_s_at |
| 201163_s_at | 208782_at | 217764_s_at |
| 201185_at | 208788_at | 218468_s_at |
| 201261_x_at | 208850_s_at | 218469_at |
| 201289_at | 208851_s_at | 218559_s_at |
| 201426_s_at | 209101_at | 218638_s_at |
| 201438_at | 209156_s_at | 219087_at |
| 201616_s_at | 209218_at | 221011_s_at |
| 201645_at | 209395_at | 221729_at |
| 201667_at | 209396_s_at | 221730_at |
| 201744_s_at | 209596_at | 221731_ x_at |
| 201792_at | 209875_s_at | 37892_at |
| 201842_s_at | 209955_s_at | 223122_s_at |
| 201852_x_at | 210095_s_at | 223235_s_at |
| 201859_at | 210495_x_at | 224560_at |
| 201893_x_at | 210511_s_at | 224694_at |
| 202237_at | 210764_s_at | 224724_at |
| 202238_s_at | 210809_s_at | 225664_at |
| 202283_at | 211161_s_at | 225681_at |
| 202291_s_at | 211571_s_at | 225710_at |
| 202310_s_at | 211719_x_at | 225799_at |
| 202311_s_at | 211813_x_at | 226237_at |
| 202403_s_at | 211896_s_at | 22631 1_at |
| 202404_s_at | 211959_at | 226694_at |
| 202450_s_at | 211964_at | 226777_at |
| 202620_s_at | 211966_at | 226930_at |
| 202766 s_at | 211980 at | 227099_s_at |
| 202859_x_at | 211981_at | 227140_at |
| 202878_s_at | 212077_at | 227566_at |
| 202917_s_at | 212344_at | 229218_at |
| 202998_s_at | 212353_at | 229802_at |
| 203083_at | 212354_at | 231579_s_at |
| 203325_s_at | 212464_s_at | 231766_s_at |
| 203382_s_at | 212488_at | 231879_at |
| 203477_at | 212489_at | 232458_at |
| 203570_at | 212667_at | 233555_s_at |
| 203645_s_at | 213125_at | 234994_at |
| 203878_s_at; and/or | | |

**(ii)**

| | | |
|---|---|---|
| COL1A2 | LGALS1 | SRGN |
| CTHRC1 | ELOVL5 | LBH |
| FN1 | MGP | CTGF |
| POSTN | MMP2 | TNC |
| SPP1 | LOXL2 | G0S2 |
| MMP1 | MYL9 | SQLE |
| SPARC | DCN | EFEMP1 |
| LUM | CALD1 | APOE |
| GREM1 | FBN1 | MSN |
| IL8 | MMP3 | IGFBP3 |
| IGFBP5 | IGFBP7 | SERPINF1 |
| SFRP2 | FSTL1 | ISLR |
| SULF1 | COL4A2 | HNT |
| ASPN | VCAN | COL5A1 |
| COL6A3 | SMOC2 | OLFML2B |
| COL8A1 | HTRA1 | KIAA1913 |
| COL12A1 | CYR61 | PALM2-AKAP2 |
| COL5A2 | FAP | SERPING1 |
| CDH11 | VIM | TYROBP |
| THBS2 | TIMP2 | ACTA2 |
| COL15A1 | SCD | COL3A1 |
| COL11A1 | TIMP3 | PLOD2 |
| S100A8 | AEBP1 | MMP11 |
| FNDC1 | GJA1 | CD163 |
| SFRP4 | NNMT | FCGR3B |
| INHBA | COL1A1 | PLAU |
| COL6A2 | SULF2 | MAFB |
| ANTXR1 | COL6A1 | LOC541471 |
| GPNMB | SPON2 | LOC387763 |
| BGN | CTSK | CHI3L1 |
| TAGLN | MXRA5 | THY1 |
| COL4A1 | CIS | LOXL1 |
| RAB31 | DKK3 | CD93 |

in a biological sample from said individual wherein a higher level of expression of the genes or transcripts of group (i) and/or group (ii) relative to a gastrointestinal adenoma cell level is indicative of a cancer or a cell predisposed to the onset of a cancerous state.
17. The method according to paragraph 15 wherein said one or more genes or transcripts are selected from:
**(i)** the gene or genes detected by Affymetrix probeset IDs: 210107_at; and/or
**(ii)** CLCA1.

18. The method according to paragraph 15 wherein said one or more genes or transcripts are selected from:
**(i)** the gene, genes or transcripts detected by Affymetrix probeset IDs: 203240_at, 219955_at, 242601_at, 204607_at, 232481_s_at, 227725_at, 223969_s_at, 228232_s_at; and/or
**(ii)** FCGBP, L1TD1, LOC253012, HMGCS2, SLITRK6, ST6GALNAC1, RETNLB, VSIG2.

19. The method according to paragraph 16 wherein said one or more genes or transcripts are selected from:
**(i)** the gene, genes or transcripts detected by Affymetrix probeset IDs: 202404_s_at, 210809_s_at, 227140_at, 225681_at, 209875_s_at, 204475_at, 212464_s_at; and/or
**(ii)** COL1A2, FN1, SPP1, CTHRC1, POSTN, MMP1.

20. The method according to paragraph 16 wherein said one or more genes or transcripts are selected from:
**(i)** the gene, genes or transcripts detected by Affymetrix probeset IDs: 200665_s_at, 218468_s_at, 211959_at, 201744_s_at, 202859_x_at; and/or
**(ii)** SPARC, GREM1, IGFBP5, LUM, IL8.

21. The method according to paragraph 16 wherein said one or more genes or transcripts are selected from:
**(i)** the gene, genes or transcripts detected by Affymetrix probeset IDs:

| | | |
|---|---|---|
| 223122_s_at | 207173_x_at | 210511_s_at |
| 212353_at | 203083_at | 209156_s_at |
| 219087_at | 203477_at | 224694_at |
| 201438_at | 37892_at | 201141_at |
| 226237_at | 202917_s_at | 213905_x_at |
| 225664_at | 226930_at | 205547_s_at |
| 221730_at | 204051_s_at; | and/or |

**(ii)**

| | | |
|---|---|---|
| SFRP2 | CDH11 | INHBA |
| SULF1 | THBS2 | COL6A2 |
| ASPN | COL15A1 | ANTXR1 |
| COL6A3 | COL11A1 | GPNMB |
| COL8A1 | S100A8 | BGN |
| COL12A1 | FNDC1 | TAGLN |
| COL5A2 | SFRP4. | |

22. The method according to paragraph 15 wherein said control level is the background level of neoplastic tissue and said one or more genes or transcripts are selected from:
**(i)** the gene, genes or transcripts detected by Affymetrix probeset IDs:

| | |
|---|---|
| 235976_at | 236894_at; and/or |

**(ii)**

| | |
|---|---|
| SLITRK6 | L1TD1. |

23. The method according to paragraph 16 wherein said control level is the background level of neoplastic tissue and said one or more genes or transcripts selected from:
**(i)** the gene, genes or transcripts detected by Affymetrix probeset IDs: 202311_s_at, 209396_s_at, 226237_at, 204320_at, 215646_s_at, 227140_at, 204475_at, 37892_at, 229802_at, 209395_at; and/or,
**(ii)** COL1A1, VCAN, CHI3L1, MMP1, COL8A1, COL11A1.

24. The method according to any one of paragraphs 1-23 wherein said cell is a colorectal cell.
25. The method according to any one of paragraphs 1 to 24 wherein said biological sample is a faecal sample, enema wash, surgical resection, tissue biopsy or blood sample.
26. The method according to any one of paragraphs 1 to 25 wherein said level of expression is mRNA expression or protein expression.
27. The method according to any one of paragraphs 1-26 wherein said individual is a human.
28. A molecular array, which array comprises a plurality of:
(i) nucleic acid molecules comprising a nucleotide sequence corresponding to any one or more of the neoplastic marker genes listed in any one of paragraphs 1-23 or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (iv) is indicative of the neoplastic state of a cell or cellular subpopulation derived from the large intestine.
29. A diagnostic kit for assaying biological samples said kit comprising an agent for detecting one or more of the neoplastic markers listed in paragraphs 1-23 and reagents useful for facilitating the detecting by said agent.
30. The method according to any one of paragraphs 1-9 wherein said control level is a non-neoplastic level.

## Claims

1. A method of screening for the onset or predisposition to the onset of a malignant large intestine neoplasm or monitoring the progress of a malignant large intestine neoplasm in an individual, said method comprising measuring the level of expression of one or more genes or transcripts selected from:
**(i)** the gene, genes or transcripts detected by Affymetrix probeset ID 205513_at; and/or
**(ii)** TCN1
in a biological sample from said individual wherein a higher level of expression of the genes or transcripts of group (i) and/or group (ii) relative to control levels is indicative of a malignant neoplastic large intestine cell or a cell predisposed to the onset of a malignant neoplastic state.

2. The method according to claim 1 wherein said control level is a non-neoplastic level.

3. The method according to any one of claims 1-2 wherein said neoplastic cell is an adenocarcinoma.

4. The method according to any one of claims 1-3 wherein said cell is a colorectal cell.

5. The method according to any one of claims 1-4 wherein said biological sample is a faecal sample, enema wash, surgical resection, tissue biopsy or blood sample.

6. The method according to any one of claims 1 to 5 wherein said level of expression is mRNA expression or protein expression.

7. The method according to any one of claims 1-6 wherein said individual is a human.

## Patentansprüche

1. Verfahren zum Screening auf den Beginn oder die Prädisposition für den Beginn eines bösartigen Dickdarmneoplasmas oder zum Überwachen des Fortschritts eines bösartigen Dickdarmneoplasmas in einem Individuum, wobei das Verfahren das Messen des Expressionsspiegels eines oder mehrerer Gene oder Transkripte umfasst, die ausgewählt sind aus:
**(i)** dem Gen, den Genen oder Transkripten, die durch den Affymetrix-Sonden-Satz-ID 205513_at nachgewiesen werden;
und/oder
**(ii)** TCN1
in einer biologischen Probe von dem Individuum, wobei ein höherer Expressionsspiegel der Gene oder Transkripte der Gruppe (i) und/oder der Gruppe (ii) in Bezug auf die Kontrollspiegel auf eine maligne neoplastische Dickdarmzelle oder eine Zelle hinweist, die für den Beginn eines malignen neoplastischen Zustands prädisponiert ist.

2. Verfahren nach Anspruch 1, wobei der Kontrollspiegel ein nicht-neoplastischer Spiegel ist.

3. Verfahren nach einem der Ansprüche 1-2, wobei die neoplastische Zelle ein Adenokarzinom ist.

4. Verfahren nach einem der Ansprüche 1-3, wobei die Zelle eine kolorektale Zelle ist.

5. Verfahren nach einem der Ansprüche 1-4, wobei die biologische Probe eine Stuhlprobe, Klistierwäsche, chirurgische Resektion, Gewebebiopsie oder Blutprobe ist.

6. Verfahren nach einem der Ansprüche 1 bis 5, wobei der Expressionsspiegel mRNA-Expression oder Proteinexpression ist.

7. Verfahren nach einem der Ansprüche 1-6, wobei das Individuum ein Mensch ist.

## Revendications

1. Méthode de dépistage de l'apparition ou d'une prédisposition à l'apparation d'un néoplasme malin du gros intestin ou de surveillance du développement d'un néoplasme malin du gros intestin chez un individu, ladite méthode comprenant une mesure du niveau d'expression d'un ou de plusieurs gènes ou de transcriptions sélectionnés à partir de :
(i) le gène, les gènes ou les transcriptions détectés par un ensemble de sondes ID 205513_at Affymetrix ; et/ou
(ii) TCN1
dans un échantillon biologique dudit individu dans lequel un niveau plus élevé d'expression des gènes ou des transcriptions du groupe (i) et/ou du groupe (ii) par rapport à des niveaux témoins est une indication d'une cellule néoplsique maligne du gros instestin ou d'une cellule prédisposée à l'apparition d'un état néoplasique malin.

2. Méthode selon la revendication 1, dans laquelle ledit niveau témoin est une niveau non néoplasique.

3. Méthode selon l'une quelconque des revendications 1 et 2, dans laquelle ladite cellule néoplasique est un adénocarcinome.

4. Méthode selon l'une quelconque des revendications 1 à 3, dans laquelle ladite cellule est une cellule colorectale.

5. Méthode selon l'une quelconque des revendications 1 à 4, dans laquelle ledit échantillon biologique est un échantillon fécal, un lavement, une résection chirurgicale, une biopsie tissulaire ou un échantillon sanguin.

6. Méthode selon l'une quelconque des revendications 1 à 5, dans laquelle ledit niveau d'expression est une expression de l'ARNm ou une expression protéique.

7. Méthode selon l'une quelconque des revendications 1 à 6, dans laquelle ledit individu est un humain.


### SEQUENCE LISTING

<110> Clinical Genomics Pty. Ltd. LaPointe, Lawrence Dunne, Rob Young, Graeme Lockett, Trevor Wilson, William
<120> A method of diagnosing neoplasms
<130> 30637616/TDO
<150> US 60/982114
   <151> 2007-10-23
<160> 206
<170> PatentIn version 3.4
<210> 1
   <211> 441
   <212> DNA
   <213> human
<400> 1
   caaggctggg ccgggaaggg cgtgggttga ggagaggctc cagacccgca cgccgcgcgc      60
   acagagctct cagcgccgct cccagccaca gcctcccgcg cctcgctcag ctccaacatg     120
   gcaaaaatct ccagccctac agagactgag cggtgcatcg agtccctgat tgctgtcttc     180
   cagaagtatg ctggaaagga tggttataac tacactctct ccaagacaga gttcctaagc     240
   ttcatgaata cagaactagc tgccttcaca aagaaccaga aggaccctgg tgtccttgac     300
   cgcatgatga agaaactgga caccaacagt gatggtcagc tagatttctc agaatttctt     360
   aatctgattg gtggcctagc tatggcttgc catgactcct tcctcaaggc tgtcccttcc     420
   cagaagcgga cctgaggacc c                                               441
<210> 2
   <211> 416
   <212> DNA
   <213> human
<400> 2
   gttggttcaa acttttggga gcacggactg tcagttctct gggaagtggt cagcgcatcc      60
   tgcagggctt ctcctcctct gtcttttgga gaaccagggc tcttctcagg ggctctaggg     120
   actgccaggc tgtttcagcc aggaaggcca aaatcaagag tgagatgtag aaagttgtaa     180
   aatagaaaaa gtggagttgg tgaatcggtt gttctttcct cacatttgga tgattgtcat     240
   aaggttttta gcatgttcct ccttttcttc accctcccct ttgttcttct attaatcaag 300
   agaaacttca aagttaatgg gatggtcgga tctcacaggc tgagaactcg ttcacctcca 360
   agcatttcat gaaaaagctg cttcttatta atcatacaaa ctctcaccat gatgtg 416
<210> 3
   <211> 541
   <212> DNA
   <213> human
<400> 3
   aaaagcgagg tggccatgtt atgctggtgg ttgccagggc ctccaaccac tgtgccactg 60
   acttgctgtg tgaccctggg caagtcactt aactataagg tgcctcagtt ttccttctgt 120
   taaaatgggg ataataatac tgacctacct caaagggcag ttttgaggca tgactaatgc 180
   tttttagaaa gcattttggg atccttcagc acaggaattc tcaagacctg agtatttttt 240
   ataataggaa tgtccaccat gaacttgata cgtccgtgtg tcccagatgc tgtcattagt 300
   ctatatggtt ctccaagaaa ctgaatgaat ccattggaga agcggtggat aactagccag 360
   acaaaatttg agaatacata aacaacgcat tgccacggaa acatacagag gatgcctttt 420
   ctgtgattgg gtgggatttt ttcccttttt atgtgggata tagtagttac ttgtgacaag 480
   aataattttg gaataatttc tattaatatc aactctgaag ctaattgtac taatctgaga 540
   t 541
<210> 4
   <211> 357
   <212> DNA
   <213> human
<400> 4
   gcagcgggaa cagagtaccc tcttcaagcc ccggtcatga tggaggtccc agccacaggg 60
   aaccatgagc tcagtggtct tggaacagct cactaagtca gtccttcctt agcctggaag 120
   ccagtagtgg agtcacaaag cccatgtgtt ttgccatcta ggccttcacc tggtctgtgg 180
   acttatacct gtgtgcttgg tttacaggtc cagtggttct tcagcccatg acagatgaga 240
   aggggctata ttgaagggca aagaggaact gttgtttgaa ttttcctgag agcctggctt 300
   agtgctgggc cttctcttaa acctcattac aatgaggtta gtacttttag tccctgt 357
<210> 5
   <211> 241
   <212> DNA
   <213> human
<400> 5
   agtgttgatg ggctctactt ctgatcttgg tcactgtgaa aaaatcaaga aggcctgtgg 60
   aaattttggc attccatgtg aacttcgagt aacatctgcg cataaaggac cagatgaaac 120
   tctgaggatt aaagctgagt atgaagggga tggcattcct actgtatttg tggcagtggc 180
   aggcagaagt aatggtttgg gaccagtgat gtctgggaac actgcatatc cagttatcag 240
   c 241
<210> 6
   <211> 312
   <212> DNA
   <213> human
<400> 6
   agatctgtgc ggttggcata accaacttac taacagaatg tcccccaatg atggacactg 60
   agtataccaa actgtggact ccattattac agtctttgat tggtcttttt gagttacccg 120
   aagatgatac cattcctgat gaggaacatt ttattgacat agaagataca ccaggatatc 180
   agactgcctt ctcacagttg gcatttgctg ggaaaaaaga gcatgatcct gtaggtcaaa 240
   tggtgaataa ccccaaaatt cacctggcac agtcacttca catgttgtct accgcctgtc 300
   caggaagggt tc 312
<210> 7
   <211> 522
   <212> DNA
   <213> human
<400> 7
   tcagaaggta ggggccgtgt cccgcggtgc tgactgaggc ctgcttcccc ctccccctcc 60
   tgctgtgctg gaattccaca gggaccaggg ccaccgcagg ggactgtctc agaagacttg 120
   atttttccgt ccctttttct ccacactcca ctgacaaacg tccccagcgg tttccacttg 180
   tgggcttcag gtgttttcaa gcacaaccca ccacaacaag caagtgcatt ttcagtcgtt 240
   gtgctttttt gttttgtgct aacgtcttac taatttaaag atgctgtcgg caccatgttt 300
   atttatttcc agtggtcatg ctcagccttg ctgctctgcg tggcgcaggt gccatgcctg 360
   ctccctgtct gtgtcccagc cacgcagggc catccactgt gacgtcggcc gaccaggctg 420
   gacaccctct gccgagtaat gacgtgtgtg gctgggacct tctttattct gtgttaatgg 480
   ctaacctgtt acactgggct gggttgggta gggtgttctg gc 522
<210> 8
   <211> 479
   <212> DNA
   <213> human
<400> 8
   tctctctttc tgtgtgtgtg tgtgtgtgtg tgtgtgtgtg tgtgtgtgtc ttgtgcttcc      60
   tcagaccttt ctcgcttctg agcttggtgg cctgttccct ccatctctcc gaacctggct     120
   tcgcctgtcc ctttcactcc acaccctctg gccttctgcc ttgagctggg actgctttct     180
   gtctgtccgg cctgcaccca gcccctgccc acaaaacccc agggacagcg gtctccccag     240
   cctgccctgc tcaggccttg cccccaaacc tgtactgtcc cggaggaggt tgggaggtgg     300
   aggcccagca tcccgcgcag atgacaccat caaccgccag agtcccagac accggttttc     360
   ctagaagccc ctcaccccca ctggcccact ggtggctagg tctcccctta tccttctggt     420
   ccagcgcaag gaggggctgc ttctgaggtc ggtggctgtc tttccattaa agaaacacc       479
<210> 9
   <211> 407
   <212> DNA
   <213> human
<400> 9
   tacagatact ctactacact cagcctctta tgtgccaagt ttttctttaa gcaatgagaa       60
   attgctcatg ttcttcatct tctcaaatca tcagaggccg aagaaaaaca ctttggctgt     120
   gtctataact tgacacagtc aatagaatga agaaaattag agtagttatg tgattatttc     180
   agctcttgac ctgtcccctc tggctgcctc tgagtctgaa tctcccaaag agagaaacca     240
   atttctaaga ggactggatt gcagaagact cggggacaac atttgatcca agatcttaaa     300
   tgttatattg ataaccatgc tcagcaatga gctattagat tcattttggg aaatctccat     360
   aatttcaatt tgtaaacttt gttaagacct gtctacattg ttatatg                   407
<210> 10
   <211> 547
   <212> DNA
   <213> human
<220>
   <221> misc_feature
   <222> (97)..(99)
   <223> n is a, c, g, or t
<220>
   <221> misc_feature
   <222> (135)..(135)
   <223> n is a, c, g, or t
<220>
   <221> misc_feature
   <222> (258)..(258)
   <223> n is a, c, g, or t
<400> 10
   agcatcggag ccattcattc ggagaaaacg ttttgatcaa aatggagact tttgtagtcg     60
   tttcaaaaga gcacctgagt catgtgtatt cccggcnnnc tttataaatg acccggtcaa    120
   gttggtttca aagtncgaca ggcttgtctg tttactagct gcgtggcctt ggacgggtgg    180
   ctgacatctg taaagaatcc tcctgtgatg aaactgagga atcgggtggc cgggcaagct    240
   gggaagagca aagccagnag ctgcgctgcc tcaataccca caaaagacca ttcccagtat    300
   acataagcac aggatgtttt tctcaagagg gatgtattta tcacttggac atctgtttat    360
   aatataaaca gacatgtgac tgggaacatc ttgctgccaa aagaatccta ggcagtggct    420
   cattgtatgt gaggttgaac cacgtgaaat tgccaatatt aggctggctt ttatctacaa    480
   agaaggagtt tcatggggtt cagcctaaca gttatggaaa ctacagtcct tataaaccat    540
   tggcatg                                                              547
<210> 11
   <211> 424
   <212> DNA
   <213> human
<400> 11
   gatgtatgtc gctgtccaag agaaggctgt ggaagaacct atacaactgt gtttaatctc     60
   caaagccata tcctctcctt ccatgaggaa agccgccctt ttgtgtgtga acatgctggc    120
   tgtggcaaaa catttgcaat gaaacaaagt ctcactaggc atgctgttgt acatgatcct    180
   gacaagaaga aaatgaagct caaagtcaaa aaatctcgtg aaaaacggga gtttggcctc    240
   tcatctcagt ggatatatcc tcccaaaagg aaacaagggc aaggcttatc tttgtgtcaa    300
   aacggagagt cacccaactg tgtggaagac aagatgctct cgacagttgc agtacttacc    360
   cttggctaag aactgcactg ctttgtttaa aggactgcag accaaggagt cgagctttct    420
   ctca                                                                 424
<210> 12
   <211> 375
   <212> DNA
   <213> human
<400> 12
   ggtctgttct tgaatcctct attaattact gtgtgtgagc cagagggagc tgtggtaagg 60
   gttgggcccc cagcctgtag ggaactttct ggactcccac tctttgaatc gatataggca 120
   tttggtctca ctacttgacc attctcaccc tgtgaaacgt cccacacttt gaagcaaata 180
   caattcacag cacagtacac acaaaaacct tggcataaga cagagaaggt tcttcttatt 240
   ttgtgggctg gttgctgtag aaacatataa caaagggcag ccctccactt ctggtataat 300
   tgtgtagccc cttttctttg ggcttgacac ctgtcttgaa taagagtgat tagagctgca 360
   taatgtccct ctctt 375
<210> 13
   <211> 533
   <212> DNA
   <213> human
<220>
   <221> misc_feature
   <222> (360)..(361)
   <223> n is a, c, g, or t
<400> 13
   gaggtcagat ttggagcttc tcattgcacg cggagattat tattgcatcg ggttccaagc 60
   caatgggaag cccgggggag gggtttggca tgaggaagcg ttggttacag cagctgattg 120
   gctgcagcca agactgtgaa aggataaaga ggcgcgaggc ggaattgggg tctgctctaa 180
   gctgcagcaa gagaaactgt gtgtgagggg aagaggcctg tttcgctgtc gggtctctag 240
   ttcttgcacg ctctttaaga gtctgcactg gaggaactcc tgccattacc agctcccttc 300
   ttgcagaagg gagggggaaa catacattta ttcatgccag tctgttgcat gcaggctttn 360
   nggcttccta ccttgcaaca aaataattgc accaactcct tagtgccgat tccgcccaca 420
   gagagtcctg gagccacagt cttttttgct ttgcattgta ggagagggac taagtgctag 480
   agactatgtc gctttcctga gctaccgaga gcgctcgtga actggaatca act 533
<210> 14
   <211> 408
   <212> DNA
   <213> human
<400> 14
   gtaaaccaca tcttttttgc acttttttta taagcaaaaa cgtgccgttt aaaccactgg 60
   atctatctaa atgccgattt gagttcgcga cactatgtac tgcgtttttc attcttgtat 120
   ttgactattt aatcctttct acttgtcgct aaatataatt gttttagtct tatggcatga 180
   tgatagcata tgtgttcagg tttatagctg ttgtgtttaa aaattgaaaa aagtggaaaa 240
   catctttgta catttaagtc tgtattataa taagcaaaaa gattgtgtgt atgtatgttt 300
   aatataacat gacaggcact aggacgtctg cctttttaag gcagttccgt taagggtttt 360
   tgtttttaaa cttttttttg ccatccatcc tgtgcaatat gccgtgta 408
<210> 15
   <211> 414
   <212> DNA
   <213> human
<400> 15
   gatgacttac catgggatgg ggtccagtcc catgaccttg gggtacaatt gtaaacctag 60
   agttttatca actttggtga acagttttgg cataatagtc aatttctact tctggaagtc 120
   atctcattcc actgttggta ttatataatt caaggagaat atgataaaac actgccctct 180
   tgtggtgcat tgaaagaaga gatgagaaat gatgaaaagg ttgcctgaaa aatgggagac 240
   agcctcttac ttgccaagaa aatgaaggga ttggaccgag ctggaaaacc tcctttacca 300
   gatgctgact ggcactggtg gtttttgctc tcgacatatc cacaatagct gacggctggg 360
   tgtttcagtt tgcaaaatat tttgttgcct tcatcttcac tgcaattttg tgta 414
<210> 16
   <211> 481
   <212> DNA
   <213> human
<400> 16
   ttcagaccca gtaactgtcc gcagctgtct gctagtggtt gtcttaacat cgtagtccta 60
   gtttgcattt tttaaatccc ctctgtttaa aaggtttgta aaacaaaaac aaaaaactaa 120
   gtctgctcag tgaaatgctg tagaacccta aataagtggt agaagagtgt cactgaattt 180
   tgtctctgaa ttcagtataa ctgagttttg tccatgctgg tgtctgggtt ataggcctga 240
   tgggcctggt agttttccat cttgttctgg cctagaggtc agtcctttgc acttcctcaa 300
   agcttgtgta cagtgctcac ctaaatccat ctgactactt gttcctgtgc cctcttgttt 360
   taggcctcgt ttacttttaa aaaatgaaat tgttcattgc tgggagaaga atgttgtaat 420
   ttttacttat taaagtcaac ttgttaagtt ttttatgtat tcctgttggg ttttcttgtt 480
   g 481
<210> 17
   <211> 545
   <212> DNA
   <213> human
<400> 17
   acaggaggaa tgcaccacgg cagctctccg ccaatttctc tcagatttcc acagagactg 60
   tttgaatgtt ttcaaaacca agtatcacac tttaatgtac atgggccgca ccataatgag 120
   atgtgagcct tgtgcatgtg ggggaggagg gagagagatg tactttttaa atcatgttcc 180
   ccctaaacat ggctgttaac ccactgcatg cagaaacttg gatgtcactg cctgacattc 240
   acttccagag aggacctatc ccaaatgtgg aattgactgc ctatgccaag tccctggaaa 300
   aggagcttca gtattgtggg gctcataaaa catgaatcaa gcaatccagc ctcatgggaa 360
   gtcctggcac agtttttgta aagcccttgc acagctggag aaatggcatc attataagct 420
   atgagttgaa atgttctgtc aaatgtgtct cacatctaca cgtggcttgg aggcttttat 480
   ggggccctgt ccaggtagaa aagaaatggt atgtagagct tagatttccc tattgtgaca 540
   gagcc 545
<210> 18
   <211> 446
   <212> DNA
   <213> human
<400> 18
   aggaaactgc tacttgtgga cctcaccaga gaccaggagg gtttggttag ctcacaggac 60
   ttcccccacc ccagaagatt agcatcccat actagactca tactcaactc aactaggctc 120
   atactcaatt gatggttatt agacaattcc atttctttct ggttattata aacagaaaat 180
   ctttcctctt ctcattacca gtaaaggctc ttggtatctt tctgttggaa tgatttctat 240
   gaacttgtct tattttaatg gtgggttttt tttctggtaa gatttagacc taaatcgcat 300
   catgccaact tgtgactttg agactattca tcaagaatga ggatatagta gccatgacat 360
   agcttgagct atagccttta attccttact ttggctatgg gtggagggtg agtttgaaga 420
   ggttctgatt ttcttgtaac ctggga 446
<210> 19
   <211> 521
   <212> DNA
   <213> human
<400> 19
   gattccgcct tgttggtctg aaattcatgc aagcttccga agatcttctc aaggaacact      60
   acgttgacct gaaggaccgt ccattctttg ccggcctggt gaaatacatg cactcagggc     120
   cggtagttgc catggtctgg gaggggctga atgtggtgaa gacgggccga gtcatgctcg     180
   gggagaccaa ccctgcagac tccaagcctg ggaccatccg tggagacttc tgcatacaag     240
   ttggcaggaa cattatacat ggcagtgatt ctgtggagag tgcagagaag gagatcggct     300
   tgtggtttca ccctgaggaa ctggtagatt acacgagctg tgctcagaac tggatctatg     360
   aatgacagga gggcagacca cattgctttt cacatccatt tcccctcctt cccatgggca     420
   gaggaccagg ctgtaggaaa tctagttatt tacaggaact tcatcataat ttggagggaa     480
   gctcttggag ctgtgagttc tccctgtaca gtgttaccat c                         521
<210> 20
   <211> 482
   <212> DNA
   <213> human
<400> 20
   agaaaaccac acttctcata ccttcactca acacttcctt ccccaaagcc agaagatgca      60
   caaggaggaa catgaggtgg ctgtgctggg ggcacccccc agcaccatcc ttccaaggtc     120
   caccgtgatt aacatccaca gcgagacctc cgtgcccgac catgtcgtct ggtccctgtt     180
   caacaccctc ttcttgaact ggtgctgtct gggcttcata gcattcgcct actccgtaaa     240
   gtctagggac aggaagatgg ttggcgacgt gaccggggcc caggcctatg cctccaccgc     300
   caagtgcctg aacatctggg ccctgattct gggcatcctc atgaccattg gattcatcct     360
   gttactggta ttcggctctg taacagtcta ccatattatg ttacagataa tacaggaaaa     420
   acggggttac tagtagccgc ccatagcctg caacctttgc actccactgt gcaatgctgg     480
   cc                                                                    482
<210> 21
   <211> 515
   <212> DNA
   <213> human
<400> 21
   gtagtgccac tgttgttttg ggggggcctt tttttctttt tccggaaaat ccttaaacct      60
   taagatacta aggacgttgt tttggttgta cttggaattc ttagtcacaa aatatatttt     120
   gtttacaaaa atttctgtaa aacaggttat aacagtgttt aaagtctcag tttcttgctt     180
   ggggaacttg tgtccctaat gtgttagatt gctagattgc taaggagctg atacttgaca     240
   gttttttaga cctgtgttac taaaaaaaag atgaatgtcg gaaaagggtg ttgggagggt 300
   ggtcaacaaa gaaacaaaga tgttatggtg tttagactta tggttgttaa aaatgtcatc 360
   tcaagtcaag tcactggtct gtttgcattt gatacatttt tgtactaact agcattgtaa 420
   aattatttca tgattagaaa ttacctgtgg atatttgtat aaaagtgtga aataaatttt 480
   ttataaaagt gttcattgtt tcgtaacaca gcatt 515
<210> 22
   <211> 393
   <212> DNA
   <213> human
<400> 22
   tcagggccaa gttcgtgggg acaccagaag tcaaccagac caccttatac cagcgttatg 60
   agatcaagat gaccaagatg tataaagggt tccaagcctt aggggatgcc gctgacatcc 120
   ggttcgtcta cacccccgcc atggagagtg tctgcggata cttccacagg tcccacaacc 180
   gcagcgagga gtttctcatt gctggaaaac tgcaggatgg actcttgcac atcactacct 240
   gcagtttcgt ggctccctgg aacagcctga gcttagctca gcgccggggc ttcaccaaga 300
   cctacactgt tggctgtgag gaatgcacag tgtttccctg tttatccatc ccctgcaaac 360
   tgcagagtgg cactcattgc ttgtggacgg acc 393
<210> 23
   <211> 432
   <212> DNA
   <213> human
<400> 23
   gtgatttttt tccacaagat ctgtaatgtt atttccactt ataaaggaaa taaaaaatga 60
   aaaacattat ttggatatca aaagcaaata aaaacccaat tcagtctctt ctaagcaaaa 120
   ttgctaaaga gagatgaacc acattataaa gtaatctttg gctgtaaggc attttcatct 180
   ttccttcggg ttggcaaaat attttaaagg taaaacatgc tggtgaacca ggggtgttga 240
   tggtgataag ggaggaatat agaatgaaag actgaatctt cctttgttgc acaaatagag 300
   tttggaaaaa gcctgtgaaa ggtgtcttct ttgacttaat gtctttaaaa gtatccagag 360
   atactacaat attaacataa gaaaagatta tatattattt ctgaatcgag atgtccatag 420
   tcaaatttgt aa 432
<210> 24
   <211> 429
   <212> DNA
   <213> human
<400> 24
   gagagcactc tatttattgt actgtgaata atgatgaagg agagtggagt ggcccaccac 60
   ctgaatgcag aggaaaatct ctaacttcca aggtcccacc aacagttcag aaacctacca 120
   cagtaaatgt tccaactaca gaagtctcac caacttctca gaaaaccacc acaaaaacca 180
   ccacaccaaa tgctcaagca acacggagta cacctgtttc caggacaacc aagcattttc 240
   atgaaacaac cccaaataaa ggaagtggaa ccacttcagg tactacccgt cttctatctg 300
   ggcacacgtg tttcacgttg acaggtttgc ttgggacgct agtaaccatg ggcttgctga 360
   cttagccaaa gaagagttaa gaagaaaata cacacaagta tacagactgt tcctagtttc 420
   ttagactta 429
<210> 25
   <211> 324
   <212> DNA
   <213> human
<400> 25
   gtatgacaac ccgggatcgt ttgcaagtaa ctgaatccat tgcgacattg tgaaggctta 60
   aatgagttta gatgggaaat agcgttgtta tcgccttggg tttaaattat ttgatgagtt 120
   ccacttgtat catggcctac ccgaggagaa gaggagtttg ttaactgggc ctatgtagta 180
   gcctcattta ccatcgtttg tattactgac cacatatgct tgtcactggg aaagaagcct 240
   gtttcagctg cctgaacgca gtttggatgt ctttgaggac agacattgcc cggaaactca 300
   gtctatttat tcttcagctt gccc 324
<210> 26
   <211> 473
   <212> DNA
   <213> human
<400> 26
   tggcctacat ggaccagcag actggcaacc tcaagaaggc cctgctcctc cagggctcca 60
   acgagatcga gatccgcgcc gagggcaaca gccgcttcac ctacagcgtc actgtcgatg 120
   gctgcacgag tcacaccgga gcctggggca agacagtgat tgaatacaaa accaccaaga 180
   cctcccgcct gcccatcatc gatgtggccc ccttggacgt tggtgcccca gaccaggaat 240
   tcggcttcga cgttggccct gtctgcttcc tgtaaactcc ctccatccca acctggctcc 300
   ctcccaccca accaactttc cccccaaccc ggaaacagac aagcaaccca aactgaaccc 360
   cctcaaaagc caaaaaatgg gagacaattt cacatggact ttggaaaata tttttttcct 420
   ttgcattcat ctctcaaact tagtttttat ctttgaccaa ccgaacatga cca 473
<210> 27
   <211> 472
   <212> DNA
   <213> human
<220>
   <221> misc_feature
   <222> (149)..(149)
   <223> n is a, c, g, or t
<220>
   <221> misc_feature
   <222> (153)..(153)
   <223> n is a, c, g, or t
<220>
   <221> misc_feature
   <222> (428)..(428)
   <223> n is a, c, g, or t
<400> 27
   gctccccatt tttataccaa aggtgctaca tctatgtgat gggtggggtg gggagggaat 60
   cactggtgct atagaaattg agatgccccc ccaggccagc aaatgttcct ttttgttcaa 120
   agtctatttt tattccttga tattttttnt ttnttttttt ttttttttgt ggatggggac 180
   ttgtgaattt ttctaaaggt gctatttaac atgggaggag agcgtgtgcg gctccagccc 240
   agcccgctgc tcactttcca ccctctctcc acctgcctct ggcttctcag gcctctgctc 300
   tccgacctct ctcctctgaa accctcctcc acagctgcag cccatcctcc cggctccctc 360
   ctagtctgtc ctgcgtcctc tgtccccggg tttcagagac aacttcccaa agcacaaagc 420
   agtttttncc cctaggggtg ggaggaagca aaagactctg tacctatttt gt 472
<210> 28
   <211> 474
   <212> DNA
   <213> human
<400> 28
   aacctgaaaa catcccagcc aagaactggt ataggagctc caaggacaag aaacacgtct 60
   ggctaggaga aactatcaat gctggcagcc agtttgaata taatgtagaa ggagtgactt 120
   ccaaggaaat ggctacccaa cttgccttca tgcgcctgct ggccaactat gcctctcaga 180
   acatcaccta ccactgcaag aacagcattg catacatgga tgaggagact ggcaacctga 240
   aaaaggctgt cattctacag ggctctaatg atgttgaact tgttgctgag ggcaacagca 300
   ggttcactta cactgttctt gtagatggct gctctaaaaa gacaaatgaa tggggaaaga 360
   caatcattga atacaaaaca aataagccat cacgcctgcc cttccttgat attgcacctt 420
   tggacatcgg tggtgctgac caggaattct ttgtggacat tggcccagtc tgtt 474
<210> 29
   <211> 356
   <212> DNA
   <213> human
<400> 29
   actttcccat gagtgtgatc cacattgtta ggtgctgacc tagacagaga tgaactgagg 60
   tccttgtttt gttttgttca taatacaaag gtgctaatta atagtatttc agatacttga 120
   agaatgttga tggtgctaga agaatttgag aagaaatact cctgtattga gttgtatcgt 180
   gtggtgtatt ttttaaaaaa tttgatttag cattcatatt ttccatctta ttcccaatta 240
   aaagtatgca gattatttgc ccaaagttgt cctcttcttc agattcagca tttgttcttt 300
   gccagtctca ttttcatctt cttccatggt tccacagaag ctttgtttct tgggca 356
<210> 30
   <211> 418
   <212> DNA
   <213> human
<400> 30
   gcaacaaccg aaaatgcacc agccccaggt cctcggacac cgaggagaat gtcaagaggc 60
   gaacacacaa cgtcttggag cgccagagga ggaacgagct aaaacggagc ttttttgccc 120
   tgcgtgacca gatcccggag ttggaaaaca atgaaaaggc ccccaaggta gttatcctta 180
   aaaaagccac agcatacatc ctgtccgtcc aagcagagga gcaaaagctc atttctgaag 240
   aggacttgtt gcggaaacga cgagaacagt tgaaacacaa acttgaacag ctacggaact 300
   cttgtgcgta aggaaaagta aggaaaacga ttccttctaa cagaaatgtc ctgagcaatc 360
   acctatgaac ttgtttcaaa tgcatgatca aatgcaacct cacaaccttg gctgagtc 418
<210> 31
   <211> 380
   <212> DNA
   <213> human
<400> 31
   ggaaacctct cagtgtcttg acatcaccct acccaggcgg tgggtctcca ccacagccac 60
   tttgagtctg tggtccctgg agggtggctt ctcctgactg gcaggatgac cttagccaag 120
   atattcctct gttccctctg ctgagataaa gaattccctt aacatgatat aatccaccca 180
   tgcaaatagc tactggccca gctaccattt accatttgcc tacagaattt cattcagtct 240
   acactttggc attctctctg gcgatggagt gtggctgggc tgaccgcaaa aggtgcctta 300
   cacactgccc ccaccctcag ccgttgcccc atcagaggct gcctcctcct tctgattacc 360
   ccccatgttg catatcaggg 380
<210> 32
   <211> 289
   <212> DNA
   <213> human
<400> 32
   atccccaact gtgacaagca tggcctgtac aacctcaaac agtgcaagat gtctctgaac 60
   gggcagcgtg gggagtgctg gtgtgtgaac cccaacaccg ggaagctgat ccagggagcc 120
   cccaccatcc ggggggaccc cgagtgtcat ctcttctaca atgagcagca ggaggcttgc 180
   ggggtgcaca cccagcggat gcagtagacc gcagccagcc ggtgcctggc gcccctgccc 240
   cccgcccctc tccaaacacc ggcagaaaac ggagagtgct tgggtggtg 289
<210> 33
   <211> 212
   <212> DNA
   <213> human
<400> 33
   ccgaacgtgg gcgccaatgg cgagatctgc gtcaacgtgc tcaagaggga ctggacggct 60
   gagctgggca tccgacacgt actgctgacc atcaagtgcc tgctgatcca ccctaacccc 120
   gagtctgcac tcaacgagga ggcgggccgc ctgctcttgg agaactacga ggagtatgcg 180
   gctcgggccc gtctgctcac agagatccac gg 212
<210> 34
   <211> 464
   <212> DNA
   <213> human
<400> 34
   ctacccttat gatgacccat tttccctcat gaccgatccc aagctcatca tttggagccc 60
   tgtgcgccgc tcagatgtgg cctggaactt tgagaagttc ctcatagggc cggagggaga 120
   gcccttccga cgctacagcc gcaccttccc aaccatcaac attgagcctg acatcaagcg 180
   cctccttaaa gttgccatat agatgtgaac tgctcaacac acagatctcc tactccatcc 240
   agtcctgagg agccttagga tgcagcatgc cttcaggaga cactgctgga cctcagcatt 300
   cccttgatat cagtcccctt cactgcagag ccttgccttt cccctctgcc tgtttccttt 360
   tcctctccca accctctggt tggtgattca acttgggctc caagacttgg gtaagctctg 420
   ggccttcaca gaatgatggc accttcctaa accctcatgg gtgg 464
<210> 35
   <211> 224
   <212> DNA
   <213> human
<400> 35
   gaagcgttta ggcatgttta acatccagca ctgtaagaag ctgtccagct gggtactgct 60
   aatgaaatac ctgggcaatg ccaccgccat cttcttccta cctgatgagg ggaaactaca 120
   gcacctggaa aatgaactca cccacgatat catcaccaag ttcctggaaa atgaagacag 180
   aaggtctgcc agcttacatt tacccaaact gtccattact ggaa 224
<210> 36
   <211> 364
   <212> DNA
   <213> human
<400> 36
   gtacccagtt aaattttcat ttcagataaa caacaaataa ttttttagta taagtacatt 60
   attgtttatc tgaaagtttt aattgaacta acaatcctag tttgatactc ccagtcttgt 120
   cattgccagc tgtgttggta gtgctgtgtt gaattacgga ataatgagtt agaactatta 180
   aaacagccaa aactccacag tcaatattag taatttcttg ctggttgaaa cttgtttatt 240
   atgtacaaat agattcttat aatattattt aaatgactgc atttttaaat acaaggcttt 300
   atatttttaa ctttaagatg tttttatgtg ctctccaaat tttttttact gtttctgatt 360
   gtat 364
<210> 37
   <211> 529
   <212> DNA
   <213> human
<400> 37
   gagaggacca accagaattc cctttggaca tttgtgtttt tttgtttttt tattttgttt 60
   tgttttttct tcttcttctt cttccttaaa gacatttaag ctaaaggcaa ctcgtaccca 120
   aatttccaag acacaaacat gacctatcca agcgcattac ccacttgtgg ccaatcagtg 180
   gccaggccaa ccttggctaa atggagcagc gaaatcaacg agaaactgga ctttttaaac 240
   cctcttcaga gcaagcgtgg aggatgatgg agaatcgtgt gatcagtgtg ctaaatctct 300
   ctgcctgttt ggactttgta attatttttt tagcagtaat taaagaaaaa agtcctctgt 360
   gaggaatatt ctctatttta aatattttta gtatgtactg tgtatgattc attaccattt 420
   tgaggggatt tatacatatt tttagataaa attaaatgct cttatttttc caacagctaa 480
   actactctta gttgaacagt gtgccctagc ttttcttgca accagagta 529
<210> 38
   <211> 516
   <212> DNA
   <213> human
<400> 38
   gtagtgtatc actgagtcat ttgcagtgtt ttctgccaca gacctttggg ctgccttata 60
   ttgtgtgtgt gtgtgggtgt gtgtgtgttt tgacacaaaa acaatgcaag catgtgtcat 120
   ccatatttct ctacatcttc tcttggagtg agggaggcta cctggagggg atcagcccac 180
   tgacagacct taatcttaat tactgctgtg gctagagagt ttgaggattg ctttttaaaa 240
   aagacagcaa actttttttt ttatttaaaa aaagatatat taacagtttt agaagtcagt 300
   agaataaaat cttaaagcac tcataatatg gcatccttca atttctgtat aaaagcagat 360
   ctttttaaaa aagatacttc tgtaacttaa gaaacctggc atttaaatca tattttgtct 420
   ttaggtaaaa gctttggttt gtgttcgtgt tttgtttgtt tcacttgttt ccctcccagc 480
   cccaaacctt ttgttctctc cgtgaaactt accttt 516
<210> 39
   <211> 459
   <212> DNA
   <213> human
<400> 39
   gccttccctg aatcagacaa ccttttcaaa tgggtaggga ccatccatgg agcagctgga 60
   acagtatatg aagacctgag gtataagctc tcgctagagt tccccagtgg ctacccttac 120
   aatgcgccca cagtgaagtt cctcacgccc tgctatcacc ccaacgtgga cacccagggt 180
   aacatatgcc tggacatcct gaaggaaaag tggtctgccc tgtatgatgt caggaccatt 240
   ctgctctcca tccagagcct tctaggagaa cccaacattg atagtccctt gaacacacat     300
   gctgccgagc tctggaaaaa ccccacagct tttaagaagt acctgcaaga aacctactca     360
   aagcaggtca ccagccagga gccctgaccc aggctgccca gcctgtcctt gtgtcgtctt     420
   tttaattttt ccttagatgg tctgtccttt ttgtgattt                            459
<210> 40
   <211> 557
   <212> DNA
   <213> human
<400> 40
   gccagtcttg accgggatga ggcccacaga caggttgtca tcagcttgtc ccattcaagc      60
   caccgagctc accacagaca cagtggagcc gcgctcttct ccagtgacac gtggacaaat     120
   gcgggctcat cagccccccc agagagggtc aggccgaacc ccatttctcc tcctcttagg     180
   tcattttcag caaacttgaa tatctagacc tctcttccaa tgaaaccctc cagtctatta     240
   tagtcacata gataatggtg ccacgtgttt tctgatttgg tgagctcaga cttggtgctt     300
   ccctctccac aacccccacc ccttgttttt caagatacta ttattatatt ttcacagact     360
   tttgaagcac aaatttattg gcatttaata ttggacatct gggcccttgg aagtacaaat     420
   ctaaggaaaa accaacccac tgtgtaagtg actcatcttc ctgttgttcc aattctgtgg     480
   gtttttgatt caacggtgct ataaccaggg tcctgggtga cagggcgctc actgagcacc     540
   atgtgtcatc acagaca                                                    557
<210> 41
   <211> 546
   <212> DNA
   <213> human
<400> 41
   caggaaatag tcactcatcc cactccacat aaggggttta gtaagagaag tctgtctgtc      60
   tgatgatgga tagggggcaa atctttttcc cctttctgtt aatagtcatc acatttctat     120
   gccaaacagg aacgatccat aactttagtc ttaatgtaca cattgcattt tgataaaatt     180
   aattttgttg tttcctttga ggttgatcgt tgtgttgttt tgctgcactt tttacttttt   240
   tgcgtgtgga gctgtattcc cgagacaacg aagcgttggg atacttcatt aaatgtagcg     300
   actgtcaaca gcgtgcaggt tttctgtttc tgtgttgtgg ggtcaaccgt acaatggtgt     360
   gggaatgacg atgatgtgaa tatttagaat gtaccatatt ttttgtaaat tatttatgtt     420
   tttctaaaca aatttatcgt ataggttgat gaaacgtcat gtgttttgcc aaagactgta     480
   aatatttatt tatgtgttca catggtcaaa atttcaccac tgaaaccctg cacttagcta 540
   gaacct 546
<210> 42
   <211> 520
   <212> DNA
   <213> human
<400> 42
   atggtaacat gtatctttgc cctgggtctg ggtgggtcca gtcagtctca gatttacaag 60
   catttaggag cctaggtaaa agctgctagt attcttttaa aagttatatt tatgacttgc 120
   aatgatagaa aactccttcc aattaaatgg cattttataa tattatgtgt gtacttcaca 180
   gtgttaaaaa taccctcata cgttattgca tttgatcttc acagaaagtg cattttaacc 240
   agtactctgg gtgcaataaa taatatgtag aaatttaagt cctccaattc cagcatatcc 300
   agtgagtttt gacagtgtgt ttatgtggaa tgtttaagga tatacaattg tactttatat 360
   aaattggttc ttgttcttct taaatgtgac atgaaataat tgtgctgcta cattatactg 420
   gaaattaaca ggggaaaagg gaagagctct tggctccctt gaggttctgc tagtggtgtt 480
   aggagtggtt acaactgagc ttttagtaac catttaaccg 520
<210> 43
   <211> 468
   <212> DNA
   <213> human
<220>
   <221> misc_feature
   <222> (399)..(399)
   <223> n is a, c, g, or t
<400> 43
   tgctaagttc aagtttcgta atgctttgaa gtatttttat gctctgaatg tttaaatgtt 60
   ctcatcagtt tcttgccatg ttgttaacta tacaacctgg ctaaagatga atatttttct 120
   actggtattt taatttttga cctaaatgtt taagcattcg gaatgagaaa actatacaga 180
   tttgagaaat gatgctaaat ttataggagt tttcagtaac ttaaaaagct aacatgagag 240
   catgccaaaa tttgctaagt cttacaaaga tcaagggctg tccgcaacag ggaagaacag 300
   ttttgaaaat ttatgaacta tcttattttt aggtaggttt tgaaagcttt ttgtctaagt 360
   gaattcttat gccttggtca gagtaataac tgaaggagnt gcttatcttg gctttcgagt 420
   ctgagtttaa aactacacat tttgacatag tgtttattag cagccatc 468
<210> 44
   <211> 477
   <212> DNA
   <213> human
<400> 44
   gtggccgtag caacttggcg gagacaggct atgagtctga cgttagagtg gttgcttcct     60
   tagcctttca ggatggagga atgtgggcag tttgacttca gcactgaaaa cctctccacc    120
   tgggccaggg ttgcctcaga ggccaagttt ccagaagcct cttacctgcc gtaaaatgct    180
   caaccctgtg tcctgggcct gggcctgctg tgactgacct acagtggact ttctctctgg    240
   aatggaacct tcttaggcct cctggtgcaa cttaattttt ttttttaatg ctatcttcaa    300
   aacgttagag aaagttcttc aaaagtgcag cccagagctg ctgggcccac tggccgtcct    360
   gcatttctgg tttccagacc ccaatgcctc ccattcggat ggatctctgc gtttttatac    420
   tgagtgtgcc taggttgccc cttatttttt attttccctg ttgcgttgct atagatg       477
<210> 45
   <211> 517
   <212> DNA
   <213> human
<400> 45
   agagtggtct tttcaacact cctcccccta ctccaccgga cctcaaccag gacttcagtg     60
   gatttcagct tctagtggat gttgcactca aacgggctgc agagatggag cttcaggcaa    120
   aacttacagc ttaacccatt ttcaagcaaa acagttctca gaaatgtcat gattgccggg    180
   gtgaaggcaa gagatgaatt gcattatttt atatattttt tattaatatt tgcacatggg    240
   attgctaaaa cagcttcctg ttactgagat gtcttcaatg gaatacagtc attccaagaa    300
   ctataaactt aaagctactg tagaaacaaa gggttttctt ttttaaatgt ttcttggtag    360
   attattcata atgtgagatg gttcccaata tcatgtgatt tttttttttc ctccccttcc    420
   ctttttttgt tattttttca gactgtgcaa tacttagaga acctatagca tcttctcatt    480
   cccatgtgga acaggatgcc cacatactgt ctaatta                             517
<210> 46
   <211> 513
   <212> DNA
   <213> human
<400> 46
   gaagccaagg gttaacccag caagctacaa agagggtgtg tcacactgaa actcaatagt 60
   tgagtttggc tgttgttgca ggaaaatgat tataactaaa agctctctga tagtgcagag 120
   acttaccaga agacacaagg aattgtactg aagagctatt acaatccaaa tattgccgtt 180
   tcataaatgt aataagtaat actaattcac agagtattgt aaatggtgga tgacaaaaga 240
   aaatctgctc tgtggaaaga aagaactgtc tctaccaggg tcaagagcat gaacgcatca 300
   atagaaagaa ctcggggaaa catcccatca acaggactac acacttgtat atacattctt 360
   gagaacactg caatgtgaaa atcacgtttg ctatttataa acttgtcctt agattaatgt 420
   gtctggacag attgtgggag taagtgattc ttctaagaat tagatacttg tcactgccta 480
   tacctgcagc tgaactgaat ggtacttcgt atg 513
<210> 47
   <211> 546
   <212> DNA
   <213> human
<400> 47
   gaagcaggtg gaaacatgag cattcagttt cttggtacag tgtacaaggt gaatatctta 60
   accagacttg ccgcagaatt gaacaaattt atgctggaaa aagtgactga ggacacaagc 120
   agtgttctgc gttccccgat gcccggagtg gtggtggccg tctctgtcaa gcctggagac 180
   gcggtagcag aaggtcaaga aatttgtgtg attgaagcca tgaaaatgca gaatagtatg 240
   acagctggga aaactggcac ggtgaaatct gtgcactgtc aagctggaga cacagttgga 300
   gaaggggatc tgctcgtgga gctggaatga aggatttata acctttcagt catcacccaa 360
   tttaattagc catttgcatg atgctttcac acacaattga ttcaagcatt atacaggaac 420
   acccctgtgc agctacgttt acgtcgtcat ttattccaca gagtcaagac caatattctg 480
   ccaaaaaatc accaatggaa attttcatga tataaatact tgtactatag atgtacttct 540
   gctgtg 546
<210> 48
   <211> 539
   <212> DNA
   <213> human
<400> 48
   tgccagcgac tgtctcagac tgggcaggga ggctttggca tgacttaaga ggaagggcag 60
   tcttgggacc cgctatgcag gtcctggcaa acctggctgc cctgtctcat ccctgtccct 120
   cagggtagca ccatggcagg actgggggaa ctggagtgtc cttgctgtat ccctgttgtg 180
   aggttccttc caggggctgg cactgaagca agggtgctgg ggccccatgg ccttcagccc 240
   tggctgagca actgggctgt agggcagggc cacttcctga ggtcaggtct tggtaggtgc 300
   ctgcatctgt ctgccttctg gctgacaatc ctggaaatct gttctccaga atccaggcca 360
   aaaagttcac agtcaaatgg ggaggggtat tcttcatgca ggagacccca ggccctggag 420
   gctgcaacat acctcaatcc tgtcccaggc cggatcctcc tgaagccctt ttcgcagcac 480
   tgctatcctc caaagccatt gtaaatgtgt gtacagtgtg tataaacctt cttcttctt 539
<210> 49
   <211> 508
   <212> DNA
   <213> human
<400> 49
   gactaatcca tacacagtta acctaatgcc aaataaatac tggttaaata aatgtatggc 60
   acagaatata atttgactat caagactttt agcataatga aaaaccctct ctctatatat 120
   atatgtgtat atgaattatg tgggcattct tgatacttca agttctagtt tgaaaaaaat 180
   acataactaa tttaatttta cacaaaaata tttatgcaga ttttcagaat ttcatatcag 240
   gaaatgacct ttttatgtct gttaaatatc aaaacaattt gctacagtgt taatctgcat 300
   ggtctttaag cctgctgtag ttgagttgca gacagtgcat gaaaaagtat tccgctggga 360
   attgagccat gccaccaaag ccaagaggag cgcatggaaa cccggtagtc tagaactaat 420
   cagattactg attttagggc acagcaccag atgaattgtt gtatatgctt gtaaaaattg 480
   attctgtgtg ttcctctgaa caaagcgg 508
<210> 50
   <211> 466
   <212> DNA
   <213> human
<400> 50
   gtccagcttg aacatctaga attcctagag aaacagaatg agcaggcgaa ggagatgcag 60
   cagatggtga aattggaagc cgagatggac cgcagaccag caacagtagt atgaaactcc 120
   aaaatgcaaa ctgaagcagc aaacccacaa agcatcaaaa gactcactca caaaacttct 180
   gaacacaaac tccatggatg aaagctgttt attttgtttc ctttatgtgt aaacaagatg 240
   atatctgaaa ccagagagac ttggaatgtc tgactgactt ctatttaaca gcttgagtat 300
   tgcatttcct tggccaaaca aaaatagcta caaatccaca aaaatttact attccagtaa 360
   ggcagagtcc aaccattgat aatacaactt aaacatgttt gctataaaat accatcacaa 420
   gtaaatgagc ttggtgtgaa caactcttcc tttgtgatgc cttagg 466
<210> 51
   <211> 440
   <212> DNA
   <213> human
<220>
   <221> misc_feature
   <222> (331)..(331)
   <223> n is a, c, g, or t
<400> 51
   ggactagagc aacatcgtgc tgcccaaagg actaacctat gcaaactagt tcacatttta 60
   gtggatgtcg cagttaatgt gtaataagac attatttccc ctgcataatg tacaacagca 120
   ttgaaatgac acattaagcc tagcatcaca ttgtatagta cagtcactca caaacccttc 180
   aaggctaccc taatcattaa cattaatatt tgtttaaaag caaatcaccg atttatctat 240
   tgaaactact taaatgacgg caaaccagga atgacagatg gctgtgtcag caatggcttt 300
   aatgtgttcc ctgcaagtgg tctcctatga ntagaactgc gttctcaaat gcactctctt 360
   cagggtctta atattctgtg ttttctctct gtatttgtaa aacattataa cacattaatt 420
   tcctatctct acacatttgg 440
<210> 52
   <211> 505
   <212> DNA
   <213> human
<400> 52
   gtcaaggcat tgtatgttgc ttctgtggtt attattctgt gatgcttaga ctacttgaac 60
   ccataaactt ggaagaatct ttgagcaaat tttctcagtt gtctgtatga cttcagtata 120
   ttcctgggaa tgccatagga ttttttgtgc ttgatacatg gtatccagtt tgcatagtat 180
   cacttctttg taatccagtt gctgttaaga atgatgtact ttaaaggaaa agagaaaact 240
   gcatcacagt cccattctcc agtgtccatg caatgaattg ctgagcattt aggaagcagc 300
   accaagtcta ttacaggcat ggtgtgaaac ttgatgtttg acctgtgatc aaaattgaac 360
   cattgtacag tttggcttct gtttgcttca aaatatgtag aattgtggtt gatgattaat 420
   ttgcgagact aactttgaga gtgtaacagt tttgaagaaa acattgaatg ttttacaaat 480
   gaaggggctt cacggaatgt tacaa 505
<210> 53
   <211> 291
   <212> DNA
   <213> human
<400> 53
   aaccagccag tcccaagaag aacattaaaa ctaggagtgc ccagaagaga acaaacccga     60
   aaagagtgtg agctaactag tttccaaagc ggagacttcc gacttcctta caggatgagg    120
   ctgggcattg cctgggacag cctatgtaag gccatgtgcc ccttgcccta acaactcact    180
   gcagtgctct tcatagacac atcttgcagc atttttctta aggctatgct tcagtttttc    240
   tttgtaagcc atcacaagcc atagtggtag gtttgccctt tggtacagaa g             291
<210> 54
   <211> 468
   <212> DNA
   <213> human
<400> 54
   ttgtgttttg tcattagctc tgcccttttt aattaaatat tttggttcat ggaccaaagg     60
   gtttacttga caaatttgtg tgacagactc cgaacaattc ctttactacg aagtataatt    120
   tataaaataa aatataccca ttttaagggt acagtttgat ttttgaccag tgaaactatg    180
   atcccaatca aggtatagat gccgtcaccc caaaaagttc cctccatatc cctttgcagt    240
   cagttcatcc ctaccctggc ccagatgatc actgatcttg tcattataga tgagttttgc    300
   cagttcaaga atttaatgga atcagatatt gtaagcattc ttgtgtaata cttcattctc    360
   tctcattatt gagattcatc catattgttg aatgtttcac tagttaatgt ttattgttca    420
   atatttttgt atatactttt aaagcctatt cacttgctga tggatctt                 468
<210> 55
   <211> 496
   <212> DNA
   <213> human
<400> 55
   cgctctcgtt tcattttctg cagcgcgcca cgaggatggc ccacaagcag atctactact     60
   cggacaagta cttcgacgaa cactacgagt accggcatgt tatgttaccc agagaacttt    120
   ccaaacaagt acctaaaact catctgatgt ctgaagagga gtggaggaga cttggtgtcc    180
   aacagagtct aggctgggtt cattacatga ttcatgagcc agaaccacat attcttctct    240
   ttagacgacc tcttccaaaa gatcaacaaa aatgaagttt atctggggat cgtcaaatct    300
   ttttcaaatt taatgtatat gtgtatataa ggtagtattc agtgaatact tgagaaatgt 360
   acaaatcttt catccatacc tgtgcatgag ctgtattctt cacagcaaca gagctcagtt 420
   aaatgcaact gcaagtaggt tactgtaaga tgtttaagat aaaagttctt ccagtcagtt 480
   tttctcttaa gtgcct 496
<210> 56
   <211> 543
   <212> DNA
   <213> human
<400> 56
   ctcatgggga ctcctaccca tttgatgggc caggaaacac gctggctcat gcctttgcgc 60
   ctgggacagg tctcggagga gatgctcact tcgatgagga tgaacgctgg acggatggta 120
   gcagtctagg gattaacttc ctgtatgctg caactcatga acttggccat tctttgggta 180
   tgggacattc ctctgatcct aatgcagtga tgtatccaac ctatggaaat ggagatcccc 240
   aaaattttaa actttcccag gatgatatta aaggcattca gaaactatat ggaaagagaa 300
   gtaattcaag aaagaaatag aaacttcagg cagaacatcc attcattcat tcattggatt 360
   gtatatcatt gttgcacaat cagaattgat aagcactgtt cctccactcc atttagcaat 420
   tatgtcaccc ttttttattg cagttggttt ttgaatgtct ttcactcctt ttattggtta 480
   aactccttta tggtgtgact gtgtcttatt ccatctatga gctttgtcag tgcgcgtaga 540
   tgt 543
<210> 57
   <211> 507
   <212> DNA
   <213> human
<400> 57
   gaaaatgtac cttggtgcca ccaacccatt ttgtgccaca tgcaagtttt gaataaggat 60
   gtatggaaaa caacgctgca tatacaggta ccatttagga aataccgatg cctttgtggg 120
   ggcagaatca cagacaaaag ctttgaaaat cataaagata taagttggtg tggctaagat 180
   ggaaacaggg ctgattcttg attcccaatt ctcaactctc cttttcctat ttgaatttct 240
   ttggtgctgt agaaaacaaa aaaagaaaaa tatatattca taaaaaatat ggtgctcatt 300
   ctcatccatc caggatgtac taaaacagtg tgtttaataa attgtaatta ttttgtgtac 360
   agttctatac tgttatctgt gtccatttcc aaaacttgca cgtgtccctg aattccgctg 420
   actctaattt atgaggatgc cgaactctga tggcaataat atatgtatta tgaaaatgaa 480
   gttatgattt ccgatgaccc taagtcc 507
<210> 58
   <211> 406
   <212> DNA
   <213> human
<400> 58
   gggtctgaat ctagcaccat gacggaacta gagacagcca tgggcatgat catagacgtc 60
   ttttcccgat attcgggcag cgagggcagc acgcagaccc tgaccaaggg ggagctcaag 120
   gtgctgatgg agaaggagct accaggcttc ctgcagagtg gaaaagacaa ggatgccgtg 180
   gataaattgc tcaaggacct ggacgccaat ggagatgccc aggtggactt cagtgagttc 240
   atcgtgttcg tggctgcaat cacgtctgcc tgtcacaagt actttgagaa ggcaggactc 300
   aaatgatgcc ctggagatgt cacagattcc tgcagagcca tggtcccagg cttcccaaaa 360
   gtgtttgttg gcaattattc ccctaggctg agcctgctca tgtacc 406
<210> 59
   <211> 485
   <212> DNA
   <213> human
<400> 59
   gtagctggac ccacgaggag gaaccaggct actttcccca gtactgaggt ggtggacatc 60
   gtctctgcca ctcctgaccc agccctgaac aaagcacctc aagtgcaagg accaaagggg 120
   gccctggctt ggagtgggtt ggcttgctga tggctgctgg aggggacgct ggctaaagtg 180
   ggtaggcctt ggcccacctg aggccccagg tgggaacatg gtcaccccca ctctgcatac 240
   cctcatcaaa aacactctca ctatgctgct atggacgacc tccagctctc agttacaagt 300
   gcaggcgact ggaggcagga ctcctgggtc cctgggaaag agggtactag gggcccggat 360
   ccaggattct gggaggcttc agttaccgct ggccgagctg aagaactggg tatgaggctg 420
   gggcggggct ggaggtggcg ccccctggtg ggacaacaaa gaggacacca tttttccaga 480
   gctgc 485
<210> 60
   <211> 410
   <212> DNA
   <213> human
<400> 60
   agaccaagac ataccggcag atcaggttaa atgagttatt aaaggaacat tcaagcacag 60
   ctaatattat tgtcatgagt ctcccagttg cacgaaaagg tgctgtgtct agtgctctct 120
   acatggcatg gttagaagct ctatctaagg acctaccacc aatcctccta gttcgtggga 180
   atcatcagag tgtccttacc ttctattcat aaatgttcta tacagtggac agccctccag 240
   aatggtactt cagtgcctag tgtagtaacc tgaaatcttc aatgacacat taacatcaca 300
   atggcgaatg gtgacttttc tttcacgatt tcattaattt gaaagcacac aggaaagctt 360
   gctccattga taacgtgtat ggagacttcg gttttagtca attccatatc 410
<210> 61
   <211> 433
   <212> DNA
   <213> human
<400> 61
   tatgattaac tctacctgca cactgtccta ttatattcat tctttttgaa atgtcaaccc 60
   caagttagtt caatctggat tcatatttaa tttgaaggta gaatgttttc aaatgttctc 120
   cagtcattat gttaatattt ctgaggagcc tgcaacatgc cagccactgt gatagaggct 180
   ggcggatcca agcaaatggc caatgagatc attgtgaagg caggggaatg tatgtgcaca 240
   tctgttttgt aactgtttag atgaatgtca gttgttattt attgaaatga tttcacagtg 300
   tgtggtcaac atttctcatg ttgaaacttt aagaactaaa atgttctaaa tatcccttgg 360
   acattttatg tctttcttgt aaggcatact gccttgttta atggtagttt tacagtgttt 420
   ctggcttaga aca 433
<210> 62
   <211> 386
   <212> DNA
   <213> human
<400> 62
   gaagaactgt ctattttctc agtcattttt aacctctaga gtcactgata cacagaatat 60
   aatcttattt atacctcagt ttgcatattt ttttactatt tagaatgtag ccctttttgt 120
   actgatataa tttagttcca caaatggtgg gtacaaaaag tcaagtttgt ggcttatgga 180
   ttcatatagg ccagagttgc aaagatcttt tccagagtat gcaactctga cgttgatccc 240
   agagagcagc ttcagtgaca aacatatcct ttcaagacag aaagagacag gagacatgag 300
   tctttgccgg aggaaaagca gctcaagaac acatgtgcag tcactggtgt caccctggat 360
   aggcaaggga taactcttct aacaca 386
<210> 63
   <211> 510
   <212> DNA
   <213> human
<400> 63
   agatgatgga ccctggttat cccaaactga ttaccaagaa cttccaagga atcgggccta     60
   aaattgatgc agtcttctat tctaaaaaca aatactacta tttcttccaa ggatctaacc    120
   aatttgaata tgacttccta ctccaacgta tcaccaaaac actgaaaagc aatagctggt    180
   ttggttgtta gaaatggtgt aattaatggt ttttgttagt tcacttcagc ttaataagta    240
   tttattgcat atttgctatg tcctcagtgt accactactt agagatatgt atcataaaaa    300
   taaaatctgt aaaccatagg taatgattat ataaaataca taatattttt caattttgaa    360
   aactctaatt gtccattctt gcttgactct actattaagt ttgaaaatag ttaccttcaa    420
   agcaagataa ttctatttga agcatgctct gtaagttgct tcctaacatc cttggactga    480
   gaaattatac ttacttctgg cataactaaa                                     510
<210> 64
   <211> 541
   <212> DNA
   <213> human
<400> 64
   tgccgtgctc ccaaaacatt ttaaatgaaa gtattggcat tcaaaaagac agcagacaaa     60
   atgaaagaaa atgagagcag aaagtaagca tttccagcct atctaatttc tttagttttc    120
   tatttgcctc cagtgcagtc catttcctaa tgtataccag cctactgtac tatttaaaat    180
   gctcaatttc agcaccgatg gccatgtaaa taagatgatt taatgttgat tttaatcctg    240
   tatataaaat aaaaagtcac aatgagtttg ggcatattta atgatgatta tggagcctta    300
   gaggtcttta atcattggtt cggctgcttt tatgtagttt aggctggaaa tggtttcact    360
   tgctctttga ctgtcagcaa gactgaagat ggcttttcct ggacagctag aaaacacaaa    420
   atcttgtagg tcattgcacc tatctcagcc ataggtgcag tttgcttcta catgatgcta    480
   aaggctgcga atgggatcct gatggaacta aggactccaa tgtcgaactc ttctttgctg    540
   c                                                                    541
<210> 65
   <211> 549
   <212> DNA
   <213> human
<400> 65
   tgctctccag tgtacccatg atggaagtat cttgatagta cccaaagaac tggtggcctc 60
   aggccacaaa aaggaaaccc aaaagggaaa gagaaagtga gaagaaactg aagatggact 120
   ctattatgtg aagtagtaat gttcagaaac tgattatttg gatcagaaac cattgaaact 180
   gcttcaagaa ttgtatcttt aagtactgct acttgaataa ctcagttaac gctgttttga 240
   agcttacatg gacaaatgtt taggacttca agatcacact tgtgggcaat ctgggggagc 300
   cacaactttt catgaagtgc attgtataca aaattcatag ttatgtccaa agaataggtt 360
   aacatgaaaa cccagtaaga ctttccatct tggcagccat cctttttaag agtaagttgg 420
   ttacttcaaa aagagcaaac actggggatc aaattatttt aagaggtatt tcagttttaa 480
   atgcaaaata gccttatttt catttagttt gttagcacta tagtgagctt ttcaaacact 540
   attttaatc 549
<210> 66
   <211> 547
   <212> DNA
   <213> human
<400> 66
   cttcgttcgc agagcttttc agattgtgga atgttggata aggaattata gacctctagt 60
   agctgaaatg caagacccca agaggaagtt cagatcttaa tataaattca ctttcatttt 120
   tgatagctgt cccatctggt catgtggttg gcactagact ggtggcaggg gcttctagct 180
   gactcgcaca gggattctca caatagccga tatcagaatt tgtgttgaag gaacttgtct 240
   cttcatctaa tatgatagcg ggaaaaggag aggaaactac tgcctttaga aaatataagt 300
   aaagtgatta aagtgctcac gttaccttga cacatagttt ttcagtctat gggtttagtt 360
   actttagatg gcaagcatgt aacttatatt aatagtaatt tgtaaagttg ggtggataag 420
   ctatccctgt tgccggttca tggattactt ctctataaaa aatatatatt taccaaaaaa 480
   ttttgtgaca ttccttctcc catctcttcc ttgacatgca ttgtaaatag gttcttcttg 540
   ttctgag 547
<210> 67
   <211> 168
   <212> DNA
   <213> human
<400> 67
   tttccagaac atgaacgggt ccgaatactt cgtgaagatc cagtccttcc tgggtggggc 60
   ccccacggag gatttgaagg cgctcagtca gcagaatgtg agcatggact tggccacgtt 120
   catgaagctg cggacggatg cggtgctgcc gttgactgtg gctgaggt 168
<210> 68
   <211> 514
   <212> DNA
   <213> human
<400> 68
   gtctggcaca ccatggatga caatgaagaa aatttggatg aatcaaccat tgacaatcta 60
   aacaaaatcc tacaagtctt tgtgttggaa tatcttcatt tgtaatactc tgatttagtt 120
   taggataatt ggttctagaa ttgaattcaa aagtcaaggc atcatttaaa ataatctgat 180
   ttcagacaaa tgctgtgtgg aaacatctat cctatagatc atcctattct tatgtgtctt 240
   tggttatcag atcaattaca gaataattgt gttgtgatat tgtgtcctaa attgctcatt 300
   aatttttatt tacagattga aaaagaggca ccgtgtaaag aaaatggcaa aataaatatc 360
   tttccaagga tcatcatcac gatagctaaa cagtacttaa atagcggttg gaactaggta 420
   gcctttcgaa ttttatgatt ttttcatatg tggaaatcta ttacatgtaa tacaaaacaa 480
   acatgtagtt tgaaggcggt cagatttctt tgag 514
<210> 69
   <211> 376
   <212> DNA
   <213> human
<400> 69
   aaggcggctg cagaagatgt caatgttact ttcgaagatc aacaaaagat aaacaaattt 60
   gcacggaata caagtagaat cacagagctg aaggaagaaa tagaagtaaa aaagaaacaa 120
   ctccaaaacc tagaagatgc ttgtgatgac atcatgcttg cagatgatga ttgcttaatg 180
   ataccttatc aaattggtga tgtcttcatt agccattctc aagaagaaac gcaagaaatg 240
   ttagaagaag caaagaaaaa tttgcaagaa gaaattgacg ccttagaatc cagagtggaa 300
   tcaattcagc gagtgttagc agatttgaaa gttcagttgt atgcaaaatt cgggagcaac 360
   ataaaccttg aagctg 376
<210> 70
   <211> 328
   <212> DNA
   <213> human
<400> 70
   cctcagcctg atcaggcttc ctggtgagaa ctgaggagcg gactcacttg atgtttcctg 60
   gaagcagagc aaaagttctc ttgtccctgt cgcgtctcat tttgtccatg tcccccgtgc 120
   acggttcaat ggtagattcg ctgtcctcag cgggggcctt gaagactccc tgatcccaga 180
   cctggtcgtc tctcccaccc cctccccaaa gccactggaa ggagcacata ctacctagaa 240
   gtaagaagag gagcctcaga agaaaacaaa gttctatttt attaattttc tatgtgttgt 300
   gtttgtagtc ttgtcttagc tctggacg 328
<210> 71
   <211> 546
   <212> DNA
   <213> human
<400> 71
   gccaacatca ccatcattga gcaccagaag tgtgagaacg cctaccccgg caacatcaca 60
   gacaccatgg tgtgtgccag cgtgcaggaa gggggcaagg actcctgcca gggtgactcc 120
   gggggccctc tggtctgtaa ccagtctctt caaggcatta tctcctgggg ccaggatccg 180
   tgtgcgatca cccgaaagcc tggtgtctac acgaaagtct gcaaatatgt ggactggatc 240
   caggagacga tgaagaacaa ttagactgga cccacccacc acagcccatc accctccatt 300
   tccacttggt gtttggttcc tgttcactct gttaataaga aaccctaagc caagaccctc 360
   tacgaacatt ctttgggcct cctggactac aggagatgct gtcacttaat aatcaacctg 420
   gggttcgaaa tcagtgagac ctggattcaa attctgcctt gaaatattgt gactctggga 480
   atgacaacac ctggtttgtt ctctgttgta tccccagccc caaagacagc tcctggccat 540
   atatca 546
<210> 72
   <211> 489
   <212> DNA
   <213> human
<400> 72
   ttcacacggc agctggccaa tgaaggctgt gacatcaatg ctatcatctt tcacacaaag 60
   aaaaagttgt ctgtgtgcgc aaatccaaaa cagacttggg tgaaatatat tgtgcgtctc 120
   ctcagtaaaa aagtcaagaa catgtaaaaa ctgtggcttt tctggaatgg aattggacat 180
   agcccaagaa cagaaagaac cttgctgggg ttggaggttt cacttgcaca tcatggaggg 240
   tttagtgctt atctaatttg tgcctcactg gacttgtcca attaatgaag ttgattcata 300
   ttgcatcata gtttgctttg tttaagcatc acattaaagt taaactgtat tttatgttat 360
   ttatagctgt aggttttctg tgtttagcta tttaatacta attttccata agctattttg 420
   gtttagtgca aagtataaaa ttatatttgg gggggaataa gattatatgg actttcttgc 480
   aagcaacaa 489
<210> 73
   <211> 502
   <212> DNA
   <213> human
<400> 73
   cccgaccggt gggcatttgt gaggcccatg gttgagaaat gaataatttc ccaattagga 60
   agtgtaagca gctgaggtct cttgagggag cttagccaat gtgggagcag cggtttgggg 120
   agcagagaca ctaacgactt cagggcaggg ctctgatatt ccatgaatgt atcaggaaat 180
   atatatgtgt gtgtatgttt gcacacttgt tgtgtgggct gtgagtgtaa gtgtgagtaa 240
   gagctggtgt ctgattgtta agtctaaata tttccttaaa ctgtgtggac tgtgatgcca 300
   cacagagtgg tctttctgga gaggttatag gtcactcctg gggcctcttg ggtcccccac 360
   gtgacagtgc ctgggaatgt acttattctg cagcatgacc tgtgaccagc actgtctcag 420
   tttcactttc acatagatgt ccctttcttg gccagttatc ccttcctttt agcctagttc 480
   atccaatcct cactgggtgg gg 502
<210> 74
   <211> 470
   <212> DNA
   <213> human
<400> 74
   aacaaagact cttcttgcgt ctctgcttca ggtaacttca acatctccgc tgatgagcct 60
   ataactgtga cacctcctga ctcacaatca tatatctccg tcaattactc tgtgagaatc 120
   aatgaaacat atttcaccaa tgtcactgtg ctaaatggtt ctgtcttcct cagtgtgatg 180
   gagaaagccc agaaaatgaa tgatactata tttggtttca caatggagga gcgctcatgg 240
   gggccctata tcacctgtat tcagggccta tgtgccaaca ataatgacag aacctactgg 300
   gaacttctga gtggaggcga accactgagc caaggagctg gtagttacgt tgtccgcaat 360
   ggagaaaact tggaggttcg ctggagcaaa tactaataag cccaaacttt cctcagctgc 420
   ataaaatcca tttgcagtgg agttccatgt ttattgtcct tatgccttct 470
<210> 75
   <211> 425
   <212> DNA
   <213> human
<400> 75
   caaacgtatt ggcaggcgaa ccccttccgt gctgtggccg agcctggcat ccaactcaag 60
   gctgtgaagt cttccacagg ccccggggaa cagctgcgga acgctctgtg gcatacagga 120
   gacacagagt cccaggtgcg gctgctgtgg aaggacccgc gaaacgtggg ttggaaggac 180
   aagaagtcct atcgttggtt cctgcagcac cggccccaag tgggctacat cagggtgcga 240
   ttctatgagg gccctgagct ggtggccgac agcaacgtgg tcttggacac aaccatgcgg 300
   ggtggccgcc tgggggtctt ctgcttctcc caggagaaca tcatctgggc caacctgcgt 360
   taccgctgca atgacaccat cccagaggac tatgagaccc atcagctgcg gcaagcctag 420
   ggacc 425
<210> 76
   <211> 555
   <212> DNA
   <213> human
<400> 76
   caccacctac ctatgatgcc gtggtacaga tggagtacct tgacatggtg gtgaatgaaa 60
   cactcagatt attcccagtt gctattagac ttgagaggac ttgcaagaaa gatgttgaaa 120
   tcaatggggt attcattccc aaagggtcaa tggtggtgat tccaacttat gctcttcacc 180
   atgacccaaa gtactggaca gagcctgagg agttccgccc tgaaaggttc agtaagaaga 240
   aggacagcat agatccttac atatacacac cctttggaac tggacccaga aactgcattg 300
   gcatgaggtt tgctctcatg aacatgaaac ttgctctaat cagagtcctt cagaacttct 360
   ccttcaaacc ttgtaaagaa acacagatcc ccttgaaatt agacacgcaa ggacttcttc 420
   aaccagaaaa acccattgtt ctaaaggtgg attcaagaga tggaacccta agtggagaat 480
   gagttattct aaggacttct actttggtct tcaagaaagc tgtgccccag aacaccagag 540
   atttcaactt agtca 555
<210> 77
   <211> 554
   <212> DNA
   <213> human
<400> 77
   tgctatgcct tgtttttgtc accaaaatcc tggacagatg cagatctggc ctgccagaag 60
   cggccctctg gaaacctggt gtctgtgctc agtggggctg agggatcctt cgtgtcctcc 120
   ctggtgaaga gcattggtaa cagctactca tacgtctgga ttgggctcca tgaccccaca 180
   cagggcaccg agcccaatgg agaaggttgg gagtggagta gcagtgatgt gatgaattac 240
   tttgcatggg agagaaatcc ctccaccatc tcaagccccg gccactgtgc gagcctgtcg 300
   agaagcacag catttctgag gtggaaagat tataactgta atgtgaggtt accctatgtc 360
   tgcaagttca ctgactagtg caggagggaa gtcagcagcc tgtgtttggt gtgcaactca 420
   tcatgggcat gagaccagtg tgaggactca ccctggaaga gaatattcgc ttaattcccc 480
   caacctgacc acctcattct tatctttctt ctgtttcttc ctccccgctg tcatttcagt 540
   ctcttcattt tgtc 554
<210> 78
   <211> 530
   <212> DNA
   <213> human
<400> 78
   tttccattcc caatctagtg ctagatgtat aaatctttct tttgattctt cctaacaaaa 60
   tattttctgg gttaaaaccc cagccaactc attgggttgt agccaaaggt tcactctcaa 120
   gaagctttaa tatttaaata aaatcatatt gaatgtttcc aacctggagt ataatattca 180
   gatataaaac agttttgtca gtctttctta gtgcctgtgt ggatttttgt gaaaatgtca 240
   aagagaaaac ttatatacta tttcccttga aattttaaac tatattttct ttacaggtat 300
   ttataatata ccaatgcttt tatcaaacag aattttaaag agcataataa attatattaa 360
   agaaccaaaa gttttcctga gaataagaaa gtttcaccca ataaaatatt tttgaaaggc 420
   atgttcctct gtcaatgaaa aaaagtacat gtatgtgttg tgatattaaa agtgacattt 480
   gtctaatagc ctaatacaac atgtagctga gtttaacatg tgtggtcttg 530
<210> 79
   <211> 540
   <212> DNA
   <213> human
<400> 79
   gaaaatcgat gcagccattt ctgataagga aaagaacaaa acatatttct ttgtagagga 60
   caaatactgg agatttgatg agaagagaaa ttccatggag ccaggctttc ccaagcaaat 120
   agctgaagac tttccaggga ttgactcaaa gattgatgct gtttttgaag aatttgggtt 180
   cttttatttc tttactggat cttcacagtt ggagtttgac ccaaatgcaa agaaagtgac 240
   acacactttg aagagtaaca gctggcttaa ttgttgaaag agatatgtag aaggcacaat 300
   atgggcactt taaatgaagc taataattct tcacctaagt ctctgtgaat tgaaatgttc 360
   gttttctcct gcctgtgctg tgactcgagt cacactcaag ggaacttgag cgtgaatctg 420
   tatcttgccg gtcattttta tgttattaca gggcattcaa atgggctgct gcttagcttg 480
   caccttgtca catagagtga tctttcccaa gagaagggga agcactcgtg tgcaacagac 540
<210> 80
   <211> 410
   <212> DNA
   <213> human
<400> 80
   cacccaggcg gagggtgcct tcgtggcctc actgattaag gagagtagca ctgatgacag 60
   caatgtctgg attggcctcc atgacccaaa aaagaaccgc cgctggcact ggagtagtgg 120
   gtccctggtc tcctacaagt cctgggacac tggatccccg agcagtgcta atgctggcta 180
   ctgtgcaagc ctgacttcat gctcaggatt caagaaatgg aaggatgaat cttgtgagaa 240
   gaagttctcc tttgtttgca agttcaaaaa ctagaggaag ctgaaaaatg gatgtctaga 300
   actggtcctg caattactat gaagtcaaaa attaaactag actatgtctc caactcagtt 360
   cagaccatct cctccctaat gagtttgcat cgctgatctt cagtaccttc 410
<210> 81
   <211> 508
   <212> DNA
   <213> human
<400> 81
   gatcttaaag ccacggagaa gcctctcatc ttatggcatt gacaaagaga agaccatcca 60
   ccttaccctg aaagtggtga agcccagtga tgaggagctg cccttgtttc ttgtggagtc 120
   aggtgatgag gcaaagaggc acctcctcca ggtgcgaagg tccagctcag tggcacaagt 180
   gaaagcaatg atcgagacta agacgggtat aatccctgag acccagattg tgacttgcaa 240
   tggaaagaga ctggaagatg ggaagatgat ggcagattac ggcatcagaa agggcaactt 300
   actcttcctg gcatcttatt gtattggagg gtgaccaccc tggggatggg gtgttggcag 360
   gggtcaaaaa gcttatttct tttaatctct tactcaacga acacatcttc tgatgatttc 420
   ccaaaattaa tgagaatgag atgagtagag taagatttgg gtgggatggg taggatgaag 480
   tatattgccc aactctatgt ttctttga 508
<210> 82
   <211> 95
   <212> DNA
   <213> human
<400> 82
   gactccaagg aagctcagat gcctgcagtc attaggtttt agcgtcccat gagccttggt 60
   atcaagaggc cacaagagtg ggaccccagg ggctc 95
<210> 83
   <211> 511
   <212> DNA
   <213> human
<400> 83
   tccacacacg gccaggcctg tttatctaca ctgctgccca ctcctctctc cagctccaca 60
   tgctgtacct ggatcattct gaagcaaatt ccgagcatta catcattttg tccataaata 120
   tttctaacat ccttaaatat acaatcggaa ttcaagcatc tcccattgtc ccacaaatgt 180
   ttggctgttt ttgtagttgg attgtttgta ttaggattca agcaaggccc atatattgca 240
   tttatttgaa atgtctgtaa gtctctttcc atctacagag tttagcacat ttgaacgttg 300
   ctggttgaaa tcccgaggtg tcatttgaca tggttctctg aacttatctt tcctataaaa 360
   tggtagttag atctggaggt ctgattttgt ggcaaaaata cttcctaggt ggtgctgggt 420
   acttcttgtt gcatcctgtc aggaggcaga taatgctggt gcctctctat tggtaatgtt 480
   aagactgctg ggtgggtttg gagttcttgg c 511
<210> 84
   <211> 459
   <212> DNA
   <213> human
<400> 84
   atactatttt tcataccacg tgcatgtgaa agggactcat gtttgggtag gcctgtataa 60
   gaatggcacc cctgtaatgt acacctatga tgaatacacc aaaggctacc tggatcaggc 120
   ttcagggagt gccatcatcg atctcacaga aaatgaccag gtgtggctcc agcttcccaa 180
   tgccgagtca aatggcctat actcctctga gtatgtccac tcctctttct caggattcct 240
   agtggctcca atgtgagtac accccacaga gctaatctaa atcttgtgct agaaaaagca 300
   ttctctaact ctaccccacc ctacaaaatg catatggagg taggctgaaa agaatgtaat 360
   ttttattttc tgaaatacag atttgagcta tcagaccaac aaaccttccc cctgaaaagt 420
   gagcagcaac gtaaaaacgt atgtgaagcc tctcttgaa 459
<210> 85
   <211> 553
   <212> DNA
   <213> human
<400> 85
   gccgaccatc tggatcacat caaggaggtg gcaggagcca gagccgtggg ttttggtggg 60
   gactttgatg gtgttccaag ggtccctgag gggctggagg acgtctccaa gtatccagac 120
   ctgatcgctg agctgctcag gaggaactgg acggaggcgg aggtcaaggg cgcactggct 180
   gacaacctgc tgagggtctt cgaggctgtg gaacaggcca gcaacctcac acaggctccc 240
   gaggaggagc ccatcccgct ggaccagctg ggtggctcct gcaggaccca ttacggctac 300
   tcctctgggg cttccagcct ccatcgccac tgggggctcc tgctggcctc cctcgctccc 360
   ctggtcctct gtctgtctct cctgtgaaac ctgggagacc agagtcccct ttagggttcc 420
   cggagctccg ggaagacccg cccatcccag gactccagat gccaggagcc ctgctgccca 480
   catgcaagga ccagcatctc ctgagaggac gcctgggctt acctgggggg caggatgcct 540
   ggggacagtt cag 553
<210> 86
   <211> 365
   <212> DNA
   <213> human
<400> 86
   ggaggatagg ataatcccgg gtggcatcta taacgcagac ctcaatgatg agtgggtaca 60
   gcgtgccctt cacttcgcca tcagcgagta taacaaggcc accaaagatg actactacag 120
   acgtccgctg cgggtactaa gagccaggca acagaccgtt gggggggtga attacttctt 180
   cgacgtagag gtgggccgaa ccatatgtac caagtcccag cccaacttgg acacctgtgc 240
   cttccatgaa cagccagaac tgcagaagaa acagttgtgc tctttcgaga tctacgaagt 300
   tccctgggag aacagaaggt ccctggtgaa atccaggtgt caagaatcct agggatctgt 360
   gccag 365
<210> 87
   <211> 331
   <212> DNA
   <213> human
<400> 87
   gagacgtggt aagtgcggtg cagttttcaa ctgacctctg gacgcagaac ttcagccatg 60
   aaggtaacag gcatctttct tctcagtgcc ttggccctgt tgagtctatc tggtaacact 120
   ggagctgact ccctgggaag agaggccaaa tgttacaatg aacttaatgg atgcaccaag 180
   atatatgacc ctgtctgtgg gactgatgga aatacttatc ccaatgaatg cgtgttatgt 240
   tttgaaggtc ggaaacgcca gacttctatc ctcattcaaa aatctgggcc ttgctgagaa 300
   ccaaggtttt gaaatcccat caggtcaccg c 331
<210> 88
   <211> 498
   <212> DNA
   <213> human
<400> 88
   gtggacctta gaatacagtt ttgagtagag ttgatcaaaa tcaattaaaa tagtctcttt 60
   aaaaggaaag aaaacatctt taaggggagg aaccagagtg ctgaaggaat ggaagtccat 120
   ctgcgtgtgt gcagggagac tgggtaggaa agaggaagca aatagaagag agaggttgaa 180
   aaacaaaatg ggttacttga ttggtgatta ggtggtggta gagaagcaag taaaaaggct 240
   aaatggaagg gcaagtttcc atcatctata gaaagctata taagacaaga actccccttt 300
   ttttcccaaa ggcattataa aaagaatgaa gcctccttag aaaaaaaatt atacctcaat 360
   gtccccaaca agattgctta ataaattgtg tttcctccaa gctattcaat tcttttaact 420
   gttgtagaag acaaaatgtt cacaatatat ttagttgtaa accaagtgat caaactacat 480
   attgtaaagc ccattttt 498
<210> 89
   <211> 489
   <212> DNA
   <213> human
<400> 89
   aagtctgtag tctttatgat cctaaaaggg aaaattgcct tggtaacttt cagattcctg 60
   tggaattgtg aattcatact aagctttctg tgcagtctca ccatttgcat cactgaggat 120
   gaaactgact tttgtctttt ggagaaaaaa aactgtactg ttgttcaaga gggctgtgat 180
   taaaatcttt aagcatttgt tcctgccaag gtagttttct tgcattttgc tctccattca 240
   gcatgtgtgt gggtgtggat gtttataaac aagactaagt ctgacttcat aagggctttc 300
   taaaaccatt tctgtccaag agaaaatgac tttttgcttt gatattaaaa attcaatgag 360
   taaaacaaaa gctagtcaaa tgtgttagca gcatgcagaa caaaaacttt aaactttctc 420
   tctcactata cagtatattg tcaatgtgaa agtgtggaat ggaagaaatg tcgatcctgt 480
   tgtaactga 489
<210> 90
   <211> 508
   <212> DNA
   <213> human
<400> 90
   gcaccctggt gtgactctag tgatctacgt agctcggctt ttttggcaca tggatcaaca 60
   aaatcggcaa ggtctcaggg accttgttaa cagtggagta actattcaga ttatgagagc 120
   atcagagtat tatcactgct ggaggaattt tgtcaactac ccacctgggg atgaagctca 180
   ctggccacaa tacccacctc tgtggatgat gttgtacgca ctggagctgc actgcataat 240
   tctaagtctt ccaccctgtt taaagatttc aagaagatgg caaaatcatc ttacattttt 300
   cagacttcat cttcaaaact gccattacca aacgattccg ccacacatcc ttttagctac 360
   agggctgata catccttctg tggcttggag atgaatagga tgattccgtg tgtgtactga 420
   ttcaagaaca agcaatgatg acccactaaa gagtgaatgc catttagaat ctagaaatgt 480
   tcacaaggta ccccaaaact ctgtagct 508
<210> 91
   <211> 428
   <212> DNA
   <213> human
<400> 91
   gaacatccaa gtctttcttc ttttttaagt tgtcaaagaa gcttccacaa aattagaaag 60
   gacaacagtt ctgagctgta atttcgcctt aaactctgga cactctatat gtagtgcatt 120
   tttaaacttg aaatatataa tattcagcca gcttaaaccc atacaatgta tgtacaatac 180
   aatgtacaat tatgtctctt gagcatcaat cttgttactg ctgattcttg taaatctttt 240
   tgcttctact ttcatcttaa actaatacgt gccagatata actgtcttgt ttcagtgaga 300
   gacgccctat ttctatgtca tttttaatgt atctatttgt acaattttaa agttcttatt 360
   ttagtataca tataaatatc agtattctga catgtaagaa aatgttacgg catcacactt 420
   atatttta 428
<210> 92
   <211> 405
   <212> DNA
   <213> human
<400> 92
   aaccgaatgc ggtgctacaa ctgtggtgga agccccagca gttcttgcaa agaggccgtg      60
   accacctgtg gcgagggcag accccagcca ggcctggaac agatcaagct acctggaaac     120
   cccccagtga ccttgattca ccaacatcca gcctgcgtcg cagcccatca ttgcaatcaa     180
   gtggagacag agtcggtggg agacgtgact tatccagccc acagggactg ctacctggga     240
   gacctgtgca acagcgccgt ggcaagccat gtggcccctg caggcatttt ggctgcagca     300
   gctaccgccc tgacctgtct cttgccagga ctgtggagcg gataggggga gtaggagtag     360
   agaagggaac aagggagcaa gggaacaagg gacatctgaa catct                     405
<210> 93
   <211> 505
   <212> DNA
   <213> human
<220>
   <221> misc_feature
   <222> (281)..(281)
   <223> n is a, c, g, or t
<400> 93
   agaacagcag ctttctaggg acagctggaa agggacttaa tgtgtttgac tatttcttac      60
   gagggttcta cttatttatg tatttatttt tgaaagcttg tattttaata ttttacatgc     120
   tgttatttaa agatgtgagt gtgtttcatc aaacatagct cagtcctgat tatttaattg     180
   gaatatgatg ggttttaaat gtgtcattaa actaatattt agtgggagac cataatgtgt     240
   cagccacctt gataaatgac agggtgggga actggagggt ngggggattg aaatgcaagc     300
   aattagtgga tcactgttag ggtaagggaa tgtatgtaca catctatttt ttatactttt     360
   ttttttaaaa aagaatgtca gttgttattt attcaaatta tctcacatta tgtgttcaac     420
   atttttatgc tgaagtttcc cttagacatt ttatgtcttg cttgtagggc ataatgcctt     480
   gtttaatgtc cattctgcag cgttt                                           505
<210> 94
   <211> 533
   <212> DNA
   <213> human
<400> 94
   ccctcaatct agaacgctac acaagaaata ttttgttttt actcagcagg tgtgccttaa      60
   cctccctatt cagaaagctc cacatcaata aacatgacac tctgaagtga aagtagccac     120
   gagaattgtg ctacttatac tggaacataa tctggaggca aggttcgact gcagtcgaac     180
   cttgcctcca gattatgaac cagtataagt agcacaattc tcgtggctac tttcacttca     240
   gagtgtcatg tttattgatg tggagctttc tgaataggga ggttaaggca cacctgctga     300
   gtaaaacaaa tatttcttgt gtagcgttct taggaatctg gtgtctgtcc ggccccggta     360
   ggcctgttgg gtttctagtc ctccttacca tcatctccat atgagagtgt gaaaatagga     420
   acacgtgctc tacctccatt tagggatttg cttgggatac agaagaggcc atgtgtctca     480
   gagctgttaa gggcttattt ttttaaaaca ttggagtcat agcatgtgtg taa            533
<210> 95
   <211> 471
   <212> DNA
   <213> human
<400> 95
   gttttgggta tgtttaatct gttatgtact agtgttctgt ttgttattgt tttgttaatt      60
   acaccataat gctaatttaa agagactcca aatctcaatg aagccagctc acagtgctgt     120
   gtgccccggt catctagcaa gctgccgaac caaaagaatt tgcaccccgc tgcgggccca     180
   cgtggttggg gccctgccct ggcagggtca tcctgtgctc ggaggccatc tcgggcacag     240
   gcccaccccg ccccacccct ccagaacacg gctcacgctt acctcaacca tcctggctgc     300
   ggcgtctgtc tgaaccacgc gggggccttg agggacgctt tgtctgtcgt gatggggcaa     360
   gggcacaagt cctggatgtt gtgtgtatcg agaggccaaa ggctggtggc aagtgcacgg     420
   ggcacagcgg agtctgtcct gtgacgcgca agtctgaggg tctgggcggc g              471
<210> 96
   <211> 382
   <212> DNA
   <213> human
<400> 96
   gattccctgc atcaactaag aaaagcctgt tttctttatt tcaaacttgg tggcgaatgt      60
   gttgcgggtc ctgttgggct gctttctgta ttgtctccta accctctagt tttaattgga     120
   cacttctttg ctgttgcaat ctatgccgtg tatttttgct ttaagtcaga accttggatt     180
   acaaaacctc gagcccttct cagtagtggt gctgtattgt acaaagcgtg ttctgtaata     240
   tttcctctaa tttactcaga aatgaagtat atggttcatt aagcttaaag gggaaccatt     300
   tgtgaatgaa tatttggaac ttaccaagtc ctaagagact tttggaagag gatatatata 360
   gcatagtacc ataccactta ta 382
<210> 97
   <211> 542
   <212> DNA
   <213> human
<400> 97
   tgcggaaata cctgaaatac agcaaaaata tcctggaccg gcaagatcct ccctctgtgg 60
   tggtcaccag ccaccaggcc ccaggagaaa agaagaaact gaagtgcctg gcctacgact 120
   tctacccagg gaaaattgat gtgcactgga ctcgggccgg cgaggtgcag gagcctgagt 180
   tacggggaga tgttcttcac aatggaaatg gcacttacca gtcctgggtg gtggtggcag 240
   tgcccccgca ggacacagcc ccctactcct gccacgtgca gcacagcagc ctggcccagc 300
   ccctcgtggt gccctgggag gccagctagg aagcaagggt tggaggcaat gtgggatctc 360
   agacccagta gctgcccttc ctgcctgatg tgggagctga accacagaaa tcacagtcaa 420
   tggatccaca aggcctgagg agcagtgtgg ggggacagac aggaggtgga tttggagacc 480
   gaagactggg atgcctgtct tgagtagact tggacccaaa aaatcatctc accttgagcc 540
   ca 542
<210> 98
   <211> 382
   <212> DNA
   <213> human
<400> 98
   gaagacttac tgttggccat agttaattgt gtgaggaaca cgccggcctt tttagccgaa 60
   agactgcatc gagccttgaa gggtattgga actgatgagt ttactctgaa ccgaataatg 120
   gtgtccagat cagaaattga ccttttggac attcgaacag agttcaagaa gcattatggc 180
   tattccctat attcagcaat taaatcggat acttctggag actatgaaat cacactctta 240
   aaaatctgtg gtggagatga ctgaaccaag aagataatct ccaaaggtcc acgatgggct 300
   tttccaacag ctccacctta cttcttctca tactatttaa gagaacaagc aaatataaac 360
   agcaacttgt gttcctaaca gg 382
<210> 99
   <211> 499
   <212> DNA
   <213> human
<400> 99
   agagattcat tgcagctcag catggctcag accagctcat acttcatgct gatctcctgc 60
   ctgatgtttc tgtctcagag ccaaggccaa gaggcccaga cagagttgcc ccaggcccgg 120
   atcagctgcc cagaaggcac caatgcctat cgctcctact gctactactt taatgaagac 180
   cgcgagacct gggttgatgc agatctctat tgccagaaca tgaattcggg caacctggtg 240
   tctgtgctca cccaggccga gggtgccttt gtggcctcac tgattaagga gagtggcact 300
   gatgacttca atgtctggat tggcctccat gaccccaaaa agaaccgccg ctggcactgg 360
   agcagtgggt ccctggtctc ctacaagtcc tggggcattg gagccccaag cagtgttaat 420
   cctggctact gtgtgagcct gacctcaagc acaggattcc agaaatggaa ggatgtgcct 480
   tgtgaagaca agttctcct 499
<210> 100
   <211> 375
   <212> DNA
   <213> human
<400> 100
   ttttaccttg gatgctgact tctaaatgaa ctgaagatgt gcccttactt ggctgatttt 60
   ttttttccat ctcataagaa aaatcagctg aagtgttacc aactagccac accatgaatt 120
   gtccgtaatg ttcattaaca gcatctttaa aactgtgtag ctacctcaca accagtcctg 180
   tctgtttata gtgctggtag tatcaccttt tgccagaagg cctggctggc tgtgacttac 240
   catagcagtg acaatggcag tcttggcttt aaagtgaggg gtgacccttt agtgagctta 300
   gcacagcggg attaaacagt cctttaacca gcacagccag ttaaaagatg cagcctcact 360
   gcttcaacgc agatt 375
<210> 101
   <211> 571
   <212> DNA
   <213> human
<400> 101
   agagagacac agctgcagag gccacctgga ttgcgcctaa tgtgtttgag catcacttag 60
   gagaagtctt ctatttattt atttatttat ttatttattt gtttgtttta gaagattcta 120
   tgttaatatt ttatgtgtaa aataaggtta tgattgaatc tacttgcaca ctctcccatt 180
   atatttattg tttattttag gtcaaaccca agttagttca atcctgattc atatttaatt 240
   tgaagataga aggtttgcag atattctcta gtcatttgtt aatatttctt cgtgatgaca     300
   tatcacatgt cagccactgt gatagaggct gaggaatcca agaaaatggc cagtaagatc     360
   aatgtgacgg cagggaaatg tatgtgtgtc tattttgtaa ctgtaaagat gaatgtcagt     420
   tgttatttat tgaaatgatt tcacagtgtg tggtcaacat ttctcatgtt gaagctttaa     480
   gaactaaaat gttctaaata tcccttggac attttatgtc tttcttgtaa gatactgcct     540
   tgtttaatgt taattatgca gtgtttccct c                                    571
<210> 102
   <211> 531
   <212> DNA
   <213> human
<400> 102
   tcctctcgtg gggtgtttac ccctgtggct ctgcccagca tccagctgtc tacacccaga       60
   tctgcaaata catgtcctgg atcaataaag tcatacgctc caactgatcc agatgctacg     120
   ctccagctga tccagatgtt atgctcctgc tgatccagat gcccagaggc tccatcgtcc     180
   atcctcttcc tccccagtcg gctgaactct ccccttgtct gcactgttca aacctctgcc     240
   gccctccaca cctctaaaca tctcccctct cacctcattc ccccacctat ccccattctc     300
   tgcctgtact gaagctgaaa tgcaggaagt ggtggcaaag gtttattcca gagaagccag     360
   gaagccggtc atcacccagc ctctgagagc agttactggg gtcacccaac ctgacttcct     420
   ctgccactcc ccgctgtgtg actttgggca agccaagtgc cctctctgaa cctcagtttc     480
   ctcatctgca aaatgggaac aatgacgtgc ctacctctta gacatgttgt g              531
<210> 103
   <211> 529
   <212> DNA
   <213> human
<400> 103
   gaatggtgca tacaaggcca tccccgttgc ccaggacctg aacgcgcctt ctgattggga      60
   cagccgtggg aaggacagtt atgaaacgag tcagctggat gaccagagtg ctgaaaccca     120
   cagccacaag cagtccagat tatataagcg gaaagctaat gatgagagca atgagcattc     180
   cgatgtgatt gatagtcagg aactttccaa agtcagccgt gaattccaca gccatgaatt     240
   tcacagccat gaagatatgc tggttgtaga ccccaaaagt aaggaagaag ataaacacct     300
   gaaatttcgt atttctcatg aattagatag tgcatcttct gaggtcaatt aaaaggagaa     360
   aaaatacaat ttctcacttt gcatttagtc aaaagaaaaa atgctttata gcaaaatgaa     420
   agagaacatg aaatgcttct ttctcagttt attggttgaa tgtgtatcta tttgagtctg 480
   gaaataactg atgtgtttga taattagttt agtttgtggc ttcatggaa 529
<210> 104
   <211> 536
   <212> DNA
   <213> human
<400> 104
   acagctttcc aaggtgacaa actcctctat gcagtgtatc gaaagctggg tgtttatgaa 60
   gttgaagacc agattacagc tgtcagaaaa ttcatagaaa tgggtttcat tgatgaaaaa 120
   agaatagcca tatggggctg gtcctatgga ggatacgttt catcactggc ccttgcatct 180
   ggaactggtc ttttcaaatg tggtatagca gtggctccag tctccagctg ggaatattac 240
   gcgtctgtct acacagagag attcatgggt ctcccaacaa aggatgataa tcttgagcac 300
   tataagaatt caactgtgat ggcaagagca gaatatttca gaaatgtaga ctatcttctc 360
   atccacggaa cagcagatga taatgtgcac tttcagaact cagcacagat tgctaaagct 420
   ctggttaatg cacaagtgga tttccaggca atgtggtact ctgaccagaa ccacggctta 480
   tccggcctgt ccacgaacca cttatacacc cacatgaccc acttcctaaa gcagtg 536
<210> 105
   <211> 409
   <212> DNA
   <213> human
<400> 105
   agtcaagtga ccagcctctg actgtgcctg tatctcccaa attctccact cgattccact 60
   gctaaactca gctgtgagct gcggataccg cccggcaatg ggacctgctc ttaacctcaa 120
   acctaggacc gtcttgcttt gtcattgggc atggagagaa cccatttctc cagactttta 180
   cctacccgtg cctgagaaag catacttgac aactgtggac tccagttttg ttgagaattg 240
   ttttcttaca ttactaaggc taataatgag atgtaactca tgaatgtctc gattagactc 300
   catgtagtta cttcctttaa accatcagcc ggccttttat atgggtcttc actctgacta 360
   gaatttagtc tctgtgtcag cacagtgtaa tctctattgc tattgcccc 409
<210> 106
   <211> 370
   <212> DNA
   <213> human
<400> 106
   gctttcaccg gcaagttcga gatggagagt gagaagaatt atgatgagtt catgaagctc 60
   cttgggatct ccagcgatgt aatcgaaaag gcccgcaact tcaagatcgt cacggaggtg 120
   cagcaggatg ggcaggactt cacttggtcc cagcactact ccgggggcca caccatgacc 180
   aacaagttca ctgttggcaa ggaaagcaac atacagacaa tggggggcaa gacgttcaag 240
   gccactgtgc agatggaggg cgggaagctg gtggtgaatt tccccaacta tcaccagacc 300
   tcagagatcg tgggtgacaa gctggtggag gtctccacca tcggaggcgt gacctatgag 360
   cgcgtgagca 370
<210> 107
   <211> 441
   <212> DNA
   <213> human
<400> 107
   aaaggagcag tcgcacagac ctttcctcat gctgcaggcc cggcagtctg aagaccaccc 60
   tcatcgccgg cgtcggcggg gcttggagtg tgatggcaag gtcaacatct gctgtaagaa 120
   acagttcttt gtcagtttca aggacatcgg ctggaatgac tggatcattg ctccctctgg 180
   ctatcatgcc aactactgcg agggtgagtg cccgagccat atagcaggca cgtccgggtc 240
   ctcactgtcc ttccactcaa cagtcatcaa ccactaccgc atgcggggcc atagcccctt 300
   tgccaacctc aaatcgtgct gtgtgcccac caagctgaga cccatgtcca tgttgtacta 360
   tgatgatggt caaaacatca tcaaaaagga cattcagaac atgatcgtgg aggagtgtgg 420
   gtgctcatag agttgcccag c 441
<210> 108
   <211> 412
   <212> DNA
   <213> human
<400> 108
   cagaccttgt gatattccag ttccccctgc agtggtttgg agtccctgcc attctgaaag 60
   gctggtttga gccttcatag gagagtttgc ttacacttac gctgccatgt atgacaaagg 120
   acccttccgg agtggcattc tgcatttctg tggcttccaa gtcttagaac ctcaactgac 180
   atatagcatt gggcacactc cagcagacgc ccgaattcaa atcctggaag gatggaagaa 240
   acgcctggag aatatttggg atgagacacc actgtatttt gctccaagca gcctctttga 300
   cctaaacttc caggcaggat tcttaatgaa aaaagaggta caggatgagg agaaaaacaa 360
   gaaatttggc ctttctgtgg gccatcactt gggcaagtcc atcccaactg ac 412
<210> 109
   <211> 372
   <212> DNA
   <213> human
<400> 109
   gtaacactct ggtacagatc tccagaagta ttgctggggt cagctcgtta ctcaactcca      60
   gttgacattt ggagtatagg caccatattt gctgaactag caactaagaa accacttttc     120
   catggggatt cagaaattga tcaactcttc aggattttca gagctttggg cactcccaat     180
   aatgaagtgt ggccagaagt ggaatcttta caggactata agaatacatt tcccaaatgg     240
   aaaccaggaa gcctagcatc ccatgtcaaa aacttggatg aaaatggctt ggatttgctc     300
   tcgaaaatgt taatctatga tccagccaaa cgaatttctg gcaaaatggc actgaatcat     360
   ccatatttta at                                                         372
<210> 110
   <211> 164
   <212> DNA
   <213> human
<220>
   <221> misc_feature
   <222> (105)..(105)
   <223> n is a, c, g, or t
<400> 110
   ggttccatca atggtgagca ccagcctgaa tgcagaagcg ctccagtatc tccaagggta      60
   ccttcaggca gccagtgtga cactgcttta aactgcattt ttctnaatgg gctaaaccca     120
   gatggtttcc taggaaatca caggcttctg agcacagctg catt                      164
<210> 111
   <211> 370
   <212> DNA
   <213> human
<400> 111
   tactggaacc tatgatctga agagcgtcct gggtcaactg ggcatcacta aggtcttcag      60
   caatggggct gacctctccg gggtcacaga ggaggcaccc ctgaagctct ccaaggccgt      120
   gcataaggct gtgctgacca tcgacgagaa agggactgaa gctgctgggg ccatgttttt     180
   agaggccata cccatgtcta tcccccccga ggtcaagttc aacaaaccct ttgtcttctt     240
   aatgattgaa caaaatacca agtctcccct cttcatggga aaagtggtga atcccaccca 300
   aaaataactg cctctcgctc ctcaacccct cccctccatc cctggccccc tccctggatg 360
   acattaaaga 370
<210> 112
   <211> 216
   <212> DNA
   <213> human
<400> 112
   gtgtgaaggt gcagttttgc caaggagtgc taaagaactt agatgtcagt gcataaagac 60
   atactccaaa cctttccacc ccaaatttat caaagaactg agagtgattg agagtggacc 120
   acactgcgcc aacacagaaa ttattgtaaa gctttctgat ggaagagagc tctgtctgga 180
   ccccaaggaa aactgggtgc agagggttgt ggagaa 216
<210> 113
   <211> 417
   <212> DNA
   <213> human
<400> 113
   attgtaaatc ttttgtgtct cctgaagact tcccttaaaa ttagctctga gtgaaaaatc 60
   aaaagagaca aaagacatct tcgaatccat atttcaagcc tggtagaatt ggcttttcta 120
   gcagaacctt tccaaaagtt ttatattgag attcataaca acaccaagaa ttgattttgt 180
   agccaacatt cattcaatac tgttatatca gaggagtagg agagaggaaa catttgactt 240
   atctggaaaa gcaaaatgta cttaagaata agaataacat ggtccattca cctttatgtt 300
   atagatatgt ctttgtgtaa atcatttgtt ttgagttttc aaagaatagc ccattgttca 360
   ttcttgtgct gtacaatgac cactgttatt gttactttga cttttcagag cacaccc 417
<210> 114
   <211> 372
   <212> DNA
   <213> human
<220>
   <221> misc_feature
   <222> (43)..(43)
   <223> n is a, c, g, or t
<400> 114
   tccaaggact gagactgacc tcctctggtg acactggcct agngcctgac actctcctaa 60
   gaggttctct ccaagccccc aaatagctcc aggcgccctc ggccgcccat catggttaat 120
   tctgtccaac aaacacacac gggtagattg ctggcctgtt gtaggtggta gggacacaga 180
   tgaccgacct ggtcactcct cctgccaaca ttcagtctgg tatgtgaggc gtgcgtgaag 240
   caagaactcc tggagctaca gggacaggga gccatcattc ctgcctggga atcctggaag 300
   acttcctgca ggagtcagcg ttcaatcttg accttgaaga tgggaaggat gttcttttta 360
   cgtaccaatt ct 372
<210> 115
   <211> 525
   <212> DNA
   <213> human
<220>
   <221> misc_feature
   <222> (44)..(44)
   <223> n is a, c, g, or t
<400> 115
   cgatgcaagt gtttctgttc tgggaggtat tggagggaaa aaancaagca ggatggctgg 60
   aacactgtac tgaggaatga atagaaaggc ttccagatgt ctaaaagatt ctttaaacta 120
   ctgaactgtt acctaggtta acaaccctgt tgagtatttg ctgtttgtcc agttcaggaa 180
   tttttgtttt gttttgtcta tatgtgcggc ttttcagaag aaatttaatc agtgtgacag 240
   aaaaaaaaat gttttatggt agcttttact ttttatgaaa aaaaaattat ttgcctttta 300
   aattcttttc ccccatcccc ctccaaagtc ttgatagcaa gcgttatttt gggggtagaa 360
   acggtgaaat ctctagcctc tttgtgtttt tgttgttgtt gttgttgttg ttttatataa 420
   tgcatgtatt cactaaaata aaatttaaaa aactcctgtc ttgctagaca aggttgctgt 480
   tgtgcagtgt gcctgtcact actggtctgt actccttgga tttgc 525
<210> 116
   <211> 116
   <212> DNA
   <213> human
<400> 116
   tacagccagg cataacatat ccactgtgtg catagagggt ctcttcacgt tgatgcttgg 60
   cattccatca gctttctcta agtctttgct caagttcaac cttaaaatga tgttag 116
<210> 117
   <211> 517
   <212> DNA
   <213> human
<220>
   <221> misc_feature
   <222> (181)..(181)
   <223> n is a, c, g, or t
<400> 117
   ggaaaacacc tcatttgacc ttgccagctg accttcaaac cctgcatttg aaccgaccaa     60
   cattaagtcc agagagtaaa cttgaatgga ataacgacat tccagaagtt aatcatttga    120
   attctgaaca ctggagaaaa accgaaaaat ggacggggca tgaagagact aatcatctgg    180
   naaaccgatt tcagtggcga tggcatgaca gagctagagc tcgggcccag ccccaggctg    240
   cagcccattc gcaggcaccc gaaagaactt ccccagtatg gtggtcctgg aaaggacatt    300
   tttgaagatc aactatatct tcctgtgcat tccgatggaa tttcagttca tcagatgttc    360
   accatggcca ccgcagaaca ccgaagtaat tccagcatag cggggaagat gttgaccaag    420
   gtggagaaga atcacgaaaa ggagaagtca cagcacctag aaggcagcgc ctcctcttca     480
   ctctcctctg attagatgaa actgttacct tacccta                               517
<210> 118
   <211> 397
   <212> DNA
   <213> human
<400> 118
   aatatccttg ttgtgtatta ggtttttaaa taccagctaa aggattacct cactgagtca     60
   tcagtaccct cctattcagc tccccaagat gatgtgtttt tgcttaccct aagagaggtt    120
   ttcttcttat ttttagataa ttcaagtgct tagataaatt atgttttctt taagtgttta    180
   tggtaaactc ttttaaagaa aatttaatat gttatagctg aatctttttg gtaactttaa    240
   atctttatca tagactctgt acatatgttc aaattagctg cttgcctgat gtgtgtatca    300
   tcggtgggat gacagaacaa acatatttat gatcatgaat aatgtgcttt gtaaaaagat    360
   ttcaagttat taggaagcat actctgtttt ttaatca                             397
<210> 119
   <211> 518
   <212> DNA
   <213> human
<400> 119
   gtgtgcacac ggagactcat cgttataatt tactatctgc caagagtaga aagaaaggct     60
   ggggatattt gggttggctt ggttttgatt ttttgcttgt ttgtttgttt tgtactaaaa     120
   cagtattatc ttttgaatat cgtagggaca taagtatata catgttatcc aatcaagatg     180
   gctagaatgg tgcctttctg agtgtctaaa acttgacacc cctggtaaat ctttcaacac     240
   acttccactg cctgcgtaat gaagttttga ttcattttta accactggaa tttttcaatg     300
   ccgtcatttt cagttagatg attttgcact ttgagattaa aatgccatgt ctatttgatt     360
   agtcttattt ttttattttt acaggcttat cagtctcact gttggctgtc attgtgacaa     420
   agtcaaataa acccccaagg acgacacaca gtatggatca catattgttt gacattaagc     480
   ttttgccaga aaatgttgca tgtgttttac ctcgactt                            518
<210> 120
   <211> 404
   <212> DNA
   <213> human
<220>
   <221> misc_feature
   <222> (374)..(374)
   <223> n is a, c, g, or t
<400> 120
   caagagctac aatgtcacct ccgtcctgtt taggaaaaag aagtgtgact actggatcag      60
   gacttttgtt ccaggttgcc agcccggcga gttcacgctg ggcaacatta agagttaccc     120
   tggattaacg agttacctcg tccgagtggt gagcaccaac tacaaccagc atgctatggt     180
   gttcttcaag aaagtttctc aaaacaggga gtacttcaag atcaccctct acgggagaac     240
   caaggagctg acttcggaac taaaggagaa cttcatccgc ttctccaaat ctctgggcct     300
   ccctgaaaac cacatcgtct tccctgtccc aatcgaccag tgtatcgacg gctgagtgca     360
   caggtgccgc cagntgccgc accagcccga acaccattga ggga                      404
<210> 121
   <211> 448
   <212> DNA
   <213> human
<400> 121
   ccttcttgtc cacggttttg ttgagttttc actcttctaa tgcaagggtc tcacactgtg      60
   aaccacttag gatgtgatca ctttcaggtg gccaggaatg ttgaatgtct ttggctcagt     120
   tcatttaaaa aagatatcta tttgaaagtt ctcagagttg tacatatgtt tcacagtaca     180
   ggatctgtac ataaaagttt ctttcctaaa ccattcacca agagccaata tctaggcatt 240
   ttcttggtag cacaaatttt cttattgctt agaaaattgt cctccttgtt atttctgttt 300
   gtaagactta agtgagttag gtctttaagg aaagcaacgc tcctctgaaa tgcttgtctt 360
   ttttctgttg ccgaaatagc tggtcctttt tcgggagtta gatgtataga gtgtttgtat 420
   gtaaacattt cttgtaggca tcaccatg 448
<210> 122
   <211> 465
   <212> DNA
   <213> human
<400> 122
   caatatctga caccactttg gactcaagag actcagtaac gtattatcct gtttatttag 60
   cttggtttta gctgtgttct ctctggataa cccacttgat gttaggaaca ttacttctct 120
   gcttattcca tattaatact gtgttaggta ttttaagaag caagttatta aataagaaaa 180
   gtcaaagtat taattcttac cttctattat cctatattag cttcaataca tccaaaccaa 240
   atggctgtta ggtagattta tttttatata agcatgttta ttttgatcag atgttttaac 300
   ttggatttga aaaaatacat ttatgagatg ttttataaga tgtgtaaata tagaactgta 360
   tttattacta tagtaaaggt tcagtaacat taaggaccat gataatgata ataaaccttg 420
   tacagtggca tattctttga tttatattgt gtttctctgc ccatt 465
<210> 123
   <211> 248
   <212> DNA
   <213> human
<220>
   <221> misc_feature
   <222> (45)..(45)
   <223> n is a, c, g, or t
<400> 123
   cacaaaaccc cagggacagc ggtctcccca gcctgccctg ctcangcctt gcccccaaac 60
   ctgtactgtc ccggaggagg ttgggaggtg gaggcccagc atcccgcgca gatgacaccg 120
   gttttcctag aagcccctca cccccactgg cccactggtg gctaggtctc cccttatcct 180
   tctggtccag cgcaaggagg ggctgcttct gaggtcggtg gctgtctttc cattaaagaa 240
   acaccgtg 248
<210> 124
   <211> 419
   <212> DNA
   <213> human
<220>
   <221> misc_feature
   <222> (30)..(30)
   <223> n is a, c, g, or t
<220>
   <221> misc_feature
   <222> (53)..(53)
   <223> n is a, c, g, or t
<220>
   <221> misc_feature
   <222> (55)..(55)
   <223> n is a, c, g, or t
<220>
   <221> misc_feature
   <222> (248)..(248)
   <223> n is a, c, g, or t
<400> 124
   gaaataaccc agacttaatc ttgaatgatn cgattatgcc caatattaag tananaatat     60
   aagaaaaggt tatcttaaat agatcttagg caaaatacca gctgatgaag gcatctgatg    120
   ccttcatctg ttcagtcatc tccaaaaaca gtaaaaataa ccactttttg ttgggcaata    180
   tgaaattttt aaaggagtag aataccaaat gatagaaaca gactgcctga attgagaatt    240
   ttgatttntt aaagtgtgtt tctttctaaa ttgctgttcc ttaatttgat taatttaatt    300
   catgtattat gattaaatct gaggcagatg agcttacaag tattgaaata attactaatt    360
   aatcacaaat gtgaagttat gcatgatgta aaaaatacaa acattctaat taaaggctt     419
<210> 125
   <211> 396
   <212> DNA
   <213> human
<220>
   <221> misc_feature
   <222> (310).. (310)
   <223> n is a, c, g, or t
<220>
   <221> misc_feature
   <222> (314)..(314)
   <223> n is a, c, g, or t
<400> 125
   tcaacaccct cttcttgaac tggtgctgtc tgggcttcat agcattcgcc tactccgtga 60
   agtctaggga caggaagatg gttggcgacg tgaccggggc ccaggcctat gcctccaccg 120
   ccaagtgcct gaacatctgg gccctgattc tgggcatcct catgaccatt ggattcatcc 180
   tgttactggt attcggctct gtgacagtct accatattat gttacagata atacaggaaa 240
   aacggggtta ctagtagccg cccatagcct gcaacctttg cactccactg tgcaatgctg 300
   gccctgcacn gctngggctg ttgcccctgc ccccttggtc ctgcccctag atacagcagt 360
   ttatacccac acacctgtct acagtgtcat tcaata 396
<210> 126
   <211> 245
   <212> DNA
   <213> human
<400> 126
   ggtgagggga tgacccctgg agatgaaggg aagaggtgaa gccttagcaa aaatgcctcc 60
   tcaccactcc ccaggagaat ttttataaaa agcataatca ctgattcctt cactgacata 120
   atgtaggaag cctctgagga gaaaaacaaa gggagaaaca tagagaacgg ttgctactgg 180
   cagaagcata agatctttgt acaatattgc tggccctggt tcacctgttt actgttatca 240
   caata 245
<210> 127
   <211> 436
   <212> DNA
   <213> human
<400> 127
   gtgattcaaa cttctgtgta ctgggtgatg cacccattgt gattgtggaa gatagaattc 60
   aatttgaact caggttgttt atgaggggaa aaaaacagtt gcatagagta tagctctgta 120
   gtggaatatg tcttctgtat aactaggctg ttaacctatg attgtaaagt agctgtaaga 180
   atttcccagt gaaataaaaa aaaattttaa gtgttctcgg ggatgcatag attcatcatt 240
   ttctccacct taaaaatgcg ggcatttaag tctgtccatt atctatatag tcctgtcttg 300
   tctattgtat atataatcta tatgattaaa gaaaatatgc ataatcagac aagcgtaatg 360
   cataatcaga caagcttgaa tattgttttt gcaccagacg aacagtgagg aaattcggag 420
   ctatacatat gtgcag 436
<210> 128
   <211> 551
   <212> DNA
   <213> human
<220>
   <221> misc_feature
   <222> (158)..(158)
   <223> n is a, c, g, or t
<220>
   <221> misc_feature
   <222> (516)..(516)
   <223> n is a, c, g, or t
<400> 128
   agtcagtgtt gtcttaatat ccttgataat gctgtaaagt ttatttttac aaatatttct      60
   gtttaagcta tttcaccttt gtttggaaat ccttcccttt taaagagaaa atgtgacact     120
   tgtgaaaagg cttgtaggaa agctcctccc tttttttnct ttaaaccttt aaatgacaaa     180
   cctaggtaat taatggttgt gaatttctat ttttgctttg tttttaatga acatttgtct     240
   ttcagaatag gattctgtga taatatttaa atggcaaaaa caaaacataa ttttgtgcaa     300
   ttaacaaagc tactgcaaga aaaataaaac atttcttggt aaaaacgtat gtatttatat     360
   attatatatt tatatataat atatattata tatttagcat tgctgagctt tttagatgcc     420
   tattgtgtat cttttaaagg ttttgaccat tttgttatga gtaattacat atatattaca     480
   ttcactatat taaaattgta cttttttact atgtgnctca ttggttcata gtctttattt     540
   tgtcctttga a                                                          551
<210> 129
   <211> 370
   <212> DNA
   <213> human
<220>
   <221> misc_feature
   <222> (227)..(227)
   <223> n is a, c, g, or t
<220>
   <221> misc_feature
   <222> (229)..(229)
   <223> n is a, c, g, or t
<220>
   <221> misc_feature
   <222> (287)..(287)
   <223> n is a, c, g, or t
<220>
   <221> misc_feature
   <222> (338)..(338)
   <223> n is a, c, g, or t
<400> 129
   tatgtcgctg tccaagagaa ggctgtggaa gaacctatac aactgtgttt aatctccaaa 60
   gccatatcct ctccttccat gaggaaagcc gcccttttgt gtgtgaacat gctggctgtg 120
   gcaaaacatt tgcaatgaaa caaagtctca ctaggcatgc tgttgtacat gatcctgaca 180
   agaagaaaat gaagctcaaa gtcaaaaaat ctcgtgaaaa acggagntng gcctctcatc 240
   tcagtggata tatccctccc aaaaggaaac aagggcaagg cttatcnttg tgtcaaaacg 300
   gagagtcacc caactgtgtg gaagacaaga tgctctcnac agttgcagta cttacccttg 360
   gctaagaact 370
<210> 130
   <211> 157
   <212> DNA
   <213> human
<400> 130
   agggcctaag ggtgacagag gtgatgctgg tcccaaaggt gctgatggct ctcctggcaa 60
   agatggcgtc cgtggtctga ccggccccat tggtcctcct ggccctgctg gtgcccctgg 120
   tgacaagggg gaccccattc ccgaggagct ttatgag 157
<210> 131
   <211> 460
   <212> DNA
   <213> human
<220>
   <221> misc_feature
   <222> (35)..(35)
   <223> n is a, c, g, or t
<400> 131
   gagttattat tatctcatag cgtatgtttt cttgnacagc cttgaataat tttgattgac 60
   ctctgggatg ttattaaagc ctaacgttcc ttctattctc acaaattttc gttatgactt 120
   cagaaggatc attaactctg gtatctgttt gtttgcttgt atggcaccaa taagcagatt 180
   tcttctttct aatctatgga ttagtataga ccaggagaag gctaatacag agactatgaa 240
   acgggaataa gtttttttaa cgatatggca aaattgtgac tctgaaagat cattcatgta 300
   tattctaaaa ttaccacagt cataaaaagt cttggacttt catgaggaaa tagcatagct 360
   agatatgaaa aaatatagaa aatcttcatc aatggaacta ttcttggggt agacactaat 420
   catatgaaaa gacaaatgct cattccctaa gatagcctga 460
<210> 132
   <211> 471
   <212> DNA
   <213> human
<400> 132
   gcttctacgt catcttcgac agagcccaga agagggtggg cttcgcagcg agcccctgtg      60
   cagaaattgc aggtgctgca gtgtctgaaa tttccgggcc tttctcaaca gaggatgtag     120
   ccagcaactg tgtccccgct cagtctttga gcgagcccat tttgtggatt gtgtcctatg     180
   cgctcatgag cgtctgtgga gccatcctcc ttgtcttaat cgtcctgctg ctgctgccgt     240
   tccggtgtca gcgtcgcccc cgtgaccctg aggtcgtcaa tgatgagtcc tctctggtca     300
   gacatcgctg gaaatgaata gccaggcctg acctcaagca accatgaact cagctattaa     360
   gaaaatcaca tttccagggc agcagccggg atcgatggtg gcgctttctc ctgtgcccac     420
   ccgtcttcaa tctctgttct gctcccagat gccttctaga ttcactgtct t              471
<210> 133
   <211> 487
   <212> DNA
   <213> human
<400> 133
   gaagtgggac gagcacattt ctattgtctt cacttggatc aaaagcaaaa cagtctctcc      60
   gccccgcacc agatcaagta gtttggacat caccctactg aaaacttgcg attcttctta     120
   gttttctgca tacttttcat cacgatgcag gaaacgattt cgagtcaaga agacttttat     180
   ttatgaacct ttgaaaggat cgtcttgtat ggtgaatttt ctaggagcga tgatgtactg     240
   taattttatt ttaatgtatt ttgatttatg attatttatt agtttttttt aaatgcttgt      300
   tctaagacat ttctgaatgt agaccatttt ccaaaaagga aactttattt tcaaaaacct     360
   aatccgtagt aattcctaat cttggagaat aaaaaagggc ggtggagggg aaaacattaa     420
   gaatttattc attatttctc gagtactttc agaaagtctg acactttcat tgttgtgcca     480
   gctggtt                                                               487
<210> 134
   <211> 521
   <212> DNA
   <213> human
<400> 134
   ggcacagagc gcggagatgt accactacca gcaccaacgg caacagatgc tgtgcctgga 60
   gcggcataaa gagccaccca aggagctgga cacggcctcc tcggatgagg agaatgagga 120
   cggagacttc acggtgtacg agtgcccggg cctggccccg accggggaaa tggaggtgcg 180
   caaccctctg ttcgaccacg ccgcactgtc cgcgcccctg ccggccccca gctcaccgcc 240
   tgcactgcca tgacctggag gcagacagac gcccacctgc tccccgacct cgaggccccc 300
   ggggaggggc agggcctgga gcttcccact aaaaacatgt tttgatgctg tgtgcttttg 360
   gctgggcctc gggctccagg ccctgggacc ccttgccagg gagacccccg aacctttgtg 420
   ccaggacacc tcctggtccc ctgcacctct cctgttcggt ttagaccccc aaactggagg 480
   gggcatggag aaccgtagag cgcaggaacg ggtgggtaat t 521
<210> 135
   <211> 399
   <212> DNA
   <213> human
<400> 135
   gccacacctt cgcgaaacct gtggtggccc accagtccta acgggacagg acagagagac 60
   agagcagccc tgcactgttt tccctccacc acagccatcc tgtccctcat tggctctgtg 120
   ctttccacta tacacagtca ccgtcccaat gagaaacaag aaggagcacc ctccacatgg 180
   actcccacct gcaagtggac agcgacattc agtcctgcac tgctcacctg ggtttactga 240
   tgactcctgg ctgccccacc atcctctctg atctgtgaga aacagctaag ctgctgtgac 300
   ttccctttag gacaatgttg tgtaaatctt tgaaggacac accgaagacc tttatactgt 360
   gatcttttac ccctttcact cttggctttc ttatgttgc 399
<210> 136
   <211> 370
   <212> DNA
   <213> human
<400> 136
   tggtgtatgc tgtgctttcc tcagcagtat ggctctgaca tctcttagat gtcccaactt 60
   cagctgttgg gagatggtga tattttcaac cctacttcct aaacatctgt ctggggttcc 120
   tttagtcttg aatgtcttat gctcaattat ttggtgttga gcctctcttc cacaagagct 180
   cctccatgtt tggatagcag ttgaagaggt tgtgtgggtg ggctgttggg agtgaggatg 240
   gagtgttcag tgcccatttc tcattttaca ttttaaagtc gttcctccaa catagtgtgt 300
   attggtctga agggggtggt gggatgccaa agcctgctca agttatggac attgtggcca 360
   ccatgtggct 370
<210> 137
   <211> 489
   <212> DNA
   <213> human
<400> 137
   gaatttctgc tggactttat ctgggcagag gaaggatgga atgaaggtag aaaaggcaga 60
   attacagctg agcggggaca acaaagagtt cttctctggg aaaagttttg tcttagagca 120
   aggatggaaa atggggacaa caaaggaaaa gcaaagtgtg acccttgggt ttggacagcc 180
   cagaggccca gctccccagt ataagccata caggccaggg acccacagga gagtggatta 240
   gagcacaagt ctggcctcac tgagtggaca agagctgatg ggcctcatca gggtgacatt 300
   caccccaggg cagcctgacc actcttggcc cctcaggcat tatcccattt ggaatgtgaa 360
   tgtggtggca aagtgggcag aggaccccac ctgggaacct ttttccctca gttagtgggg 420
   agactagcac ctaggtaccc acatgggtat ttatatctga accagacaga cgcttgaatc 480
   aggcactat 489
<210> 138
   <211> 532
   <212> DNA
   <213> human
<400> 138
   gagacagact tggcaaggga ccccctggtt ctgagccagt agctgccatc tggaaattcc 60
   tcttttagcc tctccttaga ggtgaatgtg aatgaagcct cccaggcacc cgctgaattt 120
   ctgaggcctt gcttaaagct cagaagtggt ttaggcattt ggaaaatctg gttcacatca 180
   taaagaactt gatttgaaat gttttctata gaaacaagtg ctaagtgtac cgtattatac 240
   ttgatgttgg tcatttctca gtcctatttc tcagttctat tattttagaa cctagtcagt 300
   tctttaagat tataactggt cctacattaa aataatgctt ctcgatgtca gattttacct 360
   gtttgctgct gagaacatct ctgcctaatt taccaaagcc agaccttcag ttcaacatgc 420
   ttccttagct tttcatagtt gtctgacatt tccatgaaaa caaaggaacc aactttgttt 480
   taaccaaact ttgtttggtt acagttttca ggggagcgtt tcttccatga ca 532
<210> 139
   <211> 458
   <212> DNA
   <213> human
<400> 139
   catgtacgac tcggacagcg acggccgcat cactctggaa gaatatcgaa atgtaaagtg     60
   gtcgaggagc tgctgtcggg aaaccctcac atctagaagg agtccgctcg ctccatcgcc    120
   gacggggcca tgatggaggc ggccagcgtg tgcatggggc agatggagcc tgatcaggtg    180
   tacgagggga tcaccttcga ggacttcctg aagatctggc aggggatcga cattgagacc    240
   aagatgcacg tccgcttcct taacatggaa accatggccc tctgccactg acccaccgcc    300
   acctccgcgg agagactgca ctttgcaatg ggccgcctcc cccgcgtagc tggagcagcc    360
   caggcccggc ggacagcctc ttcctgcagc gccggtacat agccaaggct cgtctgcgca    420
   ccttgtgtct tgtagggtat ggtatgtggg acttcgct                            458
<210> 140
   <211> 491
   <212> DNA
   <213> human
<400> 140
   gaggaggaac tgacgcagct acgccacgaa ctggagcggc agaacaatga ataccaagtg     60
   ctgctgggca tcaaaaccca cctggagaag gaaatcacca cgtaccgacg gctcctggag    120
   ggagagagtg aagggacacg ggaagaatca aagtcgagca tgaaagtgtc tgcaactcca    180
   aagatcaagg ccataaccca ggagaccatc aacggaagat tagttctttg tcaagtgaat    240
   gaaatccaaa agcacgcatg agaccaatga aagtttccgc ctgttgtaaa gtctattttc    300
   ccccaaggaa agtccttgca cagacaccag tgagtgagtt ctaaaagata cccttggaat    360
   tatcagactc agaaactttt attttttttt ttctgtaaca gtctcaccag acttctcata    420
   atgctcttaa tatattgcac ttttctaatc aaagtgcgag tttatgaggg taaagctcta    480
   ctttcctact g                                                         491
<210> 141
   <211> 562
   <212> DNA
   <213> human
<400> 141
   aatcctgcaa ttctcaatct tgcactgcag cctcgacctc ccaggctcca gtgactctcc     60
   cacctcagcc tcctaagtag ctgggagtac aggcgcgcac caccacgcct agctgatttt 120
   tgtatttttt tgtagagacg ggggtttggc catgttgccg aggctaactc ctgggattac 180
   aggcatgagc tgtgctggcc gggttttttt ttcttgatgt aaacgtgtac agctgtttta 240
   ttagttaagg tctaattttt actctaggtg ccttttatgt tcagaactct ttccactgga 300
   ctggtatttg ctcaaaaata aataatggta gagaagaaaa ctataaaaat ggacaaggct 360
   ttcttctatc agtagcgttt accctttgtc accagtggct ttggtatttc catgtctggc 420
   attgcataaa cttctctggt gtgaaaggat aaatatgcct ttctaaagtt gtatatcaaa 480
   attgtatcaa tttttatttt ctatgatttc tagaaacaaa tgtaataaat atttttaaaa 540
   tctcctttct actggttatg ta 562
<210> 142
   <211> 414
   <212> DNA
   <213> human
<400> 142
   ctcgttgcaa tcgcctttgc agtcaaccac ttctggtgcc aggaggagcc ggagcctgca 60
   cacatgatcc tgaccgtcgg aaacaaggca gatggagtcc tggtgggaac agatggaagg 120
   tactcttcga tggcggccag tttcaggtcc agtgagcatg agaatgccta tgagaatgtg 180
   cccgaggagg aaggcaaggt ccgcagcacc ccgatgtaac cttctctgtg gctccaaccc 240
   caagactccc aggcacatgg gatggatgtc cagtgctacc acccaagccc cctccttctt 300
   tgtgtggaat ctgcaatagt gggctgactc cctccagccc catgccggcc ctacccgccc 360
   ttgaagtata gccagccaag gttggagctc agaccgtgtc taggttgggg ctcg 414
<210> 143
   <211> 561
   <212> DNA
   <213> human
<400> 143
   gagagaaaac aatatagccc cctacccttt tcccaatcct ttgccctcaa atcagtgacc 60
   caagggaggg ggggatttaa agggaaggag tgggcaaaac acataaaatg aatttattat 120
   atctaagctc tgtagcagga ttcatgtcgt tctttgacag ttctttctct ttcctgtata 180
   tgcaataaca aggttttaaa aaaaaaaaaa aaaagtgaga ctattagaca aagtatttat 240
   gtaattattt gataactctt gtaaataggt ggaatatgaa tgcttggaaa attaaacttt 300
   aatttattga cattgtacat agctctgtgt aaatagaatt gcaactgtca ggttttgtgt 360
   tcttgttttc ctttagttgg gtttatttcc aggtcacaga attgctgtta acactagaaa 420
   acacacttcc tgcaccaaca ccaataccct ttcaaaagag ttgtctgcaa catttttgtt 480
   ttctttttta atgtccaaaa gtgggggaaa gtgctatttc ctattttcac caaaattggg 540
   gaaggagtgc cactttccag c 561
<210> 144
   <211> 409
   <212> DNA
   <213> human
<400> 144
   ttagcactga aagtctcttg ccccaggaaa ccccatcagt cccaggcaga ttgggacagc 60
   tggtcacctt acgcaagagc caggctgaaa catcccctcc atactcagct ctttaacttt 120
   tcttttcctt tttcatcggg ctctttccta aaaagctgag ctgtaaaata ttttacatcg 180
   aggtataata aataatcatg tacatgtttt accaccaccc aggtcaagac atagaatgtt 240
   tcaacatttc catcacccca gaaactcccc ttgtaccccc ttccacttcg tctcccctag 300
   ctcctagaag caaccactga tgtgatttct accaaatcca gttttggtcc tactaaatat 360
   actcttttga gactggcctc ttttactcac cataatgcct ttgtaattc 409
<210> 145
   <211> 491
   <212> DNA
   <213> human
<400> 145
   tagctgtttc agagagagta cggtatattt atggtaattt tatccactag caaatcttga 60
   tttagtttga tagtgtgtgg aattttattt tgaaggataa gaccatggga aaattgtggt 120
   aaagactgtt tgtacccttc atgaaataat tctgaagttg ccatcagttt tactaatctt 180
   ctgtgaaatg catagatatg cgcatgttca actttttatt gtggtcttat aattaaatgt 240
   aaaattgaaa attcatttgc tgtttcaaag tgtgatatct ttcacaatag cctttttata 300
   gtcagtaatt cagaataatc aagttcatat ggataaatgc atttttattt cctatttctt 360
   tagggagtgc tacaaatgtt tgtcacttaa atttcaagtt tctgttttaa tagttaactg 420
   actatagatt gttttctatg ccatgtatgt gccacttctg agagtagtaa atgactcttt 480
   gctacatttt a 491
<210> 146
   <211> 562
   <212> DNA
   <213> human
<400> 146
   gtgtaccaga attcggccat gagccgctac atactcatca tggggctcct gtacaaggtg 60
   ctgggcgtcc tcttctttgc catagcctgc ttcttataca agcccctgtc ggagtcttca 120
   gatggcctgg aaacttgtct gcccagccag tcctcagccc ctgacagtgc cacagatagc 180
   cagctccaga gcagcgtctg accaccgccc gcgcccaccc ggccacggcg ggcactcagc 240
   atttcctgat gacagaacag tgccgttggg tgatgcaatc acacgggaac ttctatttga 300
   cctgcaacct tctacttaac ctgtggttta aagtcggctg tgacctcctg tccccagagc 360
   tgtacggccc tgcagtgggt gggaggaact tgcataaata tatatttatg gacacacagt 420
   ttgcatcaga acgtgtttat agaatgtgtt ttatacccga tcgtgtgtgg tgtgcgtgag 480
   gacaaactcc gcaggggctg tgaatcccac tgggagggcg gtgggcctgc agcccgagga 540
   aggcttgtgt gtcctcagtt aa 562
<210> 147
   <211> 524
   <212> DNA
   <213> human
<400> 147
   gaagttgcaa cattcgtttg ataggaattc cagaaaagga gagttatgag aatagggcag 60
   aggacataat taaagaaata attgatgaaa actttgcaga actaaagaaa ggttcaagtc 120
   ttgagattgt cagtgcttgt cgagtaccta gtaaaattga tgaaaagaga ctgactccta 180
   gacacatctt ggtgaaattt tggaattcta gtgataaaga gaaaataata agggcttcta 240
   gagagagaag agaaattacc taccaaggaa caagaatcag gttgacagca gacttatcac 300
   tggacacact ggatgctaga agtaaatgga gcaatgtctt caaagttctg ctggaaaaag 360
   gctttaatcc tagaatccta tatccagcca aaatggcatt tgattttagg ggcaaaacaa 420
   aggtatttct tagtattgaa gaatttagag attatgtttt gcatatgccc accttgagag 480
   aattactggg gaataatata ccttagcacg ccagggtgac taca 524
<210> 148
   <211> 194
   <212> DNA
   <213> human
<400> 148
   ttgcttgttc cccggaggtt gaagctacag tgagccttga ttgtgtcact gcactccagc 60
   ctgggcaaca ggtaagactc tgtctcaaaa aaaaaacaaa aaagaagaag aaaagtactt 120
   ctacagccat gtcctattcc ttgatcatcc aaagcacctg cagagtccag tgaaatgata 180
   tattctggct gggc 194
<210> 149
   <211> 536
   <212> DNA
   <213> human
<400> 149
   gacggcgtca aggtcgtggg acgtgacacg accgctgcgg cgtcagctca gccttgcaag 60
   accccaggcg cccgcgctgc acctgcgact gtcgccgccg ccgtcgcagt cggaccaact 120
   gctggcagaa tcttcgtccg cacggcccca gctggagttg cacttgcggc cgcaagccgc 180
   cagggggcgc cgcagagcgc gtgcgcgcaa cggggaccac tgtccgctcg ggcccgggcg 240
   ttgctgccgt ctgcacacgg tccgcgcgtc gctggaagac ctgggctggg ccgattgggt 300
   gctgtcgcca cgggaggtgc aagtgaccat gtgcatcggc gcgtgcccga gccagttccg 360
   ggcggcaaac atgcacgcgc agatcaagac gagcctgcac cgcctgaagc ccgacacggt 420
   gccagcgccc tgctgcgtgc ccgccagcta caatcccatg gtgctcattc aaaagaccga 480
   caccggggtg tcgctccaga cctatgatga cttgttagcc aaagactgcc actgca 536
<210> 150
   <211> 460
   <212> DNA
   <213> human
<400> 150
   tggaattaga ccatttggcc tttgaacttt cataggaaaa atgacccaac atttcttagc 60
   atgagctacc tcatctctag aagctgggat ggacttacta ttcttgttta tattttagat 120
   actgaaaggt gctatgcttc tgttattatt ccaagactgg agataggcag ggctaaaaag 180
   gtattattat ttttccttta atgatggtgc taaaattctt cctataaaat tccttaaaaa 240
   taaagatggt ttaatcacta ccattgtgaa aacataactg ttagacttcc cgtttctgaa 300
   agaaagagca tcgttccaat gcttgttcac tgttcctctg tcatactgta tctggaatgc 360
   tttgtaatac ttgcatgctt cttagaccag aacatgtagg tccccttgtg tctcaatact 420
   ttttttttct taattgcatt tgttggctct attttaattt 460
<210> 151
   <211> 446
   <212> DNA
   <213> human
<400> 151
   tagattccgg tatatcgttc ttcaagacac ttgctctaag cggaatggaa atgtgggcaa 60
   gactgtcttt gaatatagaa cacagaatgt ggcacgcttg cccatcatag atcttgctcc 120
   tgtggatgtt ggcggcacag accaggaatt cggcgttgaa attgggccag tttgttttgt 180
   gtaaagtaag ccaagacaca tcgacaatga gcaccaccat caatgaccac cgccattcac 240
   aagaactttg actgtttgaa gttgatcctg agactcttga agtaatggct gatcctgcat 300
   cagcattgta tatatggtct taagtgcctg gcctccttat ccttcagaat atttatttta 360
   cttacaatcc tcaagtttta attgatttta aatatttttc aatacaacag tttaggttta 420
   agatgaccaa tgacaatgac cacctt 446
<210> 152
   <211> 552
   <212> DNA
   <213> human
<220>
   <221> misc_feature
   <222> (63)..(63)
   <223> n is a, c, g, or t
<220>
   <221> misc_feature
   <222> (72)..(72)
   <223> n is a, c, g, or t
<220>
   <221> misc_feature
   <222> (74)..(74)
   <223> n is a, c, g, or t
<220>
   <221> misc_feature
   <222> (85)..(85)
   <223> n is a, c, g, or t
<220>
   <221> misc_feature
   <222> (138)..(138)
   <223> n is a, c, g, or t
<400> 152
   tttcagcacc gatggccatg taaataagat gatttaatgt tgattttaat cctgtatata 60
   aantaaaaag tncncaatga gtttngggca tatttaatga tgattatgga gccttagagg 120
   tctttaatca ttggttcngg ctgcttttat gtagtttagg ctggaaatgg tttcacttgc 180
   tctttgactg tcagcaagac tgaagatggc ttttcctgga cagctagaaa acacaaaatc 240
   ttgtaggtca ttgcacctat ctcagccata ggtgcagttt gcttctacat gatgctaaag 300
   gctgcgaatg ggatcctgat ggaactaagg actccaatgt cgaactcttc tttgctgcat 360
   tcctttttct tcacttacaa gaaaggcctg aatggaggac ttttctgtaa ccaggaacat 420
   tttttagggg tcaaagtgct aataattaac tcaaccaggt ctacttttta atggctttca 480
   taacactaac tcataaggtt accgatcaat gcatttcata cggatataga cctagggctc 540
   tggagggtgg gg 552
<210> 153
   <211> 253
   <212> DNA
   <213> human
<400> 153
   gtaaatagtt aaccttcagt agtctattaa ggcattaata cttctctgga catgcgcgtt 60
   tgagggtgga ggggtcctgt aaggtgcttc atcgtctgtg attactgctt gggatgtgtt 120
   ctttggcagc ttgtgagatt actttaccta gtgtttataa agtaggaagt taagtgaatc 180
   atagattaga atttaatact cttatggaaa taatttttta acatcttaat tgacaatggc 240
   gtttttttat aca 253
<210> 154
   <211> 239
   <212> DNA
   <213> human
<400> 154
   tagtccatac tgagtgtcat caacaatcca gactgaagtc ttctatttta atctcaatcc 60
   ccttttctga tttgccaccc atgcctcttc aggctggaaa caatctcttg gttccctaaa 120
   gcactttctt ctgactgctg tgattcagtg aaccttgccc tttgctttct attacttgtg 180
   catttgcctc acctgacaat gttttaaatc gcctttgtat ctccttagct gctcaataa 239
<210> 155
   <211> 313
   <212> DNA
   <213> human
<400> 155
   aatatgtcag tgcttgcttg atggaaactt ctcttgtgtc tgttgagact ttaagggaga 60
   aatgtcggaa tttcagagtc gcctgacggc agagggtgag cccccgtgga gtctgcagag 120
   aggccttggc caggagcggc gggctttccc gaggggccac tgtccctgca gagtggatgc 180
   ttctgcctag tgacaggtta tcaccacgtt atatattccc taccgaagga gacacctttt 240
   cccccctgac ccagaacagc ctttaaatca caagcaaaat aggaaagtta accacggagg 300
   caccgagttc cag 313
<210> 156
   <211> 436
   <212> DNA
   <213> human
<400> 156
   ggctggggga gagccgggtt cattccctgt cctcattggt cgtccctatg aattgtacgt 60
   ttcagagaaa ttttttttcc tatgtgcaac acgaagcttc cagaaccata aaatatcccg 120
   tcgataagga aagaaaatgt cgttgttgtt gtttttctgg aaactgcttg aaatcttgct 180
   gtactataga gctcagaagg acacagcccg tcctcccctg cctgcctgat tccatggctg 240
   ttgtgctgat tccaatgctt tcacgttggt tcctggcgtg ggaactgctc tcctttgcag 300
   ccccatttcc caagctctgt tcaagttaaa cttatgtaag ctttccgtgg catgcggggc 360
   gcgcacccac gtccccgctg cgtaagactc tgtatttgga tgccaatcca caggcctgaa 420
   gaaactgctt gttgtg 436
<210> 157
   <211> 493
   <212> DNA
   <213> human
<400> 157
   gtgagtatgg cccaatgctt tctgtggcta aacagatgta atgggaagaa ataaaagcct 60
   acgtgttggt aaatccaaca gcaagggaga tttttgaatc ataataactc ataaggtgct 120
   atctgttcag tgatgccctc agagctcttg ctgttagctg gcagctgacg ctgctaggat 180
   agttagtttg gaaatggtac ttcataataa actacacaag gaaagtcagc caccgtgtct 240
   tatgaggaat tggacctaat aaattttagt gtgccttcca aacctgagaa tatatgcttt 300
   tggaagttaa aatttaaatg gcttttgcca catacataga tcttcatgat gtgtgagtgt 360
   aattccatgt ggatatcagt taccaaacat tacaaaaaaa ttttatggcc caaaatgacc 420
   aacgaaattg ttacaataga atttatccaa ttttgatctt tttatattct tctaccacac 480
   ctggaaacag acc 493
<210> 158
   <211> 457
   <212> DNA
   <213> human
<220>
   <221> misc_feature
   <222> (87)..(87)
   <223> n is a, c, g, or t
<400> 158
   catggtttac atttactcag ctactatata tgcagtgtgg tgcacatttt cacagaattc 60
   tggcttcatt aagatcatta tttttgnctg cgtagcttac agacttagca tattagtttt 120
   ttctactcct acaagtgtaa attgaaaaat ctttatatta aaaaagtaaa ctgttatgaa 180
   gctgctatgt actaataata ctttgcttgc caaagtgttt gggttttgtt gttgtttgtt 240
   tgtttgtttg tttttggttc atgaacaaca gtgtctagaa acccattttg aaagtggaaa 300
   attattaagt cacctatcac ctttaaacgc ctttttttaa aattataaaa tattgtaaag 360
   cagggtctca acttttaaat acactttgaa cttcttctct gaattattaa agttctttat 420
   gacctcattt ataaacacta aattctgtca cctcctg 457
<210> 159
   <211> 426
   <212> DNA
   <213> human
<220>
   <221> misc_feature
   <222> (170)..(170)
   <223> n is a, c, g, or t
<400> 159
   gccgctgtgc tttcgtggaa atgacagttc cttgtttttt ttgtttctgt ttttgtttta 60
   cattagtcat tggaccacag ccattcagga actaccccct gccccacaaa gaaatgaaca 120
   gttgtaggga gacccagcag cacctttcct ccacacacct tcattttgan gttcgggttt 180
   ttgtgttaag ttaatctgta cattctgttt gccattgtta cttgtactat acatctgtat 240
   atagtgtacg gcaaaagagt attaatccac tatctctagt gcttgacttt aaatcagtac 300
   agtacctgta cctgcacggt cacccgctcc gtgtgtcgcc ctatattgag ggctcaagct 360
   ttcccttgtt ttttgaaagg ggtttatgta taaatatatt ttatgccttt ttattacaag 420
   tcttgt 426
<210> 160
   <211> 502
   <212> DNA
   <213> human
<400> 160
   ggagtggttt aagagtgcca ggcgaagggc aaactgtaga tcgatcttta tgctgttatt 60
   acaggagaag tgacatactt tatatatgtt tatattagca aggtctgttt ttaataccat 120
   atactttata tttctataca tttatatttc taataataca gttatcactg atatatgtag 180
   acacttttag aatttattaa atccttgacc ttgtgcatta tagcattcca ttagcaagag 240
   ttgtaccccc tccccagtct tcgccttcct ctttttaagc tgttttatga aaaagaccta 300
   gaagttcttg attcattttt accattcttt ccataggtag aagagaaagt tgattggttg 360
   gttgtttttc aattatgcca ttaaactaaa catttctgtt aaattaccct atcctttgtt 420
   ctctactgtt ttctttgtaa tgtatgacta cgagagtgat actttgctga aaagtctttc 480
   ccctattgtt tatctattgt ca 502
<210> 161
   <211> 482
   <212> DNA
   <213> human
<400> 161
   ttcctgtgca agtaccgacc atagagcaag aatcaagatt ctgctaactc ctgcacagcc 60
   ccgtcctctt cctttctgct agcctggcta aatctgctca ttatttcaga ggggaaacct 120
   agcaaactaa gagtgataag ggccctacta cactggcttt tttaggctta gagacagaaa 180
   ctttagcatt ggcccagtag tggcttctag ctctaaatgt ttgccccgcc atccctttcc 240
   acagtatcct tcttccctcc tcccctgtct ctggctgtct cgagcagtct agaagagtgc 300
   atctccagcc tatgaaacag ctgggtcttt ggccataaga agtaaagatt tgaagacaga 360
   aggaagaaac tcaggagtaa gcttctagac cccttcagct tctacaccct tctgccctct 420
   ctccattgcc tgcaccccac cccagccact caactcctgc ttgtttttcc tttggccata 480
   gg 482
<210> 162
   <211> 490
   <212> DNA
   <213> human
<400> 162
   ggagctcaga gatctaagct gctttccatc ttttctccca gccccaggac actgactctg     60
   tacaggatgg ggccgtcctc ttgcctcctt ctcatcctaa tcccccttct ccagctgatc     120
   aacccgggga gtactcagtg ttccttagac tccgttatgg ataagaagat caaggatgtt    180
   ctcaacagtc tagagtacag tccctctcct ataagcaaga agctctcgtg tgctagtgtc    240
   aaaagccaag gcagaccgtc ctcctgccct gctgggatgg ctgtcactgg ctgtgcttgt    300
   ggctatggct gtggttcgtg ggatgttcag ctggaaacca cctgccactg ccagtgcagt    360
   gtggtggact ggaccactgc ccgctgctgc cacctgacct gacagggagg aggctgagaa    420
   ctcagttttg tgaccatgac agtaatgaaa ccagggtccc aaccaagaaa tctaactcaa    480
   acgtcccact                                                           490
<210> 163
   <211> 362
   <212> DNA
   <213> human
<400> 163
   gcaaagttta tttcagttca catgtaaggt attgcaaata aattcttgga caattttgta     60
   tggaaacttg atattaaaaa ctagtctgtg gttctttgca gtttcttgta aatttataaa    120
   ccaggcacaa ggttcaagtt tagattttaa gcacttttat aacaatgata agtgcctttt    180
   tggagatgta acttttagca gtttgttaac ctgacatctc tgccagtcta gtttctgggc    240
   aggtttcctg tgtcagtatt ccccctcctc tttgcattaa tcaaggtatt tggtagaggt    300
   ggaatctaag tgtttgtatg tccaatttac ttgcatatgt aaaccattgc tgtgccattc    360
   aa                                                                   362
<210> 164
   <211> 60
   <212> DNA
   <213> human
<400> 164
   agacggcctt gagtctcagt acgagtgtgc gtgagtgtga gccaccttgg caagtgcctg 60
<210> 165
   <211> 495
   <212> DNA
   <213> human
<220>
   <221> misc_feature
   <222> (44)..(44)
   <223> n is a, c, g, or t
<400> 165
   ggagtgtggt acttctccta gttgcagtca ggcttcatac gctnttgtcc tgcccgttag     60
   agcagccagc gggtacagaa tggattttgg aagagggagt caccactgga cctccaagga    120
   agccacgtgc agacatctac aaccttcgat ctcctgacga gtttattgtt ggccaaaacc    180
   aggctttgat tgaaccagga tgaatgcggg tgttggaagt agaatatata tatacatata    240
   aaattggttg ggagccacgt gtaccagtgt gtgttgatct tggcttgatt cagtctgcct    300
   tgtaacagaa actggcgatg gaatatgaga ggagccctct ggaaagaaaa ggacagaccc    360
   tgtgctttca tgaaagtgaa gatctggctg aaccagttcc acaaggttac tgtatacata    420
   gcctgagttt aaaaggctgt gcccacttca agaatgtcat tgttagactt tgaaatttct    480
   aactgcctac ctgca                                                     495
<210> 166
   <211> 527
   <212> DNA
   <213> human
<220>
   <221> misc_feature
   <222> (235)..(238)
   <223> n is a, c, g, or t
<220>
   <221> misc_feature
   <222> (271)..(274)
   <223> n is a, c, g, or t
<220>
   <221> misc_feature
   <222> (467)..(468)
   <223> n is a, c, g, or t
<400> 166
   agggctgtaa cagttgctgc tagtattagg gttccacatc attctaatgt atagtttcaa     60
   gtcttaatag acaatctgaa ttccactaca tttcttttgg ctccaacatt ccttttagct    120
   tgaccagtct aatttaaaat gtgtttgttg gaggtcatta acgttacttg tacaatgctg    180
   tcactgtgtg acatccatat gaattttggt atatatcaat caatcaatca atcannnnca    240
   ttgcattcaa tcaatcagct gtgattgatt nnnnatgctt agaaatacta tagtaactag 300
   atgcagtgtg aattttttcc attaacaaac aaacaagtca gtggcttaaa tgtgattatg 360
   gtcctgcaag gtgattcttg ctaaaatatc taaacttttg ttttgtttta actgaatcat 420
   tttttaactt aaaaagctgg aaaatatcaa atgctgtttt ttttttnnca ttgtcaacag 480
   tggtgtgtca ttttatgtat gttcctaatg cttatggaac tcctcca 527
<210> 167
   <211> 464
   <212> DNA
   <213> human
<220>
   <221> misc_feature
   <222> (402)..(402)
   <223> n is a, c, g, or t
<400> 167
   ggaacgatga gcaccatgcc aggactgccc acccggccct gcttttatga catagatctt 60
   gataccgaaa cagaacaagt taaaggcttg ttctaagtgg acaaggctct cacaggaccc 120
   gatgcagact cctgaaacag actactcttt gcctttttgc tgcagttgga gaagaaactg 180
   aatttgaaaa atgtctgtta tgcaatgctg gagacatggt gaaataggcc aaagatttct 240
   tcttcgttca agatgaattc tgttcacagt ggagtatggt gttcggcaaa aggacctcca 300
   ccaagactga aagaaactaa tttatttctg tttctgtgga gtttccatta tttctactgc 360
   ttacacttta gaatgtttat tttatgggga ctaagggatt angagtgtga actaaaaggt 420
   aacattttcc actctcaagt tttctacttt gtctttgaac tgaa 464
<210> 168
   <211> 456
   <212> DNA
   <213> human
<220>
   <221> misc_feature
   <222> (26)..(30)
   <223> n is a, c, g, or t
<220>
   <221> misc_feature
   <222> (33)..(33)
   <223> n is a, c, g, or t
<220>
   <221> misc_feature
   <222> (35)..(47)
   <223> n is a, c, g, or t
<220>
   <221> misc_feature
   <222> (50)..(50)
   <223> n is a, c, g, or t
<400> 168
   gaaacacaac caagacgcga ggatcnnnnn ctntnnnnnn nnnnnnnctn gcaagatggc 60
   gccgcagaaa gacaggaagc ccaagaggtc aacctggagg tttaatttgg accttactca 120
   tccagtagaa gatggaattt ttgattctgg aaattttgag caatttctac gggagaaggt 180
   taaagtcaat ggcaaaactg gaaatctcgg gaatgttgtt cacattgaac gcttcaagaa 240
   taaaatcaca gttgtttctg agaaacagtt ctctaaaagg tatttgaaat accttaccaa 300
   gaaatacctt aagaagaaca atcttcgtga ttggcttcga gtggttgcat ctgacaagga 360
   gacctacgaa cttcgttact tccagattag tcaagatgaa gatgaatcag agtcggagga 420
   ctaggcaaag gctcccctta cagggctttg cttatt 456
<210> 169
   <211> 351
   <212> DNA
   <213> human
<400> 169
   ggaacccaga gctgctgtgt atttcgagcg ggcagtttat cttttgctat acttattttc 60
   aattcaatta caccacgatt caaataattc ccctcctaaa accaaaaagg agggaaacgt 120
   caactccatt gcaattactt atcttcctct tctatctctg ttatacgccg gggcatagaa 180
   tgctcgtata catctcttta acaaccacaa accttaagcc atgtagatga agttagtgca 240
   tcaacgggat acagttccat attgccttaa acctccttgt tttagacaca ctaacattta 300
   taccaaattg cagattattc tgcagagagg gaattgcatg tttgtgttgt a 351
<210> 170
   <211> 506
   <212> DNA
   <213> human
<400> 170
   aattaatatt catcgcactt cttctgtgga aggactttgt gaaggaattg gtgctggatt 60
   agtggatgtt gctatctggg ttggcacttg ttcagattac ccaaaaggag atgcttctac 120
   tggatggaat tcagtttctc gcatcattat tgaagaacta ccaaaataaa tgctttaatt    180
   ttcatttgct acctcttttt ttattatgcc ttggaatggt tcacttaaat gacattttaa    240
   ataagtttat gtatacatct gaatgaaaag caaagctaaa tatgtttaca gaccaaagtg    300
   tgatttcaca ctgtttttaa atctagcatt attcattttg cttcaatcaa aagtggtttc    360
   aatatttttt ttagttggtt agaatacttt cttcatagtc acattctctc aacctataat    420
   ttggaatatt gttgtggtct tttgtttttt ctcttagtat agcattttta aaaaaatata    480
   aaagctacca atctttgtac aatttg                                          506
<210> 171
   <211> 384
   <212> DNA
   <213> human
<220>
   <221> misc_feature
   <222> (192)..(192)
   <223> n is a, c, g, or t
<400> 171
   ccgtgtgagc gatcgcggtg ggttcgggcc ggtgtgacgc gtgcgccggc cggccgccga     60
   ggggctgccg ttctgcctcc gaccggtcgt gtgtgggttg acttcggagg cgctctgcct    120
   cggaaggaag gaggtgggtg gacggggggg cctggtgggg ttgcgcgcac gcgcgcaccg    180
   gccgggcccc cngccctgaa cgcgaacgct cgaggtggcc gcgcgcaggt gtttcctcgt    240
   accgcagggc cccctccctt ccccaggcgt ccctcggcgc ctctgcgggc ccgaggagga    300
   gcggctggcg ggtgggggga gtgtgaccca ccctcggtga gaaaagcctt ctctagcgat    360
   ctgagaggcg tgccttgggg gtac                                            384
<210> 172
   <211> 360
   <212> DNA
   <213> human
<220>
   <221> misc_feature
   <222> (128)..(128)
   <223> n is a, c, g, or t
<220>
   <221> misc_feature
   <222> (190)..(190)
   <223> n is a, c, g, or t
<220>
   <221> misc_feature
   <222> (206)..(206)
   <223> n is a, c, g, or t
<400> 172
   aaatgactgg atggtcgctg ctttttaagt ttcaaattga cattccagac aagcggtgcc     60
   tgagcccgtg cctgtcttca gatcttcaca gcacagttcc tgggaaggtg gagccaccag    120
   cctctccntg aataactggg agatgaaaca ggaagctcta tgacacactt gatcgaatat    180
   gacagacacn gaaaatcacg actcancccc ctccagcacc tctacctgtt gcccgccgat    240
   cacagccgga atgcagctga aagattccct ggggcctggt tccaaccgcc cactgtggac    300
   tctgaggcct ctgcatttgc gggtggtctg cctgtgatat tttggtcatg ggctggtctg    360
<210> 173
   <211> 437
   <212> DNA
   <213> human
<220>
   <221> misc_feature
   <222> (97)..(97)
   <223> n is a, c, g, or t
<220>
   <221> misc_feature
   <222> (357)..(357)
   <223> n is a, c, g, or t
<400> 173
   tcttctcagg tcacttgtac acttggtttc ctagtagaag ctcacttgcc acctctcagg     60
   ggggtcccgg attgcatcca tcacaatccc aaaactngag ttggggggaa ctggagggag    120
   caaaacactg atttgatact agtcagtttg cttgaaacta gttcacctaa agctagatct    180
   cttaaaacca atttactgaa aacttgtttg cttaaagtta atgacttaat gactaatttg    240
   ccaaaagctc aattcctatt ttggtgtgtt tatatccatt taggtgtcct attctttttt    300
   gtcatgcttt ggatatttca aggatttata tctattcatc caagagtact tctgagntat    360
   tatcagcaac ataaatttat caaatttgca gcactttgta aatgatgaga ttgcttccta    420
   cctttatgga tgtcttt                                                   437
<210> 174
   <211> 539
   <212> DNA
   <213> human
<220>
   <221> misc_feature
   <222> (147)..(147)
   <223> n is a, c, g, or t
<400> 174
   aatgcattac tttcacttaa cactagacac caggtcgaaa attttcaagg ttatagtact 60
   tatttcaaca attcttagag atgctagcta gtgttgaagc taaaaatagc tttatttatg 120
   ctgaattgtg atttttttat gccaaanttt ttttagttct aatcattgat gatagcttgg 180
   aaataaataa ttatgccatg gcatttgaca gttcattatt cctataagaa ttaaattgag 240
   tttagagaga atggtggtgt tgagctgatt attaacagtt actgaaatca aatatttatt 300
   tgttacatta ttccatttgt attttaggtt tccttttaca ttctttttat atgcattctg 360
   acattacata ttttttaaga ctatggaaat aatttaaaga tttaagctct ggtggatgat 420
   tatctgctaa gtaagtctga aaatgtaata ttttgataat actgtaatat acctgtcaca 480
   caaatgcttt tctaatgttt taaccttgag tattgcagtt gctgctttgt acagaggtt 539
<210> 175
   <211> 487
   <212> DNA
   <213> human
<400> 175
   gtacttctcc tagttgcagt caggcttcat acgctattgt cctgcccgtt agagcagcca 60
   gcgggtacag aatggatttt ggaagaggga gtcaccactg gacctccaag gaagccacgt 120
   gcagacatct acaaccttcg atctcctgac gagtttattg ttggccaaaa ccaggctttg 180
   attgaaccag gatgaatgcg ggtgttggaa gtagaatata tatatacata taaaattggt 240
   tgggagccac gtgtaccagt gtgtgttgat cttggcttga ttcagtctgc cttgtaacag 300
   aaactggcga tggaatatga gaggagccct ctggaaagaa aaggacagac cctgtgcttt 360
   catgaaagtg aagatctggc tgaaccagtt ccacaaggtt actgtataca tagcctgagt 420
   ttaaaaggct gtgcccactt caagaatgtc attgttagac tttgaaattt ctaactgcct 480
   acctgca 487
<210> 176
   <211> 427
   <212> DNA
   <213> human
<400> 176
   gaagaggagc aacatctatg ccaaatactg tgcattctac aatggtgcta atctcagacc 60
   taaatgatac tccatttaat ttaaaaaaga gttttaaata attatctatg tgcctgtatt 120
   tcccttttga gtgctgcaca acatgttaac atattagtgt aaaagcagat gaaacaacca 180
   cgtgttctaa agtctaggga ttgtgctata atccctattt agttcaaaat taaccagaat 240
   tcttccatgt gaaatggacc aaactcatat tattgttatg taaatacaga gttttaatgc 300
   agtatgacat cccacagggg aaaagaatgt ctgtagtggg tgactgttat caaatatttt 360
   atagaataca atgaacggtg aacagactgg taacttgttt gagttcccat gacagatttg 420
   agacttg 427
<210> 177
   <211> 485
   <212> DNA
   <213> human
<400> 177
   aaacgacgca aatctctgag ctggggacca cttggagaac cggcttagta acagtcctga 60
   tcttcgcaag ccagcttctt ctgcatctga ggggctcctg gcgcccagag gaggcagaca 120
   gatgtcttct agctgagttt ctaaccgcat gatgagactc agaccttccg ctgcactaga 180
   aaatctgcaa cagtgtccct gagtcacttc tccttagtgg gcagactcgt gttagatttg 240
   tggaacccag ctctctgatt tactcctttt ggaaaaccca tggaatttca tgtataaggc 300
   tttcatttgt attttaaggt ttttctgttt gttttgagta tatacatggt gctcaatagc 360
   aacatcttag cagatgaagc agtttatgat tccactccct cctgtatgac aggtagccac 420
   tatactgaat caaggtgctg aactcaaatc acaaaattct ggcttaccga tacaacaacc 480
   aatac 485
<210> 178
   <211> 564
   <212> DNA
   <213> human
<220>
   <221> misc_feature
   <222> (172)..(172)
   <223> n is a, c, g, or t
<220>
   <221> misc_feature
   <222> (338)..(338)
   <223> n is a, c, g, or t
<400> 178
   gtcccactgc tcacatactt atgtgctgct agtctctact cgaagttcgt gcaggactaa 60
   tgcttttaaa atgaggtcta aaaaataatt actagtcgag actattattc tttaaacaga 120
   actgcctttt tctactcttt atgtaaactc tttctattgt gttggtctaa cnaggcacta 180
   ttttaaaatt ttttaatttt tcccatagca cttaaaagag attttgtaaa gaccttgctg 240
   taaagatttt gtaataaaat ggtctaaggg ctctttttcc aacattacca tttttaaaaa 300
   atgttttaaa agctagaaga caacttatgt atattctnta tatgtatagc agcacatttc 360
   atttatggaa atatgttctc agaatattta tttactaata tatttatctt aagccatgtc 420
   ttatgttgag agtgtgacat tgttggaata atcattgaaa atgactaaca caagaccctg 480
   taaatacatg ataattgcac acagatttta catatttgca gaccaaaaat gatttaaaac 540
   aagttgtagt cttctatggt tttg 564
<210> 179
   <211> 487
   <212> DNA
   <213> human
<220>
   <221> misc_feature
   <222> (296)..(296)
   <223> n is a, c, g, or t
<400> 179
   tataaggtaa ctctttagtc ctccatttag cacattttaa atcctccaaa gaataagtat 60
   catgtgatta ttttagcttt acaaaaaaaa agttgaatgg cgttttattt tcatggccta 120
   taagcaggta ccttagtagg gcagatatag gaaaaacaaa ttagagcaaa acaaatcctc 180
   tacaaatcca aggcaggaaa agtggtggca gagtgactca ttctcctgtc cctcccatca 240
   ggtcaaatca ggaggctgca gtgaatgcct gttctttgaa tgtgtagcag ttgttncctg 300
   taactcttta aaacttggct ataggctgtt tagcacagta cagattaaag atacagttac 360
   gtaaacagca aagtaatttt atagtgcttc atccatttat catgctttgg tttgctaatt 420
   ttttcacata cctttttcta tcacagtctg ttgcttttgt acacatttct catattgggg 480
   ttcgaca 487
<210> 180
   <211> 222
   <212> DNA
   <213> human
<400> 180
   ggagtgtggt acttctccta gttgcagtca ggcttcatac gctattgtcc tgcccgttag 60
   agcagccagc gggtacagaa tggattttgg aagagggagt caccactgga cctccaagga 120
   agccacgtgc agacatctac aaccttcgat ctcctgacga gtttattgtt ggccaaaacc 180
   aggctttgat tgaaccagga tgaatgcggg tgttggaagt ag 222
<210> 181
   <211> 516
   <212> DNA
   <213> human
<400> 181
   ttaccctcta tttaaatgct ttgaaaaaca gtgcattgac aatgggttga tatttttctt 60
   taaaagaaaa atataattat gaaagccaag ataatctgaa gcctgtttta ttttaaaact 120
   ttttatgttc tgtggttgat gttgtttgtt tgtttgtttc tattttgttg gttttttact 180
   ttgttttttg ttttgttttg ttttgttttg catactacat gcagttcttt aaccaatgtc 240
   tgtttggcta atgtaattaa agttgttaat ttatatgagt gcatttcaac tatgtcaatg 300
   gtttcttaat atttattgtg tagaagtact ggtaattttt ttatttacaa tatgtttaaa 360
   gagataacag tttgatatgt tttcatgtgt ttatagcaga agttatttat ttctatggca 420
   ttccagcgga tattttggtg tttgcgaggc atgcagtcaa tattttgtac agttagtgga 480
   cagtattcag caacgcctga tagcttcttt ggcctt 516
<210> 182
   <211> 507
   <212> DNA
   <213> human
<220>
   <221> misc_feature
   <222> (357)..(357)
   <223> n is a, c, g, or t
<400> 182
   ctggcacaac cctgacatta ctaagtggaa atgttaggat ttttcggcat cgcatgttag 60
   aatctctaaa atttaaacat tcctgttaaa tgactaaggt ttgcttttat caatatgaat 120
   tctgaaggcc aatatcatac cattaactat gaaagctttt aattcctaaa aatagtttta 180
   gagatattca agcaatgctc tcctaatatc catacgcaag tgtgtttatg acacaaattc 240
   actagtctgt ttaaaaatga attctttata ttgactggtg ttccacatat ttcagtaatt 300
   tctgttatga gaggacttga aatagcaaat tgccacacag ttaactggat agaccangta 360
   cgtggtgatc ataaccactt ggtactacac ccagaaactc aaaattgtct ttctcctgat 420
   gagatatggg tgtccttttg tacgtctagg cctaggtaac cagtggagtg attatattag 480
   caaatgtgtt tgtatccaga gtcttcc 507
<210> 183
   <211> 430
   <212> DNA
   <213> human
<400> 183
   ccaggcttcg tcttatttct actgtttttg tcgcaacttc cattgattta tgtcccttcc 60
   ctccccccta agtacatcag ggaacctttc cacactataa atgatatgac tactgtttgg 120
   ggtttctggg cccccatccg tgtacgtatg tggcatttcc aggtatgact gagtgtgaga 180
   gacatgtcag aggctcttca gtgatttctt gctattgacc gatgcttcac tgtgccaaaa 240
   gagaaaaaaa atgttgggtt ttgtaattaa attatttata tatttttgaa acccgaattg 300
   aaaatgttgc aggcaacggg ctacagcttt attagtggtt ctctaactgt ggtctccttg 360
   ggccaagcaa tttctttaaa ggaaaagttg attatgtatg tggggtgcca ggaccactgc 420
   cttgaaagca 430
<210> 184
   <211> 473
   <212> DNA
   <213> human
<220>
   <221> misc_feature
   <222> (260)..(260)
   <223> n is a, c, g, or t
<220>
   <221> misc_feature
   <222> (384)..(384)
   <223> n is a, c, g, or t
<400> 184
   tagcccaacc ctatcatttt catattatga aactgagtcc aggtaagtga atctgtccaa 60
   ggtcacccag caaggtatca gtagccctga gggtaaggac tctgataagg ctcgggaggg 120
   tcctggaaag cctgaggcgg caggaagagt gtgcagagtt gagcgtgtct ggaaggctga 180
   tccactgctg ggcccacatc aaagccccca tggggagcag acccgactgc acatggctct 240
   tttgctggaa gaagagcatn gctgcgcaga ggactaaaat ttcatctggg aaggcttctt 300
   ttgactgtca gtagcaggat gtcaccagat gagggtgcta tgggaccaca gctgtctttg 360
   ttcccattgc aactcaaccc tgcnggaggc cgcctgcatc cctgagagcc ttctggagcc 420
   tacagaggag acattggcca gccaaaagga aaggagtggc cagggtacga cct 473
<210> 185
   <211> 451
   <212> DNA
   <213> human
<400> 185
   aattatccct tatcattcca aaaatgaaat gctgtgttaa atatctccag ggcaaagtgg 60
   tatgttgact gggacaaacg ttagaaattg tattgttcat tgcacttgtt gccctgttcc 120
   ccaagcttgt caatgtttag agatactatt cgggttgcta aagccattat tcatagaaaa 180
   tttctgcccc tacagaagtg tgtgcatggg ccttggaaaa tctacatgtg tatatctgag 240
   tagcgaagca cagattcact ctaattgaaa gcagcagttt ggttttgtaa atgtaattgc 300
   aattgacact ttcttttccc tttcagttat tatttttttt aaaggacgtt atgagaaggc 360
   actatgaaaa gcctaattgg aatagcatta tgaaccatgt aatgcatgcc catgcacact 420
   gtgatttgca aacatatgtc cgctcttcaa t 451
<210> 186
   <211> 498
   <212> DNA
   <213> human
<220>
   <221> misc_feature
   <222> (394)..(394)
   <223> n is a, c, g, or t
<400> 186
   ggtgggtgtc actacagaca tgttctggcg tgttctccga gggatggagc atcctgttat 60
   atatttgact tcaaattgag atgttggctt catttttttt ttttacccaa ttaatctccc 120
   aatccctagc aactgtgact ctgtatttag cacaagagaa agctgagaat gtgggtcttg 180
   cctccttcca gaaatatgtc tggctcatca ggacattttt ttaaaacttc aaaatatttt 240
   taagatattt taaactttta taaaaaaaaa atcaaccaac aagagacttt tctgaggagg 300
   aacatttgta tttgaacaag atccttggtg tgtagttcag tcttgcagta tacaagcttt 360
   tgtgtataaa tgttttatga tatgattccc tgtnttttgc aggggttttt ttctcttttg 420
   ctttttagat aaatatgtat atcaatattt taaattcatc tttgcttttt ttagaggagt 480
   ttgtaatcac cttataac 498
<210> 187
   <211> 462
   <212> DNA
   <213> human
<400> 187
   gaaaaaagct atcagctgta tgttaagaga gactcttact aacatgttgt aaatattaca 60
   attcatgaaa tgttattgta agtctgtaac ttaatttttt ccctgtttta gttatacagg 120
   ttggtttgga aatttgtgtt ttggcataaa caagtaaaat gtgcccattt tatggtttcc 180
   atgcttttgt aatcctaaaa atattaatgt ctagttgttc tatattataa ccacatttgc 240
   gctctatgca agcccttgga acagaacata ctcatcttca tgtaggacct atgaaaattg 300
   tctattttta tctatatatt taaagttttc taaaaatgat aaaaggttat tacgaatttt 360
   gttgtacaaa atctgtacaa aaatctgttt ttacatcata atgcaagaat tggaaatttt 420
   tctatggtag cctagttatt tgagcctggt ttcaatgtga ga 462
<210> 188
   <211> 446
   <212> DNA
   <213> human
<220>
   <221> misc_feature
   <222> (37)..(37)
   <223> n is a, c, g, or t
<220>
   <221> misc_feature
   <222> (43)..(43)
   <223> n is a, c, g, or t
<220>
   <221> misc_feature
   <222> (286)..(287)
   <223> n is a, c, g, or t
<220>
   <221> misc_feature
   <222> (289)..(295)
   <223> n is a, c, g, or t
<400> 188
   gccgcggtgg aaacgggctt ggagctggcc ccataanggg ctngcggctt cctccgacgc    60
   cgcccctccc cacagcttct cgactgcagt ggggcggggg gcaccaacac ttggagattt   120
   ttccggaggg gagaggattt tctaagggca cagagaatcc attttctaca cattaacttg   180
   agctgctgga gggacactgc tggcaaacgg agacctattt ttgtacaaag aacccttgac   240
   ctggggcgta ataaagatga cctggacccc tgcccccact atctgnngnn nnnnntgctg   300
   gccaagatct ggacacgagc agtccctgag gggcggggtc cctggcgtga ggcccccgtg   360
   acagcccacc ctggggtggg tttgtgggca ctgctgctct gctagggaga agcctgtgtg   420
   gggcacacct cttcaaggga gcgtga                                        446
<210> 189
   <211> 433
   <212> DNA
   <213> human
<400> 189
   gacatgattg tctataatct cgctagcctt gtactgtgtg tgcatagcaa ttacagggaa    60
   gtaatctagc tcctgactat tatgttgaac tatgtcgctg ctttttacaa acttgtcttg   120
   atccaaagca gtcacaatga taaccctgca tatctgggaa tcataagtca actatgtatc   180
   tctgtgtgtg tatatatatg tatgtatgta tctattttca aactgtgatt taatatttaa   240
   atattcctac tgccattttt gtgactgaaa aactacacat gaggaaacgt cttagaattt   300
   tccaatagag gaaaaataac acttgggcaa tctgtcatgt ttcacaacag ttctcatttt   360
   tctcatgatt tgtgtagcgt ggaatgtgtt tgctcaatgt gaagggtttt cattgctcaa   420
   tttctctgtg taa                                                      433
<210> 190
   <211> 361
   <212> DNA
   <213> human
<220>
   <221> misc_feature
   <222> (71)..(71)
   <223> n is a, c, g, or t
<220>
   <221> misc_feature
   <222> (295)..(304)
   <223> n is a, c, g, or t
<400> 190
   ggttccggct aacacatttt ctaagtcgcc agtgctgctt acagtttgaa tacatgaaaa 60
   tcctgtttct nagatgtttg cgcacgtgct tattaggaaa tgagtctgta tggaaatctc 120
   accacagata atggttaacg aaccgggtcg acatcacaaa ggagggtgga gactcttttt 180
   actaacttga atgagacaaa agcagtggtg tcagtttata atcctgatgc atttcagtaa 240
   taatgtagaa aaacattatt ttaaaaaagt tccaacacac agccatgagg agccnnnnnn 300
   nnnntcagtt ttgaaagagg tgcataataa aactactaac cagaggagtc tatgccattt 360
   t 361
<210> 191
   <211> 415
   <212> DNA
   <213> human
<220>
   <221> misc_feature
   <222> (42)..(42)
   <223> n is a, c, g, or t
<220>
   <221> misc_feature
   <222> (95)..(95)
   <223> n is a, c, g, or t
<220>
   <221> misc_feature
   <222> (353)..(353)
   <223> n is a, c, g, or t
<400> 191
   gagtttcaac tttaaatgtt cactatgtca tttagtgtcc anctttacgg ataggttgac 60
   tatctaaata ggcattttta gtcattaaaa aaaantctag tcaccaggag gatccctata 120
   actcaaaata acttgtttgt aaaagaaaat ttgtttactt acccattagt aagttcctgc 180
   atattcatta taagatggca aatcaaactt ttctaggatg aagacagctt atttttaagt 240
   tgtatagtct tagttggttt agggtctcaa ttttaattaa taaaatactt ggtttttatt 300
   tgcttgtcct tttgaattcc tgttttaata attttaaaat gagcacaaag aangttgaag 360
   ttcagattaa tctcttctga atgatgtttt tttcctctgt gatgagttgt ttctg 415
<210> 192
   <211> 297
   <212> DNA
   <213> human
<220>
   <221> misc_feature
   <222> (117)..(118)
   <223> n is a, c, g, or t
<220>
   <221> misc_feature
   <222> (120)..(120)
   <223> n is a, c, g, or t
<220>
   <221> misc_feature
   <222> (122)..(122)
   <223> n is a, c, g, or t
<220>
   <221> misc_feature
   <222> (165)..(165)
   <223> n is a, c, g, or t
<220>
   <221> misc_feature
   <222> (177)..(177)
   <223> n is a, c, g, or t
<220>
   <221> misc_feature
   <222> (193)..(193)
   <223> n is a, c, g, or t
<220>
   <221> misc_feature
   <222> (240)..(241)
   <223> n is a, c, g, or t
<220>
   <221> misc feature
   <222> (254)..(254)
   <223> n is a, c, g, or t
<220>
   <221> misc_feature
   <222> (256)..(260)
   <223> n is a, c, g, or t
<400> 192
   caccaagtta cgtcaaagtc tcaggagcag ctccggtctc catagaggct gggtcagcag     60
   tgggcaaaac aacttccttt gctgggagct ctgcttcctc ctacagcccc tcggaanncn    120
   cnctcaagaa cttcacccct tcagagacac cgaccatgga catcncaacc aaggggncct    180
   tccccaccag canggaccct cttccttctg tccctccgac tacaaccaac agcagccgan    240
   ngacgaacag cacntnnnnn aagatcacaa cctcagcgaa gaccacgatg aagcccc       297
<210> 193
   <211> 549
   <212> DNA
   <213> human
<220>
   <221> misc_feature
   <222> (222)..(224)
   <223> n is a, c, g, or t
<220>
   <221> misc_feature
   <222> (226)..(226)
   <223> n is a, c, g, or t
<220>
   <221> misc_feature
   <222> (416)..(416)
   <223> n is a, c, g, or t
<220>
   <221> misc_feature
   <222> (434)..(434)
   <223> n is a, c, g, or t
<220>
   <221> misc_feature
   <222> (440)..(441)
   <223> n is a, c, g, or t
<220>
   <221> misc_feature
   <222> (448)..(448)
   <223> n is a, c, g, or t
<220>
   <221> misc_feature
   <222> (516)..(516)
   <223> n is a, c, g, or t
<400> 193
   gtacagcatg aaaggcttcc tctacaagac actagtcaaa gagttgagag ctgcggtttc    60
   taatctttgt ccattactcc cttactccct atgagactgt ggacctgtca cttggcctct   120
   ctggtcttca gttttctcac cagtaaaaca aggaacttga accaaatgac ctctagtgtt   180
   ccccttgggt ttaaatgtct ataaatgttc aatgactaga annnantgcg tttttcttta   240
   ttctttttgc tttgagaaaa gagaatgtga tttaagagta ataatttgaa taccaattat   300
   ccacattaaa attgtgtcct ctatgtgtaa ggcatagcac atttagcaca catacataag   360
   cacactaagc accttacaaa tatcctcatt tattctttac ataatctttt gaaatngatt   420
   atgtaataca cacngttttn naacaatngg tgacttccag ctgtttaaaa caaactacag   480
   tatggtgctt gagtactgac ttaggaggtc agcatnggtt tcactaggag cttctcaaag   540
   cacgctgcc                                                           549
<210> 194
   <211> 436
   <212> DNA
   <213> human
<400> 194
   gggatcactg ggagaagcca tggcattatc ttcaggcaat ttagtctgtc ccaaataaaa      60
   taaatccttg catgtaaatc attcaagggt tatagtaata tttcatatac tgaaaagtgt     120
   ctcataggag tcctcttgca catctaaaaa ggctgaacat ttaagtatcc cgaattttct     180
   tgaattgctt tccctataga ttaattacaa ttggatttca tcatttaaaa accatacttg     240
   tatatgtagt tataatatgt aaggaataca ttgtttataa ccagtatgta cttcaaaaat     300
   gtgtattgtc aaacatacct aactttcttg caataaatgc aaaagaaact ggaacttgac     360
   aattataaat agtaatagtg aagaaaaaat agaaaggttg caattatata ggccatgggt     420
   ggctcaaaac tttgaa                                                     436
<210> 195
   <211> 552
   <212> DNA
   <213> human
<220>
   <221> misc_feature
   <222> (215)..(215)
   <223> n is a, c, g, or t
<220>
   <221> misc_feature
   <222> (226)..(227)
   <223> n is a, c, g, or t
<220>
   <221> misc_feature
   <222> (233)..(235)
   <223> n is a, c, g, or t
<400> 195
   ggacgatgaa gccatcattg ctgcttggag acgccggcaa gaagaaacca ggaccaagct      60
   gcagaaaagg agggaggact gagctgggga aaatctgaga acactgaaag aaaccactca 120
   cgttagcata gggctcaggg cacacgttgc caccactcat cgcaggatga ggatacagag 180
   aggatcttcc agaggggcag agccaaaatg agagntacca agcatnnggg cannngaggt 240
   ggagtaggga ggaggcaagg agggggagaa ccatcaatac gaatacgagg tccgaatgcg 300
   gaccaactga taccattttc tgttgctcag cgccctctaa gctttggtgt ttcacttaat 360
   gtatttgaca gtgttcatca caggctagag aggtgagctt ggaaaagcac tgtagtttgt 420
   cagagactcc agtttacatc cagaaaggcc atgaacatag gacacgcttc tgtctgtaga 480
   ggcttcatat gagacccaga aagtctatcc tatggcaagt ctgacctctc ctggcaatgc 540
   tcagttctga tt 552
<210> 196
   <211> 559
   <212> DNA
   <213> human
<400> 196
   gaagtccatc ctttggtcca aagcatctgg aagaggaaga agagaggaat gagaaagaag 60
   gaagtgatgc aaaacatctc caaagaagtc ttttggaaca ggaaaatcat tcaccactca 120
   cagggtcaaa tatgaaatac aaaaccacga accaatcaac agaattttta tccttccaag 180
   atgccagctc attgtacaga aacattttag aaaaagaaag ggaacttcag caactgggaa 240
   tcacagaata cctaaggaaa aacattgctc agctccagcc tgatatggag gcacattatc 300
   ctggagccca cgaagagctg aagttaatgg aaacattaat gtactcacgt ccaaggaagg 360
   tattagtgga acagacaaaa aatgagtatt ttgaacttaa agctaattta catgctgaac 420
   ctgactattt agaagtcctg gagcagcaaa catagatgga gagtttgagg gctttcgcag 480
   aaatgctgtg attctgtttt aagtccatac cttgtaaata agtgccttac gtgagtgtgt 540
   catcaatcag aacctaagc 559
<210> 197
   <211> 448
   <212> DNA
   <213> human
<400> 197
   accaagatgg tgcaagttcc ctttgcagat ggcgtgggca cacttgattt ttattatgag 60
   tgaatgtaat ctttctgtat tttaccagag ttacagcaat tacctgaaaa gtttcctaac 120
   attttaataa tgttagggat ttcgttttgg ttttagttgt cctcaagaga caacaggttc 180
   acagtaattt ccatgatgtt gggtgtggct aagctgggga ttggttctgt tccccctgct 240
   cccgtgtaga gaaaagctat atttatactg cattctttct caactttcag gtaaaacaaa 300
   ctatgattta aaaaaagaaa aaagaaaaga caggtacttt tacttcaaag agtgctttgc 360
   tacattttta tttaaaccaa aaatcaaata aaataaggag gggggctggg tatactttaa 420
   acaaaaccag tcctgaaatg ctgttatt 448
<210> 198
   <211> 324
   <212> DNA
   <213> human
<400> 198
   ttcttgtcca gctgttcaca gttttatttt tatatagatg gtgatataaa tatttccaaa 60
   tgcatttgta aacattctaa atattctcaa gtcatgttca atgtttccta aaccttcaat 120
   tttggccaaa gtccccaaac acatcattgc cacactctga agtagagaaa gaaaatttag 180
   gggccagttc tcaaggaaca caggtccttt atttttattt taactaagtt gaagacccac 240
   tcaaaaagct cttgtggttt tatgttcttg acctttcaac tggagtcctc tcattcagca 300
   ggtggcccgt gagacacaga atac 324
<210> 199
   <211> 431
   <212> DNA
   <213> human
<220>
   <221> misc_feature
   <222> (311)..(311)
   <223> n is a, c, g, or t
<220>
   <221> misc_feature
   <222> (391)..(391)
   <223> n is a, c, g, or t
<400> 199
   cagtgctgct gtgaactaaa gtatgtcatt tatgctcaaa gtttaattct tcttcttggg 60
   atattttaaa aatgctactg agattctgct gtaaatatga ctagagaata tattgggttt 120
   gctttatttc ataggcttaa ttctttgtaa atctgaatga ccataataga aatacatttc 180
   ttgtggcaag taattcacag ttgtaaagta aataggaaaa attattttat ttttattgat 240
   gtacattgat agatgccata aatcagtagc aaaaggcact tctaaaggta agtggtttaa 300
   gttgcctcaa nagagggaca atgtagcttt attttacaag aaggcatagt tagatttcta 360
   tgaaatattt attctgtaca gttttatata nttttggttc acaaaagtaa ttattcttgg 420
   gtgcctttca a 431
<210> 200
   <211> 322
   <212> DNA
   <213> human
<220>
   <221> misc_feature
   <222> (37)..(38)
   <223> n is a, c, g, or t
<400> 200
   aaagtatatt gtgctagctt gtctaagaat aaacttnnat actgttgggg gagggctgca 60
   cctgtcaaga taacctgtca atgtagtagg aaaacaggag gggacagtaa cagaaaagca 120
   cgggaaaaga tggcaaggtt agttaaaata gaaaagtgct cagttcctca tacctgtaat 180
   cccagcagtt tagggggcca aggaaggtgg gtcacttgag cccaagagtt caaggccatc 240
   ctgggcaatg tggcgaaagt gtctacaaaa aaatacaaaa agaggaagaa atgatatttc 300
   acaagtttgt atcatttgtc at 322
<210> 201
   <211> 415
   <212> DNA
   <213> human
<220>
   <221> misc_feature
   <222> (31)..(31)
   <223> n is a, c, g, or t
<220>
   <221> misc feature
   <222> (35)..(35)
   <223> n is a, c, g, or t
<220>
   <221> misc_feature
   <222> (40)..(40)
   <223> n is a, c, g, or t
<220>
   <221> misc_feature
   <222> (50)..(50)
   <223> n is a, c, g, or t
<220>
   <221> misc_feature
   <222> (57)..(57)
   <223> n is a, c, g, or t
<220>
   <221> misc_feature
   <222> (62)..(62)
   <223> n is a, c, g, or t
<220>
   <221> misc feature
   <222> (66)..(66)
   <223> n is a, c, g, or t
<220>
   <221> misc feature
   <222> (68)..(68)
   <223> n is a, c, g, or t
<220>
   <221> misc_feature
   <222> (74)..(74)
   <223> n is a, c, g, or t
<220>
   <221> misc feature
   <222> (89)..(90)
   <223> n is a, c, g, or t
<220>
   <221> misc_feature
   <222> (93)..(97)
   <223> n is a, c, g, or t
<220>
   <221> misc_feature
   <222> (99)..(102)
   <223> n is a, c, g, or t
<220>
   <221> misc_feature
   <222> (104)..(106)
   <223> n is a, c, g, or t
<400> 201
   caaaaccacg attgtgtgcc ccttttttat naaanctggn atgtttgaan gttgtantac     60
   angctntnct tctntgttgc caattctgnn acnnnnntnn nnannngaaa aaatagtaga    120
   agctattcta caagaaaaaa tgtacttgta tatgccaaag ttgttatact tcatgatgtt    180
   tcttaaaagc tttttgcccc tcaagacagg actgcttata gctgactatt tgggcatcct    240
   tcatgcaatg gatggctttg ttgaccaaaa gaagaagctc taaagaccaa ctctatggct    300
   aaggtcatct gatacacagt gttacataat gcgtacttca atgaagaaaa gtatttttgt    360
   ctgacagtgg aatatatctg gagaccacaa gtaccactcc tattctgtta tctgg         415
<210> 202
   <211> 249
   <212> DNA
   <213> human
<400> 202
   agccgttggt ctttgaaatt tcctgtgatg tgtttcaatc tagatgcaaa gaacatggaa 60
   aaatcaaagt gctcgagtgg tttaaatatg ttttgggtat tcctgtttat agactataat 120
   acttttccaa ttaaaatcct cagttgtcac gcagaagaag gttaagctgt atttgattgc 180
   cagttttact gaaaatgctt agtattttac agtatcacca aatatatttt gtttagccaa 240
   ggtatagga 249
<210> 203
   <211> 541
   <212> DNA
   <213> human
<220>
   <221> misc_feature
   <222> (131)..(131)
   <223> n is a, c, g, or t
<220>
   <221> misc_feature
   <222> (134)..(134)
   <223> n is a, c, g, or t
<220>
   <221> misc_feature
   <222> (209)..(209)
   <223> n is a, c, g, or t
<220>
   <221> misc_feature
   <222> (279)..(279)
   <223> n is a, c, g, or t
<220>
   <221> misc_feature
   <222> (326)..(326)
   <223> n is a, c, g, or t
<400> 203
   acccaacaag agctgtgcgg ctccctgatt cctcgccagt gttgctaccg cccttggctc 60
   ttcttgcatg gctggctctt gagacccctg gaagctgatg gaggcaacgt gagaagcaca 120
   tggacatccg nccntgagct tgagaggcag aggcctgagt tctagttaca gccccagcag 180
   taccagttgt gtggactggg agggaggcna tcacgtacat actccaagcc tccaagcctg 240
   tttccccttc tgacacagga tcttttgtgg ctggtatana gtgggcactc aataaatgct 300
   gtctggtcgt ctggctggca tgcctnatgg gcctgagaat tgaatagaat tacagtgata 360
   gaagcatgct ggtattgtaa gtggtttgta agtgtgagga ctaaattatt attaaatagt 420
   aatcacatct aatcttggat aaattagtaa aagcaagaat gggagcagta aaaacctaag 480
   caacccgaac taaaatttta ttgaattaat tcaatttctt gtcatgtaac acaaccccag 540
   a 541
<210> 204
   <211> 354
   <212> DNA
   <213> human
<220>
   <221> misc_feature
   <222> (54)..(54)
   <223> n is a, c, g, or t
<400> 204
   gtttctgttt cagtcacaaa ttagggttat tgtgatgtgt atttatgatg accnttgaac 60
   aaatgtgaag aatactgtga attctatgac tttatcaaaa tcagccacat ccaggagctt 120
   gcagttgttg accaaatgaa tgatgacata gagtagttca gatctatcat gtgctcttct 180
   atctaatcag tcaatatttc cttggccctc aagccaacat tcatttttta tgtataacct 240
   tcttcatgat tttgaaattt tgatagggta actgctaatg agttcacaaa tgtagcactt 300
   taaaaggaaa ataaatggag agtgaaaaca acttggctac gtataattgt gggt 354
<210> 205
   <211> 398
   <212> DNA
   <213> human
<220>
   <221> misc_feature
   <222> (80)..(80)
   <223> n is a, c, g, or t
<220>
   <221> misc_feature
   <222> (82)..(83)
   <223> n is a, c, g, or t
<220>
   <221> misc_feature
   <222> (85)..(85)
   <223> n is a, c, g, or t
<220>
   <221> misc_feature
   <222> (87)..(87)
   <223> n is a, c, g, or t
<220>
   <221> misc feature
   <222> (97)..(97)
   <223> n is a, c, g, or t
<400> 205
   caacccattt tgtgccacat gcaagttttg aataaggatg gtatagaaaa caacgctgca     60
   tatacaggta ccatttaggn annancngat gcctttntgg gggcagaatc acatggcaaa    120
   agctttgaaa atcataaaga tataagttgg tgtggctaag atggaaacag ggctgattct    180
   tgattcccaa ttctcaactc tccttttcct atttgaattt ctttggtgct gtagaaaaca    240
   aaaaaagaaa aatatatatt cataaaaaat atggtgctca ttctcatcca tccaggatgt    300
   actaaaacag tgtgtttaat aaattgtaat tattttgtgt acagttctat actgttatct    360
   gtgtccattt ccaaaacttg cacgtgtccc tgaattcc                            398
<210> 206
   <211> 457
   <212> DNA
   <213> human
<220>
   <221> misc_feature
   <222> (64)..(95)
   <223> n is a, c, g, or t
<220>
   <221> misc_feature
   <222> (104)..(104)
   <223> n is a, c, g, or t
<220>
   <221> misc_feature
   <222> (226)..(226)
   <223> n is a, c, g, or t
<220>
   <221> misc_feature
   <222> (344)..(344)
   <223> n is a, c, g, or t
<220>
   <221> misc_feature
   <222> (387)..(402)
   <223> n is a, c, g, or t
<400> 206
   acagacttgg caagggaccc cctggttctg agccagtagc tgccatctgg aaattcctct     60
   tttnnnnnnn nnnnnnnnnn nnnnnnnnnn nnnnnctccc aggnacccgc tgaatttctg    120
   aggccttgct taaagctcag aagtggttta ggcatttgga aaatctggtt cacatcataa    180
   agaacttgat ttgaaatgtt ttctatagaa acaagtgcta agtgtnaccg tattatactt    240
   gatgttggtc atttctcagt cctatttctc agttctatta ttttagaacc tagtcagttc    300
   tttaagatta taactggtcc tacattaaaa taatgcttct cgangtcaga ttttacctgt    360
   ttgctgctga gaacatctct gcctaannnn nnnnnnnnnn nncttcagtt caacatgctt    420
   ccttagcttt tcatagttgt ctgacatttc catgaaa                              457